# EUROPÄISCHE PATENTSCHRIFT

(11) **EP 3 504 338 B1**
(45) Veröffentlichungstag und Bekanntmachung des Hinweises auf die Patenterteilung: **09.12.2020**
(21) Anmeldenummer: 17757734.3
(22) Anmeldetag: 21.08.2017
(51) Int. Cl.: C12Q 1/6848, C12Q 1/6853

(54) **VERFAHREN ZUR AMPLIFIKATION VON NUKLEINSÄUREN UND VERWENDUNG EINES KITS ZU DESSEN DURCHFÜHRUNG**
METHOD FOR AMPLIFICATION OF NUCLEIC ACIDS AND USE OF A KIT FOR SAME
PROCEDE D'AMPLIFICATION D'ACIDES NUCLEIQUES ET UTILISATION D'UN KIT D'EXECUTION

(30) Priorität: 25.08.2016 EP 16185624
(43) Veröffentlichungstag der Anmeldung: 03.07.2019
(60) Teilanmeldung: 20198086.9
(73) Patentinhaber: AGCT GmbH, 23562 Luebeck (DE)
(72) Erfinder: CHERKASOV, Dmitry, 35039 Marburg (DE); BASLER, Norbert, 22927 Groß Hansdorf (DE); BECKER, Claus, 76470 Ötigheim (DE); HESS, Hans-Jörg, Berlin 13467 (DE); MÜLLER-HERMANN, Andreas, 80796 München (DE)
(74) Vertreter: Keller, Günter
(86) Internationale Anmeldenummer: PCT/EP2017/071011
(87) Internationale Veröffentlichungsnummer: WO 2018/036955

(56) Entgegenhaltungen:
- EP-A1- 2 746 395
- WO-A1-2012/058488
- WO-A1-2016/077291
- WO-A1-2016/100388
- WO-A2-2008/097929
- CN-A- 104 164 487
- N. SRINIVAS ET AL: "On the biophysics and kinetics of toehold-mediated DNA strand displacement", NUCLEIC ACIDS RESEARCH, Bd. 41, Nr. 22, 9. September 2013 (2013-09-09), Seiten 10641-10658, XP055341398, ISSN: 0305-1048, DOI: 10.1093/nar/gkt801 in der Anmeldung erwähnt
- DAVID YU ZHANG ET AL: "Control of DNA Strand Displacement Kinetics Using Toehold Exchange", JOURNAL OF THE AMERICAN CHEMICAL SOCIETY, Bd. 131, Nr. 47, 2. Dezember 2009 (2009-12-02), Seiten 17303-17314, XP055016993, ISSN: 0002-7863, DOI: 10.1021/ja906987s

### SEQUENZPROTOKOLL

<110> AGCT GmbH
<120> Verfahren zur Amplifikation von Nukleinsäuren und Kit zu dessen Durchführung
<130> PWO00293AGC
<150> EP16185624
   <151> 2016-08-25
<160> 38
<170> PatentIn version 3.5
<210> 1
   <211> 59
   <212> DNA
   <213> artificial sequence
<220>
   <223> target
<400> 1
   ccgaagctcg caggaactca gagtgtggag aggacgatag ctagtcagat gatgaagtt 59
<210> 2
   <211> 48
   <212> DNA
   <213> artificial sequence
<220>
   <223> complementary
<400> 2
   catcatctga ctagctatcg tcctctccac actctgagtt cctgcgag 48
<210> 3
   <211> 45
   <212> DNA
   <213> artificial sequence
<220>
   <223> variant
<400> 3
   catcatctga ctagctatcg tcctctccac actctgagtt cctgc 45
<210> 4
   <211> 42
   <212> DNA
   <213> artificial sequence
<220>
   <223> activator oligonucleotide
<400> 4
   cguccucucc acacucugag uuccugcgag cttcggtaca ag 42
<210> 5
   <211> 58
   <212> DNA
   <213> artificial sequence
<220>
   <223> activator oligonucleotide
<400> 5
   aaaacaaacu agcuaucguc cucuccacac ucugaguucc ugcgagcttc ggtacaag 58
<210> 6
   <211> 50
   <212> DNA
   <213> artificial sequence
<220>
   <223> activator oligonucleotide
<400> 6
   ctagctatcg tcctctccac acucugaguu ccugcgagct tcggtacaag 50
<210> 7
   <211> 56
   <212> DNA
   <213> artificial sequence
<220>
   <223> negative control
<400> 7
   auucaaaugu guucucaacg uccucuacuc auguuccugc gagcttcggt acaaaa 56
<210> 8
   <211> 55
   <212> DNA
   <213> artificial sequence
<220>
   <223> A6 8510
<400> 8
   tttttctagc tatcgtcctc tccacactct gagttcctgc gagcttcggt acaag 55
<210> 9
   <211> 55
   <212> DNA
   <213> artificial sequence
<220>
   <223> A6 8512
<400> 9
   tttttctagc tatcgtcatc tccacactct gagttactgc gagcttcggt acaag 55
<210> 10
   <211> 53
   <212> DNA
   <213> artificial sequence
<220>
   <223> A6-8514-1
<400> 10
   tttttctagc tatcgtcctc tccacactga gttcctgcga gcttcggtac aag 53
<210> 11
   <211> 20
   <212> DNA
   <213> artificial sequence
<220>
   <223> primer
<400> 11
   gtaccgaagc tcgcaggaac 20
<210> 12
   <211> 48
   <212> DNA
   <213> artificial sequence
<220>
   <223> target
<400> 12
   atactacaat gtcacttacg tcctctccac actctgagtt cctgcgag 48
<210> 13
   <211> 20
   <212> DNA
   <213> artificial sequence
<220>
   <223> primer
<400> 13
   gtaccgaagc tcgcaggaac 20
<210> 14
   <211> 27
   <212> DNA
   <213> artificial sequence
<220>
   <223> second primer
<400> 14
   aaaaaaacat actacaatgt cacttac 27
<210> 15
   <211> 50
   <212> DNA
   <213> artificial sequence
<220>
   <223> activator oligonucleotide
<400> 15
   cuagcuaucg uccucuccac acucugaguu cctgcgagct tcggtacaag 50
<210> 16
   <211> 57
   <212> DNA
   <213> artificial sequence
<220>
   <223> elongation product
<400> 16
   gtaccgaagc tcgcaggaac tcagagtgtg gagaggacgt aagtgacatt gtagtat 57
<210> 17
   <211> 56
   <212> DNA
   <213> artificial sequence
<220>
   <223> second primer elongation product
<400> 17
   aaaaaaacat actacaatgt cacttacgtc ctctccacac tctgagttcc tgcgag 56
<210> 18
   <211> 65
   <212> DNA
   <213> artificial sequence
<220>
   <223> amplification product
<400> 18
   gtaccgaagc tcgcaggaac tcagagtgtg gagaggacgt aagtgacatt gtagtatgtt 60
ttttt 65
<210> 19
   <211> 56
   <212> DNA
   <213> artificial sequence
<220>
   <223> amplification product
<400> 19
   gagcgtcctt gagtctcaca cctctcctgc attcactgta acatcataca aaaaaa 56
<210> 20
   <211> 65
   <212> DNA
   <213> artificial sequence
<220>
   <223> amplification product
<400> 20
   gtaccgaagc tcgcaggaac tcagagtgtg gagaggacgt aagtgacatt gtagtatgtt 60
   ttttt 65
<210> 21
   <211> 44
   <212> DNA
   <213> artificial product
<220>
   <223> target
<400> 21
   gtagttctca cttacgtcct ctccacactc tgagttcctg cgag 44
<210> 22
   <211> 41
   <212> DNA
   <213> artificial product
<220>
   <223> target with one mismatch
<400> 22
   gtagttctca cttacgtcct ctccctctga gttcctgcga g 41
<210> 23
   <211> 7
   <212> DNA
   <213> artificial sequence
<220>
   <223> first primer
<400> 23
   caggaac 7
<210> 24
   <211> 20
   <212> DNA
   <213> artificial sequence
<220>
   <223> second primer
<400> 24
   cacgagtagt tctcacttac 20
<210> 25
   <211> 66
   <212> DNA
   <213> artificial sequence
<220>
   <223> activator oligonucleotide
<400> 25
   ctagctatcg tcctctccac actctgagtt cctgcgagct tcggtacaag cgagcttcgg 60
   tacaag 66
<210> 26
   <211> 49
   <212> DNA
   <213> artificial sequence
<220>
   <223> MIP Gene
<400> 26
   ctacaatgtc acttacacgt tcttaacaag tttcagccgt tcctgcgag 49
<210> 27
   <211> 44
   <212> DNA
   <213> artificial sequence
<220>
   <223> target with one mismatch
<400> 27
   ctacaatgtc acttacacgt tcttaacaag tttgttcctg cgag 44
<210> 28
   <211> 33
   <212> DNA
   <213> artificial sequence
<220>
   <223> second primer oligonucleotide
<400> 28
   ctacaatgtc acttacacgt tcttaacaag ttt 33
<210> 29
   <211> 73
   <212> DNA
   <213> artificial sequence
<220>
   <223> activator oligonucleotide
<400> 29
   aaaacaaaaa ccgaacaaca aaugaaagac guucuuaaca aguuucagcc guuccugcga 60
   gcttcggtac aag 73
<210> 30
   <211> 11
   <212> DNA
   <213> artificial sequence
<220>
   <223> first primer
<400> 30
   ctcgcaggaa c 11
<210> 31
   <211> 7
   <212> DNA
   <213> artificial sequence
<220>
   <223> first primer variant
<400> 31
   caggaac 7
<210> 32
   <211> 10
   <212> DNA
   <213> artificial sequence
<220>
   <223> first primer variant
<400> 32
   tcgcaggaac 10
<210> 33
   <211> 30
   <212> DNA
   <213> artificial sequence
<220>
   <223> second primer
<400> 33
   gctcatacta caatgtcact tacgtcctct 30
<210> 34
   <211> 41
   <212> DNA
   <213> artificial sequence
<220>
   <223> elongation product
<400> 34
   tcagagtgtg gagaggacgt aagtgacatt gtagtatgag c 41
<210> 35
   <211> 44
   <212> DNA
   <213> artificial sequence
<220>
   <223> template with one mismatch
<400> 35
   gtagttctca cttacgtcct ctccacacta tgagttcctg cgag 44
<210> 36
   <211> 44
   <212> DNA
   <213> artificial sequence
<220>
   <223> template with one mismatch
<400> 36
   gtagttctca cttacgtcct ctccaaactc tgagttcctg cgag 44
<210> 37
   <211> 44
   <212> DNA
   <213> artificial sequence
<220>
   <223> template with one mismatch
<400> 37
   gtagttctca cttacgtcct atccacactc tgagttcctg cgag 44
<210> 38
   <211> 44
   <212> DNA
   <213> artificial sequence
<220>
   <223> template with one mismatch
<400> 38
   gtagttctca cttacgtact ctccacactc tgagttcctg cgag 44

## Beschreibung

Die Synthese von Nukleinsäureketten nimmt heute eine zentrale Rolle in der Biotechnologie ein. Verfahren wie PCR haben sowohl die Forschungslandschaft als auch industrielle Applikationsfelder, wie Diagnostik in der Medizin und Lebensmittelindustrie, signifikant weiterentwickelt. Die Verbindung der PCR mit anderen Technologien, wie Sequenzierung, Real-Time-Nachweis, Microarray-Technologie, Mikrofluidic-Managment etc. hat zur technologischen Entwicklung der Basistechnologie beigetragen und einige Barrieren der Grundtechnologie der PCR teilweise überwinden können. Es wurden auch weitere Amplifikations-Verfahren, wie isothermale Amplifikationstechniken (LAMP, HDA, RPA, TMA, SDA etc.) entwickelt. Deren Einsatz wurde besonders für Bereich von POCT vorgesehen.

Trotz enormer Fortschritte auf diesem Gebiet, nimmt PCR die zentrale Rolle ein und bestimmt somit die einzelnen technologischen Barrieren der Applikationen.

Eine der Eigenschaften von verbreiteten Amplifikationsverfahren, wie PCR, besteht darin, dass während des Amplifikationsvorgangs der Nukleinsäure keine Kontrolle der amplifizierten Sequenzabschnitte zwischen beiden Primern stattfindet. Im Wesentlichen steht die Primer-Bindung im Fokus von Optimierungen von PCR-Amplifikationsreaktionen. Zu Beginn der PCR-Amplifikation und in ihrem Verlauf kommt es kontinuiertlich zu mehr oder weniger spezifischen Primer-Bindung und Initiierung der Synthese von Hauptprodukten und Nebenprodukten. Die Nebenprodukte können beispielsweise als Folge eines unspezifischen Primer-Verlängerungsereignisses in einem Synthese-Zyklus generiert werden. Bei ggf. erfolgenden Rücksynthese-Reaktion wird der unspezifisch verlängerte Primer als Matrize abgelesen, was in der Regel zu Ausbildung einer vollständigen Primer-Bindungsstelle führt. Somit erfolgt eine Übertragung einer fehlerhaften Sequenzinformation von einem Synthese-Zyklus auf den nächsten Synthese-Zyklus, was in Summe von Synthese-Zyklen nicht nur zu initialen Entstehung, sondern vor allem zur exponentiellen Vermehrung von Nebenprodukten führt.

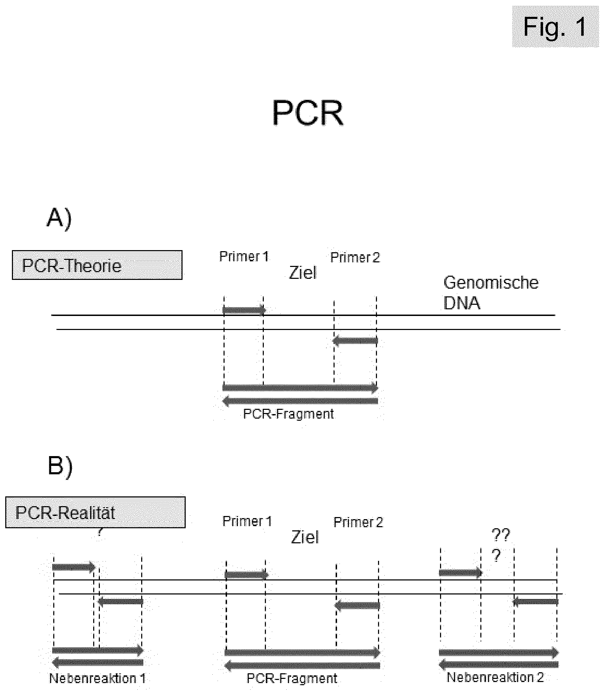

Solche Nebenreaktionen können unter Umständen zu initialen Entstehung und exponentiellen Vermehrung von Fragmenten führen, welche die Hauptreaktion (Amplifikation einer Zielsequenz) stören bzw. zu Interfernzen in nachfolgenden Schritten der Analyse führen. Solche Nebenprodukte umfassen typischerweise Primersequenzen und entsprechende Primersequenzen, so dass ihre Amplifikation parallel zu der Hauptreaktion stattfinden kann (Fig. 1). Anstatt einer Zielsequenz umfassen solche Nebenprodukte allerdings eine andere Nukleinsäuresequenz. Um die Synthese der Hauptprodukte zu optimieren und die Entstehung von Nebenprodukten zu minimieren werden typischerweise Primer-Sequenzen optimiert und solche Reaktionsbedingungen gewählt, welche die Spezifität von Primer-Bindungen unterstützen. Wenn es dennoch zu Initiierung von Nebenprodukten gekommen ist, so können diese in der Regel parallel zu Hauptprodukten vervielfältigt werden, da während des Amplifikationsverfahren alle neugebildeten Stränge unabhängig von ihrer Zusammensetzung denaturiert werden. Während dieses Schrittes werden Primer-Bindungsstellen regeneriert, was die Voraussetzung für neue Synthese-Runde darstellt. In Verfahren wie PCR oder HDA oder SDA werden Mittel eingesetzt, welche keine sequenzabhängige Strang-Trennung bewirken (in PCR wird TemperaturErhöhung eingesetzt, in HDA eine Helicase und ATP, in SDA eine zu Strangverdrängung fähige Polymerase). Folglich, werden sowohl in Hauptprodukten als auch bei Nebenprodukten Primer-Bindungsstellen für erneute Bindungen und anschließende Synthesereaktion regeneriert. Nach einer einmaligen Initiierung können sowohl Hauptprodukte als auch Nebenprodukte parallel vervielfältig werden, wenn verwendete Primer entsprechende Primerbindungsstellen vorfinden. Besonders anfällig für solche Nebenreaktionen sind Amplifikationsverfahren, welche unter teilweise schwer zu kontrollierenden Reaktionsbedinungen ablaufen müssen (z.B. Point-Of-Care Testing) oder eine starke Präsenz von Nebenreaktionen-begünstigenden Faktoren aufweisen (z.B. Amplifikaton von Sequenz-Fragmenten mit geringfügigen Sequenz-Abweichungen, wie beispielsweise Mutationen oder SNV, in Anwesenheit von hohen Mengen von Wildtyp-Sequenzen, Solche Analysen können beispielsweise in Forensik, Pränatal-Diagnostik oder beispielsweise bei ctDNA-Nachweis im Rahmen von Liquid-Biopsy Untersuchung von Bedeutung sein).

Vorrangig wird Spezifität der PCR Amplifikation durch Optimierung der Primer-Bindung an Zielsequenzen erreicht. Dabei können beispielsweise zusätzliche Oligonukleotide verwendet werden, welche an Primer teilweise binden können und somit bei Primer-Bindung an andere Nukleinsäureketten kompetetiv mitwirken. Solche Sonden binden in der Regel an einen Sequenz-Abschnitt des Primers und lassen am Primer einen einzelsträngigen Sequenzabschnitt frei, so dass der Primer mit diesem Abschnitt an die Zielnukleinsäure binden kann und eine Synthese Reaktion initiieren kann. Dabei soll die Spezifität einer Primer-Bindung dadurch verbessert werden, da Primer-Matrizen Mismatches durch solche Oligonukleotide kompetetiv verdrängt werden können. Im Ergebnis kann Spezifität der Initiierung von PCR-Reaktionen in der Regel verbessert werden. Die Wirkung solcher Oligonukleotide ist auf die Interaktion mit Primer-Sequenzen beschränkt. Solche zusätzlichen Oligonukleotide interagieren nicht mit der zu amplifizierenden Nukleinsäurekette in Abschnitten zwischen beiden Primern. Aufgrund eines molaren Überschusses an Primern kann es während einer Amplifikation dennoch zu unspezifischen Interaktionen von Primern mit Matrizen kommen. Falls es zu einem solchen unspezifischen Ereignis der Primer-Verlängerung kommt (Initiierung einer exponentiellen Nebenreaktion), kommt es zu Ausbildung einer vollfunktionsfähigen Primer-Bindungsstelle als Folge bzw. im Rahmen von Rücksynthese eines komplementären Stranges des Nebenproduktes. Das Vorliegen einer solchen vollständigen Primerbindungsstelle im Nebenprodukt führt zu einem Verlust der kompetetiven Wirkung von solchen zusätzlichen Oligonukleotiden auf Primer-Bindung. Kontrolle der Spezifität der Bindung eines Primers an die Matrize durch solche Oligonukleotide macht somit nur Initiierung einer Nebenreaktion unwahrscheinlicher, kann allerdings nach Entstehung eines Nebenproduktes seine expontielle Amplifikation kaum beeinflussen.

Weiterhin werden üblicherweise markierte Oligonukleotid-Sonden im Rahmen von Detektionsverfahrens zu Verbesserung der Spezifität der Analyse zu einem spezifischen Nachweis von bestimmten Sequenzen eingesetzt, Diese Sonden beeinflussen im Wesentlichen die exponentielle Amplifikation nicht. Im Allgemeinen benötigen Sonden eine hinreichend hohe Konzentration von Nukleinsäureketten und entfalten ihre Wirkung deshalb auf Endstadien einer Amplifikation, wenn genügend Produkt vorliegt (z.B. Taqman-Sonden oder Light-Cycler Sonden).

Im Allgemeinen kann eine Verbesserung der Spezifität der Synthese eines Amplifikationsverfahrens mit reduzierter Entstehung und Co-Amplifikation von Nebenprodukten, welche sich von Zielsequenz unterscheiden, beispielsweise zu einer Verbesserung von diagnostischen Verfahren beitragen.

Eine Aufgabe der vorliegenden Offenbarung ist es, Verfahren und Komponenten bereitzustellen, welche die enzymatische Synthese und Amplifikation von Nukleinsäureketten ermöglichen. Es soll ein neues enzymatisches Verfahren und Komponenten für die Synthese von Nukleinsäureketten, sowie die Amplifikation von Nukleinsäureketten bereitgestellt werden.

Eine weitere Aufgabe der Offenbarung ist es, ein Verfahren mit verbesserter Spezifität der Synthese von Zielnukleinsäureketten in einer exponentiellen Amplifikation bereitzustellen.

Eine weitere Aufgabe der Offenbarung ist es, Mittel für Umsetzung eines exponentiellen Amplifikationsverfahren mit verbesserter Spezifität der Synthese zur Verfügung zu stellen.

Eine weitere Aufgabe der Offenbarung ist es, Mittel und Verfahren zur Verfügung zu stellen, welche synthetisierte Sequenzabschnitte zwischen Primern überprüfen und Effizienz einer Amplifikationsreaktion bzw. Dauer einer Amplifikationssreaktion von Sequenzen in Abhängigkeit von ihrer Übereinstimmung mit einer Sequenz-Vorgabe beeinflussen können.

Eine weitere Aufgabe der Offenbarung ist es, Mittel und Verfahren zur Verfügung zu stellen, welche eine Überprüfung der synthetisierten Sequenzen in Echtzeit ermöglichen und eine Kontrolle über Verwendbarkeit der synthetisierten Sequenzen in weiteren Amplifikationszyklen ausüben (real-time controlling / On-line quality control).

Eine weitere Aufgabe der Offenbarung ist es, Mittel und Verfahren zur Verfügung zu stellen, welche ein exponentielles Amplifikationsverfahren für Nukleinsäurekette mit einer Feedback-Funktion der Überprüfung von Inhalten von Nukleinsäureketten zur Verfügung stellt. Die Feedback-Funktion der Überprüfung soll dabei parallel zu exponentiellen Amplifikation erfolgen. Die einzelnen Amplifikations-Elemente bilden dabei einen RegelKreis.

Mit dem erfindungsgemäßen Verfahren sollen Nukleinsäureketten mit definierter Sequenz-Zusammensetzung synthetisiert bzw. amplifiziert werden können.

Die Aufgabe der Offenbarung wird gelöst durch Bereitstellung von Amplifikationsverfahren und entsprechenden Mitteln zu seiner Ausführung.

Vorzugsweise erfolgt die Amplifikation als exponentielle Amplifikation, bei welcher neusynthetisierte Produkte beider Primer (Primer-Verlängerungsprodukte) als Matrizen für weitere Syntheseschritte auftreten. Dabei werden Primersequenzen zumindest teilweise kopiert, so dass komplementäre Primer-Bindungsstellen entstehen, welche unmittelbar nach ihrer Synthese als Sequenzsegmente eines Doppelstranges vorliegen. Im Amplifikatonsverfahren erfolgen Synthese-Schritte von beiden Strängen und Doppelstrang-Öffnungs-Schritten der neusynthetisierten Sequenzabschnitte in gegenseitiger Abwechselung. Eine hinreichende Doppelstrang-Trennung nach einer Synthese stellt eine wichtige Voraussetzung für weitere Synthese dar. Insgesamt kann eine solche Abwechslung aus Synthese- und Doppelstrang-Trennungsschritten zu einer exponentiellen Amplifikation führen.

Im erfindungsgemäßen Amplifikationsverfahren erfolgt die Doppelstrang-Öffnung von Hauptprodukten der Amplifikation (Amplifikation von Zielsequenz umfassenden Nukleinsäureketten) unter anderem mittels eines Oligonukleotides, genannt Aktivator-Oligonukleotid. Das Aktivator-Oligonukleotid umfasst vorzugsweise Sequenzsegmente, welche der Zielsequenz entsprechen.

Im Einzelnen, wird die Strangtrennung erfindungsgemäß durch Einsatz von Aktivator-Oligonukleotide mit vordefinierten Sequenzen erreicht, welche vorzugsweise mittels einer sequenzabhängigen nukleinsäurevermittelten Strangverdrängung einen neusynthetisierten Doppelstrang bestehend aus beiden spezifischen Primer-Verlängerungsprodukten trennen. Die dabei entstehenden einzelsträngigen Segmente von Primer-Verlängerungsprodukten umfassen die Zielsequenz, sowie entsprechende Primer-Bindungsstellen, welche als Bindungsstellen für weitere Primer-Oligonukleotide dienen können, so dass eine exponentielle Amplifikation von zu amplifizierenden Nukleinsäureketten erreicht wird. Die Primer-Verlängerungsreaktionen und Strangverdrängungsreaktionen finden vorzugsweise im Ansatz gleichzeitig statt. Die Amplifikation erfolgt vorzugsweise unter Reaktionsbedinungen, welche eine spontane Trennung von beiden spezifischen synthetisierten Primer-Verlängerungsprodukten nicht zulassen.

Eine spezifische exponentielle Amplifikation einer Zielsequenz-umfassenden Nukleinsäurekette umfasst eine Wiederholung aus Synthese-Schritten und Doppelstrang-Öffnungs-Schritten (Aktivierungs-Schritten für Primer-Bindungsstellen) als zwingende Voraussetzung für Vermehrung der Nukleinsäurekette.

Die Öffnung von synthetisierten Doppelsträngen wird als Reaktions-Schritt umgesetzt, welcher sequenzspezifisch durch das Aktivator-Oligonukleotid beeinflusst werden soll. Diese Öffnung kann vollständig erfolgen, bis hin zu Dissoziation von beiden komplementären Primer-Verlängerungsprodukten, oder auch partiell sein.

Das Aktivator-Oligonukleotid umfasst erfindungsgemäß Sequenzabschnitte, welche mit der Zielsequenz interagieren können und weitere Sequenzabschnitte, welche diese Interaktion herbeiführen, bzw. ermöglichen, bzw. begünstigen. Im Rahmen der Interaktion mit dem Aktivator-Oligonukleotid werden doppelsträngige Abschnitte der synthetisierten Primer-Verlängerungsprodukte via sequenzspezifischer Strangverdrängung einzelsträngige Form überführt. Dieser Vorgang ist sequenzabhängig: erst wenn die Sequenz des synthetisieren Doppelstranges ein gewisses Maß an Komplementarität mit der entsprechenden Sequenz des Aktivator-Oligonukleotides aufweist, kommt es zu einer hinreichenden Doppelstrang-Öffnung, so dass die für die Fortführung der Synthese wesentlichen Sequenzabschnitte, wie z.B. Primer-Bindungsstellen, in einzelsträngige Form überführt werden, was einem "aktiven Zustand" entspricht. Das Aktivator-Oligonukleotid "aktiviert" somit spezifisch die neusynthetisierten Primer-Verlängerungsprodukte, welche die Zielsequenz umfassen, für weitere Synthese-Schritte.

Hingegen werden Sequenzabschnitte, welche keine Zielsequenz umfassen, nicht in einzelsträngigen Zustand überführt und verbleiben als Doppelstrang, was einem "inaktiven" Zustand entspricht. Die potenziellen Primerbindungsstellen sind in einem solchen Doppelstrang an Interaktion mit neuen Primern benachteiligt bzw. vollständig gehindert, so dass weitere Synthese-Schritte an solchen "nicht aktivierten" Strängen im Allgemeinen nicht stattfinden. Diese fehlende oder verminderte Aktivierung (d.h. Überführung in einen einzelsträngigen Zustand) von synthetisierten Nukleinsäuresträngen nach einem Synthese-Schritt führt dazu, dass im darauffolgenden Synthese-Schritt nur eine verminderte Menge an Primern an einer Primer-Verlängerungsreaktion erfolgreich teilnehmen kann.

Aufgrund einer anzustrebenden exponentiellen Amplifikation von Hauptprodukten (einer zu amplifizierenden Nukleinsäurekette, welche Zielsequenzen umfasst) werden mehrere Synthese-Schritte und Aktivierungs-Schritte (Doppelstrang-Öffnungs-Schritte) zu einem Amplifikations-Verfahren zusammengefasst und so lange ausgeführt, bzw. so oft wiederholt, bis die gewünschte Menge der spezifischen Nukleinsäurekette bereitgestellt ist.

Die Reaktionsbedinungen (z.B. Temperatur) werden dabei dermassen gestaltet, dass eine spontane Trennung von komplementären Primer-Verlängerungsprodukten in Abwesenheit eines Aktivator-Oligonukleotdies unwahrscheinlich oder signifikant verlangsamt ist.

Die anzustrebende Erhöhung der Spezifität einer Amplifikation resultiert somit aus der Sequenzabhängigkeit der Trennung von komplementären Primer-Verlängerungsprodukten, welche eine Zielsequenz umfassen: Aktivator-Oligonukleotid ermöglicht bzw. begünstigt diese Doppelstrang-Trennung als Folge der Übereinstimmung seiner Sequenzabschnitte mit vorgegebenen Sequenzabschnitten der Primer-Verlängerungsprodukte. Diese Übereinstimmung wird nach jedem Synthese-Zyklus durch Aktivator-Oligonukleotid überprüft. Die exponentielle Amplifikation resultiert als Folge aus erfolgreichen Wiederholungen aus Synthese-Vorgängen und sequenzspezifischen Strangverdränungen durch Aktivator-Oligonukleotid, d.h. "Aktivierungen" (Doppelstrang-Öffnungen / Doppelstrang-Trennungen / Strangverdrängungs-Vorgängen resultierend in einzelsträngiger Form von entsprechenden Primer-Bindungsstellen) von neusynthetisierten Primer-Verlängerungsprodukten.

Die Gestaltung von Aktivator-Oligonukleotid und den Reaktionsbedinungen führt dazu, dass die Interaktion des Aktivator-Oligonukleotides mit unspezifischen Primer-Verlängerungsprodukten anders abläuft. Aufgrund unzureichender Komplementarität zwischen Aktivator-Oligonukleotid und einem unspezifischen Primer-Verlängerungsprodukt kommt es nicht zu einer hinreichenden Doppelstrang-Trennung / Strangverdrängung von einem solchen unspezifischen Primer-Verlängerungsprodukt von seinem Matrizenstrang. Das unspezifiche Produkt bleibt somit vorwiegend in einem "inaktiven", doppelsträngigen Zustand.

Der Einsatz von vordefiniertem Aktivator-Oligonukleotid ermöglicht somit eine sequenzabhängige Überprüfung der Inhalte von Primer-Verlängerungsprodukten zwischen einzelnen Synthese-Schritten während der exponentiellen Amplifikation und Herbeiführung einer Selektion bzw. einer Auswahl von Sequenzen für nachfolgende Synthese-Schritte. Dabei kann zwischen "aktiven", einzelsträngigen Zuständen von neusynthetisierten spezifischen Primer-Verlängerungsprodukten als Folge einer erfolgreichen Interaktion mit einem Aktivator-Oligonukleotid, und "inaktiven" doppelsträngigen Zuständen von neusynthetisierten unspezifischen Primer-Verlängerungsprodukten als Folge einer mangelhaften und / oder unzureichenden und /oder verminderten und / oder verlangsamten Interaktion mit einem Aktivator-Oligonukleotid unterschieden werden.

Für eine exponentielle Amplifikation ergeben sich daraus folgende Effekte:
Unter nicht-denaturierenden Bedingungen erfolgt die Trennung von spezifisch synthetisierten Strängen unter Mitwirkung von Aktivator-Oligonukleotid.

Exponentielle Amplifikation von Zielsequenz umfassenden Nukleinsäureketten erfolgt sequenzkontrolliert (Hauptreaktion). Diese Sequenz-Kontrolle erfolgt nach jedem Synthese-Schritt und schließt Sequenzsegmente ein, welche zwischen Primern liegen und eine Zielsequenz umfassen. Die erfolgreiche Überprüfung des Ergebnisses der Synthese nach jedem Synthese-Schritt resultiert in Trennng von beiden spezifischen Primer-Verlängerungsprodukten, was die Voraussetzung für weitere spezifische Synthese-Schritte darstellt.

Während der Amplifikation kann eine initiale Entstehung / Generierung von unspezifischen Primer-Verlängerungsprodukten nicht prinziell ausgeschlossen werden (Nebenprodukte). Aufgrund einer Matrizenabhängigkeit liegen solche unspezifischen Primer-Verlängerungsprodukte unmittelbar nach ihrer Synthese in der Regel in doppelsträngiger Form vor. Die Interaktion mit dem Aktivator-Oligonukleotid bleibt allerdings entweder vollständig aus oder ist eingeschränkt, so dass es nicht zu einer Strangtrennung kommt oder die Strangtrennung verlangsamt gegenüber der Hauptreaktion ist. Die Übertragung einer fehlerhaften Sequenzinformation von einem Synthese-Zyklus auf den nächsten findet somit nicht statt.

Durch die Wahl der Reaktions-Bedingungen und die Gestaltung von Aktivator-Oligonukleotid ist es somit möglich, einen gezielten Einfluss auf Effizienz der Regenerierung von korrekten Nukleinsäureketten-Matrizen zwischen einzelnen Synthese-Schritten im Rahmen eines Amplifikationsverfahrens auszuüben. Im Allgemeinen, je höher das Maß der Übereinstimmung der synthetisierten Sequenz mit der vorgegebenen Sequenz des Aktivator-Oligonukleotides, umso erfolgreicher ist die Trennung der synthetisierten Produkte, umso erfolgreicher ist die Regenerierung von korrekten Matrizen von einem Synthese-Schritt zum nächsten. Umgekehrt führt eine Sequenz-Abweichung bei Nebenprodukten zu einer insuffizienten Regenerierung von Matrizensträngen und somit zu einer Verlangsamung der Synthese-Initiierung bzw. zu Reduzierung der Ausbeute in jedem nachfolgenden Zyklus. Die gesamte exponentielle Amplifikation von Nebenprodukten verläuft entweder langsamer oder findet gar nicht statt und / oder bleibt auf einem nicht detektierbaren Niveau..

Reduzierung der Kinetik der Vermehrung von Nebenprodukten oder gar eine Verhinderung der Ausbildung von gewissen Arten von Nebenprodukten kann in einer Erhöhung der Spezifität der Amplifikation resultieren und somit Einfluss auf Spezifität der gesamten Amplifikations-basierten Tests haben.

Das Verfahren ermöglicht somit eine Überprüfung der synthetisierten Sequenzen in Echtzeit, d.h. ohne Reaktions-Unterbrechung und stellt somit Potenzial für Entwicklung von homogenen Assays dar, bei welchen alle Komponenten des Assays bereits zu Beginn einer Reaktion im Reaktionsgemisch vorliegen.

Das erfindungsgemäße Verfahren stellt ein exponentielles Amplifikationsverfahren für Nukleinsäureketten mit einer Feedback-Funktion (Regelungs-Funktion) dar. In vereinfachter Form kann die Funktion einzelner Strukturen folgendermassen zusammengefasst werden (in Anlehnung an Blockschaltbild nach DIN EN 60027-6):

Das Aktivator-Oligonukleotid zusammen mit beiden Primern definieren durch ihre vorausgewählte Sequenzen einen "Sollwert" der Sequenz, welche im Rahmen der Amplifikation synthetisiert werden muss (die zu Amplifizierende Nukleinsäurekette umfassend die Zielsequenz). Die in jedem Synthese-Zyklus generierten neuen Primer-Verlängerungsprodukte bilden die "Regelgröße" (Ist-Wert). Das Aktivator-Oligonukleotid bildet einen Teil eines Regel-Systems (umfassend "Sensor", "Regler" und "Stellglied"), welches sowohl den Abgleich der Komplentarität zwischen neu synthetisierten Strängen und dem Strang des Aktivator-Oligonukleotides als auch die "Stellgröße" beeinflussen kann (als Folge dieses Abgleichs). Unter "Stellgröße" wird dabei die Überführung der synthetisierten Stränge in einzelsträngige Form ("Aktivierung von Primerbindungsstellen" bzw. "Regenerierung der Matrizenstränge") bei hinreichenden Übereinstimmung zwischen "Ist-Wert" und "Soll-Wert" verstanden oder beibehalten der doppelsträngigen Form der synthetisierten Stränge im Falle einer nicht hinreichenden Übereinstimmung ("Ausbleiben der Aktivierung", Primerbindungsstellen bleiben in doppelsträngigem Zustand und somit unzugängig für erneute Primer-Bindung).

Da der Inhalt der synthetisierten Sequenzen innerhalb / während der Amplifikation fortlaufend kontrolliert wird, kann das Aktivator-Oligonukleotide auch als "Kontroll-Oligonukleotid" angesehen werden.

Die Kontrolle der synthetisierten Sequenzen durch das Aktivator-Oligonukleotid geht wesentlich weiter als Primer-Bereiche bzw. Primer-Bindungsstellen, und umfasst interne Sequenzbereiche von synthetisierten Nukleinsäureketten, welche zwischen beiden Primern liegen. In einer Ausführungsform kann die Gesamtsequenz der Zielsequenz bzw. der zu amplifizierenden Nukleinsäurekette kontrolliert werden.

Diese Kontrolle (Überprüfung der Sequenz) erfolgt bereits im Stadium der exponentiellen Synthese und hat Auswirkung auf das Ergebnis / Ausbeuten der Synthesereaktion und nicht erst im Anschluß an die exponentielle Amplifikation.

Insgesamt kann durch Verwendung von vordefinierten Sequenzen im Aktivator-Oligonukleotid in Kombination mit Reaktionsbedinungen ein Einfluss auf die exponentielle Amplifikation ausgeübt werden, welcher über die Primer-Bereiche hinausgeht. Dieser Einfluss kann von einer Toleranz zu bestimmten Sequenzabweichungen in der Zielsequenz oder einer Unterdrückung der Reaktonsgeschwindigkeit der Amplifikationsreaktion bis zu vollständigen Blockade der exponentiellen Amplifikation führen.

### Begriffe und Definitionen:

Im Rahmen der vorliegenden Offenbarung haben die verwendeten Begriffe folgende Bedeutung:
Der Begriff **"Oligonukleotid"** wie er hier bezugnehmend auf Primer, Aktivator-Oligonukleotid, Sonden, zu amplifizierende Nukleinsäurekette verwendet wird, ist als ein Molekül definiert, das zwei oder mehr, bevorzugt mehr als drei Desoxyribonukleotide und / oder Ribonukleotide und / oder Nukleotid-Modifikationen und / oder Nicht-Nukleotid-Modifikationen umfasst. Seine Länge umfasst beispielsweise Bereiche zwischen 3 bis 300 Nukleotid-Einheiten oder ihrer Analoga, bevorzugt zwischen 5 bis 200 Nukleotid-Einheiten oder ihrer Analoga. Seine genaue Größe hängt von vielen Faktoren ab, die ihrerseits von der letztendlichen Funktion oder Verwendung der Oligonukleotide abhängen.

Der Begriff **"Primer"**, wie er hier verwendet wird, betrifft ein Oligonukleotid, unabhängig davon, ob es natürlicherweise z. B. in einer gereinigten Restriktionsspaltung vorkommt oder synthetisch produziert wurde. Ein Primer ist fähig, als Initiationspunkt der Synthese zu wirken, wenn er unter Bedingungen eingesetzt wird, bei denen die Synthese eines zu einem Nukleinsäurestrang komplementären Primer-Verlängerungsproduktes induziert wird, d. h. in Gegenwart von Nukleotiden und einem induzierenden Agens, wie z.B. DNA-Polymerase bei einer geeigneten Temperatur und einem geeigneten pH-Wert. Der Primer ist für eine maximale Wirksamkeit bei der Amplifikation bevorzugt einzelsträngig. Der Primer muss genügend lang sein, um in Gegenwart des induzierenden Agens die Synthese des Verlängerungsproduktes zu initiieren. Die genaue Länge des Primers hängt von vielen Faktoren ab, einschließlich der Reaktions-Temperatur und der Primerquelle und der Anwendung des Verfahrens. Beispielsweise beträgt die Länge der Oligonukleotid-Primer bei diagnostischen Anwendungen, je nach Komplexität der Zielsequenz zwischen 5 bis 100 Nukleotide, vorzugsweise 6 bis 40 und besonders bevorzugt 7 bis 30 Nukleotide. Kurze Primer-Moleküle erfordern im allgemeinen für Ausübung ihrer Primer-Funktion niedrigere Reaktions-Temperaturen, um genügend stabile Komplexe mit der Matrize zu bilden, oder höhere Konzentrationen von anderen Reaktionskomponenten, beispielsweise von DNA-Polymerasen, so dass eine ausreichende Verlängerung von gebildeten Primer-Matrizen-Komplexen erfolgen kann.

Die hier verwendeten Primer sind so ausgewählt, das sie "im Wesentlichen" komplementär zu den verschiedenen Strängen jeder spezifischen zu amplifizierenden Sequenz sind. Das bedeutet, dass die Primer genügend komplementär sein müssen, um mit ihren betreffenden Strängen zu hybridisieren und eine Primer-Verlängerungsreaktion zu initiieren. Somit braucht die Primersequenz beispielsweise nicht die genaue Sequenz der Zielsequenz wiederzuspiegeln. Zum Beispiel kann ein nicht-komplementäres Nukleotidfragment an dem 5'-Ende des Primers angeheftet sein, wobei der Rest der Primersequenz komplementär zu dem Strang ist. In einer anderen Ausführungsform können einzelne nicht-komplementäre Basen oder längere nicht-komplementäre Sequenzen in einem Primer eingefügt sein, vorausgesetzt, dass die Primersequenz eine genügend große Komplementarität mit der zu amplifizierenden Sequenz des Stranges aufweist, um damit zu hybridisieren und so ein Primer-Matrizen-Komplex fähig zur Synthese des Verlängerungsproduktes zu erzeugen.

Im Rahmen der enzymatischen Synthese eines zu Matrize komplementären Stranges wird ein Primer-Verlängerungsprodukt erzeugt, was vollständig zum Matrizen-Strang komplementär ist.

### Tm-Schmelztemperatur

Als Schmelztemperatur eines komplementären oder partiell komplementären Doppelstranges wird im Allgemeinen ein Messwert einer Reaktionstemperatur verstanden, bei welchem ca. die Hälfte der Stränge als Doppelstrang vorliegt und die andere Hälfte als Einzelstrang vorliegt. Das System (Assoziation und Dissoziation von Strängen) befindet sich im Gleichgewicht.

Aufgrund einer Vielzahl von Faktoren, welche die Tm eines Doppelstranges beeinflussen können (z.B. Sequenzlänge, CG-Gehalt der Sequenz, Puffer-Bedinungen, Konzentration von divalenten Metal-Kationen etc.) sollte die Bestimmung der Tm einer zu amplifizierenden Nukleinsäure unter gleichen Bedinungen erfolgen, wie die beabsichtigte Amplifikationsreaktion.

Wegen Abhängigkeit der messbaren Schmelztemperatur von multiplen Reaktions-Parametern, z.B. von jeweiligen Puffer-Bedingungen und jeweiligen Konzentrationen der Reaktionsteilnehmer, wird unter Schmelztemperatur ein Wert verstanden, welcher in gleichem Reaktionspuffer gemessen wurde wie die exponentielle Amplifikation, bei Konzentrationen von beiden komplementären Komponenten eines Doppelstranges von etwa 0,1µmol/l bis etwa 10 µmol/l, bevorzugt in Konzentration von etwa 0,3 µmol/ bis ca. 3 µmol/l, vorzugsweise bei ca. 1 µmol/l. Bei dem jeweiligen Wert der Schmelztemperatur handelt sich um einen Richtwert, welcher mit der Stabilität eines jeweiligen Doppelstranges korreliert.

Die **Desoxyribonukleosid-Triphosphate** (dNTPs) dATP, dCTP, dGTP und TTP (oder dUTP, oder dUTP/TTP-Gemisch) werden in adäquaten Mengen zum Synthesegemisch gegeben. In einer Ausführungsform können zusätzlich zu dNTPs mindestens eine weitere Art von dNTP-Analoga zum Synthesegemisch zugegeben werden. Diese dNTP-Analoga umfassen in einer Ausführungsform beispielsweise eine charakteristische Markierung (z.B. Biotin oder Fluoreszenzfarbstoff), so dass wenn in Nukleinsäurestrang eingebaut, auch diese Markierung in den Nukleinsäurestrang integriert ist. In einer anderen Ausführungsform umfassen diese dNTP-Analoga mindestens eine Modifikation von Zucker-Phosphat-Anteil des Nukleotids, z.B. alpha-Phosphorothioat-2'-Desoxyribonukleosid-Triphosphate (oder andere Modifikationen, welche einem Nukleinsäurestrang eine Nuklease-Resistenz verleihen), 2',3'-Dideoxy-Ribonukleosid-Triphosphate, Azyklo-Nukleosid-Triphosphate (oder andere zur Termination einer Synthese führende Modifikationen). In einer weiteren Ausführungsform umfassen diese dNTP-Analoga mindestens eine Modifikation von Nukleobase, z.B. Iso-Cytosine, Iso-Guanosine (oder auch andere Modifikationen der Nukleobasen des erweiterten genetischen Alphabets), 2-Amino-Adenosine, 2-Thiouridine, Inosine, 7-deazy-adenosine, 7-deaza-guanosine, 5-Me-Cytosine, 5-Propyl-Uridine, 5-Propyl-Cytosine (oder auch andere Modifikationen von Nukleobasen, welche gegenüber natürlichen Nukleobasen durch eine Polymerase eingebaut werden können und zur Änderung der Strang-Stabilität führen). In einer weiteren Ausführungsform umfasst ein dNTP-Analog sowohl eine Modifikation der Nukleobase als auch eine Modifikation des Zucker-Phosphat-Anteils. In einer weiteren Ausführungsform wird statt mindestens eines natürlichen dNTP-Substrates mindesten eine weitere Art von dNTP-Analoga zum Synthesegemisch zugegeben.

Das die Nukleinsäure-Synthese induzierende Agens kann eine Enzyme einschließende Verbindung oder ein System sein, das so wirkt, dass dadurch die Synthese der Primer-Verlängerungsprodukte bewirkt wird. Geeignete Enzyme für diesen Zweck umfassen z.B. **DNA-Polymerasen** wie Bst Polymerase und ihre Modifikationen, Vent Polymerase und andere - bevorzugt hitzestabile DNA-Polymerasen, die den Einbau der Nukleotide in der korrekten Weise ermöglichen, wodurch die Primer-Verlängerungsprodukte gebildet werden, die zu jedem synthetisierten Nukleinsäurestrang komplementär sind. Im Allgemeinen wird die Synthese am 3'-Ende eines jeden Primers initiiert und schreitet dann in 5'- Richtung entlang des Matrizenstranges fort, bis die Synthese beendet ist oder unterbrochen wird.

Bevorzugt werden Polymerasen verwendet, welche zu Strangverdrängung fähig sind. Dazu zählen beispielsweise großes Fragment der Bst Polymerase oder ihre Modifikationen (z.B. Bst 2.0 DNA Polymerase), Klenow Fragment, Vent exo minus Polymerase, Deepvent exo minus DNA Polymerase, großes Fragment der Bsu DNA Polymerase, großes Fragment der Bsm DNA Polymerase.

In einer Ausführungsform werden bevorzugt Polymerasen eingesetzt, welche keine 5'-3'-Exonuklease-Aktivität aufweisen, bzw. keine 5'-3'-FEN-Aktivität aufweisen.

In einer Ausführungsform werden mindestens zwei verschiedene Polymerasen eingesetzt, beispielsweise Polymerasen welche zu Strangverdrängung fähig sind und solche, welche eine 3'-5'-Proofreading Aktivität aufweisen.

In bevorzugten Ausführungsformen werden Polymerasen mit einer Hotstart-Funktion eingesetzt, welche erst beim Erreichen einer bestimmten Temperatur ihre Funktion entfalten können.

### Reaktionsbedingungen

Reaktionsbedingungen umfassen unter anderem Puffer-Bedingungen, Temperatur-Bedingungen, Zeitdauer der Reaktion und Konzentrationen von jeweiligen Reaktionskomponenten.

Im Verlauf der Reaktion akkumuliert die Menge der spezifischen produzierten zu amplifizierenden Nukleinsäure in einer exponentiellen Weise. Die Reaktion umfassend die Synthese der Verlängerungsprodukte kann zur Produktion der gewünschten Menge der spezifischen Nukleinsäuresequenz so lange wie nötig durchgeführt werden. Das erfindungsgemäße Verfahren wird bevorzugt kontinuierlich durchgeführt. In bevorzugter Ausführungsform läuft die Amplifikations-Reaktion bei gleicher Reaktionstemperatur ab, wobei die Temperatur bevorzugt zwischen 50°C und 70°C liegt. In einer anderen Ausführungsform kann die Reaktions-Temperatur auch variabel gesteuert werden, so dass einzelne Schritte der Amplifikation bei jeweils unterschiedlichen Temperaturen verlaufen.

Die für die exponentielle Amplifikation benötigten Reagenzien sind vorzugsweise bereits zu Beginn einer Reaktion im gleichen Ansatz vorhanden. In einer anderen Ausführungsform können Reagenzien auch in späteren Stadien des Verfahrens zugegeben werden.

Bevorzugt werden keine Helicasen oder Recombinasen in der Reaktionsmischung zur Trennung der neu synthetisieren Doppelstränge der zu amplifizierenden Nukleinsäure verwendet.

In einer bevorzugten Ausführungsform beinhaltet die Reaktionsmischung keine biochemischen Energie-spendenden Verbindungen wie ATP.

Die Menge der zu Beginn der Reaktion vorliegenden zu amplifizierenden Nukleinsäure kann zwischen wenigen Kopien und mehreren Milliarden Kopien in einem Ansatz vorliegen. Bei diagnostischen Einsätzen kann die Menge der zu amplifizierenden Nukleinsäurekette unbekannt sein.

In der Reaktion können auch weitere, nicht zu amplifizierende Nukleinsäuren vorliegen. Diese Nukleinsäuren können von natürlichen DNA oder RNA oder ihren Äquivalenten abstammen. In einer Ausführungsform liegen Kontroll-Sequenzen im gleichen Ansatz vor, welche parallel zu der zu amplifizierenden Nukleinsäure ebenfalls amplifiziert werden sollen.

Bevorzugt wird ein molarer Überschuss von ungefähr 10³:1 bis ungefähr 10¹⁵:1 (Verhältnis Primer:Matrize) der eingesetzter Primer und des Aktivator-Oligonukleotides zu der Reaktionsmischung gegeben, welche Matrizen-Stränge für die Synthese der zu amplifizierenden Nukleinsäurekette umfasst.

Es kann die Menge der Zielnukleinsäuren nicht bekannt sein, wenn das erfindungsgemäße Verfahren in diagnostischen Anwendungen benutzt wird, so dass die relative Menge des Primers und des Aktivator-Oligonukleotides bezüglich des komplementären Stranges nicht mit Sicherheit bestimmt werden kann. Die Menge des zugefügten Primers wird im Allgemeinen im molaren Überschuss bezüglich der Menge des komplementären Stranges (Matrize) vorhanden sein, wenn die zu amplifizierende Sequenz in einem Gemisch von komplizierten langkettigen Nukleinsäuresträngen enthalten ist. Ein großer molarer Überschuss wird bevorzugt, um die Effizienz des Verfahrens zu verbessern.

Die verwendeten Konzentrationen von Primer-1, Primer-2 und Aktivator-Oligonukleotide liegen beispielsweise in Bereichen zwischen 0,01 µmol/l und 100 µmol/l, bevorzugt zwischen 0,1 µmol/l und 100 µmol/l, bevorzugt zwischen 0,1 µmol/l und 50 µmol/l, besser zwischen 0,1 µmol/l und 20 µmol/l. Die hohe Konzentration von Komponenten kann die Geschwindigkeit der Amplifikation erhöhen. Die jeweiligen Konzentrationen einzelner Komponenten können unabhängig von einander variiert werden, um das gewünschte Reaktionsergebnis zu erzielen.

Die Konzentration von Polymerase liegt in Bereichen zwischen 0,001 µmol/l und 50 µmol/l, vorzugsweise zwischen 0,01 µmol/l und 20 µmol/l, besser zwischen 0,1µmol/l und 10 µmol/l.

Die Konzentration von einzelnen dNTP-Substraten liegt in Bereichen zwischen 10 µmol/l und 10 mmol/l, vorzugsweise zwischen 50 µmol/l und 2 mmol/l, besser zwischen 100 µmol/l und 1 mmol/l. Die Konzentration von dNTP kann die Konzentration von divalenten Metal-Kationen beeinflußen. Gegebenenfalls wird diese entsprechend angepasst.

Als divalente Metal-Kationen werden beispielsweise Mg2+ verwendet. Als entsprechendes Anion können beispielsweise Cl, Acetat, Sulfat, Glutamat etc. verwendet werden.

Die Konzentration von divalente Metal-Kationen wird beispielsweise an den für jeweilige Polymerase optimalen Bereich angepasst und umfasst Bereiche zwischen 0,1 mmol/l und 50 mmol/l, besser zwischen 0,5 mmol/l und 20 mmol/l, bevorzugter zwischen 1 mmol/l und 15 mmol/l.

Die enzymatische Synthese erfolgt im Allgemeinen in einer gepufferten wässrigen Lösung. Als Puffer-Lösungen können gelöste konventionelle Puffer-Substanzen, wie Tris-HCI, Tris-Acetat, Kalium-Glutamat, HEPES-Puffer, Natrium-Glutamat in gängigen Konzentrationen verwendet werden. Der pH-Wert dieser Lösungen liegt üblicherweise zwischen 7 und 9,5, bevorzugt etwa bei 8 bis 8,5. Die Puffer-Bedingungen können beispielsweise nach Empfehlung des Herstellers der verwendeten Polymerase angepasst werden.

Weitere Substanzen, wie sogenannte Tm-Depressoren (z.B. DMSO, Betaine, TPAC) etc. können zum Puffer zugegeben werden. Solche Substanzen verringern die Schmelztemperatur ("Tm-Depressoren") von Doppelsträngen und können somit einen positiven Einfluss auf die Öffnung von Doppelsträngen haben. Auch Polymerase-stabilisierende Komponenten, wie Tween 20 oder Triton 100 können in üblichen Mengen zum Puffer zugegeben werden. EDTA oder EGTA kann zu Komplexierung von Schwermetallen in konventionellen Mengen zugegeben werden. Polymerase-stabilisierende Substanzen, wie Trehalose oder PEG 6000 können ebenfalls zum Reaktionsgemisch zugegeben werden.

Vorzugsweise enthält die Reaktionsmischung keine Inhibitoren der Strangverdrängungsreaktion und keine Inhibitoren einer Polymerasenabhängigen Primer-Verlängerung.

In einer Ausführungsform enthält die Reaktionsmischung DNA-bindende Farbstoffe, bevorzugt interkalierenden Farbstoffe, wie z.B. EvaGreen oder SybrGreen. Solche Farbstoffe können ggf. bei Detektion der Neubildung von Nukleinsäureketten ermöglichen.

Die Reaktionsmischung kann weiterhin Proteine bzw. andere Substanzen enthalten, welche beispielsweise aus einem Original-Material stammen und welche die Amplifikation vorzugsweise nicht beeinflussen.

### Temperatur-Bedingungen

Die Temperatur hat einen wesentlichen Einfluss auf die Stabilität der Doppelstränge.

In einer bevorzugten Ausführungsform werden während der Amplifikationsreaktion keine Temperaturbedinungen verwendet, welche im Wesentlichen zur Trennung von Doppelsträngen der zu amplifizierenden Nukleinsäure in Abwesenheit von Aktivator-Oligonukleotid führen. Damit soll sichergestellt werden, dass die Doppelstrang-Trennung von zu amplifizierenden Nukleinsäureketten von Anwesenheit des Aktivator-Oligonukleotides im gesamten Verlauf der Amplifikation abhängig ist.

Bei Temperatur etwa in Höhe von der gemessenen Schmelztemperatur (Tm) der zu amplifizierenden Nukleinsäure kommt es zu einer spontanen Trennung von beiden Strängen der zu amplifizierenden Nukleinsäure, so dass Einfluss des Aktivator-Oligonukleotids auf Trennung von synthetisierten Strängen und somit auf die Sequenzspezifität der Amplifikation minimal bis begrenzt ist.

Bei einer exponentiellen Amplifikation, welche weniger sequenzspezifisch (d.h. wenig Aktivator-Oligonukleotid-abhängig) ablaufen muss, kann die Reaktionstemperatur beispielsweise um die Schmelztemperatur (d.h. Tm plus / minus 3° bis 5°C) der zu amplifizierenden Nukleinsäure liegen. Bei einer solchen Temperatur können Sequenzunterschiede zwischen Aktivator-Oligonukleotid und dem synthetisierten Primer-Verlängerungsprodukt im Rahmen einer Strangverdrängungsreaktion im Allgemeinen gut toleriert werden.

Auch im Temperatur-Bereich von ca. (Tm minus 3° C) bis ca. (Tm minus 10°C) kann es immer noch zu einer spontanen Strangtrennung von synthetisierten Primer-Verlängerungsprodukten kommen, obwohl mit geringerer Effizienz. Der Einfluss von Aktivator-Oligonukleotid auf die Sequenzspezifität der zu amplifizierenden Nukleinsäure ist höher als bei Temperaturbedingungen um die Schmelztemperatur (Tm) der zu amplifizierenden Nukleinsäurekette.

Zwar findet die Strangtrennung mit sinkender Reaktionstemperatur im Wesentlichen dank Interaktion des neusynthetisierten Doppelstranges mit Aktivator-Oligonukleotid statt, Allerdings können Duplexe aus Primer bei Verlängerungstemperatur unter den genannten Bedingungen auch spontan zerfallen, d.h. ohne sequenzabhängige Strangverdrängung durch Aktivator-Oligonukleotid. Beispielsweise liegt die Reaktionstemperatur bei einer vermindert sequenzspezifischen Amplifikation in Bereichen zwischen ca. (Tm minus 3°C) und ca. (Tm minus 10°C), bevorzugt zwischen ca. (Tm minus 5°C) und ca. (Tm minus 10°C). Bei einer solchen Temperatur werden Sequenzunterschiede zwischen Aktivator-Oligonukleotid und dem synthetisierten Primer-Verlängerungsprodukt im Rahmen einer Strangverdrängungsreaktion schlechter toleriert.

Eine hohe Sequenzspezifität der Amplifikation des Verfahrens wird vor allem dann erreicht, wenn die neu synthetisierten Stränge der zu amplifizierenden Nukleinsäure unter Reaktionsbedinungen nicht spontan in Einzelstränge dissoziieren können. In einem solchen Fall spielt die sequenzspezifische Strangverdrängung durch Aktivator-Oligonukleoid für eine sequenzspezifische Strangtrennung eine entscheidende Rolle und ist für die Sequenzspezifität der Amplifikatonsreaktion maßgeblich verantwortlich. Dies kann im allgemeinen erreicht werden, wenn die Reaktionstemperatur deutlich unter der Schmelztemperatur von beiden Strängen der zu amplifizierenden Nukleinsäure liegt und keine weiteren Komponenten für eine Strangtrennung verwendet werden, beispielsweise keine Helicasen oder Recombinasen. Beispielsweise liegt die Reaktionstemperatur bei einer sequenzspezifischen Amplifikation in Bereichen zwischen ca. (Tm minus 10°C) und ca. (Tm minus 50°C), bevorzugt zwischen ca. (Tm minus 15°C) und ca. (Tm minus 40°C), besser zwischen ca. (Tm minus 15°C) und ca. (Tm minus 30°C).

In einer bevorzugten Ausführungsform der Amplifikation wird die maximale Reaktions-Temperatur im Verlauf der gesamten Amplifikationsreaktion nicht über die Schmelztemperatur der zu amplifizierenden Nukleinsäurekette erhöht.

In einer weiteren Ausführungsform der Amplifikation kann die Reaktionstemperatur über die Schmelztemperatur der zu amplifizierenden Nukleinsäurketten mindestens einmal erhöht werden. Die Erhöhung der Temperatur kann beispielsweise zu Beginn der Amplifikationsreaktion erfolgen und zu Denaturierung von Doppelsträngen einer genomischen DNA führen. Dabei muss beachet werden, dass während eines solchen Schrittes die Abhängigkeit der Doppelstrang-Trennung von der Wirkung des Aktivator-Oligonukleotides aufgehoben ist oder zumindest signifikant reduziert ist.

Die Reaktionstemperaturen der einzelnen Schritte der Amplifikationsreaktion können im Bereich von ca. 15°C bis ca. 85°C, besser im Bereich von ca. 15°C bis ca. 75°C, bevorzugt im Bereich von ca. 25°C bis ca. 70°C liegen.

In den nachfolgend beschriebenen Beispielen 2 und 3 wurde eine Reaktionstemperatur der Amplifikationsreaktion von 65°C verwendet, wobei die Tm der zu amplifizierenden Nukleinsäuren zwischen ca. 75°C und ca. 80°C lag. Somit war der Doppelstrang der zu amplifizierenden Nukleinsäure unter Reaktionsbedingungen stabil und die Amplifikationsreaktion sequenzspezifisch (siehe Beispiel 3).

Im Allgemeinen kann für die Reaktionstemperatur für jeden einzelnen Reaktionsschritt optimal eingestellt werden, so dass für jeden Reaktionsschritt eine solche Temperatur herbeigeführt wird. Die Amplifikationsreaktion umfasst somit eine sich wiederholende Änderung von Temperaturen, welche zyklisch wiederholt werden. In einer vorteilhaften Ausführungsform des Verfahrens werden Reaktionsbedingungen für mehrere Reaktionsschritte vereinheitlicht, so dass die Anzahl von Temperatur-Schritten geringer ist als die Anzahl von Reaktionsschritten. In einer solchen bevorzugten Ausführungsform der Erfindung erfolgt mindestens einer der Schritte der Amplifikation bei einer Reaktionstemperatur, welche sich von der Reaktionstemperatur anderer Schritte der Amplifikation unterscheidet. Die Reaktion verläuft somit nicht isothermal, sondern die Reaktionstemperatur wird zyklisch geändert.

Es werden beispielsweise während Amplifikation mindestens zwei Temperatur-Bereiche verwendet, welche abwechselnd herbeigeführt werden (zyklische Änderung von Temperaturen zwischen einzelnen Temperatur-Bereichen). In einer Ausführungsform umfasst der untere Temperatur-Bereich beispielsweise Temperaturen zwischen 25°C und 60°C, besser zwischen 35°C und 60°C, bevorzugt zwischen 50°C und 60°C und der obere Temperatur-Bereich umfasst beispielsweise Temperaturen zwischen 60°C und 75°C, besser zwischen 60°C und 70°C.

In einer weiteren Ausführungsform umfasst der untere Temperatur-Bereich beispielsweise Temperaturen zwischen 15°C und 50°C, besser zwischen 25°C und 50°C, bevorzugt zwischen 30°C und 50°C und der obere Temperatur-Bereich umfasst beispielsweise Temperaturen zwischen 50°C und 75°C, besser zwischen 50°C und 65°C.

In einer weiteren Ausführungsform umfasst der untere Temperatur-Bereich beispielsweise Temperaturen zwischen 15°C und 40°C, besser zwischen 25°C und 40°C, bevorzugt zwischen 30°C und 40°C und der obere Temperatur-Bereich umfasst beispielsweise Temperaturen zwischen 40°C und 75°C, besser zwischen 40°C und 65°C.

Die Temperatur kann im jeweiligen Bereich konstant gehalten werden oder als TemperaturGradient (abfallend oder aufsteigend) geändert werden.

Weitere Erläuterungen zu Temperatur-Einstellungen werden in folgenden Abschnitten bei Ausführungsformen im Einzelnen angeführt.

Jede herbeigeführte Temperatur kann eine gewisse Zeit aufrechterhalten werden, so dass dabei ein Inkubations-Schritt resultiert. Das Reaktionsgemisch kann somit während einer Amplifikation bei einer ausgewählten Temperatur eine gewisse Zeit inkubiert werden. Diese Zeit kann unterschiedlich lange für jeweiligen Inkubations-Schritt sein und kann sich an den Fortschritt der jeweiligen Reaktion bei gegebener Temperatur richten (z.B. Primer-Verlängerung oder Strangverdrängung usw.). Die Zeit eines Inkubationsschrittes kann folgende Bereiche umfassen: zwischen 0,1 sec und 10.000 sec, besser zwischen 0,1 sec und 1000 sec, bevorzugt zwischen 1 sec und 300 sec, noch bevorzugter zwischen 1 sec und 100 sec.

Durch eine solche Temperatur-Änderung können einzelne Reaktionsschritte bevorzugt bei einer ausgewählten Temperatur ausgeführt werden. Dadurch können Ausbeuten von einem jeweiligen Reaktionsschritt verbessert werden. Innerhalb eines Synthese-Zyklus kann Temperatur-Änderung bzw. Temperatur-Wechsel zwischen einzelnen Temperatur-Bereichen ggf. mehrmals herbeigeführt werden. Synthese-Zyklus kann somit mindestens ein Temperatur-Wechsel umfassen. Ein solcher Temperatur-Wechsel kann beispielsweise in einem PCR-Gerät / Thermocycler routinemäßig als zeitliches Programm ausgeführt werden.

In einer Ausführungsform wird ein Amplifikationsverfahren bevorzugt, bei welchem mindestens eines der Schritte, welche Strangverdrängung umfassen, und mindestens eines der Schritte, welche Primer-Verlängerungsreaktionen umfassen, gleichzeitig bzw. parallel stattfinden und unter gleichen Reaktionsbedingungen erfolgen. In einer solchen Ausführungsform kann beispielsweise eine Primer-Verlängerungsreaktion von mindestens einem Primer-Oligonukleotid (z.B. vom ersten Primer-Oligonukleotid) bevorzugt bei Temperaturbedinungen im unteren Temperatur-Bereich erfolgen. Hingegen erfolgt die Strangverdrängung unter Mitwirkung von Aktivator-Oligonukletid und die eine weitere Primer-Verlängerungsreaktion (z.B. vom zweiten Primer-Oligonukleotid) bevorzugt im Reaktionsschritt im oberen Temperatur-Bereich.

In einer weiteren Ausführungsform wird ein Amplifikationsverfahren bevorzugt, bei welchem mindestes eines der Schritte, welche Strangverdrängung durch das Aktivator-Oligonukleotid umfassen und mindestens eines der Schritte, welche Primer-Verlängerungsreaktion umfassen, bei unterschiedlichen Temperaturen durchgeführt. In einer solchen Ausführungsform können beispielsweise Primer-Verlängerungsreaktionen von mindestens einem Primer-Oligonukleotid (z.B. vom ersten Primer-Oligonukleotid und / oder von zweiten Primer-Oligonukleotid) bevorzugt bei Temperaturbedinungen im unteren Temperatur-Bereich erfolgen. Hingegen erfolgt die Strangverdrängung unter Mitwirkung von Aktivator-Oligonukletid bevorzugt im Reaktionsschritt im oberen Temperatur-Bereich.

In einer weiteren bevorzugten Ausführungsform verlaufen sämltliche Schritte einer Amplifikatons-Reaktion unter gleichen Reaktionsbedingungen.

In einer solchen Ausführungsform kann das Amplifikationsverfahren unter isothermalen Bedingungen durchgeführt werden, d.h. dass keine Temperaturänderungen für die Ausführung des Verfahrens erforderlich sind. In einer solchen bevorzugten Ausführungsform der Erfindung erfolgt die gesamte Amplifikationsreaktion unter konstanter Temperatur, d.h. die Reaktion ist isothermal. Die Zeit einer solchen Reaktion umfasst beispielsweise folgende Bereiche: zwischen 100 sec und 30.000 sec, besser zwischen 100 sec und 10.000 sec, noch besser zwischen 100 sec und 1000 sec.

Im Abschnitt "Beispiele" wird gezeigt, dass es möglich ist, Strukturen einzelner Reaktionskomponenten und entsprechende Reaktionsschritte dermassen aneinander abzustimmen, dass eine isothermale Reaktion möglich ist.

Die Summe aller Verfahrens-Schritte, welche zu einer Verdoppelung der Menge einer zu amplifizierenden Nukleinsäurekette führt, kann als Synthese-Zyklus bezeichnet werden. Ein solcher Zyklus kann entsprechend isothermal ablaufen oder auch durch Änderungen der Temperatur in seinem Verlauf gekennzeichnet sein. Die Temperatur-Änderungen können sich von Zyklus zu Zyklus wiederholen und identisch gestaltet werden.

Besonders vorteilhaft sind Amplifikationsverfahren, bei welchen die maximal erreichbare Temperatur eine Strang-Trennung unter Mitwirkung von Aktivator-Oligonukleotid nur dann im Wesentlichen zulässt, wenn mehr als 5 Nukleotide des dritten Bereichs des Aktivator-Oligonukleotides eine komplementäre Bindung mit dem ersten Primer-Verlängerungsprodukt eingehen können, vorteilhafter ist es, wenn mehr als 10, noch vorteilhafter, wenn mehr als 20 Nukleotide des Aktivator-Oligonukleotides mit dem ersten Primer-Verlängerungsprodukt eine Bindung eingehen. Im Allgemeinen gilt, je länger die erforderliche Bindung zwischen Aktivator-Oligonukleotid und dem komplementären Strang des ersten Primer-Verlängerungsproduktes, bevor die synthetisierten Stränge unter Reaktionsbedinungen dissoziieren, desto spezifischer ist die Amplifikations-Reaktion. Im Einzelnen, kann durch Verlängerung bzw. Verkürzung des dritten Abschnittes des Aktivator-Oligonukleotides das gewünschte Maß an Spezifität ermittelt werden.

Ein Verfahrensschritt kann bei seiner Wiederholung bei konstanter Temperatur erfolgen über die Gesamt-Dauer des Verfahrens oder auch bei unterschiedlichen Temperaturen.

Einzelne Verfahrensschritte können jeweils hintereinander durch Zugabe von einzelnen Komponenten ausgeführt werden. Bei einer vorteilhaften Ausführungsform liegen sämtliche für die Ausführung einer Amplifikation notwendige Reaktions-Komponenten zu Beginn einer Amplifikation in einem Reaktionsgemisch vor.

Der Start einer Amplifikations-Reaktion kann durch Zugabe einer Komponente erfolgen, z.B. durch Zugabe einer Nukleinsäurekette umfassend eine Zielsequenz (z.B. eine Start-Nukleinsäurekette), oder einer Polymerase oder divalenten Metal-Ionen, oder auch durch Herbeiführung von Reaktions-Bedingungen, welche für Amplifikation notwendig sind, z.B. Einstellung einer erforderlichen Reaktionstemperatur für einen oder mehrere Verfahrenschritte.

Die Amplifikation kann so lange ausgeführt werden, bis die gewünschte Menge an zu amplifizierenden Nukleinsäure erreicht ist. In einer anderen Ausführungsform wird die Amplifikationsreaktion über eine Zeit ausgeführt, welche bei Vorliegen einer zu amplifizierenden Nukleinsäure ausgereicht hätte, um eine ausreichende Menge zu bekommen. In einer anderen Ausführungsform wird die Amplifikationsreaktion über eine hinreichede Anzahl von Synthese-Zyklen (Verdoppelungszeiten) ausgeführt, welche bei Vorliegen einer zu amplifizierenden Nukleinsäure ausgereicht hätte, um eine ausreichende Menge zu bekommen.

Die Reaktion kann durch verschiedene Eingriffe gestoppt werden. Beispielsweise durch Änderung der Temperatur (z.B. Abkühlen oder Erhitzen, wobei beispielsweise Polymerase in ihrer Funktion gestört wird) oder durch Zugabe einer Substanz, welche eine Polymerase-Reaktion stoppt, z.B. EDTA oder Formamid.

Im Anschluss an die Amplifikation kann die amplifizierte Nukleinsäurekette für weitere Analysen verwendet werden. Dabei können synthetisierte Nukleinsäureketten durch verschiedene Detektionsverfahren analysiert werden. Beispielsweise können fluoreszenzmarkierte Oligonukleotid-Sonden verwendet werden oder Sequenzierungsverfahren (Sanger-Sequenzierung oder Next-Generation Sequenzierung), fest-Phasen-Analysen wie Microarray oder Bead-Array-Analysen usw. Die synthetisierte Nukleinsäurekette kann als Substrat / Matrize in weiteren Primer-Verlängerungsreaktionen verwendet werden.

In einer vorteilhaften Ausführungsform wird der Fortschritt der Synthese-Reaktion während der Reaktion überwacht. Das kann beispielsweise durch Einsatz von interkalierenden Farbstoffen erfolgen, z.B. Sybrgreen oder Evagreen, oder unter Einsatz von markierten Primern (z.B. Lux-Primer oder Scorpion-Primer) oder unter Einsatz von fluoreszenzmarkierten Oligonukleotid-Sonden.

Das Amplifikationsverfahren kann zur Überprüfung der Anwesenheit einer Zielnukleinsäurekette in einem biologischen Material oder einem diagnostischen Material im Rahmen eines diagnostischen Verfahrens eingesetzt werden.

### Erstes Primer-Oligonukleotid:

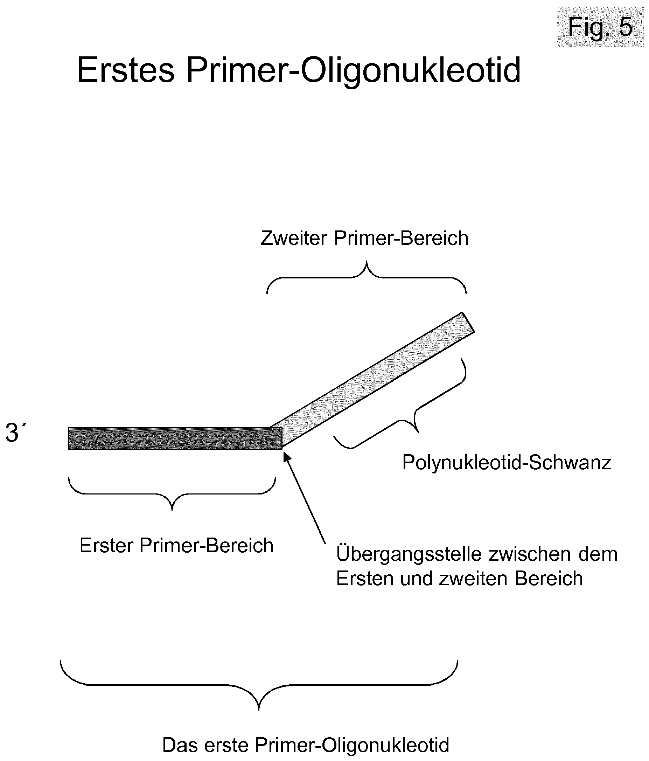

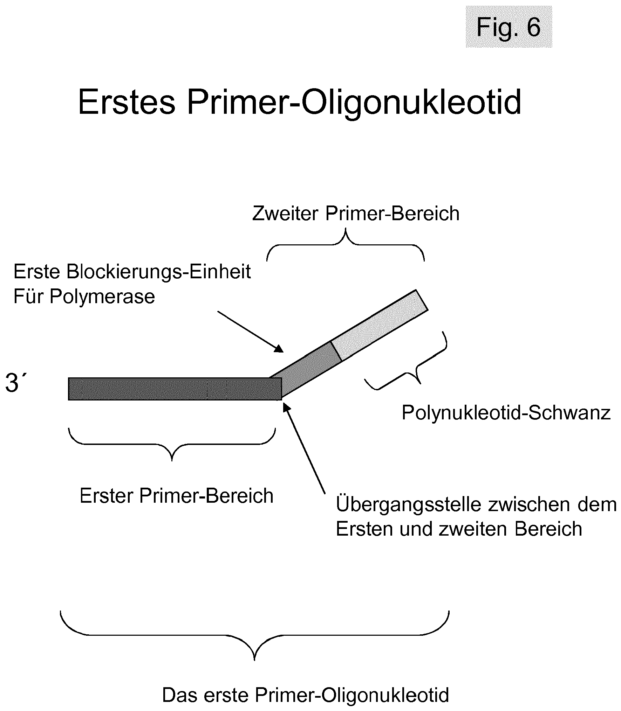

Das erste Primer-Oligonukleotid (Fig. 5 und 6) umfasst einen ersten Primer-Bereich und einen zweiten Bereich. Der erste Primer-Bereich ist in der Lage, an eine im Wesentlichen komplementäre Sequenz innerhalb der zu amplifizierenden Nukleinsäure oder ihrer Äquivalente zu binden und eine Primer-Verlängerungsreaktion zu initiieren. Der zweite Bereich umfasst einen Polynukleotid-Schwanz, welcher in der Lage ist, ein Aktivator-Oligonukleotid zu binden und damit eine räumliche Nähe zwischen dem Aktivator-Oligonukleotid und den anderen Teilen des ersten Primer-Verlängerungsproduktes zu bewirken, welche ausreichend ist, um eine Strangverdrängung durch das Aktivator-Oligonukleotid zu initiieren. Der zweite Bereich des ersten Primer-Oligonukleotids umfasst weiterhin mindestens eine Modifikation (eine Nukleotid-Modifikation oder eine nicht-Nukleotid-Modifikation), welche die Polymerase am Kopieren des Polynukleotid-Schwanzes hindert, indem die Fortführung der Polymerasenabhängigen Synthese gehemmt wird. Diese Modifikation ist beispielsweise am Übergang zwischen dem ersten und dem zweiten Bereich des ersten Primer-Oligonukleotid lokalisiert. Der erste Primer-Bereich des ersten Primer-Oligonukleotides ist folglich durch eine Polymerase kopierbar, so dass eine komplementäre Sequenz zu diesem Bereich während der Synthese des zweiten Primer-Verlängerungsproduktes von der Polymerase erzeugt werden kann. Der Polynukleotid-Schwanz des zweiten Bereichs des ersten Primer-Oligonukleotides wird vorzugsweise durch die Polymerase nicht kopiert. In einer Ausführungsform wird dies durch die Modifikation im zweiten Bereich erreicht, welche die Polymerase vor dem Polynukleotid-Schwanz stoppt. In einer weiteren Ausführungsform wird dies durch Nukleotid-Modifikationen im zweiten Bereich erreicht, wobei der gesamte Polynukleotid-Schwanz im Wesentlichen aus solchen Nukleotid-Modifikationen besteht und somit für Polymerase unkopierbar ist.

In einer Ausführungsform ist ein jedes erstes Primer-Oligonukleotid spezifisch für je eine zu amplifizierende Nukleinsäure.

In einer Ausführungsform ist ein jedes erstes Primer-Oligonukleotid spezifisch für mindestens zwei der zu amplifizierenden Nukleinsäuren, welche jeweils im Wesentlichen unterschiedliche Sequenzen umfassen.

In einer Ausführungsform ist das erste Primer-Oligonukleotid mit einem charakteristischen Marker markiert, z.B. einem Fluoreszenzfarbstoff (z.B. TAMRA, Fluoreszein, Cy3, Cy5) oder einem Affinitätsmarker (z.B. Biotin, Digoxigenin) oder einem zusätzlichen Sequenzfragment, z.B. zur Bindung einer spezifischen Oligonukleotid-Sonde für Detektion oder Immobilisierung oder zur Barcode-Markierung.

### Zweites Primer-Oligonukleotid:

Ein Oligonukleotid, welches mit seinem 3'-Segment in der Lage ist, an eine im Wesentlichen komplementäre Sequenz innerhalb der zu amplifizierenden Nukleinsäure oder ihrer Äquivalente zu binden und eine spezifische zweite Primer-Verlängerungsreaktion zu initiieren. Dieses zweite Primer-Oligonukleotid ist somit in der Lage, an das 3'-Segment eines ersten spezifischen Primer-Verlängerungsproduktes des ersten Primer-Oligonukleotids zu binden und eine Polymerase-abhängige Synthese eines zweiten Primer-Verlängerungsproduktes zu initiieren.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden.

In einer Ausführungsform ist ein jedes zweite Primer-Oligonukleotid spezifisch für je eine zu amplifizierende Nukleinsäure.

In einer Ausführungsform ist ein jedes zweite Primer-Oligonukleotid spezifisch für mindestens zwei der zu amplifizierenden Nukleinsäuren, welche jeweils im Wesentlichen unterschiedliche Sequenzen umfassen.

In einer Ausführungsform ist das zweite Primer-Oligonukleotid mit einem charakteristischen Marker markiert, z.B. einem Fluoreszenzfarbstoff (z.B. TAMRA, Fluoreszein, Cy3, Cy5) oder einem Affinitätsmarker (z.B. Biotin, Digoxigenin) oder einem zusätzlichen Sequenzfragment, z.B. zur Bindung einer spezifischen Oligonukleotid-Sonde für Detektion oder Immobilisierung oder zur Barcode-Markierung.

### Primer-Verlängerungsprodukt:

Ein Primer-Verlängerungsprodukt (auch Primer-Extensionsprodukt genannt) entsteht durch enzymatische (Polymerase-abhängige) Verlängerung eines Primer-Oligonukleotides als Ergebnis einer matrizenabhängigen Synthese, welche durch eine Polymerase katalysiert wird.

Ein Primer-Verlängerungsprodukt umfasst die Sequenz des Primer-Oligonukleotid in seinem 5'-Segment und die Sequenz des Verlängerungsproduktes (auch ExtensionsProdukt genannt), welches durch eine Polymerase in matrizenabhängiger Form synthetisiert wurde. Das durch die Polymerase synthetisierte Verlängerungsprodukt ist komplementär zum Matrizenstrang, an welchem es synthetisiert wurde.

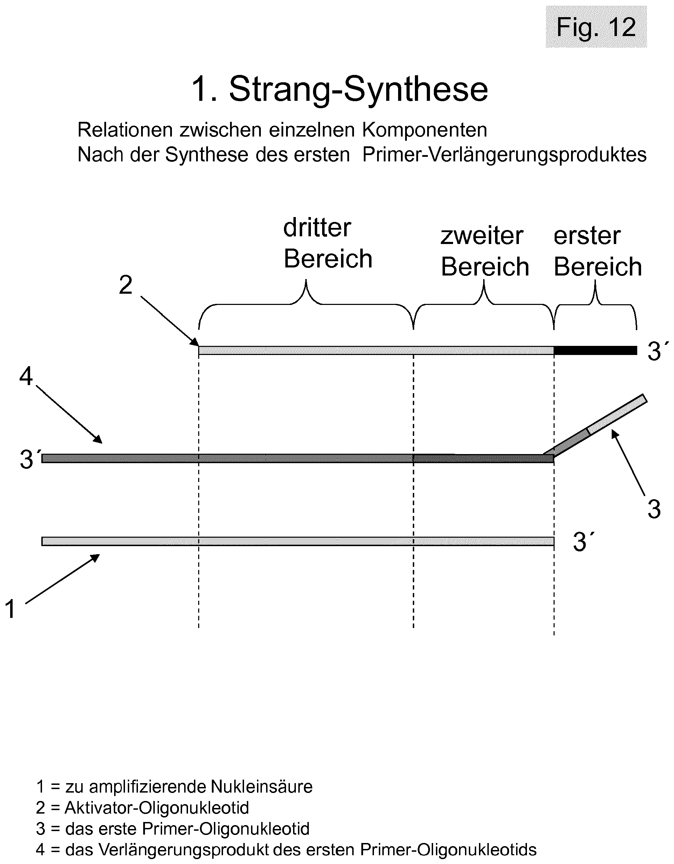

Ein spezifisches Primer-Verlängerungsprodukt (Fig. 12 bis 14) (Hauptprodukt) umfasst Sequenzen der zu amplifizierenden Nukleinsäurekette. Es ist Ergebnis einer spezifischen Synthese bzw. einer korrekten Ausführung einer beabsichtigten Primer-Verlängerungsreaktion bei welcher die spezifisch zu amplifizierende Nukleinsäurekette als Matrize dient. In einer bevorzugten Ausführungsform stimmt die Sequenz der synthetisierten Primer-Verlängerungsprodukte mit der zu erwartenden Sequenz einer zu amplifizierenden Nukleinsäure komplett überein. In einer anderen Ausführungsform können Abweichungen in der erhaltenen Sequenz von der theoretisch zu erwartenden Sequenz toleriert werden. In einer Ausführungsform liegt das Ausmaß der Übereinstimmung der erhaltenen Sequenz infolge einer Amplifikation mit der Sequenz der theoretisch zu erwartenden zu amplifizierenden Nukleinsäure beispielsweise zwischen 90 % und 100 %, bevorzugt liegt die Übereinstimmung bei über 95 %, idealerweise liegt die Übereinstimmung über 98 % (gemessen am Anteil der synthetisierten Basen).

Die Länge des Verlängerungsproduktes eines spezifischen Primer-Verlängerungsproduktes kann zwischen 10 und 300 Nukleotiden, besser zwischen 10 und 180 Nukleotiden liegen, bevorzugt zwischen 20 und 120 Nukleotiden, besonders bevorzugt zwischen 30 und 80 Nukleotiden.

Ein unspezifisches Primer-Verlängerungsprodukt (Nebenprodukt) umfasst beispielsweise Sequenzen, welche als Ergebnis einer unspezifischen bzw. inkorrekten bzw. nicht beabsichtigten Primer-Verlängerungsreaktion entstanden sind. Diese schließen beispielsweise Primer-Verlängerungsprodukte ein, welche als Folge eines falschen Initiierungs-Ereignisses (falsches Primings) oder als Folge anderer Nebenreaktionen, einschließlich polymerase-abhängigen Sequenzveränderungen wie Basen-Substitution, Deletion etc., entstanden sind. Das Ausmaß an Sequenzabweichungen von unspezifischen Primer-Verlängerungsprodukten übersteigt im Allgemeinen die Fähigkeit von Aktivator-Oligonukleotide, solche doppelsträngige Nebenprodukte erfolgreich von ihren Matrizen zu verdrängen, so dass Amplifikation von solchen Nebenprodukten langsamer verläuft oder vollständig ausbleibt. Das Ausmaß der Akzeptanz bzw. die Toleranzgrenze zu Abweichungen sind abhängig beispielsweise von Reaktionstemperatur und Art und Weise der Sequenabweichung. Beispiele für unspezifische Primer-Verlängerungsprodukte bilden Primer-Dimere oder Sequenzvarianten, welche nicht der zu amplifizierenden Nukleinsäure entsprechen, z.B. Sequenzen, welche keine Zielsequenz umfassen.

Die Beurteilung einer hinreichenden Spezifität der Amplifikation hängt häufig mit der Aufgabenstellung zusammen. In vielen Amplifikationsverfahren kann ein gewisses Maß der Unspezifität der Amplifikationsreaktion toleriert werden, solange das gewünschte Ergebnis erreicht werden kann. In einer bevorzugten Ausführungsform beträgt der Anteil von zu amplifizierenden Nukleinsäureketten am Gesamtergebnis der Reaktion mehr als als 1 %, besser mehr als 10 %, noch bevorzugter mehr als 30 % gemessen an Gesamtmenge von neusynthetisierten Stränge.

### Die zu amplifizierende Nukleinsäure

Die zu amplifizierende Nukleinsäure stellt eine Nukleinsäurekette dar, welche sequenzspezifisch oder zumindest vorwiegend sequenzspezifisch mit Hilfe der exponentiellen Amplifikation unter Einsatz von Primern und Aktivator-Oligonukleoid durch die Polymerase amplifiziert werden soll.

Die Länge der zu amplifizierenden Nukleinsäure kann zwischen 20 und 300 Nukleotiden, besser zwischen 30 und 200 Nukleotiden liegen, bevorzugt zwischen 40 und 150 Nukleotiden, besonders bevorzugt zwischen 50 und 100 Nukleotiden.

Die zu amplifizierende Nukleinsäurekette kann eine oder mehrere Zielsequenzen oder ihre Äquivalente umfassen. Weiterhin kann eine zu amplifizierende Nukleinsäure die im Wesentlichen zu einer Zielsequenz komplementären Sequenzen umfassen, welche mit ähnlicher Effizienz wie eine Zielsequenz in einer Amplifikationsreaktion vermehrt werden und Zielsequenz oder ihre Abschnitte umfasst. Zusätzlich zu einer Zielsequenz kann die zu amplifizierende Nukleinsäure weitere Sequenzsegmente einschließen, beispielsweise Primer-Sequenzen, Sequenzen mit Primer-Bindungsstellen und /oder Sequenzsegmente für Bindung von Detektions-Sonden, und / oder Sequenzsegmente für Sequenz-Kodierung von Strängen durch Barcode-Sequenzen und / oder Sequenzsegmente für Bindung an eine feste Phase. Die Primer-Sequenzen oder ihre Sequenzanteile, sowie Primer-Bindungsstellen oder ihre Sequenzanteile können beispielsweise zu Sequenzabschnitten einer Zielsequenz gehören.

In einer Ausführungsform entspricht die zu amplifizierende Nukleinsäure der Zielsequenz.

In einer anderen Ausführungsform bildet die Zielsequenz einen Teil der Sequenz der zu amplifizierenden Nukleinsäurekette. Eine solche Zielsequenz kann von 3'-Seite und / oder von 5'-Seite von weiteren Sequenzen flankiert sein. Diese weiteren Sequenzen können beispielsweise Bindungsstellen für Primer oder ihre Anteile umfassen, und / oder Primer-Sequenzen oder ihre Anteile umfassen, und/ oder Bindungsstellen für Detektions-Sonden umfassen, und / oder Adaptor-Sequenzen für komplementäre Bindung an eine feste Phase (z.B. Im Rahmen von Microarrays, oder Bead-basierten Analysen) umfassen und / oder Barcoding-Sequenzen für eine digitale Signatur von Sequenzen umfassen.

Damit die Amplifikation starten kann, muss zu Beginn der Reaktion eine Nukleinsäurekette in das Reaktionsgemisch gegeben werden, welche als initiale Matrize für die Synthese der zu amplifizierenden Nukleinsäurekette auftritt. Diese Nukleinsäurekette wird als Start-Nukleinsäurekette bezeichnet. Diese Start-Nukleinsäurekette gibt die Anordnung einzelner Sequenz-Elemente vor, welche für die Bildung / Synthese / exponentielle Amplifikation einer zu amplifizierenden Nukleinsäurekette von Bedeutung sind.

In einer bevorzugten Ausführungsform entspricht die initiale Matrize (Start-Nukleinsäurekette), welche einer Amplifikationsreaktion zu Beginn zugeführt wird, bzw. welche in das Reaktionsgemisch gegeben wird, der Sequenz-Zusammensetzung der zu amplifizierenden Nukleinsäurekette.

In anfänglichen Stadien der Amplifikatons-Reaktion und in ihrem weiteren Verlauf binden die jeweiligen Primer an die entsprechenden Bindungsstellen in der Start-Nukleinsäurekette und initiieren die Synthese von spezifischen Primer-Verlängerungsprodukten. Solche spezifischen Primer-Verlängerungsprodukte akkumulieren im Verlauf der Amplifikation in exponentieller Art und Weise und übernehmen zunehmend die Rolle von Matrizen für Synthese komplementärer Primer-Verlängerungsprodukte bei einer exponentiellen Amplifikation.

Durch die wiederholten matrizen-abhängige Synthese-Vorgänge im Rahmen einer exponentiellen Amplifikation wird somit die zu amplifizierende Nukleinsäurekette gebildet. Gegen Ende einer Amplifikationsreaktion kann das Hauptprodukt der Reaktion (die zu amplifizierende Nukleinsäure) vorwiegend einzelsträngig sein oder vorwiegend einen komplementären Doppelstrang bilden. Dies kann beispielsweise durch die relative Konzentrationen von beiden Primern und entsprechende Reaktionebedinungen bestimmt werden.

Äquivalente der zu amplifizierenden Nukleinsäure umfassen Nukleinsäuren mit im Wesentlichen identischen Informationsgehalt. Beispielsweise haben komplementäre Stränge einer zu amplifizierenden Nukleinsäure identischen Informationsgehalt und können als äquivalent bezeichnet werden.

### Zielsequenz

Eine Zielsequenz ist in einer Ausführungsform ein Segment einer zu amplifizierenden Nukleinsäurekette, welches als charakteristische Sequenz der zu amplifizierenden Nukleinsäure dienen kann. Diese Zielsequenz kann als Marker für die Anwesenheit oder Abwesenheit einer anderen Nukleinsäure dienen. Diese andere Nukleinsäure dient somit als Quelle der Zielsequenz und kann beispielsweise eine genomische DNA oder RNA oder Teile der genomischen DNA oder RNA (z.B. mRNA), oder Äquivalente der genomischen DNA oder RNA eines Organismus sein (z.B. cDNA, modifizierte RNA wie rRNA, tRNA, microRNA etc.), oder definierte Veränderungen der genomischen DNA oder RNA eines Organismus umfassen, beispielsweise Mutationen (z.B. Deletionen, Insertionen, Substitutionen, Additionen, Sequenzvermehrung, z.B. Repeat-Vermehrung im Rahmen von Mikrosatellit-Instabilität), Splice-Varianten, Rearrangement-varianten (z.B. T-Zell-Rezeptor Varianten) usw. Die einzelnen Zielsequenzen können für ein phänotypisches Merkmal stehen, beispielsweise für Antibiotika-Resistenz oder prognostische Information haben und somit für diagnostische Assays / Teste vom Interesse sein. Als Quelle / Ursprung einer Zielsequenz kann eine solche Nukleinsäure beispielsweise die Zielsequenz als Sequenz-Element ihres Stranges umfassen. Eine Zielsequenz kann somit als charakteristischer Marker für einen bestimmten Sequenzinhalt einer anderen Nukleinsäure dienen.

Die Zielsequenz kann einzelsträngig oder doppelsträngig sein. Sie kann mit der zu amplifizierenden Nukleinsäure im Wesentlichen identisch sein oder nur einen Teil der zu amplifizierenden Nukleinsäure darstellen.

Äquivalente der Zielsequenz umfassen Nukleinsäuren mit im wesentlichen identischen Informationsgehalt. Beispielsweise haben komplementäre Stränge einer Zielsequenz identischen Informationsgehalt und können als äquivalent bezeichnet werden, RNA und DNA Varianten einer Sequenz sind ebenfalls Beispiele für äquivalenten Informationsgehalt.

Im Rahmen der Material-Vorbereitung vor Amplifikations-Reaktion kann eine solche Zielsequenz aus ihrer ursprünglichen Sequenzumgebung isoliert werden und für die Amplifikationsreaktion vorbereitet werden.

In einer bevorzugten Ausführungsform umfasst eine zu amplifizierende Nukleinsäure eine Zielsequenz. In einer Ausführungsform entspricht die Zielsequenz der zu amplifizierenden Nukleinsäure. In einer weiteren bevorzugten Ausführungsform umfasst eine Start-Nukleinsäurekette eine Zielsequenz. In einer Ausführungsform entspricht die Zielsequenz einer Start-Nukleinsäurekette.

### Start-Nukleinsäurekette

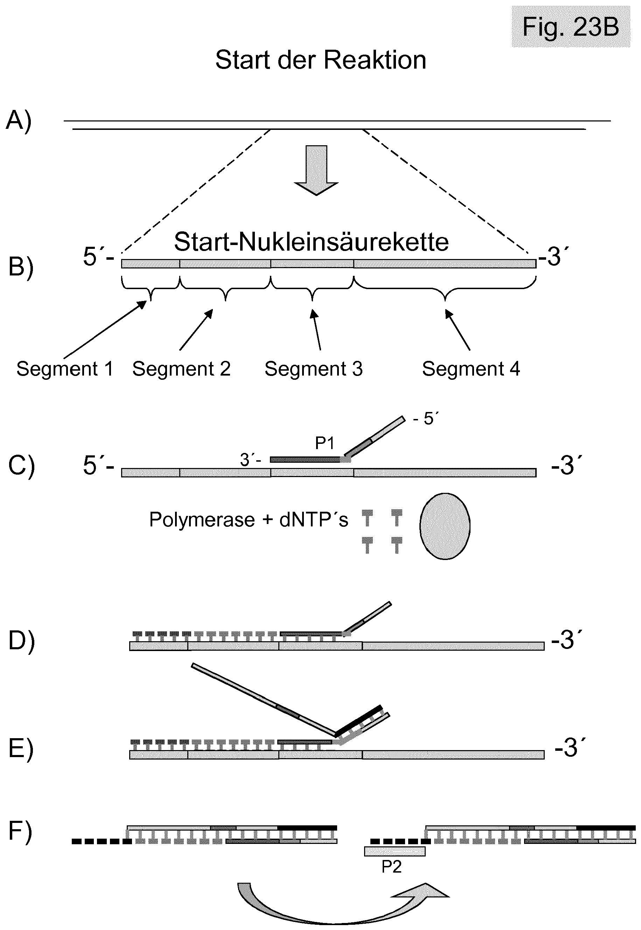

Damit die Amplifikation starten kann, muss zu Beginn der Reaktion eine Nukleinsäurekette in das Reaktionsgemisch gegeben werden, welche als initiale Matrize für die Synthese der zu amplifizierenden Nukleinsäurekette auftritt (Fig. 23B). Diese Nukleinsäurekette wird als Start-Nukleinsäurekette bezeichnet. Diese Start-Nukleinsäurekette gibt die Anordnung einzelner Sequenz-Elemente vor, welche für die Bildung / Synthese / exponentielle Amplifikation einer zu amplifizierenden Nukleinsäurkette von Bedeutung sind.

Eine solche Start-Nukleinsäurekette kann einzelsträngig oder doppelsträngig zu Beginn der Reaktion vorliegen. Wenn die komplementären Stränge der Start-Nukleinsäurekette voneinander getrennt sind, können die Stränge unabhängig davon, ob die Nukleinsäure ursprünglich doppel- oder einzelsträngig vorlag, als Matrize für die Synthese von spezifischen komplementären Primer-Verlängerungsprodukte dienen.

### Aktivator-Oligonukleotid:

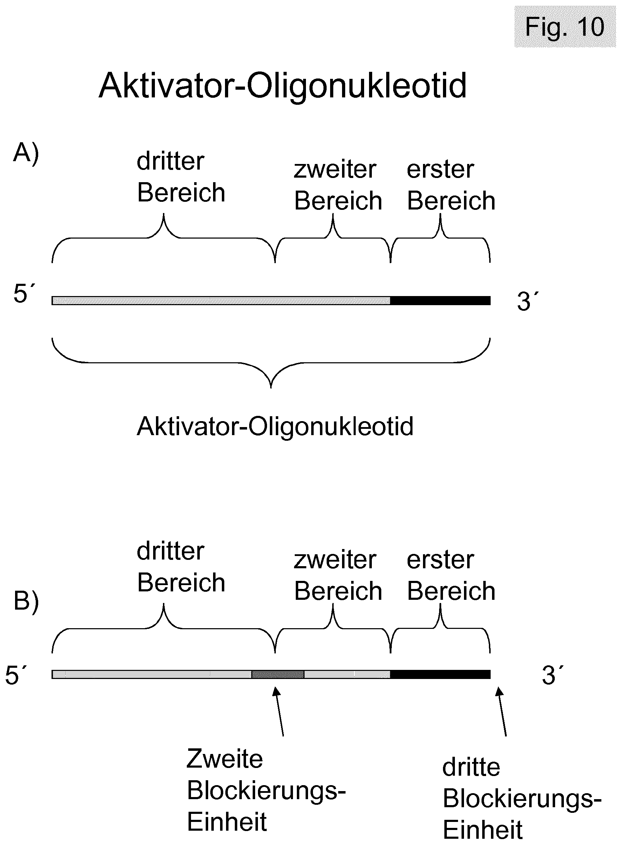

Das Aktivator-Oligonukleotid (Fig. 10) ist eine bevorzugt einzelsträngige Nukleinsäurekette, welche eine vordefinierte im wesentlichen komplementäre Sequenz in einem Teil des ersten Primer-Verlängerungsprodukts einschließt, welches im Rahmen der Amplifikation der zu amplifizierenden Nukleinsäure spezifisch generiert wird. Dadurch kann das Aktivator-Oligonukleotid an das erste Primer-Oligonukleotid und mindestens an das 5'-Segment des spezifischen Verlängerungsprodukts des ersten Primer-Oligonukleotid im Wesentlichen komplementär binden. Das Aktivator-Oligonukleotid umfasst in seinen inneren Sequenzsegment in einer Ausführungsform Nukleotid-Modifikationen, welche die Polymerase daran hindern, einen komplementären Strang unter Verwendung des Aktivator-Oligonukleotides als Matrize zu synthetisieren, wenn das erste Primer-Oligonukleotid an das Aktivator-Oligonukleotid komplementär gebunden ist. Das Aktivator-Oligonukleotid ist weiterhin in der Lage, unter den gewählten Reaktionsbedingungen das zweite spezifische Primer-Verlängerungsprodukt vollständig oder teilweise aus der Bindung mit dem ersten spezifischen Primer-Verlängerungsprodukt via Strangverdrängung zu verdrängen. Dabei lagert sich das Aktivator-Oligonukleotid mit seinen komplementären Bereichen an das erste spezifische Primer-Verlängerungsprodukt an. Bei erfolgreicher Bindung zwischen dem Aktivator-Oligonukleotid und dem ersten spezifischen Primer-Verlängerungsprodukt führt dies zu Wiederherstellung eines einzelsträngigen Zustandes des 3'-ständigen Segments des zweiten spezifischen Primer-Verlängerungsproduktes, welches für die Bindung des ersten Primer-Oligonukleotides geeignet ist, so dass eine neue Primer-Verlängerungsreaktion stattfinden kann. Während der Synthese des zweiten Primer-Verlängerungsproduktes kann das Aktivator-Oligonukleotid aus der Bindung mit dem ersten Primer-Verlängerungsprodukt mittels Strangverdrängung beispielsweise durch Polymerase und/oder durch das zweite Primer-Oligonukleotid getrennt werden.

### Strangverdrängung (Strand Displacement):

Hiermit wird ein Vorgang bezeichnet, welcher durch Einwirkung eines geeigneten Mittels zu einer vollständigen oder partiellen Trennung eines ersten Doppelstranges (bestehend beispielsweise aus A1 und B1-Strang) führt, und zur gleichzeitigen / parallelen Ausbildung eines neuen zweiten Doppelstranges, wobei mindestens einer der Stränge (A1 oder B1) an der Ausbildung dieses neuen zweiten Stranges beteiligt sind. Man kann hier zwei Formen der Strangverdrängung unterschieden.

In einer ersten Form der Strangverdrängung kann die Ausbildung eines neuen zweiten Doppelstranges unter Verwendung eines bereits existierenden komplementären Stranges erfolgen, welcher zu Beginn der Reaktion im Allgemeinen in einzelsträngiger Form vorliegt. Dabei wirkt das Mittel der Strangverdrängung (beispielsweise ein vorgebildeter einzelsträngiger Strang C1, welcher eine komplementäre Sequenz zum Strang A1 aufweist, auf den ersten bereits ausgebildeten Doppelstrang (A1 und B1) und geht mit dem Strang A1 eine komplementäre Bindung ein, wodurch der Strang B1 aus der Bindung mit dem Strang A1 verdrängt wird. Falls die Verdrängung des B1 vollständig abläuft, so ist das Ergebnis der C1-Wirkung ein neuer Doppelstrang (A1:C1) und ein einzelsträngiger Strang B1. Falls die Verdrängung von B1 unvollständig abläuft, so hängt das Ergebnis von mehreren Faktoren ab. Beispielsweise kann ein Komplex aus partiell doppelsträngigen A1:B1 und A1:C1 als Zwischenprodukt vorliegen.

In einer zweiten Form der Strangverdrängung kann die Ausbildung eines neuen zweiten Doppelstranges unter einer gleichzeitig ablaufenden enzymatischen Synthese des komplementären Stranges erfolgen, wobei ein Strang des ersten vorgebildeten Doppelstranges als Matrize für die Synthese durch die Polymerase auftritt. Dabei wirkt das Mittel der Strangverdrängung (beispielsweise Polymerase mit einer Strangverdrängungs-Fähigkeit), auf den ersten bereits vorgebildeten Doppelstrang (A1 und B1) und synthetisiert einen zu Strang A1 neuen komplementären Strang D1, wobei gleichzeitig der Strang B1 aus der Bindung mit dem Strang A1 verdrängt wird.

In der wissenschaftlichen Literatur sind viele Beispiele aus der Natur und Molekular-Biologie / BioNanotechnologie beschrieben, welche den Vorgang der Strangverdrängung einschließen. Es wurden Beispiele für sowohl protein-vermittelte bzw. enzym-vermittelte Strangverdrängung als auch Bespiele für Nukleinsäureketten-vermittelte Strangverdrängung beschrieben. Ebenfalls wurden Beispiele für Strangverdrängungsvorgänge mit bereits vorgebildeten einzelsträngigen Nukleinsäuren beschrieben als auch Strangverdrängungsvorgänge, bei welchen ein neuer Strang während der Strangverdrängung erst neu synthetisiert wird.

Bei dem protein- bzw. enzym-vermittelten Strangverdrängungs-Vorgang (z.B. durch Polymerase oder Helicase vermittelt) erfolgt die Trennung einer Duplex unter Mithilfe eines Proteins oder Enzyms. Strangverdrängung mit Hilfe einer DNA-Polymerase findet beispielsweise unter gleichzeitiger Synthese eines komplementären Stranges während Strand-Displacement Amplification (SDA) statt (US Pat No.: 5,455,166 und Walker et al. PNAS 89, 1992, 392-396). Die verwendete Polymerase muss die Nukleinsäure Polymerisation bewirken und den stromabwärts gelegenen Strang verdrängen, jedoch nicht durch die eine 5'→3' Exonuklease Aktivität abbauen. Derartige Eigenschaften haben beispielsweise das Klenow-Fragment der E.coli Polymerase I, Bsu-Polymerase oder das große Fragment von Bacillus stearothermophilus (Bst Polymerase und ihre Modifikationen), sowie Vent Polymerase, DeepVent-Polymerase und andere Polymerase mit Fähigkeit zu Strangverdrängung.

Ein anderes Beispiel für Polymerase-assoziierte Strangverdrängung ist die Amplifikations-Methode LAMP (Loop mediated isothermal Amplification). Ein anderes Beispiel für protein-vermittelte bzw. enzym-vermittelte Strangverdrängung stellt die Recombinase-Reaktion dar, z.B. mit RecA. Hier führt eine Interaktion zwischen einem Protein-Nukleinsäurestrang-Komplex und einem Doppelstrang zu einer partiellen Strang-Verdrängung des Doppelstranges und Dissoziation des Protein-Nukleinsäurestrang-Komplexes. Eine Recombinase-Polymerase Amplification (RPA) macht Gebrauch von diesem Vorgang und ist beschrieben in Patentanmeldung WO 2008035205. Ein protein- bzw. enzym-vermittelter Strangverdrängungs-Vorgang geht häufig mit einem Energie-Verbrauch einher, z.B. in Form von ATP bei RPA.

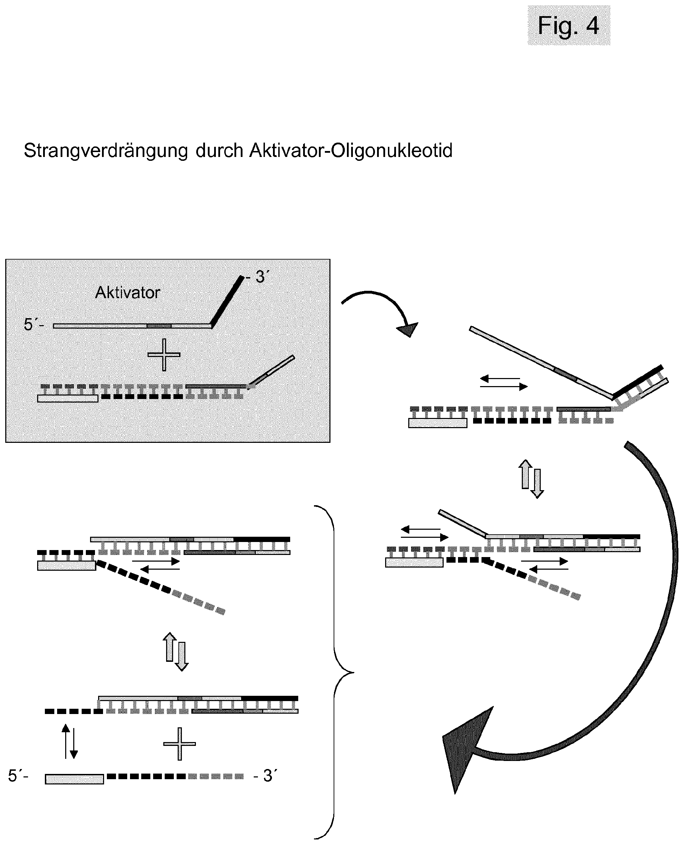

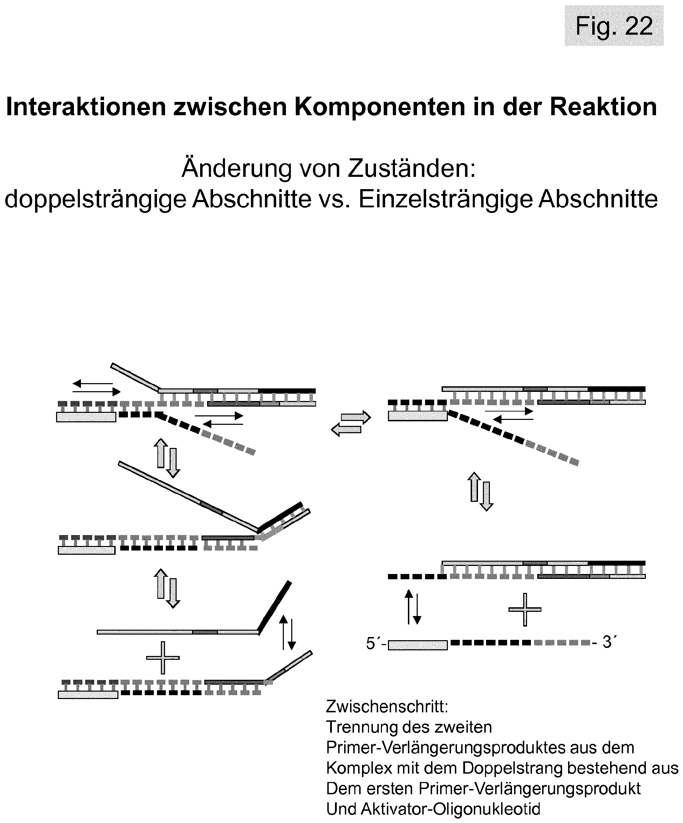

Bei einer nukleinsäurevermittelten Strangverdrängung (Fig. 4 und 22) erfolgt ein Austausch von identischen oder ähnlichen Strängen unter gleichzeitiger / parallelen Ausbildung neuer Doppelstränge. In der Literatur wird dieser Vorgang synonym als Strangersatzreaktion (engl. Strand-Replacement) bezeichnet. Dieses Phänomen ist involviert / maßgeblich beteiligt in Zweigmigration (engl. Branch-Migration) von homologen Duplexen, Überwindung von intramolekularen Haarnadel-Strukturen mittels einzelsträngiger Oligonukleotide, DNA-Klonierung, DNA-basierte "Toehold"-vermittelte Kaskadenreaktionen und SNP-Analysen. Die Natur des Phänomens der nukleinsäurebasierten bzw. nukleinsäurevermittelten Strangverdrängung wurde seit über 40 Jahren ausführlich studiert und ist in vielen Artikel abgebildet ("The Kinestics of Oligonukleotide Replacement", Reynaldo et al. J.Mol.Biol. 2000, 297, 511-; "Formation of a single base mismatch impedes spontaneous DNA branch migration" Panyutin et al. J.Mol.Biol. 1993, 230, 413-; "The kinetics of spontaneous DNA branch migration" Panyutin et al. PNAS 1994, 91,2021-; "Programmable energy landscapes for Kinetic control of DNA strand displacement" Machinek et al. Nature Commun 2014, 5, 5324-; "Remote Toehold: A mechanism for flexiblecontrol of DNA hybridization Kinetics" Genot et al. JACS, 2011, 133, 2177-; "A new class of homogeneous nucleic acid probes based on specific displacement hybridization" Li et al. NAR, 20102, v30, No.2 e5; "On the biophysics and kinetics of toehold-mediated DNA strand displacement", Srinivas et al. NAR 2013, 10641-58; "Modelling toehold-mediated RNA strand displacement" Sulc et al. Biophysical Journal, 2015, 1238-; "Branch Migration through DNA sequence heterology" Biswas et al. J.Mol.Biol. 1998, 279, 795-; "Toehold-mediated nonenzymatic DNA strand displacement as a platform for DNA genotyping" Khodakov et al. JACS 2013, 135, 5612-; "Protected DNA Strand Displacement for enhanced single nucleotide discrimination in double stranded DNA" Khodakov et al. Nature, Scientific Reports, 2015, 5, 8721-; "Allele-Specific Holliday Junction Formation: a new mechanism of allelic discrimination for SNP Scoring" Yang et al. Genome Research 2003, 1754-; "Conditionally fluorescent molecular probes for detecting single base changes in double-stranded DNA" Chen et al. Nature Chemistry, 2013, 782-; "Optimizing the specificity of nucleic acid hybridization" Zhang et al. Nature Chemistry, 2012, 208-). In diesen und anderen Quellen des Standes der Technik sind strukturelle Voraussetzungen von beteiligten Nukleinsäuresträngen für einen erfolgreichen Strangverdrängungs-Vorgang, die Initiierung der Strangverdrängung, die Kinetik der Strangverdrängung, Reaktionsbedingungen sowie der Einfluss von Sequenzunterschieden in einzelnen Abschnitten der beteiligten Strukturen auf Strangverdrängung detailliert studiert und beschrieben worden. Dabei kommt der Sequenzabhängigkeit des Strangverdrängungsvorgangs eine besonders große Rolle zu. Dank der Möglichkeit, vordefinierte, spezifische Nukleinsäureketten für eine nukleinsäurevermittelte Strangverdrängung zu verwenden, ist das Ergebnis dieses Vorgangs ebenfalls sequenzspezifisch. Diese Sequenzabhängigkeit führte in Vergangenheit zum Einsatz beim Nachweis von Unterschieden in Nukleinsäureketten, unter Verwendung von Branch-Migration (dieser Vorgang ist verwandt mit Strangverdrängung und ist in Patentanmeldung beschrieben: Lishanski et al. US Pat No: 6,232,104. Verwendung von modifizierten Nukleinsäuresträngen beim Einsatz in Branch-Migration ist beschrieben in Wetmur et al US Pat No: 5,958,681.)

Nukleinsäurevermittelte Strangverdrängung wird in der Literatur auch als ein Beispiel für kompetitor-induzierte Dissoziation von molekularen Komplexen angesehen, siehe "A general mechanism for competitor-induced dissociation of molecular complexes", Paramanathan et al. Natur Commun, 2014, 5207-).

Einem Fachmann sind mehrere Formen des nukleinsäurevermittelten StrangverdrängungsVorgangs bekannt, z.B. "Toehold-Mediated" Strand Displacement oder "3-Branch-Migration".

Unter dem Begriff "nukleinsäurevermittelte Strang-Verdrängung" wird eine Summe / Reihe von Zwischenschritten zusammengefasst, welche untereinander im Gleichgewicht stehen können, und im Ergebnis zur vorübergehenden oder dauerhaften Öffnung einer ersten vorgeformten Duplex (bestehend aus komplementären Strängen A1 und B1) und Ausbildung einer neuen zweiten Duplex (bestehend aus komplementären Strängen A1 und C1), wobei A1 und C1 einander komplementär sind. Dieser Vorgang ist im Beispiel 1 veranschaulicht (s.u.).

Es ist bekannt, dass eine wesentliche strukturelle Voraussetzung für die Initiierung einer Strangverdrängung die Herbeiführung einer räumlichen Nähe zwischen einem Duplex-Ende (vorgeformter erster Duplex aus A1 und B1) und einem einzelsträngigen Strang (C1) ist, welcher die Strangverdrängung initiiert (wobei A1 und C1 einen komplementären Strang bilden können). Eine solche räumliche Nähe kann vorzugsweise mittels eines einzelsträngigen Überhanges (in der Literatur sind Beispiele mit kurzen Überhängen bekannt, im Englischen genannt "Toehold", siehe Literatur oben) herbeigeführt werden, welcher den einzelsträngigen Strang (C1) vorübergehend oder dauerhaft komplementär bindet, und somit komplementäre Seqmente des Stranges C1 und A1 in hinreichende Nähe bringt, so dass eine erfolgreiche Strangverdrängung des Stranges B1 initiiert werden kann. Die Effizienz der Initiierung der nukleinsäurevermittelten Strang-Verdrängung ist im Allgemeinen um so höher, je näher die komplementäre Segmente des Stranges A1 und C1 zueinander positioniert werden.

Eine weitere wesentliche strukturelle Voraussetzung für die effiziente Fortführung einer nukleinsäurevermittelten Strangverdrängung in inneren Segmenten liegt in hoher Komplementarität zwischen Strängen (z.B. zwischen A1 und C1), welche einen neuen Doppelstrang ausbilden müssen. So können beispielsweise einzelne Nukleiotid-Mutationen (in C1) zur Unterbrechung einer Strangverdrängung (beschrieben z.B. für Branch-Migration) führen.

Die vorliegende Erfindung macht Gebrauch von der Fähigkeit komplementärer Nukleinsäuren zu einer sequenzabhängigen nukleinsäurevermittelten Strangverdrängung.

Bevorzugte Ausführungsformen der Erfindung werden in den Figuren und Beispielen näher erläutert.
Figur 1 zeigt die Problematik bei der Nukleinsäureamplifikation durch PCR. Theoretisch sollte nur die gewünschte Zielsequenz amplifiziert werden. Da die Primer aber auch an anderen, nicht relevanten Teilen der in der Reaktionsmischung vorhandenen Nukleinsäure binden können, entstehen aufgrund von (unerwünschten) Nebenreaktionen Nebenprodukte, die das Ergebnis stören können.

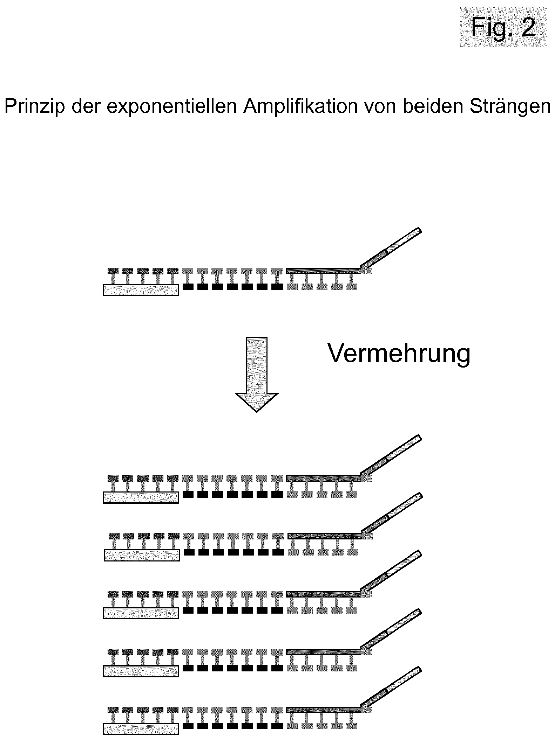

Figur 2 zeigt die Amplifikation einer zu amplifizierenden Nukleinsäure unter Einsatz des ersten Primer-Oligonukleotids und des zweiten Primer-Oligonukleotids.

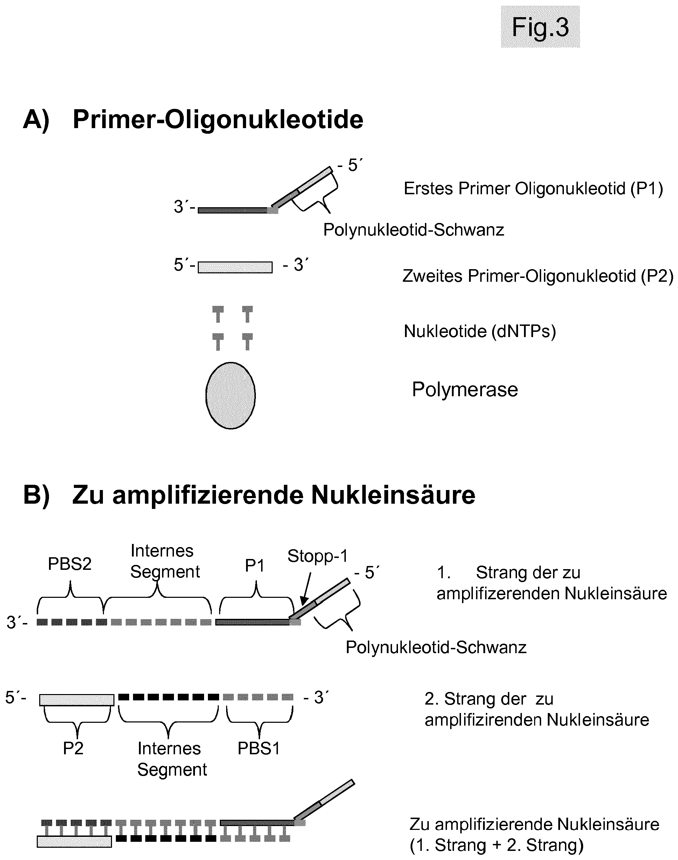

Figur 3 beschreibt Komponenten der in Fig. 2 dargestellten Strukturen
Figur 4 zeigt schematisch den Strangverdrängungsmechanismus
Figur 5 - 6 zeigt schematisch die Struktur des ersten Primer-Oligonukleotids.

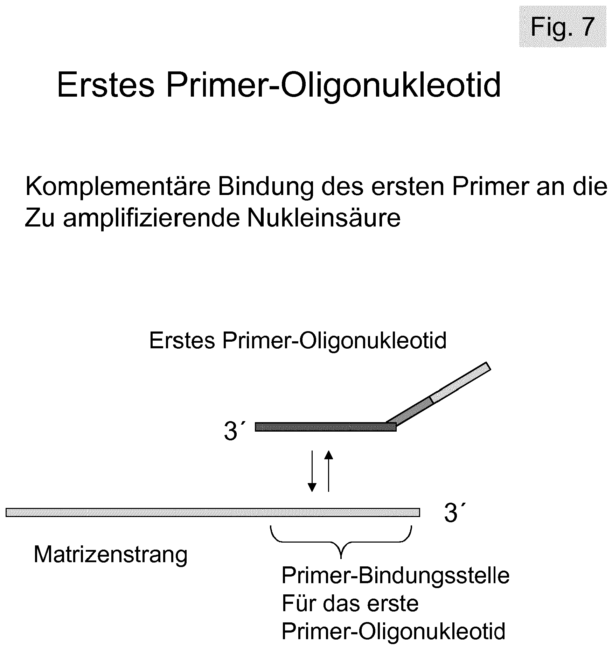

Figur 7 - 8 zeigt schematisch die Interaktion zwischen dem ersten Primer-Oligonukleotid und der Matrize, sowie die Synthese des ersten Primer-Verlängerungsprodukts.
Figur 9 zeigt schematisch die Struktur des Primer-Verlängerungsprodukts des ersten Primer-Oligonukleotids.
Figur 10 zeigt schematisch die Struktur des Aktivator-Oligonukleotids.
Figur 11 - 12 zeigt schematisch die Interaktion zwischen Strukturen während der Primer-Verlängung des ersten Primer-Oligonukleotids.
Figur 13 - 14 zeigt schematisch die Interaktion zwischen Strukturen während der Primer-Verlängerung des zweiten Primer-Oligonukleotids.
Figur 15 - 21 zeigt schematisch die Interaktion zwischen einzelnen Bereichen von Komponenten während der Amplifikation.
Figur 22 zeigt schematisch das Zusammenwirken der Strukturen bei Strangverdrängung.
Figur 23A zeigt schematisch das Zusammenwirken der Strukturen während der Nukleinsäureamplifikation durch einerseits die Amplifikation von erstem Primer-Oligonukleotid und zweitem Primer-Oligonukleotid und weiterhin die Einwirkung des Aktivator-Oligonukleotids und dabei resultierende Strangverdrängung.
Figur 23B - 23D zeigt schematisch einige Ausführungsformen von Strukturen einer Start-Nukleinsäurekette und ihre Verwendung als Matrize zu Beginn der Reaktion.
Fig. 24 - 29 zeigt die Ergebnisse der Strangverdrängung im Beispiel 1.
Figur 30 zeigt die Entwicklung der Signalintensität im Verlauf einer exponentiellen Amplifikation wie in Beispiel 2 beschrieben.
Figur 31 zeigt den Verlauf einer Kapillarelektrophorese nach Ablauf einer exponentiellen Amplifikation wie in Beispiel 2 beschrieben.
Figur 32 zeigt die Entwicklung einer Signalintensität im Verlauf einer erfindungsgemäßen Amplifikation, wie in Beispiel 3 beschrieben.
Figur 33 zeigt die Entwicklung einer Signalintensität im Verlauf einer erfindungsgemäßen Amplifikation, wie in Beispiel 4 beschrieben.

Die mit →1 gekennzeichnete Reaktion entspricht dem Einsatz einer Matrize mit Perfect Match (SEQ ID NO:26) in einer Konzentration von 10 pmol/l. →2 zeigt die Reaktion mit einer Mismatch-Matrize (SEQ ID NO:27) in einer Menge von 10 pmol/l und die mit →3 gekennzeichnete Reaktion stellt die Negativkontrolle dar.

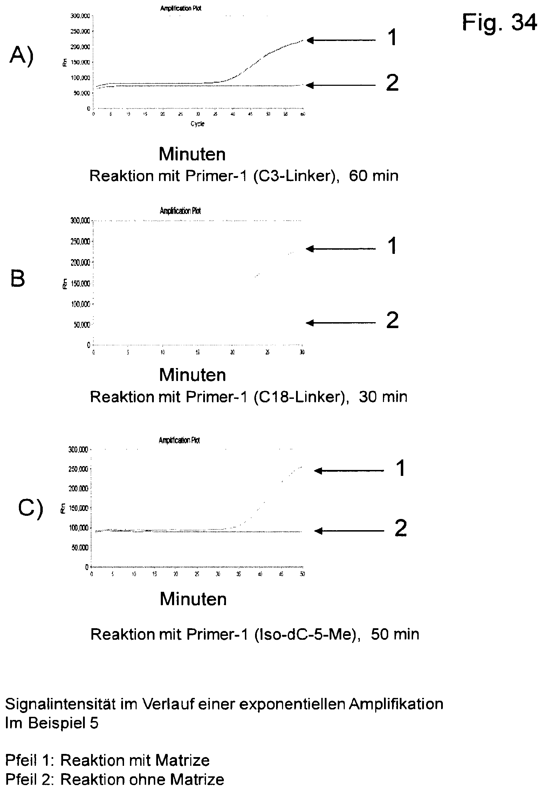

Figur 34 zeigt die Entwicklung einer Signalintensität im Verlauf einer erfindungsgemäßen Amplifikation, wie in Beispiel 5 beschrieben.

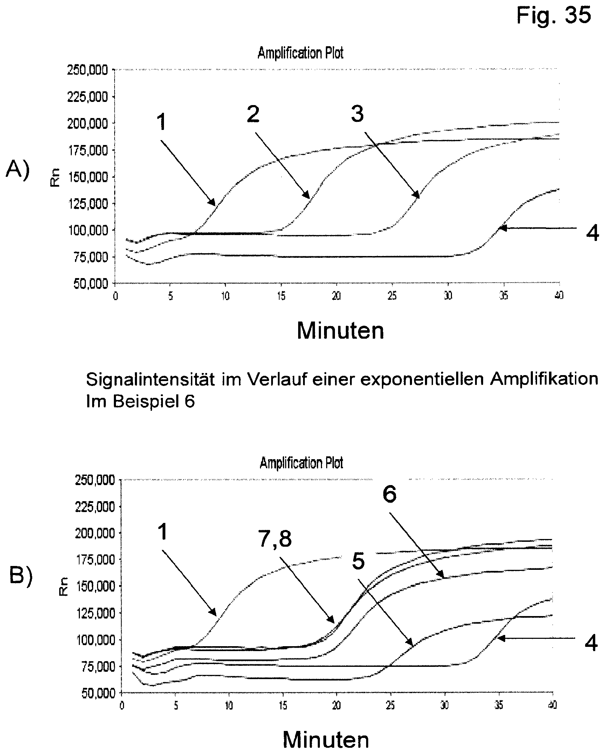

Figur 35 zeigt die Entwicklung einer Signalintensität im Verlauf einer erfindungsgemäßen Amplifikation, wie in Beispiel 6 beschrieben.

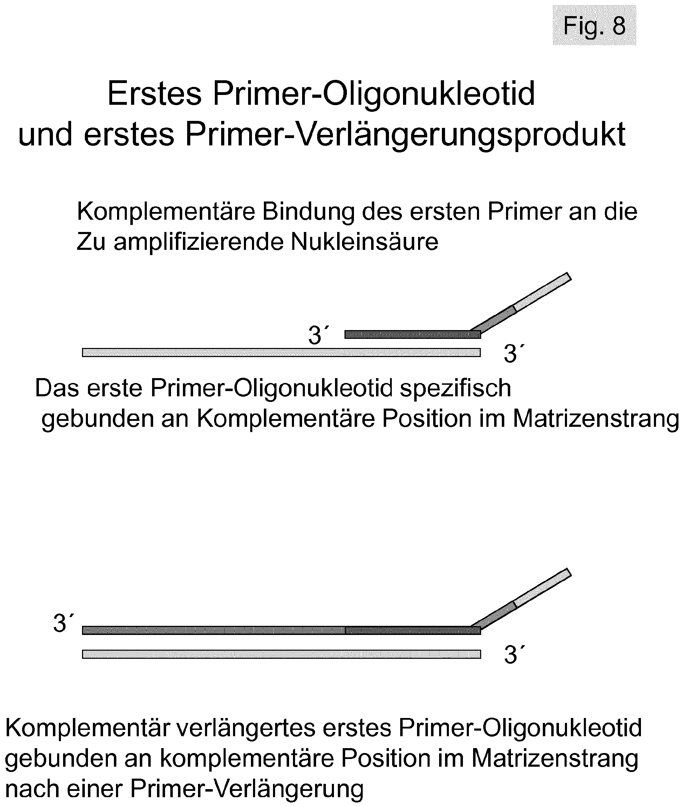

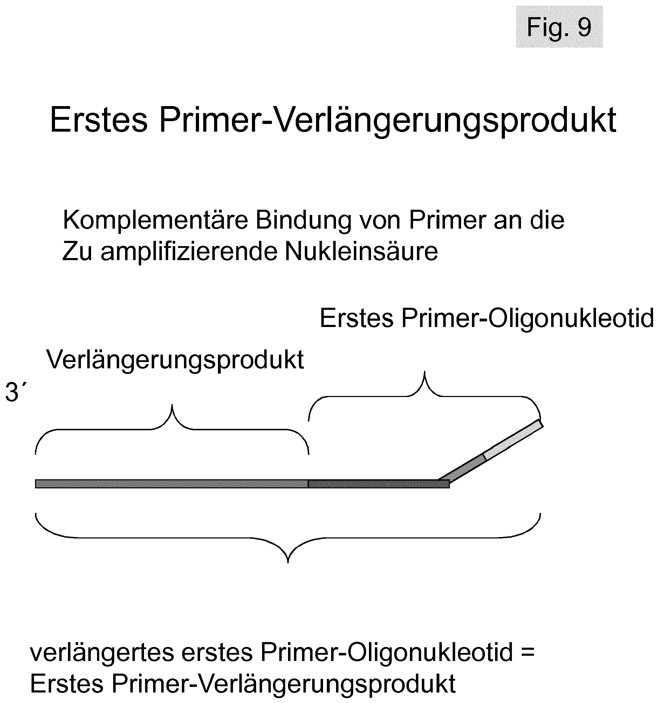

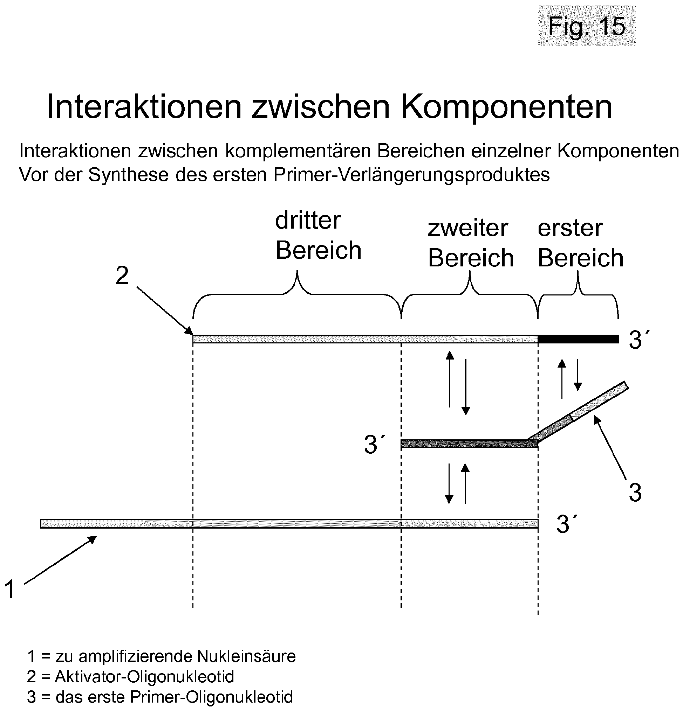

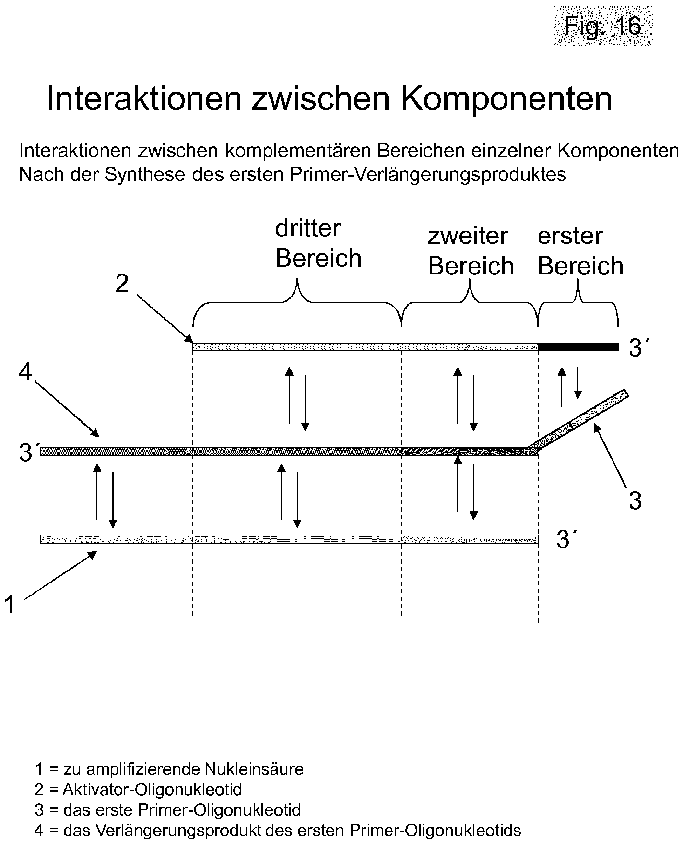

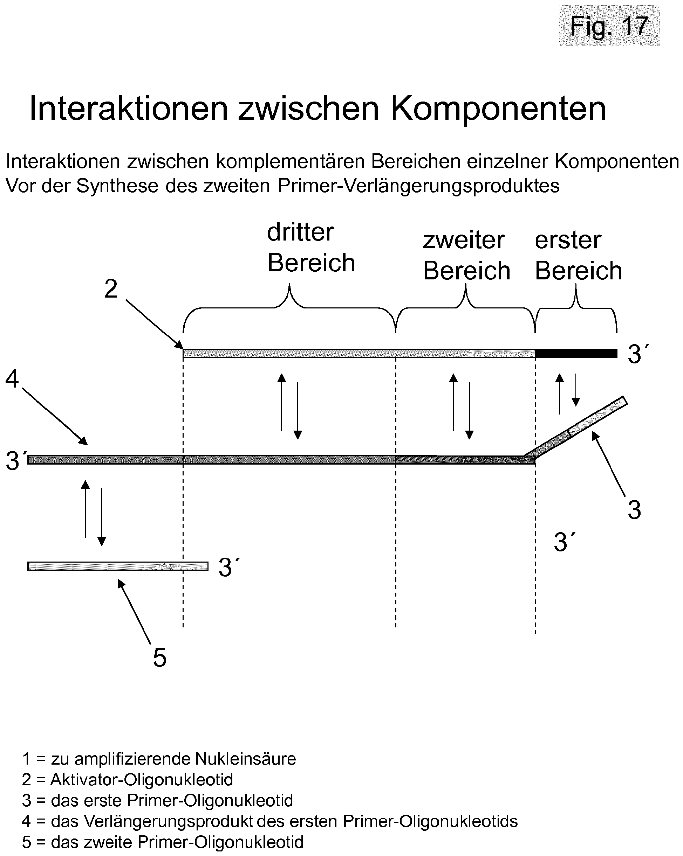

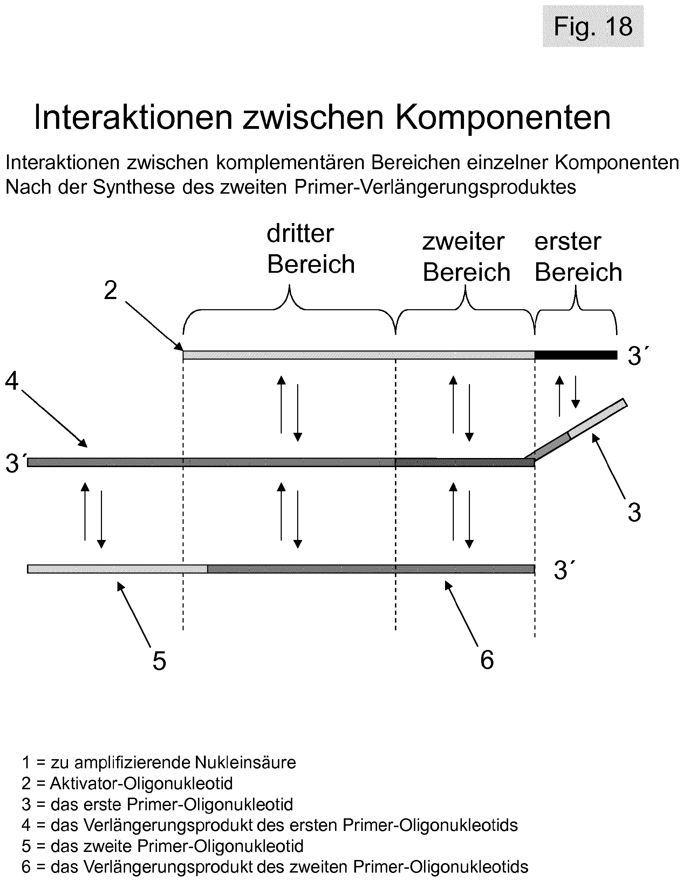

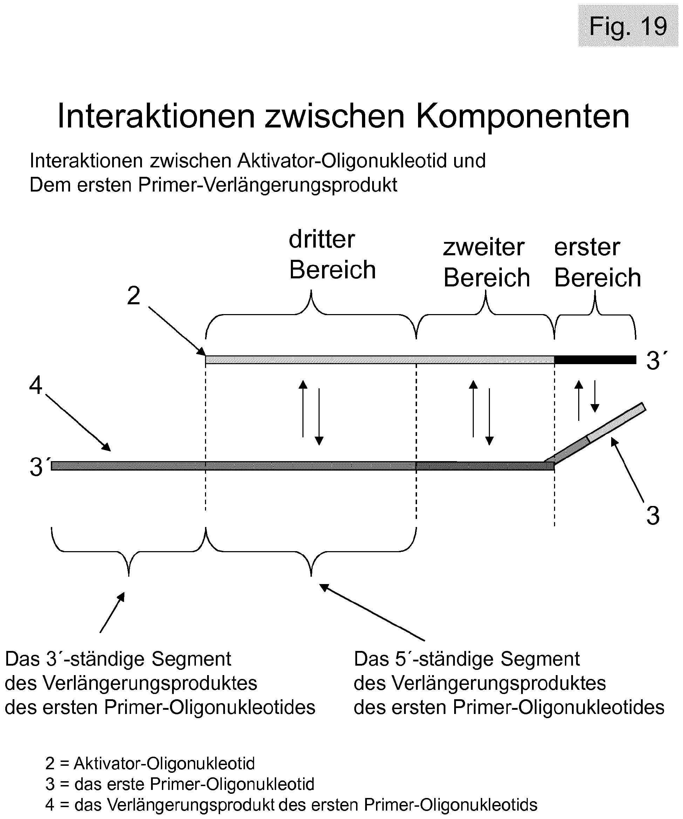

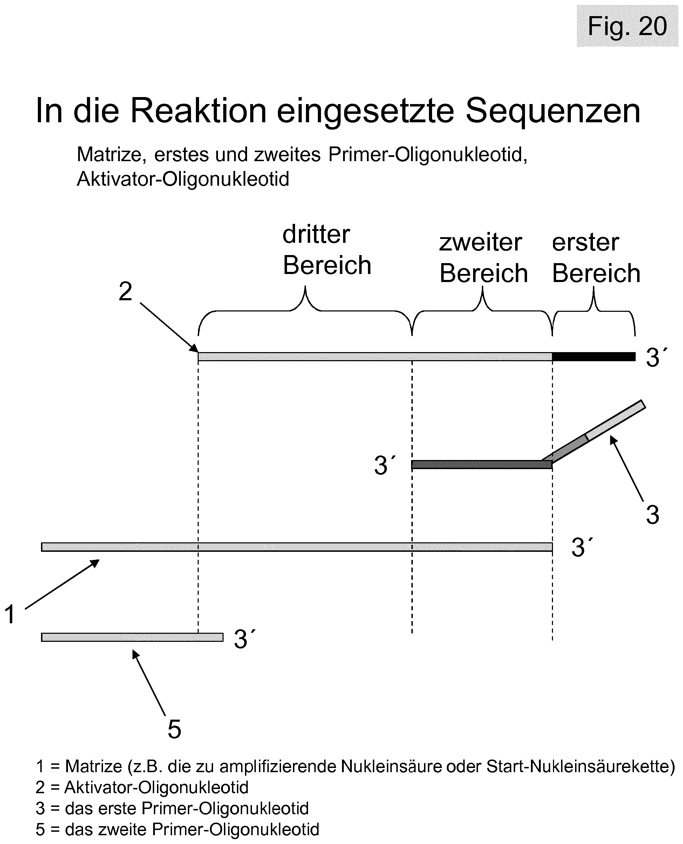

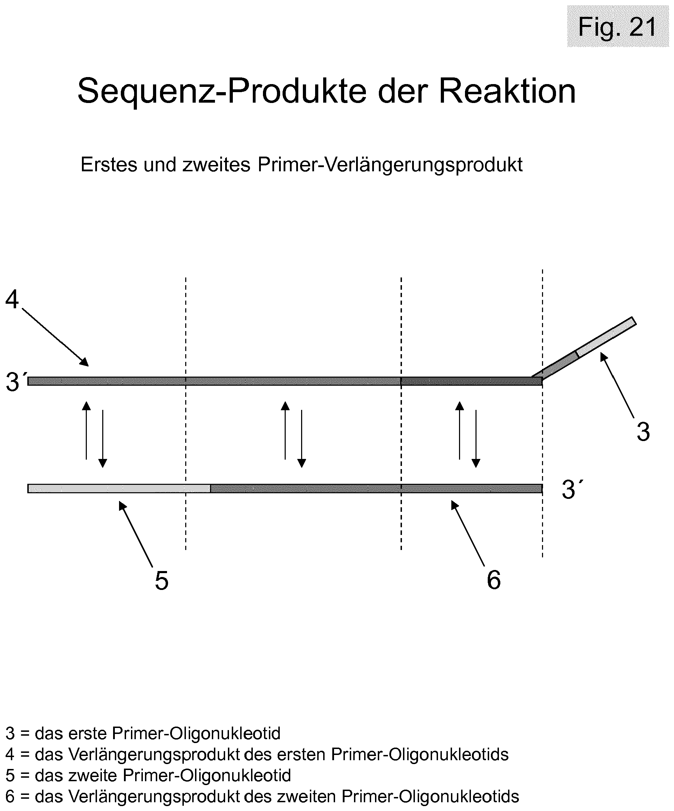

In dem erfindungsgemäßen Verfahren (Fig. 8 bis 21) kann als Ausgangsmaterial eine einzelsträngige Nukleinsäurekette dienen oder eine doppelsträngige Nukleinsäure, welche in einzelsträngige Form überführt wurde. Die Amplifikation erfolgt bevorzugt sequenzspezifisch, d.h. vorzugsweise wird die zu amplifizierende Nukleinsäure vervielfältigt.

Als Komponenten des Amplifikationssystems dienen eine zu amplifizierende Nukleinsäurekette, ein erstes spezifisches Primer-Oligonukleotid, ein zweiter Primer und ein Aktivator-Oligonukleotid, welches bei der Trennung der neu synthetisierten Stränge mitwirkt, sowie eine geeignete Polymerase und Substrate wie dNTP's. Die Amplifikation verläuft in einer Puffer-Lösung unter Bedingungen, welche eine Primer-Verlängerungsreaktion von beiden Primern zulassen, sowie welche eine Strangverdrängung durch Aktivator-Oligonukleotid zur Trennung von beiden Primer-Verlängerungsprodukten unterstützen.

In einer Ausführungsform sollen alle Verfahrensschritte unter Bedingungen verlaufen, welche keine Trennung von synthetisierten Primer-Verlängerungsproduken in Abwesenheit eines geeigneten Aktivator-Oligonukleotides zulassen. Beispielsweise ist die Temperatur der Reaktionslösung dermaßen gewählt, dass die Tm eines Doppelstranges aus beiden Primer-Verlängerungsprodukten signifikant über der Reaktionstemperatur liegt.

Unter diesen Voraussetzungen erfolgt eine Trennung von beiden Primer-Verlängerungsprodukten in Abhängigkeit von der Wirkung des Aktivator-Oligonukleotides. Dieses Aktivator-Oligonukleotid ist in der Lage, komplementär an das erste Primer-Verlängerungsprodukt zu binden und dadurch das zweite Primer-Verlängerungprodukt aus seiner Bindung mit dem ersten Primerverlängerungsprodukt zu verdrängen. Um diese Strangverdrängungsreaktion zu initiieren, ist das erste Primer-Oligonukleotid mit einem Polynukleotid-Schwanz in seinem zweiten Bereich versehen, welcher in der Lage ist, das Aktivator-Oligonukleotid unter Reaktionsbedingungen vorübergehend zu binden und damit eine räumliche Nähe zu anderen Bereichen des ersten Primer-Verlängerungsnukleotides herbeizuführen. Nach der Initiierung der Strangverdrängung durch das Aktivator-Oligonukleotid wird das zweite Primer-Verlängerungsprodukt aus seiner Bindung mit dem ersten Primer-Verlängerungsprodukt verdrängt. Sein 3'-ständiges Segment wird damit frei und steht für weitere Bindung eines ersten Primer-Oligonukleotides zur Verfügung.

Der Polynukleotid-Schwanz des ersten Primer-Oligonukleotides ist vorzugsweise nicht von einer Polymerase kopierbar. Das kann entweder durch Verwendung entsprechender Modifikationen in diesem Bereich erreicht werden oder durch Einführung einer ersten Blockierungseinheit zwischen dem ersten Primer-Bereich und dem zweiten Primer-Bereich des ersten Primer-Oligonukleotides.

Die Synthese des zweiten Primer-Verlängerungsproduktes erfolgt nach der Bindung des zweiten Primer-Oligonukleotides an das erste Primer-Verlängerungsprodukt in seinem 3'-ständigen Segment. Dieses Segment geht vorzugsweise keine Bindung mit dem Aktivator-Oligonukleotid ein und ist hinreichend lang, um das zweite Primer-Oligonukleotid zu binden und eine erfolgreiche Primer-Verlängerungsreaktion zu unterstützen. Die Synthese des zweiten Primer-Verlängerungsproduktes erfolgt unter Verdrängung des Aktivator-Oligonukleotides aus der Bindung mit dem ersten Primer-Verlängerungsprodukt. Dies kann beispielsweise durch Polymerase-abhängige Strangverdrängung oder auch durch Strangverdrängung mittels des zweiten Primers erfolgen.

Beide Primer-Verlängerungsprodukte schließen kopierbare Bereiche ein und dienen gegenseitig als Matrize. Das Aktivator-Oligonukleotid dient nicht als Matrize. Dies kann bevorzugt durch Verwendung von Nukleotid-Modifikationen erreicht werden, welche zwar eine komplementäre Bindung mit dem ersten Primer-Verlängerungsprodukt eingehen können, nicht aber von der Polymerase als Matrize akzeptiert werden. Beispiele für solche Nukleotid-Modifikationen stellen Nukleotid-Verbindungen mit modifizierten Phosphat-Zucker-Rückgrad-Anteilen, z.B. 2'-O-Alkyl-RNA Modifikationen (z.B. 2'-OMe), LNA-Modifikationen oder Morpholino-Modifkationen dar. Im Allgemeinen hindert die Anwesenheit solcher Modifikationen in einem Strang eine DNA-Abhängige Polymerase beim Ablesen eines solchen Stranges. Die Anzahl von solchen Modifikationen kann unterschiedlich groß sein, in der Regel können wenige Modifikationen (zwischen 1 und 20) hinreichend sein, um eine Polymerase beim Ablesen eines solchen Stranges zu hindern. Solche Nukleotid-Modifikationen können beispielsweise an oder um die Bindungsstelle des ersten Primer-Oligonukleotides an das Aktivator-Oligonukleotid verwendet werden und/oder als Bestandteile des zweiten Bereichs des ersten Primer-Oligonukleotides.

Dank der Verwendung solcher Modifikationen kommt es zu einer lokalen Hinderung der Polymerase-Funktion, so dass bestimmte Segmente der verwendeten Strukturen nicht durch die Polymerase kopiert werden können und vorwiegend einzelsträngig bleiben. In dieser einzelsträngigen Form können sie weitere Reaktionskomponenten binden und somit ihre Funktion ausführen.

Unter Reaktionsbedingungen, welche einen Doppelstrang nicht denaturieren, führt die Verwendung der sequenzabhängigen nukleinsäurevermittelten Strangverdrängung zur sequenzspezifischen Trennung der beiden Primer-Verlängerungsprodukte während der Amplifikationsreaktion im beschriebenen Verfahren: eine hinreichende Komplementarität zwischen neusynthetisierten Verlängerungsfragmenten der Primer-Oligonukleotide mit der zu Beginn einer Amplifikation vorgegebenen Sequenz eines Aktivator-Oligonukleotides stellt eine Voraussetzung für eine erfolgreiche Strangverdrängung dar und kann somit Einfluss auf die Effizienz der Strangtrennung eines Doppelstranges (bestehend aus dem ersten und dem zweiten Primer-Verlängerungsprodukt) haben. Bei geringfügigen Abweichungen kommt es zu einer Verlangsamung der Strangverdrängung und somit auch zur Verlangsamung der Strangtrennung. Diese kann sich in einer Verlangsamung der Gesamtreaktion auswirken. Mit steigender Differenz in der Sequenz der neusynthetisierten Verlängerungsprodukte und der zu Beginn der Reaktion vorgegebenen Sequenz des Aktivator-Oligonukleotides kommt es zu einer immer höheren Hinderung der Strangverdrängung, welche im Endeffekt keine ausreichende Trennung von beiden Primer-Verlängerungsprodukten mehr leisten kann. Die beiden neu synthetisierten Stränge können nicht mehr ausreichend voneinander getrennt werden, so dass ihre Bindungsstellen für Primer-Oligonukleotide nicht mehr zugängig sind. Das führt im Allgemeinen zum Abbruch einer Amplifikation von Sequenzen mit Sequenzabweichungen.

Zusammengefasst können nicht nur die Spezifitäten der Bindung von beiden Primern mit ihren Matrizen, sondern auch die Beschaffenheit der Sequenzsegmente zwischen den Primern auf die Amplifikation Auswirkung haben, nämlich indem diese Abschnitte eine hinreichende Strangverdrängung zulassen oder nicht, je nach ihrer Übereinstimmung in der zu Beginn der Reaktion vorgegebenen Sequenz des Aktivator-Oligonukleotides. Das beschrieben Verfahren kann dadurch unter Umständen insgesamt eine höhere Spezifität vorweisen, als die herkömmlichen Amplifikations-Verfahren.

Das erfindungsgemäße Verfahren verwendet vorzugsweise keine spezifischen Proteine, welche einen Strang-Austausch zwischen Aktivator-Oligonukleotid und einem Doppelstrang bewirken, z.B. RecA. Das erfindungsgemäße Verfahren macht vielmehr Gebrauch von der zeitlichen Kopplung zwischen einer Strangverdrängung mittels einer zu Beginn der Reaktion vorgegebenen Nukleinsäurekette (Aktivator-Oligonukleotid) und einer gleichzeitig stattfindenden matrizenabhängigen Strang-Polymerisation mittels Polymerase. Diese gleichzeitige Kopplung von beiden Prozessen wird zum einen durch sequenzspezifische Strukturen von Komponenten (sequenzspezifische Aktivator-Oligonukleotide und sequenzspezifische Primer) sowie ihre spezifische Kombination ermöglicht. Zum anderen wird die Aktivität der Polymerase sequenzspezifisch kontrolliert: beispielsweise dient Aktivator-Oligonukleotid nicht als Matrize und die Synthese des zweiten Primer-Verlängerungsproduktes wird am ersten Primer-Oligonukleotid positionsspezifisch gestoppt.

Die spezifische Amplifikation resultiert weiterhin durch Verwendung der Komponenten bei Reaktionsbedinungen, welche bevorzugt eine spontane Trennung von neu synthetisierten Primer-Verlängerungsprodukte nicht zulassen.

Überraschenderweise ist es gelungen, diese Vorgänge vorteilhaft miteinander zu verknüpfen, und zwar in einer Art und Weise, dass beide Vorgänge (sequenzspezifische Strangverdrängung und Primer-Verlängerung) vorzugsweise in einem Ansatz ablaufen können. Dadurch kann ein homogenes Amplifikationssystem zusammengestellt werden, welches sequenzspezifische Primer-Oligonukleotide, ein sequenzspezifisches Aktivator-Oligonukleotid, Polymerase und Nukleotid-Substrate (dNTP) umfasst. Mit einem solchen Amplfikationssystem kann eine zu amplifizierende Nukleinsäure vervielfältigt werden. Die Sequenzen der beiden Primer-Oligonukleotide und die Sequenz des Aktivator-Oligonukleotides sind auf die zu amplifizierende Nukleinsäure abgestimmt.

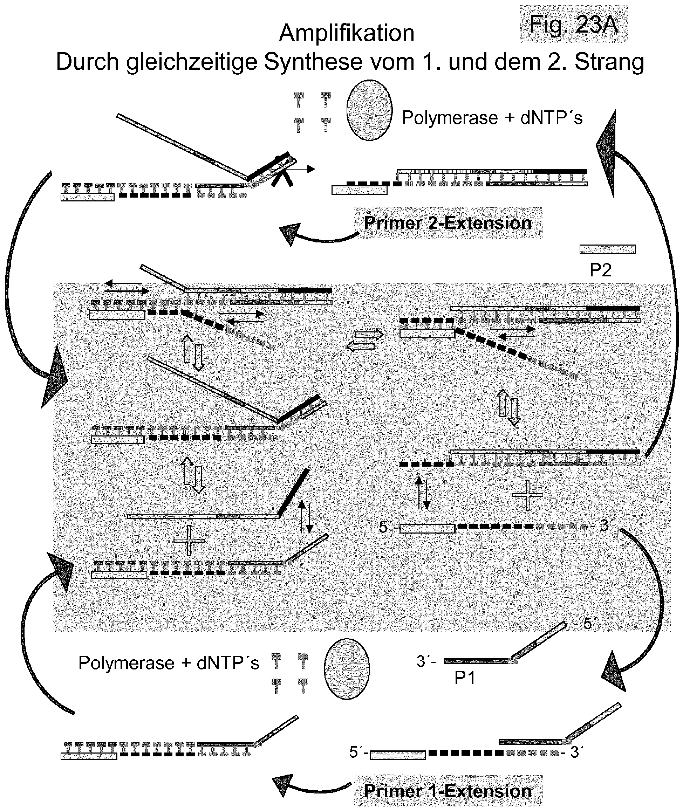

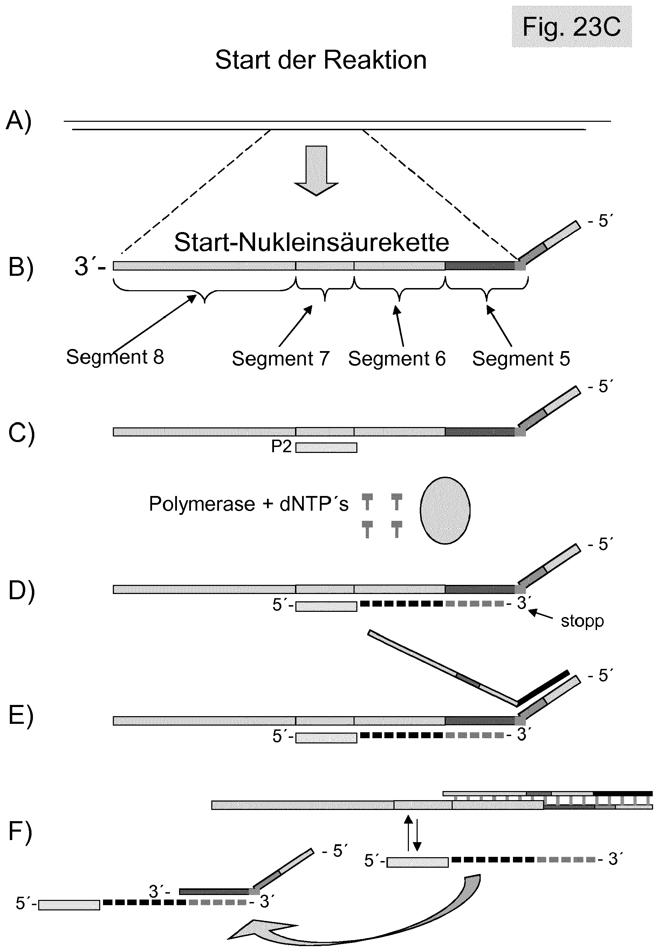

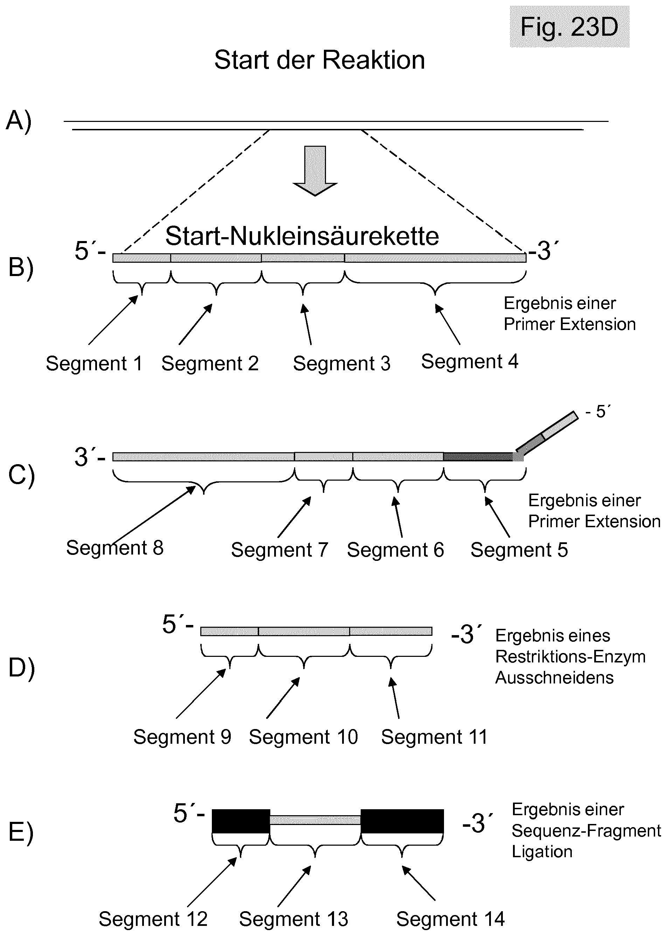

Das Verfahren (Fig. 23) umfasst mehrere Vorgänge, welche nachfolgend beschrieben sind. Diese Vorgänge können in einem Ansatz oder in getrennten Ansätzen ausgeführt werden. Wenn die Vorgänge in einem Ansatz durchgeführt werden sollen, so können diese unter gleichen Bedingungen, z.B. isothermal, oder unter unterschiedlichen Bedingungen, z.B. beim Thermocycling, ausgeführt werden. Vorzugsweise sind Primer-Oligonukleotide und Aktivator-Oligonukleotid zu Beginn der Reaktion präsent. Eine sequenzielle Zugabe einzelner Reagenzien ist allerdings auch möglich.

Ebenfalls sind Kombinationen mit anderen Amplifikationsverfahren möglich, z.B. mit PCR, wobei die PCR beispielsweise erst über 1 bis 10 Zyklen durchgeführt werden und anschließend wird beispielsweise unter isothermalen Bedingungen weitergearbeitet.

In einer vorteilhaften Form umfasst das Verfahren zur spezifischen Amplifizierung von Nukleinsäureketten folgende Schritte:
a) Hybridisierung eines ersten Primer-Oligonukleotids an das 3'-Segment eines Stranges einer zur amplifizierenden Nukleinsäurekette, wobei die zu amplifizierende Nukleinsäurekette eine Zielsequenz umfasst, wobei das erste Primer-Oligonukleotid umfasst:
   - Einen ersten Primer-Bereich im 3'-Segment des ersten Primer-Oligonukleotides, der an einen Strang einer zu amplifizierenden Nukleinsäurekette sequenzspezifisch binden kann,
   - einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedinungen im Wesentlichen unkopiert bleibt.
b) Extension des ersten Primer-Oligonukleotids mit Hilfe einer Polymerase zur Bildung eines ersten Primer-Verlängerungsproduktes, das zur Zielsequenz und/oder zu der zu amplifizierenden Nukleinsäurekette (a) komplementäre Sequenzabschnitte umfasst,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
   - Einen ersten einzelsträngigen Bereich, welcher an den Polynukleotid-Schwanz des zweiten Bereichs des ersten Primer-Oligonukleotides binden kann,
   - einen zweiten einzelsträngigen Bereich, welcher zu dem ersten Bereich des ersten Primer-Oligonukleotides im Wesentlichen komplementär ist und an diesen binden kann,
   - einen dritten einzelsträngigen Bereich, welcher zumindest zu einem Segment des von Polymerase synthetisierten Verlängerungsproduktes des ersten Primer-Verlängerungsprodukt im Wesentlichen komplementär ist, wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, wobei das 3'-Segment des ersten Primer-Verlängerungsproduktes einzelsträngig wird
f) Hybridisierung eines zweiten Oligonukleotid-Primers an das erste Primer-Verlängerungsprodukt, wobei das 3'-Segment des zweiten Oligonukleotid-Primers eine Sequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist.

In einer Ausführungsform wird das Verfahren unter Bedingungen ausgeführt, welche keine Trennung von komplementären Strängen der zu amplifizierenden Nukleinsäure in Abwesenheit von Aktivator-Oligonukleotid zulassen.

In einer Ausführungsform wird das Kopieren des Polynukleotid-Schwanzes im zweiten Primer-Bereich durch einen Stopp Bereich für die Polymerase bewirkt, der zwischen dem ersten und dem zweiten Bereich angeordnet ist.

In einer Ausführungsform ist der dritte einzelsträngige Bereich des Aktivator-Oligonukleotid zum Segment des von Polymerase synthetisierten Verlängerungsprodukts des ersten Primer-Verlängerungsprodukts, welches unmittelbar an den ersten Primer-Bereich anschließt, im Wesentlichen komplementär ist, wobei:

In einer Ausführungsform ist der dritte einzelsträngige Bereich des Aktivator-Oligonukleotides zum genannten 5'-Segment des Verlängerungsprodukts des ersten Primer-Verlängerungsprodutkes vollständig komplementär, wobei die Länge dieses komplemetären Sequenzabschnittes folgende Bereiche umfasst: von mindestens 3 bis 70 Nukleotide, besser von mindestens 5 bis 50 Nukleotide, bevorzugt von 5 bis 40 Nukleotide, weiter bevorzugt von 5 bis 30 Nukleotide, besonders bevorzugt von 5 bis 20 Nukleotide.

In einer weiteren Ausführungsform umfassen die Sequenzen des dritten einzelsträngigen Bereich des Aktivator-Oligonukleotides und die entsprechenden Sequenz des genannten 5'-Segments des Verlängerungsprodukts des ersten Primer-Verlängerungsproduktes komplementäre Sequenzen bis auf eine Sequenzposition (ein Nukleotid-Paar / ein Basenpaar) mit nicht komplementärer Basenpaarung (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide.

In einer weiteren Ausführungsform umfassen die Sequenzen des dritten einzelsträngigen Bereich des Aktivator-Oligonukleotides und die entsprechenden Sequenz des genannten 5'-Segments des Verlängerungsprodukts des ersten Primer-Verlängerungsproduktes komplementäre Sequenzen bis auf zwei Sequenzpositionen (ein Nukleotid-Paar / ein Basenpaar) mit nicht komplementärer Basenpaarung (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide.

In einer weiteren Ausführungsform umfassen die Sequenzen des dritten einzelsträngigen Bereichs des Aktivator-Oligonukleotides und die Sequenz des genannten 5'-Segments des Verlängerungsprodukts des ersten Primer-Verlängerungsprodutkes komplementäre Sequenzen (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide, weiterhin umfassen genannte Segmente nicht komplementäre Bereiche an mindestens drei Sequenzpositionen, wobei diese Positionen im 5'-Segment des dritten Abschnitt des Aktivator-Oligonukleotides liegen.

In einer weiteren Ausführungsform umfassen die Sequenzen des dritten einzelsträngigen Bereichs des Aktivator-Oligonukleotides und die Sequenz des genannten 5'-Segments des Verlängerungsprodukts des ersten Primer-Verlängerungsprodutkes komplementäre Sequenzen bis auf mindestens eine und maximal zehn Sequenzpositionen mit nicht komplementärer Basenpaarung (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide, wobei an Sequenzpositionen mit nicht komplementärer Basenpaarung (im Sinne von Watson-Crick Basenpaarung) mindestens ein modifiziertes Nukleotid mit modifizierten Nukleobasen beteiligt sind. Solche modifizierte Nukleobasen umfassen beispielsweise Nukleobasen mit verstärkter Bindung von natürlichen Nukleobasen (z.B. 2-Amino-Adenine), oder mit abgeschwächter Bindung, wie beispielsweise so genannte universale Basen, wie Inosine oder 5-Nitroindol. Die modifizierten Nukleobasen sind vorzugsweise im dritten Sequenzbereich des Aktivator-Oligonukleotides lokalisiert.

In einer weiteren Ausführungsform des Verfahrens wird Schritt (e) des Verfahrens weiterhin modifiziert und umfasst:
Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette bis dieser komplementärer Strang der zu amplifizierenden Nukleinsäure vom ersten Primer-Verlängerungsprodukt abgelöst wird, wobei das 3'-Segment des ersten Primer-Verlängerungsproduktes einzelsträngig wird

In einer weiteren Ausführungsform des Verfahrens wird Schritt (f) des Verfahrens weiterhin modifiziert und umfasst:
Hybridisierung eines zweiten Oligonukleotid-Primers an das erste Primer-Verlängerungsprodukt, wobei gleichzeitige zumindest partielle Verdrängung des Aktivator-Oligonukleotides aus der Bindung mit dem ersten Verlängerungsprodukt durch Strangverdrängung erfolgt.

In einer weiteren Ausführungsform des Verfahrens wird Schritt (g) des Verfahrens weiterhin modifiziert und umfasst eine Verdrängung des Aktivator-Oligonukleotides aus der Bindung mit dem ersten Primer-Verlängerungsprodukt unter Mitwirkung der Polymerase.

In einer weiteren Ausführungsform des Verfahrens wird Schritt (h) des Verfahrens weiterhin modifiziert und umfasst: ggf. Bindung des Aktivator-Oligonukleotides an den unkopierten Polynukleotid-Schwanz des ersten verlängerten Primer-Oligonukleotid und Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oiigonukleotides.

In einer weiteren Ausführungsform des Verfahrens wird Verfahren weiterhin modifiziert und umfasst: h) Fortführung der Reaktion unter Bedinungen, welche Wiederholung von Schritten (a) bis (g) zulassen.

In einer weiteren Ausführungsform des Verfahrens wird des Verfahrens weiterhin modifiziert und umfasst: gleichzeitige Amplifizierung des ersten und zweiten Primer-Verlängerungsproduktes in einer exponentiellen Reaktion unter Verwendung der ersten und zweiten Primer-Oligonukleotid und des Aktivator-Oligonukleotides, wobei die gebildeten Primer-Verlängerungsprodukte als Matrizen für gegenseitige Synthese auftreten.

### Bevorzugte Ausführungsformen einer Start-Nukleinsäurekette:

Die zu Beginn der Amplifikations-Reaktion eingesetzte bzw. einzusetzende Nukleinsäurekette kann als Start-Nukleinsäurekette bezeichnet werden (Fig. 23B-23D).

Ihre Funktion kann dahingehend gesehen werden, dass sie die anfängliche Matrize darstellt, welche eine korrekte Positionierung von Primern, der Synthese-Abschnitte zwischen beiden Primern, sowie die Initiierung von Bindungs- und Verlängerungsvorgängen ermöglicht. In einer bevorzugten Ausführungsform umfasst eine Start-Nukleinsäurekette eine Zielsequenz.

Durch Bindung von Primern an ihre entsprechenden Primer-Bindungsstellen (PBS 1 und PBS 2) und Initiierung von entsprechenden Primer-Verlängerungsreaktionen erfolgt durch Generierung von ersten Primer-Verlängerungsprodukten. Diese werden als spezifische Kopien der zu Begin der Reaktion vorliegenden Nukleinsäurekette synthetisiert.

In einer Ausführungsform kann diese in das Reaktionsgemisch vor Beginn der Amplifikatons-Reaktion einzusetzende Nukleinsäurekette (Start-Nukleinsäurekette) mit der zu amplifizierenden Nukleinsäurekette identisch sein. Durch die Amplifikations-Reaktion wird lediglich die Menge einer solchen Nukleinsäurekette vermehrt.

In einer weiteren Ausführungsform unterscheiden sich die zu amplifizierende Nukleinsäure und die Start-Nukleinsäurekette dahingehend, dass die Start-Nukleinsäurekette zwar die Anordnung einzelner Sequenzelemente der zu amplifizierenden Nukleinsäurekette vorgibt, doch kann die Sequenzzusammensetzung der Start-Nukleinsäurekette von der Sequenz der zu amplifizierenden Nukleinsäurekette abweichen. Beispielsweise können im Rahmen der Primer-Bindung und Verlängerung während einer Amplifikation neue Sequenzinhalte (bezogen auf Start-Nukleinsäurekette) in die zu amplifizierende Nukleinsäurekette integriert werden. Weiterhin können sich Sequenzelemente einer zu amplifizierenden Nukleinsäurekette von solchen Sequenzelementen einer Start-Nukleinsäurekette in ihrer Sequenzzusammensetzung unterscheiden (z.B. Primer-Bindungsstellen oder Primer-Sequenzen). Die Start-Nukleinsäure dient nur als initiale Matrize für die spezifische Synthese der zu amplifizierenen Nukleinsäurekette. Diese initiale Matrize kann im Reaktionsgemisch bis zum Ende der Amplifikation verbleiben. Durch den exponentiellen Charakter der Amplifikation überwiegt allerdings die Menge der zu amplifizierenden Nukleinsäurekette am Ende einer Amplifikationsreaktion die Menge einer in die Reaktion zugegebenen Start-Nukleinsäurekette.

In einer weiteren Ausführungsform kann die Start-Nukleinsäurekette mindestens einen Sequenzabschnitt umfassen, welcher nicht amplifiziert wird. Eine solche Start-Nukleinsäurekette ist somit nicht mit der zu amplifizierenden Sequenz identisch. Solche nicht zu amplifizierenden Abschnitte können beispielsweise als Folge von Sequenzvorbereitungsschritten bzw. als Folge von vorangegangenen Sequenz-Manipulations-Schritten einen Sequenzabschnitt einer Start-Nukleinsäurekette repräsentieren.

In einer bevorzugten Ausführungsform schließt die in das Reaktionsgemisch vor Beginn der Reaktion einzusetzende Start-Nukleinsäurekette mindestens eine Zielsequenz ein.

In einer weiteren Ausführungsform schließt eine solche Start-Nukleinsäurekette mindestens eine Zielsequenz (engl. Target-Sequence) und noch weitere Sequenzen ein, welche nicht Zielsequenz (engl. Non-target Sequences) sind. Während der Amplifikation werden Sequenzsegmente umfassend die Zielsequenz exponentiell vermehrt und andere Sequenzsegmente werden dabei entweder gar nicht oder nur teilweise exponentiell vermehrt.

### Aufbau einer Start-Nukleinsäurekette

Beispiel für eine solche Start-Nukleinsäurekette stellt eine Nukleinsäurekette dar, welche eine Zielsequenz einschließt und welche ein Sequenzfragment-A umfasst und ein Sequenzfragment-B umfasst.

Das Sequenzfragment-A der Start-Nukleinsäurekette umfasst eine Sequenz, die mit der Sequenz eines der beiden in der Amplifikation verwendeten Primer eine signifikante Homologie aufweist oder im Wesentlichen mit dem kopierbaren Anteil des 3'-Segments des einen Primers identisch ist. Bei Synthese eines komplementären Stranges zu diesem Segment wird eine komplementäre Sequenz generiert, welche eine entsprechende Primer-Bindungs-Stelle darstellt.

Das Sequenzfragment-B der Start-Nukleinsäurekette umfasst eine Sequenz, welche zur komplementären Bindung eines entsprechenden weiteren Primers oder seines 3'-Segmentes unter Ausbildung eines extensionsfähigen Primer-Matrizen-Komplexes geeignet ist, wobei Sequenzfragment-A und Sequenzfragment-B im Verhältnis zu einander vorwiegend / bevorzugt nicht komplementär sind.

In einer bevorzugten Ausführungsform wird zum Reaktionsgemisch eines Amplifikationsverfahrens eine Start-Nukleinsäurekette gegeben, welche folgende Eigenschaften aufweist:
Vorzugsweise liegt das Sequenzfragment-A im 5'-Segment der Start-Nukleinsäurekette. Vorzugsweise bildet dieses Sequenzfragment-A eine Begrenzung des Nukleinsäurekette-Stranges in 5'-Richtung.

Vorzugsweise liegt das Sequenzfragment-B stromabwärts vom Sequenzfragment-A.

In einer bevorzugten Ausführungsform bildet dieses Sequenzfragment-B eine Begrenzung des Nukleinsäurekette-Stranges in 3'-Richtung. In einer weiteren Ausführungsform stellt dieses Sequenzfragment-B keine Begrenzung des Nukleinsäurekette-Stranges in 3'-Richtung dar, sondern wird von weiteren Sequenzen von der 3'-Seite flankiert. Vorzugsweise sind diese Sequenzen keine Zielsequenzen und nehmen an der exponentiellen Amplifikation nicht teil.

In einer Ausführungsform umfasst die Zielsequenz mindestens eines von beiden Sequenzfragment-A oder Sequenzfragment-B. In einer weiteren Ausführungsform liegt die Zielsequenz zwischen Sequenzfragment-A und Sequenzfragment-B.

In einer Ausführungsfrom kann eine solche Start-Nukleinsäurekette als Matrize für die Synthese eines ersten Primer-Verlängerungsproduktes dienen (Fig. 23B). Die Start-Nukleinsäurekette kann dabei beispielsweise im Rahmen einer Primer-Verlängerungsreaktion unter Verwendung des zweiten Primer-Oligonukleotides als ein Sequenzsegment einer längeren Ausgangs-Nukleinsäurekette (Fig. 23B, Abschnitt A) während eines vorbereitenden Schrittes vor der exponentiellen Amplifikation bereitgestellt werden und in einer einzelsträngige Form überführt werden. Die Ausgangsnukleinsäurekette kann beispielsweise eine genomische DNA oder RNA sein und dient als Quelle der Zielsequenz (schematisch dargestellt in Fig. 23B Abschnitt A als Doppelstrang). Die Start-Nukleinsäurekette umfasst (Fig. 23B, Abschnitt B) im 5'-Segment der Nukleinsäurekette ein Segment 1, was Sequenzabschnitte des zweiten Primer-Oligonukleotides umfasst und Begrenzung in 5'-Richtung darstellt, ein Segment 2, was eine Zielsequenz oder ihre Anteile umfasst, ein Segment 3, was eine Primerbindungsstelle für das erste Primer-Oligonukleotid umfasst und ein Segment 4 was eine nicht-Zielsequenz im 3'-Segment der Start-Nukleinsäurekette lokalisiert ist und somit das Segment 3 auf der 3'-Seite flankiert.

Im Rahmen der Initiierung der Amplifikation kann der erste Primer zumindest mit 3'-Segment seines ersten Bereichs an eine solche Start-Nukleinsäurekette im Segment 3 binden (Fig. 23B Abschnitt C) und in Anwesenheit einer Polymerase und Nukleotide entsprechend verlängert werden. Dabei entsteht ein erstes Primer-Verlängerungsprodukt, welches dem Matrizenstrang der Start-Nukleinsäurekette komplementär ist und in seiner Länge begrenzt ist (Fig. 23B Abschnitt D). Im Rahmen der Amplifikationsreaktion kann ein solches Primer-Verlängerungsprodukt via Aktivator-Oligonukleotid von seinem Matrizenstrang sequenzspezifisch abgelöst werden (Fig. 23B Abschnitt E-F), so dass entsprechende Sequenzabschnitte im 3'-Segment des nun synthetisierten ersten Primer-Verlängerungsproduktes als Bindungsstelle für das zweite Primer-Oligonukleoid zur Verfügung stehen (Fig. 23B Abschnitt F).

In einer bevorzugten Ausführungsform umfasst eine Start-Nukleinsäurekette somit folgende Sequenzfragmente (Fig. 23B):
- Sequenzsegment-1 (in Fig. 23B als Segment 1 bezeichnet), welches eine Sequenz umfasst, die mit der Sequenz des zweiten Primers eine signifikante Homologie aufweist oder im Wesentlichen mit dem kopierbaren Anteil des 3'-Segments des zweiten Primers identisch ist. Dieses Sequenzsegment-1 liegt im 5'-Segment der Start-Nukleinsäurekette, vorzugsweise bildet dieses Sequenzsegment-1 eine Begrenzung des Nukleinsäurekette-Stranges in 5'-Richtung.
- Das Sequenzsegment-3 (in Fig. 23B als Segment 3 bezeichnet),, welches eine Sequenz umfasst, welche zu komplementären Bindung des ersten Bereichs des ersten Primers oder seines 3'-Segmentes unter Ausbildung eines extensionsfähigen Primer-Matrizen-Komplexes geeignet ist. Vorzugsweise liegt das Sequenzsegment-3 stromabwärts vom Sequenzsegment-1.
- Zielsequenz , welche teilweise oder ganz zwischen Segment 1 und Segment 3 liegt (in Fig. 23B dieser Anteil der Zielsequenz wird als Segment 2 bezeichnet). In einer bevorzugten Ausführungsform umfasst die Zielsequenz mindestens eines von Segment 1 und / oder Segment 3.
- Optional umfasst die Start-Nukleinsäurekette im 3'-Segment flankierende Sequenzabschnitte (in Fig. 23B als Segment 4 bezeichnet), welche nicht amplifiziert werden.

In einer weiteren Ausführungsfrom kann eine solche Start-Nukleinsäurekette als Matrize für die Synthese eines zweiten Primer-Verlängerungsproduktes dienen (Fig. 23 C).

Die Start-Nukleinsäurekette kann dabei beispielsweise im Rahmen einer Primer-Verlängerungsreaktion unter Verwendung des ersten Primer-Oligonukleotides als ein Sequenzsegment einer längeren Ausgangs-Nukleinsäurekette (Fig. 23C, Abschnitt A) während eines vorbereitenden Schrittes vor der exponentiellen Amplifikation bereitgestellt werden und in einer einzelsträngige Form überführt werden. Die Ausgangsnukleinsäurekette kann beispielsweise eine genomische DNA oder RNA sein und dient als Quelle der Zielsequenz (schematisch dargestellt in Fig. 23C Abschnitt A als Doppelstrang). Die Start-Nukleinsäurekette umfasst (Fig. 23C, Abschnitt B) im 5'-Segment der Nukleinsäurekette ein Segment 5, was Sequenzabschnitte des ersten Primer-Oligonukleotides umfasst und Begrenzung in 5'-Richtung darstellt, ein Segment 6, was eine Zielsequenz oder ihre Anteile umfasst, ein Segment 7, was eine Primerbindungsstelle für das zweite Primer-Oligonukleotid umfasst und ein Segment 8 was eine nicht-Zielsequenz im 3'-Segment der Start-Nukleinsäurekette lokalisiert ist und somit das Segment 7 auf der 3'-Seite flankiert.

Im Rahmen der Initiierung der Amplifikation kann der zweite Primer zumindest mit 3'-Segment seines ersten Bereichs an eine solche Start-Nukleinsäurekette im Segment 7 binden (Fig. 23C Abschnitt C) und in Anwesenheit einer Polymerase und Nukleotide entsprechend bis zu hin zum Stopp-Abschnitt des ersten Primer-Oligonukleotides verlängert werden. Dabei entsteht ein zweites Primer-Verlängerungsprodukt, welches dem Matrizenstrang der Start-Nukleinsäurekette komplementär ist und in seiner Länge begrenzt ist (Fig. 23C Abschnitt D). Im Rahmen der Amplifikationsreaktion kann ein solches Primer-Verlängerungsprodukt via Aktivator-Oligonukleotid von seinem Matrizenstrang sequenzspezifisch abgelöst werden (Fig. 23C Abschnitt E-F), so dass entsprechende Sequenzabschnitte im 3'-Segment des nun synthetisierten zweiten Primer-Verlängerungsproduktes als Bindungsstelle für das erste Primer-Oligonukleoid zur Verfügung stehen (Fig. 23C Abschnitt F).

In dieser bevorzugten Ausführungsform umfasst eine Start-Nukleinsäurekette folgende Sequenzfragmente (Fig. 23 C):
- Sequenzsegment-5 (genannt Segment 5), welches eine Sequenz umfasst, die mit der Sequenz des ersten Bereichs des ersten Primers eine signifikante Homologie aufweist oder im Wesentlichen mit dem kopierbaren Anteil des ersten Bereichs des ersten Primers identisch ist. Dieses Segment-7 liegt im 5'-Segment der Start-Nukleinsäurekette und ist von einem nicht kopierbaren Oligonukleotid-Schwanz flankiert (analog zum ersten Primer-Oligonukleotid), vorzugsweise bildet dieses Segment - 7 eine Begrenzung des kopierbaren Nukleinsäurekette-Stranges in 5'-Richtung.
- Das Sequenzfragment-7 (genannt Segment 7), welches eine Sequenz umfasst, welche zur komplementären Bindung eines zweiten Primers oder seines 3'-Segmentes unter Ausbildung eines extensionsfähigen Primer-Matrizen-Komplexes geeignet ist. Vorzugsweise liegt das Segment 7 stromabwärts vom Segment 5.
- Zielsequenz , welche teilweise oder ganz zwischen Segment 5 und Segment 7 liegt (in Fig. 23C dieser Anteil der Zielsequenz wird als Segment 6 bezeichnet). In einer bevorzugten Ausführungsform umfasst die Zielsequenz mindestens eines von Segment 5 und / oder Segment 7.
- Optional umfasst die Start-Nukleinsäurekette im 3'-Segment flankierende Sequenzabschnitte (in Fig. 23C als Segment 8 bezeichnet), welche nicht amplifiziert werden.

### Funktionsweise der Start-Nukleinsäurekette

Zu Beginn der Amplifikations-Reaktion dient die Start-Nukleinsäurekette als Matrize für initiale Entstehung von jeweiligen Primer-Verlängerungsprodukten. Sie stellt somit die Ausgangs-Matrize für die zu amplifizierende Nukleinsäurekette dar. Die Start-Nukleinsäurekette braucht nicht zwingend mit der zu amplifizierenden Nukleinsäurekette identisch zu sein. Durch Bindung und Verlängerung von beiden Primern während der Amplifikations-Reaktion bestimmen im Wesentlichen die beiden Primer, welche Sequenzen an beiden endständigen Segmenten der zu amplifizierenden Nukleinsäurekette im Rahmen der Amplifikation generiert werden.

In einer bevorzugten Ausführungsform des Verfahrens werden für einen Doppelstrang nicht-denaturierenden Reaktions-Bedinungen während des exponentiellen Amplifikationsvorgangs aufrechterhalten. Daher ist es vorteilhaft, wenn Start-Nukleinsäurekette eine Begrenzung in ihrem durch eine Polymerase verlängerbaren 5'-Sequenzsegment aufweist, was zu einem Stopp in der enzymatischen Verlängerung eines entsprechenden Primers führt (z.B. Segment 1 oder 5 oder 9 oder 12 in Fig. 23D). Damit wird die Länge der unter Reaktionsbedinungen generierten Primer-Verlängerungsfragmente begrenzt. Das kann sich vorteilhaft auf Strangverdrängung durch Aktivator-Oligonukleotid auswirken und zu einer Dissoziation des jeweiligen Stranges führen, so dass Primer-Bindungsstellen in einzelsträngigen Zustand überführt werden und somit für eine erneute Bindung von Primern zugängig werden.

In einer weiteren bevorzugten Ausführungsform des Verfahrens werden während initialen Synthese-Schritte (beispielsweise initiale eine bis zehn Synthese-Wiederholungen) für einen Doppelstrang denaturierenden Reaktions-Bedingungen verwendet (z.B. TemperaturErhöhung bis auf über 90°C), allerdings in darauffolgenden Wiederholungen der Synthese-Schritte werden nicht-denaturierende Bedingungen während des exponentiellen Amplifikationsvorgangs aufrechterhalten. Für eine solche Kombination von TemperaturBedingungen spielt es keine Rolle, ob Start-Nukleinsäurekette eine Begrenzung in ihrem 5'-Sequenzsegment aufweist oder nicht. Durch initiale Denaturierung erfolgt Trennung von synthetisierten Strängen unabhängig von ihrer Länge in Analogie zu PCR.

### Erstellung einer Start-Nukleinsäurekette

Eine Start-Nukleinsäurekette kann vor einer exponentiellen Amplifikation in einem separaten Reaktions-Vorgang erstellt werden. Einem Fachmann sind viele Methoden bekannt, wie man eine Nukleinsäurekette umfassend eine Zielsequenz sowie weitere vorgegebene Sequenzabschnitte erstellen und verwenden kann. Im Rahmen der Erstellung einer Start-Nukleinsäurekette können Verfahren verwendet werden, welche beispielsweise matrizenabhängige Primer-Verlängerungs-Schritte, Restriktions-Enzym-Verdau-Schritte, Ligations-Schritte, Polyadenylierung-Schritte umfassen. Diese Verfahrenschritte können einzeln oder in Kombination verwendet werden. Im Rahmen solcher Reaktionen können Start-Nukleinsäureketten erstellt werden, welche unterschiedliche Strukturen aufweisen, Fig. 23D.

In Fig. 23D wird eine doppelsträngige DNA als Ausgangsmaterial schematisch dargestellt (A), wobei ein Sequenzabschnitt aus dieser DNA als Material bzw. Matrize zur Herstellung einer Start-Nukleinsäurekette dienen soll, welcher eine Zielsequenz umfasst. Geeignete Start-Nukleinsäureketten mit unterschiedlichen Strukturen können aus einem solchen Material unter Verwendung entsprechender Verfahren erstellt werden.

Beispielsweise kann eine zu erstellende Start-Nukleinsäurekette (Fig. 23D, Abschnitt B) folgende Sequenzsegmente umfassen: ein Segment 1 im 5'-Segment der Start-Nukleinsäurekette, was Sequenzabschnitte des zweiten Primer-Oligonukleotides umfasst und Begrenzung in 5'-Richtung darstellt, ein Segment 2, was eine Zielsequenz oder ihre Anteile umfasst, ein Segment 3, was eine Primerbindungsstelle für das erste Primer-Oligonukleotid umfasst, und ein Segment 4, was eine nicht-Zielsequenz im 3'-Segment der Start-Nukleinsäurekette lokalisiert ist und somit das Segment 3 auf der 3'-Seite flankiert. Eine solche Start-Nukleinsäurekette kann beispielsweise im Rahmen einer Primer-Verlängerungsreaktion synthetisiert werden oder im Rahmen eines Restriktions-Enzym-Verdau / Restriktions-Enzym-Ausscheidens unter Verwendung eines oder mehreren Restriktionsenzyms generiert werden.

Weiterhin kann beispielsweise eine zu erstellende Start-Nukleinsäurekette (Fig. 23D, Abschnitt C) folgende Sequenzsegmente umfassen: ein Segment 5 im 5'-Segment der Start-Nukleinsäurekette, was Sequenzabschnitte des ersten Primer-Oligonukleotides umfasst und Begrenzung in 5'-Richtung darstellt, ein Segment 6, was eine Zielsequenz oder ihre Anteile umfasst, ein Segment 7, was eine Primerbindungsstelle für das zweite Primer-Oligonukleotid umfasst und ein Segment 8, was eine nicht-Zielsequenz im 3'-Segment der Start-Nukleinsäurekette lokalisiert ist und somit das Segment 7 auf der 3'-Seite flankiert. Eine solche Start-Nukleinsäurekette kann beispielsweise im Rahmen einer Primer-Verlängerungsreaktion synthetisiert werden.

Weiterhin kann beispielsweise eine zu erstellende Start-Nukleinsäurekette (Fig. 23D, Abschnitt D) folgende Sequenzsegmente umfassen: ein Segment 9 im 5'-Segment der Start-Nukleinsäurekette, was Sequenzabschnitte des ersten Primer-Oligonukleotides umfasst und Begrenzung in 5'-Richtung darstellt, ein Segment 10, was eine Zielsequenz oder ihre Anteile umfasst, ein Segment 11, was eine Primerbindungsstelle für das zweite Primer-Oligonukleotid umfasst. Eine solche Start-Nukleinsäurekette kann beispielsweise im Rahmen eines Restriktions-Enzym-Verdau / Restriktions-Enzym-Ausscheidens unter Verwendung eines oder mehreren Restriktionsenzyms generiert werden.

Weiterhin kann beispielsweise eine zu erstellende Start-Nukleinsäurekette (Fig. 23D, Abschnitt E) folgende Sequenzsegmente umfassen: ein Segment 12 im 5'-Segment der Start-Nukleinsäurekette, was Sequenzabschnitte des ersten Primer-Oligonukleotides umfasst und Begrenzung in 5'-Richtung darstellt, ein Segment 13, was eine Zielsequenz oder ihre Anteile umfasst, ein Segment 14, was eine Primerbindungsstelle für das zweite Primer-Oligonukleotid umfasst. Eine solche Start-Nukleinsäurekette kann beispielsweise im Rahmen eines Restriktions-Enzym-Verdau / Restriktions-Enzym-Ausscheidens unter Verwendung eines oder mehreren Restriktionsenzyms generiert werden sowie einer Ligation von mindestens einem der Segmente 12 und / oder 14 an das Segment 13 unter Verwendung entsprechender Ligase-Enzym-Systeme.

Im Rahmen der Erstellung kann resultierende Start-Nukleinsäurekette in doppelsträngigen oder einzelsträngigen Form vorliegen. Je nach Struktur der Start-Nukleinsäurekette und des Verfahrens ihrer Erstellung kann es notwendig sein, vor der Amplifikation die Start-Nukleinsäurekette in einzelsträngige Form zu überführen. Dies kann beispielsweise als separater Schritt erfolgen oder im Rahmen einer Aufreinigungs-Prozedur der Start-Nukleinsäurekette von begleitenden Komponenten.

Beispielsweise kann eine Start-Nukleinsäurekette im Rahmen einer separaten Primer-Extensions-Reaktion unter Verwendung eines in der exponentiellen Amplifikation verwendeten Primers (z.B. das erste Primer-Oligonukleotid oder das zweite Primer-Oligonukleotid), einer einzelsträngigen DNA oder RNA, welche eine Zielsequenz umfasst, sowie einer Polymerase und dNTPs synthetisiert werden. Es resultiert dabei eine Start-Nukleinsäurekette, welche an ihre Matrize gebunden ist. Zur Verwendung in der exponentiellen Amplifikation kann eine solche Start-Nukleinsäurekette vor der exponentiellen Amplifikation in einzelsträngige Form überführt werden. Dabei können mehrere bekannte Methoden verwendet werden, z.B. temperaturabhängige Denaturierung, Alkalie-Denaturierung, partielle oder vollständige Strang-degradation (z.B. mit Hilfe von Rnase H, oder Dnase oder 5'-Exonuklease). Zusätzlich kann eine weitere Primer-Verlängerungsreaktion durchgeführt werden, mit einem zusätzlichen Primer, welcher stromaufwärts positioniert ist (sogenannter Bumper-Primer bzw. äußerer Primer), so dass Start-Nukleinsäurekette durch polymerase-abhängige Strangverdrängung in einzelsträngige Form überführt wird.

Zwecks Überführung von Start-Nukleinsäurekette in eine einzelsträngige Form können weitere enzymatische Systeme sowie ihre Kofaktoren bzw. Energie-Träger zum Amplifikationsgemisch zugegeben werden, welche in der Lage sind, doppelsträngige Nukleinsäureketten in einzelsträngige Form zu überführen und somit eine erzeugte Start-Nukleinsäurekette aus einem doppelsträngigen Zustand in einen einzelsträngigen Zustand überführen. Zu solchen enzymatischen Systemen zählen beispielsweise Helicasen und Recombinasen (mit ATP oder dATP als Energie-Träger). In einer bevorzugten Ausführungsform werden solche Enzyme vor der exponentiellen Amplifikationsreaktion inaktiviert, damit es im Rahmen der Amplifikationsreaktion nicht zu einer sequenzunspezifischen Strang-Dissoziation kommt.

In einer vorteilhaften Ausführungsform wird eine solche Start-Nukleinsäurekette vor der exponentiellen Amplifikation erstellt und bedarf keiner separaten Aufbereitung.

In einer weiteren vorteilhaften Ausführungsform wird die Start-Nukleinsäurekette in Anwesenheit von Komponenten einer Amplifikations-Reaktion / bzw. zu Amplifikations-Reaktion bereitgestellten Reaktions-Gemisches erstellt. Das ermöglicht eine nahtlose Überführung einer Start-Nukleinsäurekette direkt nach ihrer Erstellung in die exponentielle Amplifikations-Reaktion. Eine solche Ausführungsform ermöglicht Design eines homogenen Assay-Formats.

In einer vorteilhaften Ausführungsform wird Ausgangs-Material für eine solche Start-Nukleinsäurekette aus folgender Gruppe ausgewählt: genomische DNA oder ihre Fragmente, Plasmid-DNA oder ihre Fragmente, mRNA oder ihre Fragmente, microRNA oder ihre Fragmente. Die als Ausgangs-Material dienende Nukleinsäure kann beispielsweise aus einer klinisch relevanten Material-Quelle abstammen.

In einer bevorzugten Ausführungsform des Verfahrens, ist die Sequenz der zu Begin der Amplifikations-Reaktion einzusetzenden Start-Nukleinsäurekette mit der Sequenz der zu amplifizierenden Nukleinsäurekette identisch. Dabei kann nur ein Strang der zu amplifizierenden Nukleinsäurekette (entweder als erstes Primer-Verlängerungsprodukt oder als zweites Primer-Verlängerungsprodukt) oder auch beide Stränge zu Beginn der Amplifikation im Amplifikations-Ansatz eingesetzt werden.

In einer Ausführungsform des Verfahrens ist eine in die Reaktion einzusetzende Start-Nukleinsäurekette vorzugsweise mindestens an einer Seite dermaßen in ihrer Länge limitiert, so dass bei Synthese des komplementären Stranges ein doppelsträngiges Fragment entsteht, welches unter Mitwirkung des Aktivator-Oligonukleotides bei gewählter Temperatur in Einzelstränge dissoziieren kann.

Dies kann beispielsweise dadurch erreicht werden, dass die in das Amplifikations-Gemisch einzusetzende Start-Nukleinsäurekette mindestens ein Sequenz-Fragment in ihrem 5'-Segment einschließt, welches einem Amplifikations-Primer oder einem Teil eines Amplifikations-Primers entspricht, und ein weiteres Sequenz-Fragment stromabwärts einschließt, welches einer Primerbindungsstelle entspricht oder als Primerbindungsstelle funktioniert, an welche im Amplifikationsverfahren ein passender Primer binden kann und eine Polymerase-abhängige Synthese-Reaktion eines komplementären Stranges zu Start-Nukleinsäurekette ermöglichen kann.

### Bevorzugte Ausführungsformen des ersten Primer-Oligonukleotids (Primer-1)

**Das erste Primer-Oligonukleotid** (Primer-1) ist eine Nukleinsäurekette welche mindestens folgende Bereiche einschließt (Fig. 5 und 6):
- Einen ersten Primer-Bereich im 3'-Segment des ersten Primer-Oligonukleotides, der an einen Strang einer zu amplifizierenden Nukleinsäurekette im Wesentlichen sequenzspezifisch binden kann
- Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz unter Reaktionsbedingungen im wesentlichen einzelsträngig bleibt, d.h. keine stabile Hairpin-Struktur oder ds-Strukturen ausbildet, und vorzugsweise nicht von Polymerase kopiert wird.

Die Gesamtlänge des ersten Primer-Oligonukleotides liegt zwischen 10 und 80, vorzugsweise zwischen 15 und 50, besser zwischen 20 und 30 Nukleotiden oder ihren Äquivalenten (z.B. Nukleotid-Modifikationen). Die Struktur des ersten Primer-Oligonukleotids ist dermaßen angepasst, dass es unter gewählten Reaktionsbedinungen eine reversible Bindung an Aktivator-Oligonukleotid eingehen kann. Weiterhin ist die Struktur des ersten Primer-Oligonukletides an seine Primer-Funktion angepasst. Weiterhin ist die Struktur so angepasst, dass eine Strangverdrängung mittels Aktivator-Oligonukleotid ausgeführt werden kann. Insgesamt sind Strukturen des ersten und des zweiten Bereichs aneinander angepasst, so dass eine exponentielle Amplifikation ausgeführt werden kann.

In einer vorteilhaften Ausführungsform der Erfindung sind der erste und der zweite Bereich des Primers in einer konventionellen 5'-3' Anordnung gekoppelt. In einer weiteren Ausführungsform der Erfindung erfolgt die Kopplung von beiden Abschnitten über eine 5'-5'-Bindung, so dass der zweite Bereich eine umgekehrte Richtung hat als der erste Bereich.

Die Kopplung von Bereichen zwischeneinander/untereinander erfolgt vorzugsweise kovalent. In einer Ausführungsform ist die Kopplung zwischen dem ersten und zweiten Bereich eine für DNA konventionelle 5'-3'-Phosphodiester-Kopplung. In einer weiteren Ausführungsform ist es eine 5'-5'-Phosphodiester-Kopplung. In einer weiteren Ausführungsform ist es eine 5'-3'-Phosphodiester-Kopplung, wobei zwischen benachbarten endständigen Nukleotiden bzw. Nukleotid-Modifikationen der beiden Bereiche mindestens ein Linker (z.B. ein C3, C6, C12 oder ein HEG-Linker oder eine abasische Modifikation) positioniert ist.

Einzelne Bereiche können unterschiedliche Nukleotid-Modifikationen einschließen. Dabei können individuelle Elemente von Nukleotiden modifiziert sein: Nukleobase und Rückgrad (Zucker-Anteil und / oder Phosphat-Anteil). Weiterhin können Modifikationen verwendet werden, welchen mindestens eine Komponente der Standard-Nukleotid-Bausteine fehlt oder modifiziert ist, z.B. PNA.

In einer weiteren Ausführungsform umfasst ein zweiter Bereich des ersten Primer-Oligonukleotides weitere Sequenzen, die nicht an das Aktivator-Oligonukleotid binden. Diese Sequenzen können zu anderen Zwecken verwendet werden, z.B. zu Bindung an die feste Phase. Diese Sequenzen sind vorzugsweise am 5'-Ende des Polynukleotid-Schwanzes lokalisiert.

In einer weiteren Ausführungsform kann ein erstes Primer-Oligonukleotid charakteristische Markierung umfassen. Beispiele für eine solche Markierung stellen Farbstoffe dar (z.B. FAM, TAMRA, Cy3, Alexa 488 etc.) oder Biotin bzw. andere Gruppen, die spezifisch gebunden werden können, z.B. Digoxigenin.

### Der erste Primer-Bereich des ersten Primer-Oligonukleotides

Die Sequenz-Länge liegt zwischen ca. 3-30 Nukleotiden, vorzugsweise zwischen 5 und 20 Nukleotiden, wobei die Sequenz vorwiegend komplementär zum 3'-Segment eines Stranges der zu amplifizierenden Nukleinsäurekette ist. Im Einzelnen, muss dieser Primer-Bereich in der Lage sein, an das komplementäre 3'-Segment eines zweiten Primer-Verlängerungsproduktes spezifisch zu binden. Dieser erste Bereich soll bei Rücksynthese kopiert werden können und dient auch als Matrize für 2. Strang. Die Nukleotid-Bausteine sind vorzugsweise untereinander via übliche 5'-3' Phosphodieser-Bindung oder Phosphothioester-Bindung verknüpft.

Der erste Primer-Bereich schließt bevorzugt Nukleotid-Monomere ein, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- Modifizierte Nukleotide, 2-Amino-dA, 2-thio-dT oder andere Nukleotid-Modifikationen mit abweichender Basen-Paarung.

In einer bevorzugten Ausführungsform ist das 3'-OH-Ende dieses Bereichs vorzugsweise frei von Modifikationen und hat eine funktionsfähige 3'-OH Gruppe, welche von Polymerase erkannt werden kann. Der erste Primer-Bereich dient als Initiator der Synthese des ersten Primer-Verlängerungsproduktes in der Amplifikation. In einer weiteren bevorzugten Ausführungsform umfasst der erste Bereich mindestens eine Phosphorothioat-Verbindung, so dass kein Abbau von 3'-Ende des Primern durch 3'-Exonuclease Aktivität von Polymerasen erfolgen kann.

Die Sequenz des ersten Bereichs des ersten Primer-Oligonukleotids und die Sequenz des zweiten Bereichs des Aktivator-Oligonukleotides sind vorzugsweise einander komplementär.

In einer Ausführungsform kann der erste Primer-Bereich oder sein 3'-Segment an Sequenzsegmente einer Zielsequenz binden.

### Der zweite Bereich des ersten Primer-Oligonukleotides

Der zweite Bereich des ersten Primer-Oligonukleotides ist vorzugsweise eine Nukleinsäuresequenz, welche mindestens einen Polynukleotid-Schwanz umfasst, welcher vorzugsweise während der Synthesereaktion von Polymerase unkopiert bleibt und welcher zur Bindung mit dem ersten Bereich des Aktivator-Oligonukleotides fähig ist. Das Segment des zweiten Bereichs, welches überwiegend diese Bindung mit dem Aktivator-Oligonukleotid eingeht, kann als Polynukleotid-Schwanz bezeichnet werden.

Weiterhin muss der zweite Bereich des ersten Primer-Oligonukleotids nicht nur das Aktivator-Oligonukleotides unter Reaktionsbedinungen spezifisch binden, sondern auch beim Vorgang der Strangverdrängung mittels Aktivator-Oligonukleotid mitwirken. Die Struktur des zweiten Bereichs muß folglich für die Herbeiführung einer räumlichen Nähe zwischen dem Aktivator-Oligonukleotid und dem entsprechenden Doppelstrang-Ende (im Einzelnen, dem 3'-Ende des zweiten Primer-Verlängerungsproduktes) geeignet sein.

Die Gestaltung der Struktur des zweiten Bereichs des ersten Primer-Oligonukleotids wird in mehreren Ausführungsformen genauer dargestellt. Dabei werden Anordnung der Oligonukleotid-Segmente und verwendete Modifikationen berücksichtigt, welche zu einem Stopp in der Polymerase-katalysierten Synthese führen.

Die Länge des zweiten Bereichs liegt zwischen 3 und 60, vorzugsweise zwischen 5 und 40, bevorzugt zwischen 6 und 15 Nukleotiden oder ihren Äquivalenten.

Die Sequenz des zweiten Bereichs kann willkürlich gewählt sein. Vorzugsweise ist sie nicht komplementär mit der zu amplifizierenden Nukleinsäure und/oder mit dem zweiten Primer-Oligonukleotid und/oder mit dem ersten Bereich des ersten Primer-Oligonukleotid. Weiterhin enthält sie vorzugsweise keine selbstkomplementären Segmente, wie Hairpins oder Stemmloops.

Die Sequenz des zweiten Bereichs ist vorzugsweise auf die Sequenz des ersten Bereichs des Aktivator-Oligonukleotids abgestimmt, so dass beide Sequenzen unter Reaktionsbedingungen eine Bindung eingehen können. In einer bevorzugten Ausführungsform ist diese Bindung unter Reaktionsbedingungen reversibel: es besteht somit ein Gleichgewicht zwischen aneinander gebundenen Komponenten und nichtgebundenen Komponenten.

Die Sequenz des zweiten Bereichs des ersten Primer-Oligonukleotides ist vorzugsweise dermaßen gewählt, dass die Anzahl von komplementären Basen, welche mit dem ersten Bereich des Aktivator-Oligonukleotid binden können, zwischen 1 und 40, besser zwischen 3 und 20, bevorzugt zwischen 6 und 15 liegt.

Die Funktion des zweiten Bereichs besteht unter anderem in Bindung des Aktivator-Oligonukleotides. In einer Ausführungsform ist diese Bindung vorzugsweise spezifisch, so dass ein zweiter Bereich eines ersten Primer-Oligonukleotides ein spezifisches Aktivator-Oligonukleotid binden kann. In einer anderen Ausführungsform kann ein zweiter Bereich mehr als nur ein Aktivator-Oligonukleotid unter Reaktionsbedinungen binden.

Es besteht im Allgemeinen keine Notwendigkeit in einer perfekten Übereinstimmung in der Sequenz zwischen dem zweiten Bereich des ersten Primer-Oligonukleotides und dem ersten Bereich des Aktivator-Oligonukleotides. Das Maß der Komplementarität zwischen dem zweiten Bereich des ersten Primer-Oligonukleotides und dem ersten Bereich des Aktivator-Oligonukleotides kann zwischen 20 % und 100 %, besser zwischen 50 % und 100 %, bevorzugt zwischen 80 % und 100 % liegen. Die jeweils komplementären Bereiche können unmittelbar aneinander anschließend positioniert sein oder auch nichtkomplementäre sequenz-Segmente dazwischen umfassen.

Der zweite Bereich des ersten Primer-Oligonukleotides kann in einer Ausführungsform mindestens eine Tm-modifizierende Modifikation einschließen. Durch Einführung solcher Modifikationen kann die Stabilität der Bindung zwischen dem zweiten Bereich des ersten Primer-Oligonukleotides und dem ersten Bereich des Aktivator-Oligonukleotides modifiziert werden. Beispielsweise können Tm-erhöhende Modifikationen (Nukleotid-Modifikationen oder nicht-Nukleotid-Modifikationen) verwendet werden, wie LNA-Nukleotide, 2-Amino-Adenosine oder MGB-Modifikationen. Andererseits können auch Tm-Senkende Modifikationen verwendet werden, wie beispielsweise Inosine-Nukleotid. In der Struktur des zweiten Bereichs können auch Linker (z.B. C3, C6, HEG-Linker) intergriert werden.

Für die Strangverdrängung muss das Aktivator-Oligonukleotid in räumliche Nähe des Doppelstrang-Endes der zu amplifizierenden Nukleinsäure gebracht werden. Dieses Doppelstrang-Ende besteht aus Segmenten des ersten Primer-Bereichs des ersten Primer-Verlängerungsproduktes und einem entsprechend komplementären dazu 3'-Segment des zweiten Primer-Verlängerungsproduktes.

Der Polynukleotid-Schwanz bindet vorwiegend komplementär das Aktivator-Oligonukleotides unter Reaktionsbedingungen und bewirkt damit eine vorübergehende Annäherung des zweiten Bereichs des Aktivator-Oligonukleotides und des ersten Bereichs eines verlängerten Primer-Verlängerungsproduktes, so dass eine komplementäre Bindung zwischen diesen Elementen im Rahmen eines Strangverdrängungsvorgangs initiiert werden kann.

In einer Ausführungsform führt die Bindung des Aktivator-Oligonukleotides an den Polynukleotid-Schwanz des ersten Primer-Oligonukleotides unmittelbar zu einem solchen Kontakt. Das bedeutet, dass der Polynukleotid-Schwanz und der erste Primer-Bereich des ersten Primer-Oligonukleotides unmittelbar aneinander gekoppelt sein müssen. Dank einer solchen Anordnung kann nach einer Bindung eines Aktivator-Oligonukleotids im seinem ersten Bereich unmittelbar zu einem Kontakt zwischen komplementären Basen des zweiten Bereichs des Aktivator-Oligonukleotides und zu entsprechenden Basen des ersten Primer-Bereichs kommen, so dass eine Strangverdrängung initiiert werden kann.

In einer weiteren Ausführungsform liegen zwischen Strukturen des Polynukleotid-Schwanzes und dem ersten Primer-Bereich andere Strukturen des zweiten Bereichs des ersten Primer-Oligonukleotides. Nach einer Bindung eines Aktivator-Oligonukleotids an den Polynukleotid-Schwanz ist dieser somit nicht unmittelbar an den ersten Primer-Bereich positioniert, sondern in einer gewissen Distanz dazu. Die Strukturen zwischen dem unkopierbarem Polynukleotid-Schwanz und dem kopierbaren ersten Primer-Bereich des Primer-Oligonukleotides können einen solchen Abstand generieren. Dieser Abstand hat einen Wert, welcher zwischen 0,1 und 20 nm, vorzugsweise zwischen 0,1 und 5 nm, besser zwischen 0,1 und 1 nm liegt.

Solche Strukturen stellen beispielsweise Linker (z.B. C3 oder C6 oder HEG-Linker) oder nicht zu Aktivator-Oligonukleotid komplementäre Segmente dar (z.B. in Form von nichtkomplementären, nicht-kopierbaren Nukleotid-Modifikationen). Die Länge dieser Strukturen kann im Allgemeinen in Ketten-Atomen ausgemessen werden. Diese Länge beträgt zwischen 1 und 200 Atomen, vorzugsweise zwischen 1 und 50 Kettenatomen, bevorzugt zwischen 1 und 10 Kettenatomen.

Damit der Polynukleotid-Schwanz von Polymerase unter Amplifikationsbedinungen unkopierbar bleibt, umfasst der zweite Bereich des ersten Primer-Oligonukleotides im allgemeinen Sequenz-Anordnungen bzw. Strukturen, welche zu einem Stopp der Polymerase in der Synthese des zweiten Primer-Verlängerungsproduktes bewirken, nachdem die Polymerase den ersten Primer-Bereich erfolgreich kopiert hat. Diese Strukturen sollen das Kopieren des Polynukleotid-Schwanzes des zweiten Bereichs verhindern. Der Polynukleotid-Schwanz bleibt somit vorzugsweise von der Polymerase unkopiert.

In einer Ausführungsform liegen solche Strukturen zwischen dem ersten Primer-Bereich und dem Polynukleotid-Schwanz.

In einer weiteren Ausführungsform kann die Sequenz des Polynukleotid-Schwanzes Nukleotid-Modifikationen einschließen, welche zum Stopp der Polymerase führen. Dadurch kann ein Sequenzsegment des zweiten Bereichs des ersten Primer-Oligonukleotids beide Funktionen umfassen: es ist sowohl ein Polynukleotid-Schwanz als auch eine zum Stopp der Polymerase führende Sequenz aus Nukleotid-Modifikationen.

Modifikationen im zweiten Bereich des ersten Primer-Oligonukleotid, welche zu einem Synthese-Stopp führen und somit den Polynukleotid-Schwanz unkopierbar lassen, werden in dieser Anmeldung unter dem Begriff "erste Blockierungs-Einheit oder einen Stopp-Bereich" zusammengefasst.

### Nachfolgend sind weitere Ausführungsformen von Strukturen angeführt, welche zum Stopp in der Synthese des zweiten Stranges führen können.

Mehrere Bausteine bei der Oligonukleotid-Synthese sind bekannt, welche die Polymerase beim Ablesen der Matrize hindern und zur Termination von Polymerase-Synthese führen. Beispielsweise sind nicht-kopierbare Nukleotid-Modifikationen oder Nicht-Nukleotid-Modifikationen bekannt. Es gibt auch Synthese-Arten/Anordnungen von Nukleotid-Monomeren innerhalb eines Oligonukleotides, welche zum Stopp der Polymerase führen (z.B. 5'-5-Anordnung oder 3'-3'Anordnung). Primer-Oligonukleotide mit einem nicht kopierbaren Polynukleotid-Schwanz sind ebenfalls im Stand der Technik bekannt (z.B. Scorpion-Primer-Strukturen bzw. Primer für Bindung an die feste Phase). Beide Primer-Varianten beschreiben Primer-Oligonukleotid-Strukturen, welche in der Lage sind, einerseits die Synthese eines Stranges zu initiieren, so dass eine Primer-Verlängerungsreaktion stattfinden kann. Es resultiert ein erster Strang, welcher im Primer-Verlängerungs-Produkt auch die Primer-Struktur mit Schwanz integriert. Bei der Synthese eines komplementären Stranges zum ersten Primer-Verlängerungs-Produkt, z.B. im Rahmen einer PCR-Reaktion, wird der zweite Strang bis zur "Blockierungs-Einheit/Stopp-Struktur" der Primer-Struktur verlängert. Beide beschriebene Primer-Strukturen sind dermaßen konzipiert, dass der 5'-Anteil des Primer-Oligonukleotides einzelsträngig bleibt und nicht von der Polymerase kopiert wird.

In einer weiteren Ausführungsform umfasst der zweite Bereich des Primer-Oligonukleotides einen Polynukleotid-Schwanz, welche in seiner gesamten Länge eine konventionelle Anordnung von 5'- nach 3'- aufweist und nicht-kopierbare Nukleotid-Modifikationen einschließt. Zu solchen nicht-kopierbaren Nukleotid-Modifikationen zählen beispielsweise 2'-O-Alkyl-RNA Modifikationen, PNA, Morpholino. Diese Modifikationen können unterschiedlich im zweiten Primer-Bereich verteilt sein.

Der Anteil von nicht-kopierbaren Nukleotid-Modifikationen kann im Polynukleotid-Schwanz zwischen 20 % und 100 % liegen, vorzugsweise beträgt er mehr als 50% der Nukleotid-Bausteine. Vorzugsweise liegen diese Nukleotid-Modifikationen im 3'-Segment des zweiten Bereichs und grenzen somit an den ersten Bereich des ersten Primer-Oligonukleotides.

In einer Ausführungsform ist die Sequenz der nicht-kopierbaren Nukleotid-Modifikationen zumindest teilweise komplementär zu der Sequenz im Matrizenstrang, so dass die Primer-Bindung an die Matrize unter Einbeziehung von zumindest einem Teil dieser Nukleotid-Modifikationen stattfindet. In einer weiteren Ausführungsform ist die Sequenz der nicht-kopierbaren Nukleotid-Modifikationen nicht-komplementär zu der Sequenz im Matrizenstrang.

Die nicht-kopierbaren Nukleotid-Modifikationen werden vorzugsweise aneinander kovalent gekoppelt und stellen somit ein Sequenz-Segment im zweiten Bereich dar. Die Länge dieses Segments umfasst zwischen 1 und 40, bevorzugt zwischen 1 und 20 Nukleotid-Modifikationen, bevorzugter zwischen 3 und 10 Nukleotid-Modifikationen.

In einer weiteren Ausführungsform umfasst der zweite Bereich des ersten Primer-Oligonukleotides einen Polynukleotid-Schwanz, welcher in seiner gesamten Länge eine konventionelle Anordnung von 5'-3' aufweist und nicht-kopierbare Nukleotid-Modifikationen einschließt (z.B. 2'-O-Alkyl-Modifikationen) und mindestens einen Nicht-Nukleotid-Linker (z.B. C3, C6, HEG-Linker) einschließt. Ein Nicht-Nukleotid-Linker hat die Funktion, benachbarte Nukleotide oder Nukleotid-Modifikationen kovalent zu verbinden und gleichzeitig die Synthese-Funktion der Polymerase ortspezifisch zu unterbrechen.

Ein solcher Nicht-Nukleotid-Linker soll die Strukturen des Polynukleotid-Schwanzes und des ersten Primer-Bereichs nicht zu weit voneinander entfernen. Vielmehr sollte der Polynukleotid-Schwanz in einer räumlichen Nähe vom ersten Primer-Bereich sein. Unter einem Nicht-Nukleotid-Linker werden Modifikationen zusammengefasst, welche in ihrer Länge nicht länger sind als 200 Ketten-Atome, noch vorteilhafter nicht länger als 50 Ketten-Atome, besonders bevorzugt nicht länger als 10 Ketten-Atome umfassen. Die Minimal-Länge eines solchen Linkers kann ein Atom betragen. Ein Beispiel für solche Nicht-Nukleotid-Linker stellen gerade oder verzweigte Alkyl-Linker mit einer Alkyl-Kette dar, welche mindestens ein Kohlenstoff-Atom einschließt, vorteilhafter mindestens 2 bis 30, bevorzugter 4 bis 18. Solche Linker sind in der Oligonukleotid-Chemie hinreichend bekannt (z.B. C3, C6 oder C12 Linker) und können während einer Festphasen-Synthese von Oligonukleotiden zwischen der Sequenz des Polynukleotid-Schwanzes und der Sequenz des ersten Bereichs des ersten Primer-Oligonukleotides eingeführt werden. Ein anderes Beispiel für solche Nicht-Nukleotid-Linker stellen Lineare oder verzweigte PolyethylenGlykol-Derivate dar. Ein bekanntes Beispiel in der Oligonukleotid-Chemie stellt Hexaethylenglycol (HEG) dar. Ein weiteres Beispiel für solche Nicht-Nukleotid-Linker stellen abasische Modifikationen dar (z.B. THF-Modifikation, als Analog von dRibose).

Wenn eine oder mehrere solche Modifikationen in einen zweiten Bereich integriert sind, können sie eine Polymerase in ihrer Kopier-Funktion während ihrer Synthese des zweiten Primer-Verlängerungsprodukts effektiv stören, so dass stromabwärts gelegene Segmente nach einer solchen Modifikation unkopiert bleiben. Die Anzahl von solchen Modifikationen im zweiten Bereich kann zwischen 1 und 100 liegen, vorzugsweise zwischen 1 und 10, bevorzugt zwischen 1 und 3.

Die Position eines solchen Nicht-Nukleotid-Linker kann am 3'-Ende des zweiten Bereichs liegen und somit den Übergang zum ersten Bereich und dem zweiten Bereich des Primer-Oligonukleotid darstellen.

Die Lage des Nicht-Nukleotid-Linkers im mittleren Segment des zweiten Bereichs kann ebenfalls verwendet werden. Damit wird ein Polynukleotid-Schwanz in mindestens zwei Segmente geteilt. In dieser Ausführungsform schließt das 3'-Segment des Polynukleotid-Schwanzes mindestens eine, besser mehrere, z.B. zwischen 2 und 20, vorzugsweise zwischen 2 und 10 nicht-kopierbaren Nukleotid-Modifikationen ein. Diese nicht-kopierbaren Nukleotid-Modifikationen liegen vorzugsweise am Übergang zwischen dem ersten und dem zweiten Bereich des Primer-Oligonukleotides.

In einer weiteren Ausführungsform umfasst der zweite Bereich des Primer-Oligonukleotides einen Polynukleotid-Schwanz, welcher in seiner gesamten Länge eine Anordnung von 5'-nach 3'- aufweist und zumindest ein Nukleotid-Monomer in einer "umgekehrten" Anordnung von 3'-nach 5'- einschließt und welche am Übergang zwischen dem ersten und dem zweiten Bereich des ersten Primer-Oligonukleotides positioniert sind.

In einer weiteren Ausführungsform umfasst der zweite Bereich des Primer-Oligonukleotides einen Polynukleotid-Schwanz, wobei ein solcher Polynukleotid-Schwanz vollständig aus Nukleotiden besteht, welche in umgekehrten Anordnung direkt an den ersten Bereich des ersten Primer-Oligonukleotides angrenzen, so dass die Kopplung des ersten und des zweiten Bereichs durch 5'-5' Position erfolgt. Ein Vorteil einer solchen Anordnung ist, dass die Polymerase nach einer Kopierung des ersten Bereichs an eine "umgekehrte" Anordnung von Nukleotiden trifft, was typischerweise zu Termination der Synthese an dieser Stelle führt.

Bei einer "umgekehrten" Anordnung von Nukleotiden in der Gesamtlänge des Polynukleotid-Schwanzes soll vorzugsweise das 3'-terminales Nukleotid des Polynukleotid-Schwanzes an seinem 3'-OH-Ende blockiert sein, um Nebenreaktionen vorzubeugen. Alternativ kann auch ein terminales Nukleotid verwendet werden, welches gar keine 3'-OH Gruppe aufweist, z.B. ein Dideoxy-Nukleotid.

In einer solchen Ausführungsform soll natürlich auch die entsprechende Nukleotid-Anordnung im Aktivator-Oligonukleotid angepasst werden. In einem solchen Fall müssen der erste und der zweite Bereich des Aktivator-Oligonukleotids in 3'-3' Anordnung verknüpft werden.

In einer weiteren Ausführungsform umfasst der zweite Bereich des Primer-Oligonukleotides einen Polynukleotid-Schwanz, welche in seiner gesamten Länge eine konventionelle Anordnung von 5'-nach 3'- aufweist und mindestens eine Nukleotid-Modifikation einschließt, welche für die Polymerase keine komplementäre Nukleobase darstellt, wenn die Synthese mit ausschließlich natürlichen dNTP (dATP, dCTP, dGTP, dTTP oder dUTP) durchgeführt wird.

Beispielsweise können Iso-dG bzw. Iso-dC Nukleotid-Modifikationen als einzelne, vorzugsweise aber mehrere (mindestens 2 bis 20) Nukleotid-Modifikationen im zweiten Bereich des ersten Primer-Oligonukleotids integriert sein. Weitere Beispiele für Nukleobasen-Modifikationen stellen verschiedene Modifikationen des erweiterten genetischen Alphabets dar. Solche Nukleotid-Modifikationen unterstützen vorzugsweise keine komplementäre Basen-Paarung mit natürlichen Nukleotiden, so dass eine Polymerase (zumindest theoretisch) kein Nukleotid aus der Reihe (dATP, dCTP, dGTP, dTTP oder dUTP) einbaut. In der Realität kann es dennoch zum rudimentären Einbau kommen, insbesondere bei höheren Konzentrationen von dNTP-Substraten und prolongierten Inkubationszeiten (z.B. 60 min oder länger). Daher sollen bevorzugt mehrere solche Nukleotid-Modifikationen positioniert an benachbarten Stellen zum Einsatz kommen. Der Stopp der Polymerase-Synthese wird durch Fehlen von passenden komplementären Substraten für diese Modifikationen bewirkt. Oligonukleotide mit Iso-dC bzw. Iso-dG können mit Standardverfahren synthetisiert werden und sind von mehreren kommerziellen Anbietern erhältlich (z.B. Trilink-Technologies, Eurogentec, Biomers GmbH). Wahlweise kann auch die Sequenz des ersten Bereichs des Aktivator-Oligonukleotids an die Sequenz eines solchen zweiten Primer-Bereichs angepasst werden. Dabei können komplementäre Nukleobasen des erweiterten genetischen Alphabets in den ersten Bereich des Aktivator-Oligonukleotides entsprechend während der chemischen Synthese intergriert werden. Beispielsweise kann Iso-dG im zweiten Bereich des ersten Primer-Nukleotids integriert sein, sein komplementäres Nukleotid (Iso-dC-5-Me) kann an der passenden Stelle im ersten Bereich des Aktivator-Oligonukleotids platziert werden.

Zusammenfassend, kann die Termination der Synthese von Polymerase im zweiten Bereich auf verschiedene Art und Weise erreicht werden. Diese Blockade erfolgt bevorzugt allerdings erst, wenn Polymerase den ersten Bereich des ersten Primer-Oligonukleotides kopiert hat. Damit wird sichergestellt, dass ein zweites Primer-Verlängerungsprodukt eine passende Primer-Bindungsstelle in seinem 3'-Segment besitzt. Diese Primer-Bindungsstelle wird im Rahmen der Strangverdrängung freigelegt und steht somit für eine erneute Bindung eines weiteren ersten Primer-Oligonukleotids zur Verfügung.

Bei der Synthese des komplementären Stranges zum ersten Primer-Verlängerungsprodukt bleibt die Primer-Verlängerungsreaktion vor dem Polynukleotid-Schwanz stehen. Da dieser Polynukleotid-Schwanz für die Interaktion mit dem Aktivator-Oligonukleotid dadurch einzelsträngig bleibt und somit für Bindung zur Verfügung steht, unterstützt dieser die Initiierung der Strangverdrängungs-Reaktion durch das Aktivator-Oligonukleotid, indem er den entsprechenden komplementäre Segmente des Aktivator-Oligonukleotids in unmittelbare Nähe des passenden Duplex-Endes bringt. Der Abstand zwischen dem komplementären Teil des Aktivator-Oligonukleotides (zweiter Bereich) und dem komplementären Teil des verlängerten Primer-Oligonukleotid (erster Bereich) wird dadurch auf ein Minimum reduziert. Solche räumliche Nähe erleichtert die Initiierung der Strangverdrängung.

Im Rahmen einer schematischen Darstellung einer nukleinsäurevermittelten Strangverdrängungs-Reaktion liegt nun eine komplementäre Sequenz von Aktivator-Oligonukleotid unmittelbar in der Nähe des passenden Duplex-Endes. Dabei kommt es zu Konkurrenz um die Bindung an den ersten Bereich des ersten Primer-Oligonukleotids zwischen dem Strang des Aktivator-Oligonukleotides und dem zum Primer komplementären Matrizenstrang. Durch repetetive Schließung und Formierung von Basenpaarung zwischen dem Primer-Bereich und dem komplementären Segment des Aktivator-Oligonukleotids (zweiter Bereich des Aktivator-Nukleotides) bzw. dem komplementären Segment des Matrizenstranges kommt es zur Initiierung des nukleinsäurevermittelten Strangverdrängungs-Vorgangs.

Im Allgemeinen ist die Ausbeute der Initiierung der Stang-Verdrängung ist umso höher, je näher der entsprechende komplementäre Sequenzabschnitt des Aktivator-Oligonukleotides zum komplementären Segment des Primer-Bereichs liegt. Bei Vergrößerung dieses Abstandes sinkt dagegen die Ausbeute der Initiierung der Strangverdrängung.

Im Rahmen der vorliegenden Erfindung ist es nicht zwingend notwendig, dass die Initiierung der Strangverdrängung mit maximaler Ausbeute funktioniert. Daher können Abstände zwischen dem 5'-Segment des ersten Primer-Bereichs des ersten Primer-Oligonukleotid, welcher eine Bindung mit einem komplementären Strang der Matrize eingeht und eine komplementäre Duplex bildet, und einem entsprechend komplementären Sequenzabschnitt im Aktivator-Oligonukleotides, wenn gebunden an Polynukleotid-Schwanz des zweiten Bereichs des ersten Primer-Oligonukleotides in folgenden Bereichen liegen: zwischen 0,1 und 20 nm, besser zwischen 0,1 und 5 nm, noch besser zwischen 0,1 und 1 nm. Im bevorzugten Fall beträgt diese Distanz weniger als 1 nm. In anderen Einheiten ausgedruckt entspricht dieser Abstand einer Strecke von weniger als 200 Atomen, noch besser weniger als 50 Atomen, noch besser weniger als 10 Atomen. Im bevorzugten Fall ist diese Distanz ein Atom. Die Angaben zur Distanz sollen lediglich zur Orientierung dienen und veranschaulichen, dass kürzere Distanzen zwischen diesen Strukturen bevorzugt werden. Die Messung dieser Distanz ist in vielen Fällen nur durch Analyse der genauen Strukturen von Oligonukleotiden und Ausmessung der Messung von Sequenzabständen bzw. von Linker-Längen möglich.

Der erste Primer kann auch noch weitere Sequenzabschnitte umfassen, welche nicht zu Interaktion mit Aktivator-Oligonukleotid oder Matrizen Strang benötigt werden. Solche Sequenzabschnitte können beispielsweise weitere Oligonukleotide binden, welche als Detektionssonden oder Immobilisierungspartner bei einer Bindung an die feste Phase verwendet werden.

### Primer-Funktion des ersten Primer-Oligonukleotides

Das erste Primer-Oligonukleotid kann in mehreren Teil-Schritten verwendet werden. In erster Linie übernimmt es eine Primer-Funktion in der Amplifikation. Dabei wird Primer-Verlängerungsreaktion unter Verwendung des zweiten Primer-Verlängerungsproduktes als Matrize ausgeführt. In einer weiteren Ausführungsform kann das erste Primer-Oligonukleotid zu Beginn der Amplifikationsreaktion die Start-Nukleinsäurekette als Matrize verwenden. In einer weiteren Ausführungsform kann das erste Primer-Oligonukleotid bei der Erstellung / Bereitstellung einer Start-Nukleinsäurekette verwendet werden.

Im Rahmen der Amplifikation dient der erste Primer als Initiator der Synthese des ersten Primer-Verlängerungsproduktes unter Verwendung des zweiten Primer-Verlängerungsproduktes als Matrize. Das 3'-Segment des ersten Primers umfasst eine Sequenz, welche vorwiegend komplementär an das zweite Primer-Verlängerungsprodukt binden kann. Die enzymatische Verlängerung des ersten Primer-Oligonukleotid unter Verwendung des zweiten Primer-Verlängerungsproduktes als Matrize führt zu Ausbildung des ersten Primer-Verlängerungsproduktes. Ein solches erstes Primer-Verlängerungsprodukt umfasst die Zielsequenz bzw. ihre Sequenz-Anteile. Im Verlauf der Synthese des zweiten Primer-Verlängerungsproduktes wird die Sequenz des kopierbaren Anteils des ersten Primer-Oligonukleotid von Polymerase als Matrize erkannt und eine entsprechende komplementäre Sequenz synthetisiert wird, so dass eine entsprechende Primer-Bindungsstelle für das erste Primer-Oligonukleotid resultiert. Die Synthese des ersten Primer-Verlängerungsproduktes erfolgt bis einschließliech 5'-Segment des zweiten Primer-Oligonukleotids. Unmittelbar nach der Synthese des ersten Primer-Verlängerungsproduktes ist dieses Produkt an das zweite Primer-Verlängerungsprodukt gebunden und bildet doppelsträngigen Komplex. Das zweite Primer-Verlängerungsprodukt wird aus diesem Komplex sequenzspezifisch durch das Aktivator-Oligonukleotid verdrängt. Dabei bindet das Aktivator-Oligonukleotid an das erste Primer-Verlängerungsprodukt. Nach einer erfolgreichen Strangverdrängung durch Aktivator-Oligonukleotid kann das zweite Primer-Verlängerungsprodukt wiederum selbst als Matrize für die Synthese des ersten Primer-Verlängerungsproduktes dienen. Das nun frei gewordene 3'-Segment des ersten Primer-Verlängerungsproduktes kann ein weiteres zweites Primer-Oligonukleotid binden, so dass eine neue Synthese des zweiten Primer-Verlängerungsproduktes initiiert werden kann.

Weiterhin kann das erste Primer-Oligonukleotid als Initiator der Synthese des ersten Primer-Verlängerungsproduktes ausgehend von Start-Nukleinsäurekette zu Begin der Amplifikation dienen. In einer Ausführungsform ist die Sequenz des ersten Primers vollständig komplementär zum entsprechenden Sequenzsegment einer Start-Nukleinsäurekette. In einer weiteren Ausführungsform ist die Sequenz des ersten Primer-Oligonukleotides nur teilweise komplementäre zum entsprechenden Sequenzsegment einer Start-Nukleinsäurekette. Diese abweichende Komplementarität soll das erste Primer-Oligonukleotid allerdings nicht daran hindern, eine vorwiegend sequenzspezifische Primer-Verlängerungsreaktion zu starten. Die jeweiligen Unterschiede in Komplementarität des ersten Primer-Oligonukleotides zur jeweiligen Position in der Start-Nukleinsäurekette liegen vorzugsweise im 5'-Segment des ersten Bereichs des ersten Primer-Oligonukleotides, so dass im 3'-Segment vorwiegend komplementäre Basenpaarung und Initiierung der Synthese möglich ist. Für die Initiierung der Synthese sollen beispielsweise besonders die ersten 4 - 10 Positionen im 3'-Segment vollkomplementär zu Matrize (Start-Nukleinsäurekette) sein. Die restlichen Nukleotidpositionen können von perfekten Komplementarität abweichen. Das Ausmaß einer perfekten Komplementarität im restlichen 5'-Segment des ersten Bereichs des ersten Primer-Oligonukleotides kann somit Bereiche umfassen zwischen 50% bis 100%, besser zwischen 80% und 100% der Basenzzusammensetzung. Je nach Länge des ersten Bereichs des ersten Primer-Oligonukleotides, umfassend die Sequenz-Abweichungen von 1 bis maximal 15 Positionen, besser 1 bis maximal 5 Positionen. Das erste Primer-Oligonukleotid kann somit eine Synthese von einer Start-Nukleinsäukette initiieren. Bei einer darauffolgenden Synthese des zweiten Primer-Verlängerungsproduktes werden kopierbare Sequenzabschnitte des ersten Primer-Oligonukleotides von Polymerase kopiert, so dass wiederum in darauf folgenden Synthese-Zyklen eine vollkomplementäre Primer-Bindungsstelle innerhalb des zweiten Primer-Verlängerungsprodukte für die Bindung des ersten Primer-Oligonukleotides ausgebildet wird und in darauf folgenden Synthese-Zyklen zur Verfügung steht.

In einer weiteren Ausführungsform kann das erste Primer-Oligonukleotid im Rahmen der Vorbereitung einer Start-Nukleinsäurekette verwendet werden. Dabei kann ein solches erste Primer-Oligonukleotid an eine Nukleinsäure (z.B. eine einzelsträngige genomische DNA oder RNA oder ihre Äquivalente umfassend eine Zielsequenz) vorwiegend / bevorzugt sequenzspezifisch binden und in Anwesenheit einer Polymerase eine matrizenabhängige Primer-Verlängerungsreaktion initiieren. Die Bindungsposition ist dermassen gewählt, dass das Primer-Verlängerungsprodukt eine gewünschte Zielsequenz umfasst. Durch die Verlängerung des ersten Primer-Oligonukleotides resultiert ein Nukleinsäure-Strang, welcher zu Matrize komplementäre Sequenz aufweist. Ein solcher Strang kann von der Matrize abgelöst werden (z.B. durch Hitze oder Alkalie) und damit in eine einzelsträngige Form überführt werden. Eine solche einzelsträngige Nukleinsäurekette kann als Start-Nukleinsäurekette zu Beginn der Amplifikation dienen. Eine solche Start-Nukleinsäurekette umfasst in ihrem 5'-Segment die Sequenzanteile des ersten Primer-Oligonukleotides, weiterhin umfasst sie eine Zielsequenz oder ihre Äquivalente und eine Primer-Bindungsstelle für das zweite Primer-Oligonukleotid. Weitere Schritte sind im Abschnitt "Start-Nukleinsäurekette" erläutert.

Die Synthese des ersten Primer-Verlängerungsproduktes ist eine Primer-Verlängerungsreaktion und bildet einen Teilschritt in der Amplifikation. Die Reaktionsbedinungen während dieses Schrittes sind entsprechend angepasst. Die Reaktions-Temperatur und die Reaktions-Zeit sind dermassen gewählt, dass die Reaktion erfolgreich stattfinden kann. Die jeweils bevorzugte Temperatur in diesem Schritt hängt von der verwendeten Polymerase ab und der Bindungsstärke des jeweiligen ersten Primer-Oligonukleotides an seine Primer-Bindungsstelle und umfasst beispielsweise Bereiche von 15°C bis 75°C, besser von 20 bis 65°C, bevorzugt von 25°C bis 65°C. Die Konzentration des ersten Primer-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

In einer Ausführungsform verlaufen alle Schritte der Amplifikation unter stringenten Bedinungen, welche Ausbildung von unspezifischen Produkten / Nebenprodukten verhindern bzw. verlangsamen. Zu solchen Bedinungen zählen beispielsweise höhere Temperaturen, beispielsweise über 50°C.

Falls mehr als eine spezifische Nukleinsäurekette in einem Ansatz amplifiziert werden müssen, werden in einer Ausführungsform bevorzugt jeweils sequenzspezifische Primer-Oligonukleotide für Amplifikation von entsprechenden jeweiligen Zielsequenzen verwendet.

Vorzugsweise sind Sequenzen des ersten, des zweiten Primer-Oligonukleotides und des Aktivator-Oligonukleotides dermassen aneinander angepasst, dass Nebenreaktionen, z.B. Primer-Dimer-Ausbildung, minimiert sind. Zu diesem Zweck sind beispielsweise die Sequenz des ersten und des zweiten Primer-Oligonukleotides aneinander demassen angepasst, daß beide Primer-Oligonukleotide nicht in der Lage sind, eine Amplifikations-Reaktion in Abwesenheit einer passenden Matrize und/oder einer Zielsequenz und/oder einer Start-Nukleinsäurekette zu starten bzw. zu unterstützen. Das kann beispielsweise dadurch erreicht werden, dass das zweite Primer-Oligonukleotid keine Primer-Bindungsstelle für das erste Primer-Oligonukleotid umfasst und das erste Primer-Oligonukleotid keine Primer-Bindungsstelle für das zweite Primer-Oligonukleotid umfasst. Weiterhin soll vermieden werden, dass die Primer-Sequenzen ausgedehnte eigen-komplementäre Strukturen (Self-complement) umfassen.

In einer Ausführungsform verläuft die Synthese des ersten und des zweiten Primer-Verlängerungsproduktes bei gleicher Temperatur. In einer weiteren Ausführungsform verläuft die Synthese des ersten und des zweiten Primer-Verlängerungsproduktes bei unterschiedlichen Temperaturen. In einer weiteren Ausführungsform verläuft die Synthese des ersten Primer-Verlängerungsproduktes und Strangverdrängung durch Aktivator-Oligonukleotid bei gleicher Temperatur. In einer weiteren Ausführungsform verläuft die Synthese des ersten Primer-Verlängerungsproduktes und Strangverdrängung durch Aktivator-Oligonukleotid bei unterschiedlichen Temperaturen.

### Bevorzugte Ausführungsformen des Aktivator-Oligonukleotids

Ein Aktivator-Oligonukleotid (Fig. 10) umfasst:
- Einen ersten einzelsträngigen Bereich, welcher an den Polynukleotid-Schwanz des zweiten Bereichs des ersten Primer-Oligonukleotides binden kann,
- einen zweiten einzelsträngigen Bereich, welcher an den ersten Bereich des ersten Primer-Oligonukleotides im wesentlich komplementär binden kann,
- einen dritten einzelsträngigen Bereich, welcher zumindest zu einem Segment des Verlängerungsprodukts des ersten Primer-Verlängerungsprodukts im Wesentlichen komplementär ist,
und das Aktivator-Oligonukleotid dient nicht als Matrize für Primerverlängerung des ersten oder des zweiten Primer-Oligonukleotids.

Im Allgemeinen wird die Sequenz des dritten Bereichs des Aktivator-Oligonukleotides an die Sequenz der zu amplifizierenden Nukleinsäure angepasst, da diese als Matrize für die Reihenfolge der Nukleotide im Verlängerungsprodukt eines ersten Primers maßgeblich ist. Die Sequenz des zweiten Bereichs von Aktivator-Oligonukleotid wird an die Sequenz des ersten Primer-Bereiches angepasst. Die Struktur des ersten Bereichs des Aktivator-Oligonukleotides wird an die Sequenz des zweiten Bereichs des ersten Primer-Oligonukleotides angepasst, vor allem an die Beschaffenheit des Polynukleotid-Schwanzes.

Ein Aktivator-Oligonukleotid kann auch weitere Sequenz-Segmente einschließen, welche nicht zum ersten, zweiten oder dritten Bereich gehören. Diese Sequenzen können beispielsweise als flankierende Sequenzen am 3'- und am 5'-Ende angebracht werden. Vorzugsweise stören diese Sequenzsegmente die Funktion des Aktivator-Oligonukleotids nicht.

Die Struktur des Aktivator-Oligonukleotids weist bevorzugt folgende Eigenschaften auf:
Die einzelnen Bereiche sind untereinander kovalent gebunden. Die Bindung kann beispielsweise via konventionelle 5'-3' Bindung erfolgen. Es kann beispielsweise eine Phospho-Diesterbindung oder eine Nuklease-resistente Phospho-Thioester Bindung verwendet werden.

Ein Aktivator-Oligonukleotid kann mittels seines ersten Bereichs an den Polynukleotid-Schwanz des ersten Primer-Oligonukleotides binden, wobei die Bindung vorwiegend durch Hybridisierung komplementärer Basen vermittelt wird. Die Länge dieses ersten Bereichs beträgt 3 - 80 Nukleotide, vorzugsweise 4 - 40 Nukleotide, besonders bevorzugt 6 - 20 Nukleotide. Das Ausmaß an Sequenzübereinstimmung zwischen der Sequenz des ersten Bereichs des Aktivator-Oligonukleotids und der Sequenz des zweiten Bereichs des ersten Primer-Oligonukleotide kann zwischen 20 % und 100 %, vorzugsweise zwischen 50 % und 100 %, besonders bevorzugt zwischen 80 % und 100 % liegen. Die Bindung des ersten Bereichs des Aktivator-Oligonukleotids sollte vorzugsweise spezifisch an den zweiten Bereich des ersten Primer-Oligonukleotid unter Reaktionsbedingungen erfolgen.

Die Sequenz des ersten Bereichs des Aktivator-Oligonukleotids ist vorzugsweise dermaßen gewählt, dass die Anzahl von komplementären Basen, welche mit dem zweiten Bereich des ersten Primer-Oligonukleotids komplementär binden können, zwischen 1 und 40, besser zwischen 3 und 20, bevorzugt zwischen 6 und 15 liegt.

Da das Aktivator-Oligonukleotid keine Matrize für Polymerase darstellt, kann es Nukleotid-Modifikationen einschließen, welche die Polymerase-Funktion nicht unterstützen, welche sowohl Basenmodifikationen und / oder Zucker-Phosphat-Rückgrad-Modifikationen sein können. Das Aktivator-Oligonukleotid kann beispielsweise in seinem ersten Bereich Nukleotide und/oder Nukleotid-Modifikationen einschließen, welche aus der folgenden Liste ausgewählt sind: DNA, RNA, LNA ("locked nucleic acids" Analoga mit 2'-4' Brückenbindung im Zuckerrest), UNA ("unlocked nucleid acids" ohne Bindung zwischen 2'-3'-Atomen des Zuckerrestes), PNA ("peptide nucleic acids" Analoga), PTO (phosphorothioate), Morpholino-Analoga, 2'-O-Alkyl-RNA Modifikationen (wie 2'-OMe, 2'-O Propargyl, 2'-O-(2-Methoxyethyl), 2'-O-Propyl-Amin), 2'-Halogen-RNA, 2'-Amino-RNA etc. Diese Nukleotide oder Nukleotid-Modifikationen sind untereinander beispielsweise durch konventionelle 5'-3'-Bindung oder 5'-2' Bindung verknüpft. Es kann beispielsweise eine Phospho-Diesterbindung oder eine nuklease resistente Phospho-Thioester Bindung verwendet werden.

Das Aktivator-Oligonukleotid kann in seinem ersten Bereich Nukleotide und/oder Nukleotid-Modifikationen einschließen, wobei die Nukleobasen aus der folgenden Liste ausgewählt sind: Adenine und seine Analoga, Guanine und seine Analoga, Cytosine und seine Analoga, Uracil und seine Analoga, Thymine und seine Analoga, Inosine oder andere Universalbasen (z.B. Nitroindol), 2-Amino-Adenin und seine Analoga, Iso-Cytosine und seine Analoga, Iso-Guanine und seine Analoga.

Das Aktivator-Oligonukleotid kann in seinem ersten Bereich Nicht-Nukleotid-Verbindungen einschließen, welche aus der folgenden Liste ausgewählt sind: Interkallierende Stoffe, welche Bindungsstärke zwischen dem Aktivator-Oligonukleotid und dem ersten Primer-Oligonukleotid beeinflussen können, z.B. MGB, Naphthalene etc. Gleiche Elemente können auch im zweiten Bereich des ersten Primers verwendet werden.

Das Aktivator-Oligonukleotid kann in seinem ersten Bereich Nicht-Nukleotid-Verbindungen einschließen, z.B. Linker wie C3, C6, HEG Linker, welche einzelne Segmente des ersten Bereichs untereinander verknüpfen.

Das Aktivator-Oligonukleotid kann mittels seines zweiten Bereichs an den ersten Primer-Bereich des ersten Primer-Oligonukleotids binden, wobei die Bindung im Wesentlichen durch Hybridisierung komplementärer Basen vermittelt wird.

Die Länge des zweiten Bereichs des Aktivator-Oligonukleotids ist an die Länge des ersten Bereichs des ersten Primer-Oligonukleotid angepasst und stimmt mit dieser vorzugsweise überein. Sie liegt zwischen ca. 3-30 Nukleotiden, vorzugsweise zwischen 5 und 20 Nukleotiden. Die Sequenz des zweiten Bereichs von Aktivator-Oligonukleotid ist vorzugsweise komplementär zum ersten Bereich des ersten Primer-Oligonukleotid. Das Maß der Übereinstimmung in Komplementarität liegt zwischen 80 % und 100 %, vorzugsweise zwischen 95 % und 100 %, bevorzugt bei 100 %. Der zweite Bereich von Aktivator-Oligonukleotid schließt vorzugsweise Nukleotid-Modifikationen ein, welche Polymerase bei Verlängerung des ersten Primer-Oligonukleotides verhindern allerdings Ausbildung von komplementären Doppelsträngen nicht blockieren oder nicht wesentlich verhindern, beispielsweise 2'-O-Alkyl RNA-Analoga (z.B. 2'-O-Me, 2'-O-(2-Methoxyethyl), 2'-O-Propyl, 2-O-Propargyl Nukleotid-Modifikationen), LNA, PNA oder Morpholino Nukleotid-Modifikationen. Einzelne Nukleotid-Monomere sind vorzugsweise via 5'-3'-Bindung verknüpft, aber auch alternative 5'-2'-Bindung zwischen Nukleotid-Monomeren kann verwendet werden.

Die Sequenz-Länge und ihre Beschaffenheit des ersten und des zweiten Bereichs des Aktivator-Oligonukleotides sind vorzugsweise dermassen gewählt, dass die Bindung dieser Bereiche an das erste Primer-Oligonukleotid unter Reaktionsbedingungen zumindestens in einem Reaktions-Schritt des Verfahrens reversibel ist. Das bedeutet, dass Aktivator-Oligonukleotid und das erste Primer-Oligonukleotid zwar spezifisch an einander binden können, diese Bindung soll allerdings nicht zu Ausbildung eines unter Reaktionsbedingungen dauerhaft stabilen Komplexes aus beiden Elementen führen. Vielmehr soll ein Gleichgewicht zwischen einer gebunden Komplex-Form aus Aktivator-Oligonukleotid und dem ersten Primer-Oligonukleotid und einer freien Form von einzelnen Komponenten unter Reaktionsbedingungen zumindest in einem Reaktions-Schritt angestrebt bzw. ermöglicht werden. Damit wird sichergestellt, dass zumindest ein Anteil der ersten Primer-Oligonukleotide unter Reaktionsbedingungen in freier Form vorliegt und mit der Matrize interagieren kann, um eine Primer-Verlängerungsreaktion zu initiieren. Andererseits, wird damit sichergestellt werden, dass entsprechende Sequenzbereiche des Aktivator-Oligonukleotides zu Bindung mit einem verlängerten Primer-Oligonukleotid zur Verfügung stehen.

Durch Temperatur-Wahl während der Reaktion kann der Anteil von freien, einzelsträngigen und somit reaktionsfähigen Komponenten beeinflusst werden: durch Absenkung der Temperatur binden erste Primer-Oligonukleotide an die Aktivator-Oligonukleotide, so dass beide Teilnehmer einen komplementären doppelsträngigen Komplex binden. Damit kann Konzentration einzelsträngiger Formen einzelner Komponenten abgesenkt werden. Eine Erhöhung der Temperatur kann zu Dissoziation beider Komponenten in einzelsträngige Form führen. Im Bereich der Schmelz-Temperatur des Komplexes (Aktivator-Oligonukleotid / erstes Primer-Oligonukleotid) liegen ca. 50% der Komponenten in einzelsträngiger Form und ca. 50% als doppelsträngiger Komplex vor. Durch Verwendung entsprechender Temperaturen kann somit die Konzentration einzelsträngiger Formen im Reaktionsgemisch beeinflusst werden.

Bei Ausführungsformen des Amplifikationsverfahrens, welche Temperatur-Wechsel zwischen einzelnen Reaktions-Schritten umfassen, können gewünschte Reaktionsbedingungen während entsprechender Reaktions-Schritte herbeigeführt werden. Beispielsweise durch Verwendung von Temperatur-Bereichen etwa in Höhe von Schmelztemperatur von Komplexen aus Aktivator-Oligonukleotid / erstes Primer-Oligonukleotid können Anteile von jeweils freien Formen einzelner Komponenten beeinflusst werden. Dabei führt verwendete Temperatur zu einer Destabilisierung von Komplexen umfassend Aktivator-Oligonukleotid / erster Primer-Oligonukleotid, so dass während dieses Reaktionsschrittes einzelne Komplex-Komponente zumindest vorübergehend einzelsträngig werden und damit in die Lage versetzt werden, mit anderen Reaktionspartnern zu interagieren. Beispielsweise kann erster Sequenzbereich des Aktivator-Oligonukleotides aus dem doppelsträngigen Komplex mit einem nichtverlängerten ersten Primer freigesetzt werden und kann somit mit dem zweiten Sequenzbereich eines verlängerten ersten Primer-Oligonukleotides interagieren und damit eine Strangverdrängung initiieren. Andererseits, führt Freisetzung eines ersten, nicht verlängerten Primer-Oligonukleotides aus einem Komplex umfassend Aktivator-Oligonukleotid / erster Primer-Oligonukleotid dazu, dass der erster Primer-Bereich einzelsträngig wird und somit mit der Matrize interagieren kann, so dass eine PrimerVerlängerung durch eine Polymerase initiiert werden kann.

Die verwendete Temperatur muss dabei nicht exakt der Schmelztemperatur des Komplexes aus Aktivator-Oligonukleotid / erstes Primer-Oligonukleotid entsprechen. Es genügt, wenn Temperatur in einem Reaktionsschritt etwa im Bereich der Schmelztemperatur verwendet wird. Beispielsweise umfasst die Temperatur in einem der Reaktionsschritte Bereiche von Tm +/- 10°C, besser Tm +/- 5°C, bevorzugt Tm +/- 3°C des Komplexes aus Aktivator-Oligonukleotid / erstes Primer-Oligonukleotid.

Eine solche Temperatur kann beispielsweise im Rahmen des Reaktions-Schrittes eingestellt werden, welcher eine sequenzspezifische Strandverdrängung durch das Aktivator-Oligonukleotid umfasst.

Bei Ausführungsformen des Amplifikationsverfahrens, welche keinen Temperatur-Wechsel zwischen einzelnen Reaktions-Schritten umfassen und Amplifikation unter isothermalen Bedinungen verläuft, werden über die Gesamtdauer der Amplifikationsreaktion Reaktionsbedinungen aufrechterhalten, bei welchen ein Gleichgewicht zwischen einer Komplex-Form aus Aktivator-Oligonukleotid und dem ersten Primer-Oligonukleotid und einer freien Form von einzelnen Komponenten möglich ist.

Das Verhältnis zwischen einer Komplex-Form aus Aktivator-Oligonukleotid und dem ersten Primer-Oligonukleotid und freien Formen einzelner Komponenten kann sowohl durch Reaktionsbedinungen (z.B. Temperatur und Mg2+ Konzentration) als auch durch Strukturen und Konzentrationen einzelner Komponenten beeinflusst werden.

Die Sequenz-Länge und ihre Beschaffenheit des ersten und des zweiten Bereichs des Aktivator-Oligonukleotides sind in einer Ausführungsform so gewählt, dass unter gegebenen Reaktionsbedinungen (z.B. im Reaktionsschritt einer Strangverdängung durch das Aktivator-Oligonukleotid) das Verhältnis zwischen einem Anteil eines freien Aktivator-Oligonukleotids und einem Anteil eines Aktivator-Oligonukleotid im Komplex mit einem ersten Primer-Oligonukleotid folgende Bereiche umfasst: von 1:100 bis 100:1, vorzugsweise von 1:30 bis 30:1, besonders bevorzugt von 1:10 bis 10:1. Das Verhältnis zwischen einem Anteil eines freien ersten Primer-Oligonukleotid und einem Anteil eines ersten Primer-Oligonukleotids im Komplex mit einem Aktivator-Oligonukleotid umfasst Bereiche von 1:100 bis 100:1, vorzugsweise von 1:30 bis 30:1, besonders bevorzugt von 1:10 bis 10:1.

In einer Ausführungsform ist die Konzentration des ersten Primer-Oligonukleotides höher als die Konzentration des Aktivator-Oligonukleotides. Dadurch liegt ein Überschuß des ersten Primers in der Reaktion vor und Aktivator-Oligonukleotid muss für seine Wirkung aus der Bindung mit dem ersten Primer durch Wahl entsprechender Reaktionstemperatur freigesetzt werden. Im Allgemeinen erfolgt das durch eine Temperaturerhöhung, bis hinreichende Konzentrationen von freien Formen des Aktivator-Oligonukleotids vorliegen.

In einer weiteren Ausführungsform ist die Konzentration des ersten Primer-Oligonukleotides niedriger als die Konzentration des Aktivator-Oligonukleotides. Dadurch liegt ein Überschuss des Aktivator-Oligonukleotids vor und das erste Primer-Oligonukleotid muss für seine Wirkung aus der Bindung mit dem Aktivator-Oligonukleotid durch Wahl entsprechender Reaktionstemperatur freigesetzt werden. Im Allgemeinen erfolgt das durch eine Temperaturerhöhung, bis hinreichende Konzentrationen von freien Formen des ersten Primer-Oligonukleotids vorliegen.

Bei isothermalen Bedingungen liegt ein Gleichgewicht vor: gewisse Anteile des ersten Primer-Oligonukleotides und Aktivator-Oligonukleotides sind aneinander gebunden, andere dagegen sind als einzelsträngige Form in der Reaktion vorhanden.

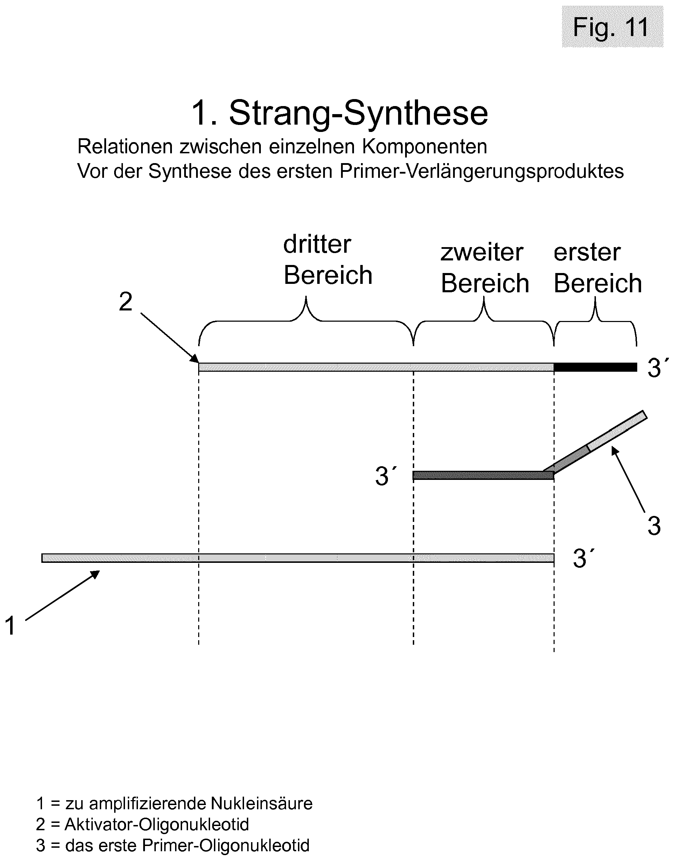

Das Aktivator-Oligonukleotid kann mittels seines dritten Bereichs an mindestens ein Segment des spezifisch synthetisierten Verlängerungsprodukts des ersten Primer-Oligonukleotids binden (Fig. 11 bis 21). Die Bindung erfolgt bevorzugt durch Hybridisierung komplementärer Basen zwischen dem Aktivator-Oligonukleotid und dem von Polymerase synthetisierten Verlängerungsprodukt.

Zu Unterstützung der Strangverdrängungsreaktion soll die Sequenz des dritten Bereichs vorzugsweise eine hohe Komplementarität zum Verlängerungsprodukt aufweisen. In einer Ausführungsform ist die Sequenz des dritten Bereichs zu 100 % dem Verlängerungsprodukt komplementär.

Die Bindung des dritten Bereichs erfolgt vorzugsweise an das Segment des Verlängerungsproduktes, welches unmittelbar an den ersten Bereich des ersten Primer-Oligonukleotides anschließt. Somit liegt das Segment des Verlängerungsprodukts vorzugsweise im 5'-Segment des gesamten Verlängerungsprodukts des ersten Primer-Oligonukleotids.

Die Bindung des dritten Bereichs des Aktivator-Oligonukleotids erfolgt vorzugsweise nicht über die gesamte Länge des Verlängerungsprodukts des ersten Primer-Oligonukleotides. Vorzugsweise bleibt ein Segment am 3'-Ende des Verlängerungsproduktes ungebunden. Dieses 3'-ständige Segment ist für die Bindung des zweiten Primer-Oligonukleotides notwendig.

Die Länge des dritten Bereichs ist entsprechend dermassen angepasst, dass der dritte Bereich zum einen an das 5'-ständige Segment des Verlängerungsproduktes bindet, andererseits das 3'-ständige Segment des Verlängerungsproduktes nicht bindet.

Die Gesamtlänge des dritten Bereichs des Aktivator-Oligonukleotids beträgt von 2 bis 100, vorzugsweise von 6 bis 60, besonders bevorzugt von 10 bis 40 Nukleotide oder ihrer Äquivalente. Das Aktivator-Oligonukleotid kann mit dem Segment des Verlängerungsproduktes über diese Länge komplementäre Bindung eingehen und damit dieses 5'-ständige Segment des Verlängerungsprodukts aus der Bindung mit seinem komplementären Matrizen-Strang verdrängen.

Die Länge des 3'-ständigen Segments des Verlängerungsproduktes, welches nicht vom Aktivator-Oligonukleotid gebunden wird, umfasst beispielsweise Bereiche zwischen 5 und 60 Nukleotide, vorzugsweise zwischen 10 und 40, bevorzugt zwischen 15 und 30 Nukleotide.

Dieses 3'-ständige Segment des Verlängerungsprodukts wird nicht vom Aktivator-Oligonukletid aus der Bindung mit dem Matrizenstrang verdrängt. Auch bei vollständig gebundenem dritten Bereich des Aktivator-Oligonukleotids an sein komplementäres Segment des Verlängerungsproduktes kann das erste Primer-Verlängerungsprodukt via sein 3'-ständiges Segment mit dem Matrizenstrang gebunden bleiben. Die Bindungsstärke dieses Komplexes ist vorzugsweise so gewählt, dass er unter Reaktionsbedinungen (Schritte)) beispielsweise spontan dissoziieren kann. Dies kann bespielsweise dadurch erreicht werden, dass die Schmelztemperatur dieses Komplexes aus dem 3'-ständigen Segment des Verlängerungsprodukts des ersten Primer-Oligonukleotid und seinem Matrizenstrang etwa im Bereich der Reaktionstemperatur liegt oder unterhalb der Reaktionstemperatur bei einem entsprechenden Reaktionsschritt (Reaktionsschritt e). Bei geringer Stabilität dieses Komplexes im 3'-Segment des Verlängerungsprodutkes führt eine vollständige Bindung des dritten Bereichs des Aktivator-Oligonukleotid an das 5'-ständige Segment des Verlängerungsproduktes zu einer schnellen Dissoziation der ersten Primer-Verlängerungsprodukts von seinem Matrizenstrang.

Das Aktivator-Oligonukleotid hat insgesamt eine passende Struktur, um seine Funktion auszuführen: unter entsprechenden Reaktionsbedinungen ist es in der Lage, das verlängerte erste Primer-Oligonukleotid aus der Bindung mit dem Matrizenstrang sequenzspezifisch zu verdrängen, wodurch der Matrizenstrang in einzelsträngige Form überführt wird und somit für weitere Bindungen mit einem neuen ersten Primer-Oligonukleotid und deren zielsequenzspezifische Verlängerung durch Polymerase zur Verfügung steht.

Um die Funktion der Strangverdrängung zu erfüllen, sollten Bereiche ein, zwei und drei des Aktivator-Oligonukleotids vorwiegend in einzelsträngiger Form unter Reaktionsbedingungen vorliegen. Daher sollten doppelsträngige selbstkomplementäre Strukturen (z.B. Hairpins) in diesen Bereichen nach Möglichkeit vermieden werden, da sie Funktionalität des Aktivator-Oligonukleotides herabsetzen können.

Das Aktivator-Oligonukleotid soll im erfindungsgemäßen Verfahren nicht als Matrize auftreten, daher soll das erste Primer-Oligonukleotid, wenn angelagert an das Aktivator-Oligonukleotid unter Reaktionsbedingungen, nicht von Polymerase verlängert werden.

Dies wird vorzugsweise durch Verwendung von Nukleotid-Modifikationen erreicht, welche die Polymerase am Kopieren des Stranges hindern. Vorzugsweise bleibt das 3'-Ende des ersten Primer-Oligonukleotid nicht verlängert, wenn das erste Primer-Oligonukleotid an das Aktivator-Oligonukleotid unter Reaktionsbedingungen bindet.

Das Ausmaß der Blockade/Hinderung/Verlangsamung/Erschwerung der Reaktion kann zwischen vollständiger Ausprägung dieser Eigenschaft (z.B. 100 % Blockade unter gegebenen Reaktionsbedingungen) und einer partiellen Ausprägung dieser Eigenschaft liegen (z.B. 30-90 % Blockade unter gegebenen Reaktionsbedingungen). Bevorzugt werden Nukleotid-Modifikationen, welche einzeln oder in einer Reihe aneinander gekoppelt (z.B. als Sequenzfragment bestehend aus modifizierten Nukleotiden)mehr als 70 %, bevorzugt mehr als 90 %, bevorzugter mehr als 95 %, und besonders bevorzugt 100 % die Verlängerung eines ersten Primers verhindern können.

Die Nukleotid-Modifikationen können Basen-Modifikationen und/oder Zucker-Phosphat-Rest Modifikationen umfassen. Die Zucker-Phosphat-Modifikationen sind bevorzugt, da durch Kombination mit konventionellen Nukleobasen eine beliebige komplementäre Sequenz eines Aktivator-Oligonukleotides zusammengestellt werden kann. Die Nukleotide mit Modifikationen im Zucker-Phosphat-Rest, welche zu Hinderung bzw. Blockade der Synthese der Polymerase führen können, schließen beispielsweise ein: 2'-O-Alkyl-Modifikationen (z.B. 2'-O-Methyl, 2'-O-(2-Methoxyethyl), 2'-O-Propyl, 2'-O-Propargyl Nukleotid-Modifikationen), 2'-Amino-2'-Deoxy-Nukleotid-Modifikationen, 2'-Amino-Alkyl-2'-Deoxy-Nukleotid-Modifikationen, PNA, Morpholino-Modifikationen usw.

Die Blockade kann sowohl durch eine einzelne Nukleotid-Modifikation erfolgen oder erst durch Kopplung von mehreren Nukleotid-Modifikationen in Reihe erfolgen (z.B. als Sequenzfragment bestehend aus modifizierten Nukleotiden). Beispielsweise können mindestens 2, vorzugsweise mindestens 5, besonders bevorzugt mindestens 10 solcher Nukleotid-Modifikationen nebeneinander im Aktivator-Oligonukleotid gekoppelt sein.

Ein Aktivator-Oligonukleotid kann eine einheitliche Art von Nukleotid-Modifikationen aufweisen oder auch mindestens zwei verschiedene Arten der Nukleotid-Modifizierung umfassen.

Die Position solcher Nukleotid-Modifikationen im Aktivator-Oligonukleotid soll vorzugsweise die Polymerase daran hindern, das 3'-Ende eines am Aktivator-Oligonukleotid gebundenen ersten Primer-Oligonukleotids zu verlängern.

In einer Ausführungsform sind solche Nukleotid-Modifikationen im zweiten Bereich des Aktivator-Oligonukleotids lokalisiert. In einer weiteren Ausführungsform sind solche Nukleotid-Modifikationen im dritten Bereich des Aktivator-Oligonukleotids lokalisiert. In einer weiteren Ausführungsform sind solche Nukleotid-Modifikationen im zweiten und im dritten Bereich des Aktivator-Oligonukleotids lokalisiert.

Beispielsweise besteht der zweite Bereich des Aktivator-Oligonukleotids zu mindestens 20 % seiner Positionen aus solchen Nukleotid-Modifikationen, vorzugsweise zu mindestens 50 %.

Beispielsweise besteht der dritte Bereich des Aktivator-Oligonukleotids zu mindestens 20 % seiner Positionen aus solchen Nukleotid-Modifikationen, vorzugsweise zu mindestens 50 %.

Die Sequenz der Nukleobasen von diesen Nukleotid-Modifikationen ist den Anforderungen an die Sequenz im jeweiligen Bereich angepasst.

Den restlichen Anteil können beispielsweise natürliche Nukleotide ausmachen, oder Nukleotid-Modifikationen, welche die Polymerasen-Funktion gar nicht oder nur unwesentlich hindern z.B. DNA-Nukleotide, PTO-Nukleotide, LNA-Nukleotide, RNA-Nukleotide. Dabei können weitere Modifikationen, beispielsweise Basenmodifikationen wie 2-Amino-Adenosin, 2-Aminopurine, 5-Methyl-Cytosine, Inosine, 5-Nitroindole, 7-Deaza-Adenosin, 7-Deaza-Guanosin, 5-Propyl-Cytosin, 5-Propyl-Uridin oder Nicht-Nukleotid-Modifikationen wie Farbstoffe, oder MGB-Modifikationen etc. z.B. zur Anpassung von Bindungsstärken von einzelnen Bereichen der Aktivator-Oligonukleotide verwendet werden. Die einzelnen Nukleotid-Monomere können via konventionelle 5'-3'-Bindung untereinander gekoppelt werden oder auch abweichend via 5'-2'-Bindung.

Ein Segment des Aktivator-Oligonikleotids mit Nukleotid-Modifikationen, welche Verlängerung von 3'-Ende eines an Aktivator-Oligonukleotid gebundenen ersten Primer-Oligonukleotids durch Polymerase verhindern, wird als "zweite Blockierungs-Einheit" bezeichnet. Die Länge dieses Segments kann zwischen 1 bis 50 Nukletid-Modifikationen einschließen, vorzugsweise zwischen 4 und 30. Dieses Segment kann beispielsweise dermaßen im Aktivator-Oligonukleotid lokalisiert sein, dass das 3'-Ende des gebundenen ersten Primer-Oligonukleotids in diesem Segment liegt. Somit kann dieses Segment die Bereiche zwei und drei überspannen.

In einer Ausführungsform werden vorzugsweise keine Linker-Strukturen oder Spacer-Strukturen, wie C3, C6, HEG-Linker zur Verhinderung der Verlängerung des 3'-Ende eines an das Aktivator-Oligonukleotid gebundenen ersten Primer-Oligonukleotids verwendet.

Das Aktivator-Oligonukleotid kann außer Bereichen eins, zwei und drei noch weitere Sequenzsegmente umfassen, welche beispielsweise im 5'-Segment oder 3'-Segment des Aktivator-Oligonukleotid die oben genannten Bereiche flankieren. Solche Sequenzelemente können beispielsweise für weitere Funktionen verwendet werden, wie beispielsweise Interaktion mit Sonden, Bindung an feste Phase etc. Solche Bereiche stören vorzugsweise die Funktion der Bereiche eins bis drei nicht. Die Länge dieser flankierenden Sequenzen kann beispielsweise zwischen 1 bis 50 Nukleotide betragen. Weiterhin kann ein Aktivator-Oligonukleotid mindestes ein Element zu Immobilisierung an einer festen Phase umfassen, z.B. ein Biotin-Rest. Weiterhin kann ein Aktivator-Oligonukleotid mindestes ein Element zu Detektion umfassen, z.B. ein Fluoreszenz-Farbstoff.

### Effekt des Aktivator-Oligonukleotides auf Doppelstrangtrennung durch sequenzabhängige Strangverdrängung (schematische Erläuterung)

Nachfolgend soll der Wirkmechamismus des Aktivator-Oligonukleotides schematisch veranschaulicht werden.

Neusynthetisierte Stränge, welche Primerbindungsstellen umfassen, sind unmittelbar nach ihrer Synthese an ihre Matrizen gebunden und somit für sofortige Bindung von weiteren Primern im Wesentlich nicht zugängig. Erst nach Auftrennung bzw. Öffnung des Doppelstranges oder seines Sequenzabschnittes mit einer Primer-Bindungsstelle, z.B. im Rahmen oder als Folge einer Strangverdrängungsreaktion oder als Folge einer Doppelstrang-Dissoziation, kann ein solcher Sequenzabschnitt mit Primer-Bindungsstelle einen neuen Primer-Oligonukleotid binden und somit steht einer weiteren Runde der Synthese-Reaktion zur Verfügung.

Überführung von neusynthetisierten Sequenzabschnitten mit Primer-Bindungsstellen in einen einzelsträngigen Zustand stellt somit eine wesentliche Voraussetzung für Wiederholung von Synthese-Vorgängen und somit auch für eine Amplifikation dar. Faktoren, welche auf diesen Vorgang Einfluss haben, können die Amplifikation beeinflussen.

Die Strangverdrängung des zweiten Primer-Verlängerungsproduktes erfolgt erfindungsgemäß unter Mitwirkung / durch Bindung des Aktivator-Oligonukletides an das erste Primer-Verlängerungsprodukt.

Die Bindung des Aktivator-Oligonukleotides an das erste Primer-Verlängerungsprodukt geht über die Sequenz des ersten Primers hinaus. Dabei wird das neusynthetisierte Strangsegment des ersten Primer-Verlängerungsprodukt durch das Aktivator-Oligonukleotid gebunden und kann aus der Bindung mit seinem Matrizenstrang, repräsentiert beispielsweise durch das zweite Primer-Verlängerungsprodukt, verdrängt werden.

Diese Strang-Verdrängung führt zu einer Öffnung des Doppelstranges bestehend aus dem ersten und dem zweiten Primer-Verlängerungsprodukt. Diese Doppelstrang-Öffnung kann lokal erfolgen (z.B. nur in Sequenz-Segmenten, welche unmittelbar durch Aktivator-Oligonukleotid verdrängt werden) oder über diese Segmente hinausgehen, beispielsweise wenn restliche Doppelstrang durch einen weiteren Einflussfaktor destabilisiert wird, was in einer vollständigen Trennung der beiden Primer-Verlängerungsprodukte führen kann (vollständige Ablösung des zweiten Primer-Verlängerungsproduktes). Zu solchen Faktoren kann beispielsweise Temperatur-Einfluss auf die Stabilität des Doppelstrangs unter gegebenen Reaktionsbedingungen zählen. Zu weiteren Faktoren zählen beispielsweise Enzyme, welche einen Doppelstrang destabilisieren bzw. öffnen können (z.B. Helicasen oder Recombinasen) oder weitere Oligonukleotide, welche mittels Strangverdrängung einen Doppelstrang öffnen können.

Als Folge der Strangverdrängung werden beiden Primer-Bindungsstellen aus ihrem doppelsträngigen Zustand in einen einzelsträngigen Zustand überführt. Somit wird ihre Fähigkeit zur Bindung von weiteren Primern wiederhergestellt.

Zu den Faktoren, welche auf diesen Vorgang Einfluss haben und Amplifikation beeinflussen, zählen beispielsweise Vollständigkeit der Trennung beider Primer-Verlängerungsprodukte

Eine vollständige Dissoziation / Ablösung des zweiten Primer-Verlägerungsproduktes vom ersten Primer-Verlängerungsprodukt (vollständige Strang-Dissoziation) kann sich positiv auf die Kinetik einzelner Schritte auswirken. Das ist insbesondere dann der Fall, wenn geringe Konzentrationen von beiden Primer-Verlängerungsprodukten im Reaktionsgemisch vorliegen. Eine Re-Assoziierung von beiden Primer-Verlängerungsprodukten zu einem Doppelstrang ist bei diesen Umständen verlangsamt und Primer-Bindungsstellen verbleiben längere Zeit in ihrem einzelsträngigen Zustand.

### Gleichgewicht / Reversibilität der Verdrängung durch Aktivator-Oligonukleotid

Die Bindung des Aktivator-Oligonukleotid an das erste Primer-Verlängerungsprodukt ist durch Ausbildung von komplementären Basenpaaren zwischen beiden Strängen bedingt und ist somit unter Reaktionsbedingungen reversibel.

Die Bindung des Aktivator-Oligonukleotides an das erste Primer-Verlängerungsprodukt kann durch mehrere Faktoren Rückgängig gemacht werden. Dazu zählen beispielsweise:
- Polymerase-abhängige Strangverdrängung während der Synthese der zweiten Primer-Verlängerungsprodukte bis hin zur Stopp-Stelle im ersten Primer,
- Strang-Verdrängung durch das zweite Primer-Verlängerungsprodukt unter Ausbildung von komplementären Strängen zwischen dem ersten und dem zweiten Primer-Verlängerungsprodukt,
- Partielle Strangverdrängung durch 3'-Segment des zweiten Primers und seine Bindung an das erste Primer-Verlängerungsprodukt mit Verdrängung von 5'-Segment des Aktivator-Oligonukleotides.

Das zweite Primer-Verlängerungsprodukt kann sich an das erste Primer-Verlängerungsprodukt unter Ausbildung komplementärer Basenpaarungen binden und dadurch das Aktivator-Oligonukleotid verdrängen. Es besteht somit eine konkurrierende Situation zwischen dem Aktivator-Oligonukleotid und dem zweiten Primer-Verlängerungsprodukt um die Bindung an das erste Primer-Verlängerungsprodukt. Solange alle drei Stränge einen Drei-Strang-Komplex (der Komplex umfasst dabei das erste Primer-Verlängerungsprodukt, welches über seine komplementären Sequenzabschnitte an das zweite Primer-Verlängerungsprodukt und das Aktivator-Oligonukleotid gebunden ist) ausbilden, kann die Strangverdrängungs-Reaktion in beide Richtungen gehen: sowohl das Aktivator-Oligonukleotid kann das zweite Primer-Verlängerungsprodukt verdrängen als auch umgekehrt, das zweite Primer-Verlängerungsprodukt kann das Aktivator-Oligonukleotid verdrängen. Diese Strang-Verdrängungs-Vorgänge befinden sich im Gleichgewicht. Die im Rahmen dieses Komplexes stattfindende Strang-Verdrängung kann dabei bis hin zu Sequenzpositionen führen, welche unter gegebenen Reaktionsbedinungen zu einer vollständigen Dissoziation / Ablösung eines Stranges aus dem Drei-Strang-Komplex führen (Fig. 22).

Dies ist insbesondere dann der Fall, wenn Reaktionsbedingungen bei Duplex-Destabilisation mitwirken (z.B. durch Verwendung von entsprechender Reaktionstemperatur, Mg2+-Konzentration und Tm-verändernde Substanzen) und die Konstruktion von Sequenzabschnitten eine solche Ablösung / Dissoziation begünstigt, z.B. durch Anpassung der Stabilitäten von entsprechenden Sequenzabschnitten. In diesem Zusammenhang sind Stabilitäten von Komplexen aus Sequenzabschnitten von Bedeutung umfassend einerseits einen Komplex aus dem ersten Abschnitt von Aktivator-Oligonukleotid und dem zweiten Abschnitt des ersten Primers, andererseits einen Komplex aus dem 3'-Segment des ersten Primer-Verlängerungsproduktes mit seiner Primer-Bindungsstelle für das zweite Primer-Oligonukleotid und entsprechend das zweite Primer-Oligonukleotid bzw. das zweite Primer-Verlängerungsprodukt. Stabilität beider Komplexe kann Einfluss auf Gleichgewicht im Drei-Strang-Komplex haben: Höhere Stabilität im Komplex aus dem Aktivator-Oligonukleotid und dem ersten Primer begünstigt im Allgemeinen die Strangverdrängung des zweiten Primer-Verlängerungsproduktes. Umgekehrt, höhere Stabilität zwischen dem 3'-Segment des ersten Primer-Verlängerungsproduktes und dem zweiten Primer-Oligonukleotid begünstigt im Allgemeinen die Verdrängung von Aktivator-Oligonukleotid.

Die Stabilität von jeweiligen Sequenzabschnitten kann beispielsweise dadurch beeinflusst werden, dass die Längen der jeweiligen Sequenz-Abschnitte verkleinert oder vergrößert verlängert werden. Im Allgemeinen, führt die Verlängerung des ersten Bereichs des Aktivator-Oligonukleotid und des entsprechenden zweiten Bereichs des ersten Primer-Oligonukleotides zu verstärkten Verdrängung des synthetisierten zweiten Primer-Verlängerungsproduktes. Umgekehrt, führt die Verlängerung des 3'-Segments des ersten Primer-Verlängerungsproduktes und entsprechend des zweiten Primer-Verlängerungsproduktes zu Verstärkung der Verdrängung des Aktivator-Oligonukleotides.

Im Rahmen von Strangverdrängungsvorganges innerhalb eines Drei-Strang-Komplex können Zwischenstadien erreicht werden, bei welchen eine Ablösung / Dissoziation eines der Teilnehmer unter gegebenen Reaktionsbedinungen besonders begünstigt ist.

In einem Fall wird das zweite Primer-Verlängerungsprodukt aus der Bindung mit dem ersten Primer-Verlängerungsprodukt vollständig abgelöst, in einem anderen Fall kann Aktivator-Oligonukleotid vollständig aus der Bindung mit dem ersten Primer-Verlängerungsprodukt abgelöst werden.

Solange alle drei Stränge im Rahmen eines Drei-Strang-Komplexes gebunden sind, besteht ein gewisses Gleichgewicht im Fortschritt der Strangverdrängung in beide Richtungen.

Dieses Gleichgewicht kann durch mehrere Faktoren beeinflusst werden.

Dazu zählen beispielsweise Strang-Ablösung, Ausmaß der Komplementarität zwischen einzelnen Strukturen bzw. Vorliegen von Sequenz-Abweichungen (z.B. Mutationen), Verwendung von Nukleotid-Modifikationen oder Nicht-Nukleotid-Modifikationen, welche sich stabilisierend oder destabilisierend auf eine komplementäre Bindung von Nukleinsäuresträngen auswirken.

Besondere Bedeutung hat das Ausmaß der Komplementarität zwischen dem Aktivator-Oligonukleotid und einem von Polymerase synthetisierten Doppelstrang auf das beschriebene Gleichgewicht: ein Sequenz-Unterschied von nur einem Nukleotid kann zu einer Verzögerung der Amplifikation führen und ein Unterschied von drei Nukleotiden kann einen Stopp der Amplifikation herbeiführen.

Im Allgemeinen, wirken sich Sequenzunterschiede zwischen Aktivator-Oligonukleotid und dem ersten Primer-Verlängerungsprodukt negativ auf die Fähigkeit des Aktivator-Oligonukleotid, einen Doppelstrang mit einem solchen ersten Primer-Verlängerungsprodukt auszubilden.

Hingegen wird ein zweites Primer-Verlängerungsprodukt durch Polymerase im Allgemeinen als perfekt-match Produkt unter Verwendung des ersten Primer-Verlängerungsproduktes synthetisiert. Die Ausbildung eines Doppelstranges wird dadurch im Allgemeinen begünstigt.

Falls eine Sequenzabweichung zwischen dem Aktivator-Oligonukleotid und dem ersten Primer-Verlängerungsprodukt vorliegt, kann es zu Verschiebung im Gleichgewicht der Strangverdrängung im Rahmen des Drei-Strang-Komplexes kommen.

Als Ergebnis der Konkurrenz um die Bindung an das erste Primer-Verlängerungsprodukt wird die Ausbildung eines perfekt-match Doppelstranges zwischen dem ersten und dem zweiten Primer-Verlängerungsproduktes begünstigt gegenüber der Ausbildung eines mismatch-Doppelstranges. Es resultiert eine Verlangsamung oder gar Verhinderung der Strangverdrängung des zweiten Primer-Verlängerungsproduktes. Dies kann zu Verlangsamung oder Reduktion der Ausbeuten oder gar zu Verhinderung der Amplifikation führen.

Durch Verschiebung des Gleichgewichts bei Strangverdrängung als Folge solcher Sequenzabhängigkeit (pefekt-match vs. Mismatch-Situation zwischen dem ersten Primer-Verlängerungsprodukt und dem Aktivator-Oligonukleotid) kann ein Einfluss auf die Reaktion ausgeübt werden. Beispielsweise wird die Geschwindigkeit der Reaktion dermassen beeinflusst, so dass perfekt-match Situation zwischen dem ersten Primer-Verlängerungsprodukt und dem Aktivator-Oligonukleotid begünstigt wird und mismatches aus der Amplifikation ausgeschlossen werden.

Dieser Einfluss der Sequenzen ist in Beispielen 1, 3 und 4 verdeutlicht.

Durch Änderungen in diesem Gleichgewicht können Dissoziations-Vorgänge von beiden Primer-Verlängerungsprodukten bescheunigt, verlangsamt oder gar aufgehoben werden. Die Amplifikation wird somit schneller, langsamer oder u.U. gar nicht stattfinden.

Durch die Design der Sequenz der komplementären Anteile im Aktivator-Oligonukleotid (Im Rahmen von Oligonukleotid-Design und chemischen Synthese) können somit Nukleinsäureketten mit vorbestimmten Sequenzen amplifiziert werden. Andere Nukleinsäureketten, welche eine abweichende Sequenz aufweisen, können von der Amplifikation ausgeschlossen werden bzw. ihre Amplifikation wird kinetisch benachteiligt.

Besonders überraschend war, dass dieser Sequenz-Effekt der Aktivator-Oligonukleotide in seinem dritten Bereich sehr ausgeprägt war. Der dritte Bereich des Aktivator-Oligonukleotids soll erfindungsgemäß an den Sequenzabschnitt des ersten Primer-Verlängerungsprodukts binden, welches von Polymerase synthetisiert wird. Dieser Sequenzabschnitt des ersten Primer-Primer-Verlängerungsproduktes liegt somit im mittleren Segment der zu amplifizierenden Nukleinsäurekette. Er kann beispielsweise die Zielsequenz umfassen.

Somit unterliegt die Amplifikation einer ständigen Kontrolle der Sequenz-Inhalte in diesem Sequenzsegment: Die Strangverdrängung des zweiten Primer-Verlängerungsproduktes und somit seine Überführung in einzelsträngige Form ist von der Übereinstimmung in der Komplementarität des Aktivator-Oligonukleotids und des ersten Primer-Verlängerungsproduktes abhängig.

Somit kann ein Aktivator-Oligonukleotid eine Sequenz-Kontrolle nach jedem Primer-Verlängerungs-Vorgang ausüben.

Als Ergebnis dieser Kontrolle können mehrere Zustände erreicht werden:
- Bei perfekt-match Übereinstimmung der synthetisierten Stränge mit Aktivator-Oligonukleotid kommt es zu einer erfolgreichen Strangverdrängung, welche bis hin zu Dissoziation des zweiten Primer-Verlängerungsproduktes verlaufen kann. Damit werden entsprechende Sequenzabschnitte mit Primer-Bindungsstellen in einzelsträngigen Zustand überführt und Voraussetzungen geschaffen, dass eine weitere Synthese-Runde stattfinden kann.
- Bei auftretenden Mismatches in der Übereinstimmung der synthetisierten Stränge mit Aktivator-Oligonukleotid (z.B. bei Sequenzen, welche nicht einer vorgegebenen Anordnung der Nukleotide im dritten Bereich des Aktivator-Oligonukleotides entsprechen) kann Strangverdrängung negativ beeinflusst werden: Das Gleichgewicht liegt auf der Seite der Doppelstrangbildung zwischen dem ersten Primer-Verlängerungsprodukt und einer entsprechenden Matrize. Der Strangverdrängungsvorgang wird langsamer oder findet nur teilweise statt, was zu einer Reduzierung der Verfügbarkeit von Sequenzabschnitten mit freien Primer-Bindungsstellen führt. Insgesamt können Mismatches somit zu Verlangsamung der Amplifikation führen, bzw. zur Unterbrechung der Amplifikation.

Dadurch kann ein Regelungs-Kreis nach jeder Synthese-Wiederholung etabliert werden: Nach einer Bindung und Verlängerung der Primer werden Doppelstränge gebildet, welche in Abwesenheit von Aktivator-Oligonukleotides unter gegebenen Reaktionsbedingungen hinreichend stabil sind, so dass keine spontane Dissoziation erfolgt. Die Sequenzabschnitte mit Primer-Bindungsstellen sind im Kontext von ausgebildeten Doppelsträngen vor Interaktion mit neuen Primern gehindert. Das System hat einen Zustand erreicht, in welchem keine weitere Synthese stattfinden kann.

In Anwesenheit von einem Aktivator-Oligonukleotid werden neusynthetisierte Sequenzen auf ihre Sequenz-Inhalte durch Interaktion mit dem Aktivator-Oligonukleotid überprüft.
- Bei korrekter Übereinstimmung mit vorgegebenen Sequenzinhalten des Aktivator-Oligonukleotides erfolgt eine Strangverdrängung, wobei die Matrizen-Stränge von neusynthetisierten Strängen abgelöst werden. Dabei werden Primer-Bindungsstellen in einzelsträngigen Zustand überführt und stehen für neue Interaktion mit Primern und somit für eine weitere Synthese zur Verfügung. Somit wird das System aus beiden Primer-Verlängerungsprodukten in einen aktiven Zustand versetzt. Das Aktivator-Oligonukleotid hat somit eine aktivierende Wirkung auf das System.
- Bei fehlender Übereinstimmung mit vorgegebenen Sequenzinhalten des Aktivator-Oligonukleotides wird Strangverdrängung der Matrizen-Stränge von neusynthetisierten Strängen beieinflusst. Die Strangverdrängung und /oder Ablösung wird entweder quantitativ verlangsamt oder komplett aufgehoben. Es kommt somit gar nicht oder seltener zu Überführung von Primer-Bindungsstellen in einzelsträngigen Zustand. Somit stehen für neue Interaktion mit Primern gar keine oder quantitativ gesehen wenige Primer-Bindungsstellen zur Verfügung. Somit wird das System aus beiden Primer-Verlängerungsprodukten seltener in einen aktiven Zustand versetzt bzw. aktiver Zustand wird gar nicht erreicht.

Die Effizienz der Doppelstrang-Öffnung der neusynthetisierten Primer-Verlängerungsprodukte nach jedem einzelnen Synthese-Schritt wirkt sich auf die potenziell erreichbare Ausbeuten in darauf folgenden Zyklen aus: je weniger freier/einzelsträngiger Primer-Bindungsstellen einer zu amplifizierenden Nukleinsäurekette zu Beginn eines Synthese-Schrittes zu Verfügung gestellt werden, um so geringer ist die Anzahl von neusynthetisierten Stränge der zu amplifizierenden Nukleinsäurekette in diesem Schritt. Anders ausgedrückt: Die Ausbeute eines Synthese-Zyklus ist proportional der Menge von Primer-Bindungsstellen, welche für Interaktion mit entsprechenden komplementären Primern zur Verfügung stehen. Insgesamt kann dadurch ein Regelungs-Kreis realisiert werden.

Dieser Regelungs-Kreis entspricht einer Echtzeit-Kontrolle (real-time / on-line control) von synthetisierten Fragmenten: Sequenz-Kontrolle erfolgt im Reaktionsansatz während Amplifikation stattfindet. Diese Sequenzkontrolle erfolgt nach einem vorgegebenen Muster und das Oligonukleotid-System (durch Strang-Öffnende Wirkung von Aktivator-Oligonukleotid) kann ohne äußere Einmischung zwischen "korrekten" und "nicht-korrekten" Zuständen entscheiden. Im korrekten Zustand wird die Synthese von Sequenzen fortgesetzt, im nicht-korrekten Zustand wird die Synthese entweder verlangsamt oder vollständig verhindert. Die resultierenden Unterschiede in Ausbeuten an "korrekten" und "nicht-Korrekten" Sequenzen nach jedem Schritt wirken sich auf die gesamte Amplifikation aus, welche eine Vielzahl solcher Schritte umfasst.

Bei einer exponentiellen Amplifikation ist diese Abhängigkeit exponentiell, so dass sogar geringfügige Unterschiede in der Effizienz im Rahmen eines einzelnen Synthese-Zyklus aufgrund Sequenzunterschiede eine signifikante zeitliche Verzögerung der Gesamtamplifikation bedeuten können, bzw. das vollständige Ausbleiben einer detektierbaren Amplifikation in einem vorgegebenen Zeitrahmen bewirken.

Dieser Effekt der Echtzeit-Kontrolle der neusynthetisierten Nukleinsäureketten ist verbunden mit dem Einsatz des Aktivator-Oligonukleotides und der Einfluss von Aktivator-Oligonukleotid im Rahmen einer Amplifikation geht somit über die Länge von Primer-Oligonukleotide deutlich hinaus.

### Weitere Einflussfaktoren

Im Allgemeinen können Faktoren, welche eine Strangverdrängungsreaktion beeinflussen können, z.B. das Ausmaß, das Gleichgewicht und/oder die Geschwindigkeit dieses Vorgangs beeinflussen, auch Einfluss auf die exponentielle Amplifikation haben.

Zu solchen Faktoren zählen beispielsweise Reaktionsbedingungen (z.B. Temperatur, Mg2+-Konzentration, DMSO, Betaine, TPAC, Buffer-Komponenten), Vorliegen von Sekundär-Strukturen innerhalb des Aktivator-Oligonukleotides (z.B. Hairpin-Strukturen oder G-Quadruplexe), Verwendung von Nukleotid-Modifikationen im Aktivator-Oligonukleotid (welche Doppelstrang stabilisieren z.B. 2-Amino-dA oder 5-propargyl-dC, LNA-Nukleotide, oder destabilisieren, z.B. Inosine) oder nicht-Nukleotid-Modifikationen, welche Doppelstrang stabilisieren, z.B. MGB, oder destabilisieren, z.B. sterisch anspruchsvolle Gruppen ("bulky groups"), welche Geometrie von A-Form oder B-Form der Nukleinsäurestränge beeinflussen (z.B. an Nukleotid gekoppelte Farbstoffe oder Liganden), oder interkalierende Substanzen, z.B. SybrGreen, EvaGreen Farbstoffe.

Im Einzelfall können solche Faktoren verwendet werden, um positiven oder negativen Einfluss auf das Gleichgewicht der Strangverdrängung auszuüben und damit Amplifikation von bestimmten Sequenzen begünstigen oder verlangsamen bzw. verhindern. Das Ausmass der Beinflussung kann experimentell bestimmt werden.

### Reaktionsbedingungen bei Strangverdrängungsreaktion

Die Verdrängung des zweiten Primer-Verlängerungsproduktes aus der Bindung mit dem ersten Primer-Verlängerungsprodukt mittels einer sequenzabhänigen Strangverdängung durch das Aktivator-Oligonukleotid bildet einen Teilschritt in der Amplifikation. Die Reaktionsbedinungen während dieses Schrittes sind entsprechend angepasst. Die Reaktions-Temperatur und die Reaktions-Zeit sind dermassen gewählt, dass die Reaktion erfolgreich stattfinden kann.

In einer bevorzugten Ausführungsform verläuft die Strangverdrängung durch das Aktivator-Oligonukleotid bis zur Ablösung / Dissoziation des zweiten Primer-Verlängerungsproduktes aus der Bindung mit dem ersten Primer-Verlängerungsprodukt. Eine solche Dissoziation des 3'-Segment des ersten Primer-Verlängrungsproduktes von komplementären Anteilen des zweiten Primerverlängerungsproduktes kann spontant erfolgen im Rahmen einer temperatur-abhängigen / temperatur-bedingten Trennung von beiden Primer-Verlängerungprodukten. Eine solche Dissoziation wirkt sich günstig auf Kinetik der Amplifikationsreaktion und kann durch die Wahl der Reaktionsbedinungen beeinflusst werden, z.B. mittels Temperatur-Bedinungen. Die Temperatur-Bedinungen werden deshalb dermassen gewählt, dass eine erfolgreiche Strangverdrängung durch komplementäre Bindung des Aktivator-Oligonukleotides eine Dissoziation des zweiten Primer-Verlängerungsproduktes vom 3'-Segment des ersten Primer-Verlängerungsproduktes begünstigt.

Die Temperatur in diesem Schritt umfasst beispielsweise Bereiche von 15°C bis 75°C, besser von 30°C bis 70°C, bevorzugt von 50°C bis 70°C.

Bei gegebener Länge des ersten Bereichs des Aktivator-Oligonukleotides und des zweiten Bereichs des ersten Primer-Oligonukleotides (umfassend beispielsweise Bereiche von 3 bis 25 Nukleotid-Monomere, besser von 5 bis 15 Nukleotid-Monomere) kann eine Strangverdrängungsreaktion im allgemeinen erfolgreich initiiert werden. Bei vollständigen Komplementarität des Aktivator-Oligonukleotid zu entsprechenden Anteilen des ersten Primer-Verlängerungsproduktes kann das Aktivator-Oligonukleotid an das erste Primer-Verlängerungsprodukt bis auf das 3'-Segment des ersten Primer-Verlängerungsproduktes binden und das zweite Primer-Verlängerungsprodukt verdrängen. Das zweite Primer-Verlängerungsprodukt verbleibt somit in Verbindung mit dem 3'-Segment des ersten Primer-Verlängerungsproduktes. Diese Stärke dieser Verbindung kann temperaturabhängig beeinflusst werden. Beim Erreichen einer kritischen Temperatur kann diese Verbindung zerfallen und beide Primer-Verlängerungsprodukte dissoziieren. Je kürzer die Sequenz des 3'-Segmentes ist, umso instabiler ist diese Verbindung und umso niedriger kann die Temperatur sein, welche eine spontane Dissoziation herbeiführt.

Eine spontane Dissoziation kann beispielsweise im Temperatur-Bereich erreicht werden, welcher etwa bei der Schmelztemperatur liegt. In einer Ausführungsform liegt die Temperatur der Schritte der Strangverdrängung durch das Aktivator-Oligonukleotid etwa bei der Schmelztemperatur (Tm +/- 3°C) des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt.

In einer Ausführungsform liegt die Temperatur der Schritte der Strangverdrängung durch das Aktivator-Oligonukleotid etwa bei der Schmelztemperatur (Tm +/- 5°C) des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt.

In einer Ausführungsform liegt die Temperatur der Schritte der Strangverdrängung durch das Aktivator-Oligonukleotid über der Schmelztemperatur des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt. Eine solche Temperatur umfasst Temperatur-Bereiche von etwa Tm + 5°C bis Tm+20°C, besser von Tm+5°C bis Tm +10°C. Durch Verwendung einer höheren Temperatur kann das Gleichgewicht in diesem Reaktionsschritt in Richtung Dissoziation verschoben werden. Dadurch kann die Kinetik der Reaktion günstig beeinflusst werden. Verwendung von zu niedrigen Temperaturen im Schritt der Strangverdrängung mittels Aktivator-Oligonukleotides kann zu einer signifikanten Verlangsamung der Amplifikation führen.

Die Temperatur der Strangverdrängungsschritte durch Aktivator-Oligonukleotid kann auch in etwa abgeschätzt werden und an die Dissoziations-Temperatur angepasst werden.

In einer weiteren Ausführungsform umfasst ein erstes Primer-Verlängerungsprodukt ein 3'-Segment, welches nicht von Aktivator-Oligonukleotid gebunden wird, und welches Sequenzlängen von 4 bis etwa 8 Nukleotiden umfasst. Bei dieser Ausführungsform kann eine spontane Dissoziation in der Regel bereits bei Temperatur-Bereichen zwischen 15°C und 50°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 9 bis etwa 18 Nukleotiden umfasst. Bei dieser Ausführungsform kann eine spontane Dissoziation in der Regel bereits bei Temperatur-Bereichen zwischen 40°C und 65°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 15 bis etwa 25 Nukleotiden umfasst. Bei dieser Ausführungsform kann eine spontane Dissoziation in der Regel bereits bei Temperatur-Bereichen zwischen 50°C und 70°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 20 bis etwa 40 Nukleotiden umfasst. Bei dieser Ausführungsform kann eine spontane Dissoziation in der Regel bereits bei Temperatur-Bereichen zwischen 50°C und 75°C erreicht werden. Auch höhere Temperaturen führen zu einer Dissoziation.

Die Zusammensetzung des 3'-Segmentes des ersten Primer-Verlängerungsproduktes und ggf. eine Einführung von Schmelztemperatur-beeinflussenden Oligonukleotid-Modifikationen (z.B. MGB) bzw. Reaktionsbedinungen (z.B. TPAC, Betaine) kann die Wahl der Temperatur beeinflussen. Entsprechende Anpassung kann daher vorgenommen werden.

In einer Ausführungsform verlaufen alle Schritte der Amplifikation unter stringenten Bedinungen, welche Ausbildung von unspezifischen Produkten / Nebenprodukten verhindern bzw. verlangsamen. Zu solchen Bedinungen zählen beispielsweise höhere Temperaturen, beispielsweise über 50°C.

In einer Ausführungsform verläufen die Einzelschritte der Strangverdrängung durch Aktivator-Oligonukleotide bei gleicher Temperatur wie die Synthese des ersten und des zweiten Primer-Verlängerungsproduktes. In einer weiteren Ausführungsform verläufen die Einzelschritte der Strangverdrängung durch Aktivator-Oligonukleotide bei Temperatur, welche von der Temperatur der jeweiligen Synthese des ersten und des zweiten Primer-Verlängerungsproduktes abweicht. In einer weiteren Ausführungsform verläuft die Synthese des ersten Primer-Verlängerungsproduktes und Strangverdrängung durch Aktivator-Oligonukleotid bei gleicher Temperatur. In einer weiteren Ausführungsform verläuft die Synthese des zweiten Primer-Verlängerungsproduktes und Strangverdrängung durch Aktivator-Oligonukleotid bei gleicher Temperatur.

Die Konzentration des Aktivator-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

### Bevorzugte Ausführungsformen des zweiten Primer-Oligonukleotides (Primer-2):

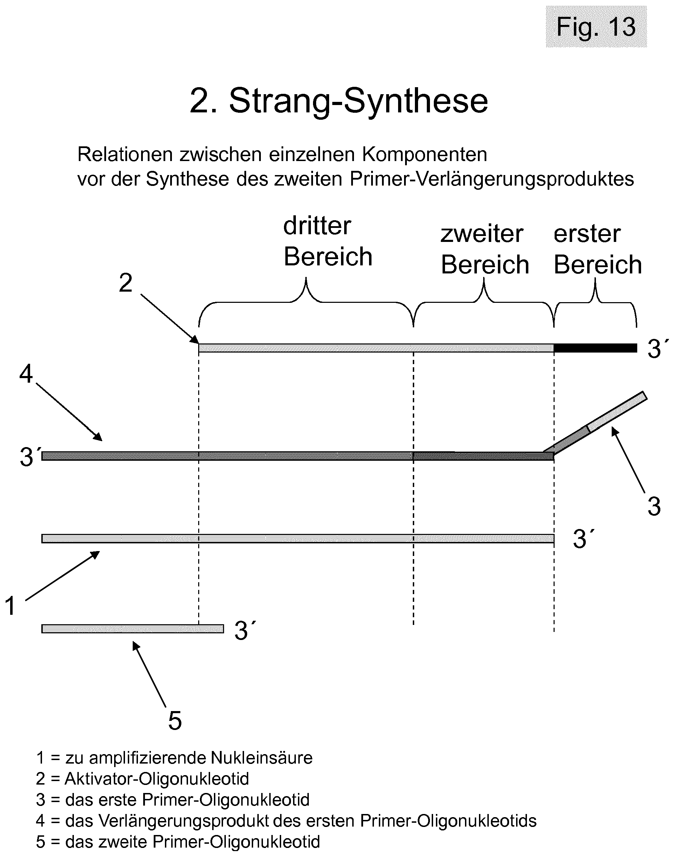

Ein Oligonukleotid, welches mit seinem 3'-Segment in der Lage ist, an eine im Wesentlichen komplementäre Sequenz innerhalb der zu amplifizierenden Nukleinsäure oder ihrer Äquivalente zu binden und eine spezifische zweite Primer-Verlängerungsreaktion zu initiieren (Fig 13). Dieses zweite Primer-Oligonukleotid ist somit in der Lage, an das 3'-Segment eines ersten spezifischen Primer-Verlängerungsproduktes des ersten Primer-Oligonukleotids zu binden und eine polymerase-abhängige Synthese eines zweiten Primer-Verlängerungsproduktes zu initiieren. In einer Ausführungsform ist ein jedes zweite Primer-Oligonukleotid spezifisch für je eine zu amplifizierende Nukleinsäure.

Der zweite Primer-Oligonukleotid soll bei Rücksynthese kopiert werden können und dient auch als selbst Matrize für im Rahmen der Synthese des ersten Primer-Verlängerungsproduktes.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden. Die Nukleotid-Bausteine sind vorzugsweise untereinander via übliche 5'-3' Phosphodieser-Bindung oder Phosphothioester-Bindung verknüpft. Ein solches Primer-Oligonukleotid kann in gewünscher Form chemisch synthetisiert werden.

In einer Ausführungsform kann das zweite Primer-Oligonukleotid Nukleotid-Monomere einschließen, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- Modifizierte Nukleotide, 2-Amino-dA, 2-thio-dT oder andere Nukleotid-Modifikationen mit abweichender Basen-Paarung (z.B. Universal-Basenpaaren, wie beispielsweise Inosine 5-Nitroindol).

In einer bevorzugten Ausführungsform ist das 3'-OH-Ende dieses Bereichs vorzugsweise frei von Modifikationen und hat eine funktionsfähige 3'-OH Gruppe, welche von Polymerase erkannt und matrizenabhänig verlängert werden kann. In einer weiteren bevorzugten Ausführungsform umfasst Das 3'-Segment des zweiten Primers mindestens eine Phosphorothioat-Verbindung, so dass kein Abbau von 3'-Ende des Primern durch 3'-Exonuclease Aktivität von Polymerasen erfolgen kann.

Das zweite Primer-Oligonukleotid kann in mehreren Teil-Schritten verwendet werden. In erster Linie übernimmt es eine Primer-Funktion in der Amplifikation. Dabei wird Primer-Verlängerungsreaktion unter Verwendung des ersten Primer-Verlängerungsproduktes als Matrize ausgeführt. In einer weiteren Ausführungsform kann das zweite Primer-Oligonukleotid zu Beginn der Amplifikationsreaktion die Start-Nukleinsäurekette als Matrize verwenden. In einer weiteren Ausführungsform kann das zweite Primer-Oligonukleotid bei der Erstellung / Bereitstellung einer Start-Nukleinsäurekette verwendet werden.

Im Rahmen der Amplifikation dient der zweiten Primer als Initiator der Synthese des zweiten Primer-Verlängerungsproduktes unter Verwendung des ersten Primer-Verlängerungsproduktes als Matrize. Das 3'-Segment des zweiten Primers umfasst eine Sequenz, welche vorwiegend komplementär an das erste Primer-Verlängerungsprodukt binden kann. Die enzymatische Verlängerung des zweiten Primer-Oligonukleotid unter Verwendung des ersten Primer-Verlängerungsproduktes als Matrize führt zu Ausbildung des zweiten Primer-Verlängerungsproduktes. Eine solche Synthese erfolgt typischerweise parallel zu Verdrängung des Aktivator-Oligonukleotides aus seiner Bindung mit dem ersten Primer-Verlängerungsprodukt. Diese Verdrängung erfolgt vorwiegend durch Polymerase und kann teilweise durch das zweite Primer-Oligonukleotid erfolgen. Ein solches zweites Verlängerungsprodukt umfasst die Zielsequenz oder ihre Segmente. Im Verlauf der Synthese des zweiten Primer-Verlängerungsproduktes wird die Sequenz des kopierbaren Anteils des ersten Primer-Oligonukleotid von Polymerase als Matrize erkannt und eine entsprechende komplementäre Sequenz synthetisiert wird. Diese Sequenz liegt im 3'-Segment des zweiten Primer-Verlängerungsproduktes und umfasst Primer-Bindungsstelle für das erste Primer-Oligonukleotid. Die Synthese des zweiten Primer-Verlängerungsproduktes erfolgt bis zur Stopp-Position im ersten Primer-Oligonukleotid. Unmittelbar nach der Synthese des zweiten Primer-Verlängerungsproduktes ist dieses Produkt an das erste Primer-Verlängerungsprodukt gebunden und bildet doppelsträngigen Komplex. Das zweite Primer-Verlängerungsprodukt wird aus diesem Komplex sequenzspezifisch durch das Aktivator-Oligonukleotid verdrängt. Nach einer erfolgreichen Strangverdrängung durch Aktivator-Oligonukleotid kann das zweite Primer-Verlängerungsprodukt wiederum selbst als Matrize für die Synthese des ersten Primer-Verlängerungsproduktes dienen.

Weiterhin kann das zweite Primer-Oligonukleotid als Initiator der Synthese des zweiten Primer-Verlängerungsproduktes ausgehend von Start-Nukleinsäurekette zu Begin der Amplifikation dienen. In einer Ausführungsform ist die Sequenz des zweiten Primers vollständig komplementär zum entsprechenden Sequenzsegment einer Start-Nukleinsäurekette. In einer weiteren Ausführungsform ist die Sequenz des zweiten Primer-Oligonukleotides nur teilweise komplementäre zum entsprechenden Sequenzsegment einer Start-Nukleinsäurekette. Diese abweichende Komplementarität soll das zweite Primer-Oligonukleotid allerdings nicht daran hindern, eine vorwiegend sequenzspezifische Primer-Verlängerungsreaktion zu starten. Die jeweiligen Unterschiede in Komplementarität des zweiten Primer-Oligonukleotides zur jeweiligen Position in der Start-Nukleinsäurekette liegen vorzugsweise im 5'-Segment des zweiten Primer-Oligonukleotides, so dass im 3'-Segment vorwiegend komplementäre Basenpaarung und Initiierung der Synthese möglich ist. Für die Initiierung der Synthese sollen beispielsweise besonders die ersten 4 - 10 Positionen im 3'-Segment vollkomplementär zu Matrize (Start-Nukleinsäurekette) sein. Die restlichen Nukleotidpositionen können von perfekten Komplementarität abweichen. Das Ausmaß einer perfekten Komplementarität im 5'-Segment kann somit Bereiche umfassen zwischen 10% bis 100%, besser zwischen 30% und 100% der Basenzzusammensetzung. Je nach Länge des zweiten Primer-Oligonukleotides, umfassen diese Abweichung von einer vollständigen Komplementarität im 5'-Segment von 1 bis 40, besser 1 bis 20 Nukleotid-Positionen. In einer weiteren Ausführungsform bindet das zweite Primer-Oligonukleotid nur mit seinem 3'-Segment an die Start-Nukleinsäurekette, aber nicht mit seinem 5'-Segment. Die Länge eines solchen zu Start-Nukleinsäurekette vollkomplementären 3'-Segment des zweiten Primer-Oligonukleotides umfasst Bereiche zwischen 6 und 40 Nukleotide, besser, zwischen 6 und 30 Nukleotide, bevorzugt zwischen 6 und 20. Die Länge eines entsprechenden, zu Start-Nukleinsäurekette nicht komplementären 5'-Segments des zweiten Primer-Oligonukleotides umfasst Bereiche zwischen 5 und 60, besser zwischen 10 und 40 Nukleotide. Das zweite Primer-Oligonukleotid kann somit eine Synthese von einer Start-Nukleinsäukette initiieren. Bei einer darauffolgenden Synthese des ersten Primer-Verlängerungsproduktes werden Sequenzabschnitte des zweiten Primer-Oligonukleotides von Polymerase kopiert, so dass wiederum in darauf folgenden Synthese-Zyklen eine vollkomplementäre Primer-Bindungsstelle innerhalb des ersten Primer-Verlängerungsprodukte für die Bindung des zweiten Primer-Oligonukleotides ausgebildet wird und in darauf folgenden Synthese-Zyklen zur Verfügung steht.

In einer weiteren Ausführungsform kann das zweite Primer-Oligonukleotid im Rahmen der Vorbereitung einer Start-Nukleinsäurekette verwendet werden. Dabei kann ein solches zweite Primer-Oligonukleotid an eine Nukleinsäure (z.B. eine einzelsträngige genomische DNA oder RNA oder ihre Äquivalente umfassend eine Zielsequenz) vorwiegend / bevorzugt sequenzspezifisch binden und in Anwesenheit einer Polymerase eine matrizenabhängige Primer-Verlängerungsreaktion initiieren. Die Bindungsposition ist dermassen gewählt, dass das Primer-Verlängerungsprodukt eine gewünschte Zielsequenz umfasst. Durch die Verlängerung des zweiten Primer-Oligonukleotides resultiert ein Strang, welche zu Matrize komplementäre Sequenz aufweist. Ein solcher Strang kann von der Matrize abgelöst werden (z.B. durch Hitze oder Alkalie) und damit in eine einzelsträngige Form überführt werden. Eine solche einzelsträngige Nukleinsäurekette kann als Start-Nukleinsäurekette zu Beginn der Amplifikation dienen. Eine solche Start-Nukleinsäurekette umfasst in ihrem 5'-Segment die Sequenzanteile des zweiten Primer-Oligonukleotides, weiterhin umfasst sie eine Zielsequenz oder ihre Äquivalente und eine Primer-Bindungsstelle für das erste Primer-Oligonukleotid. Weitere Schritte sind im Abschnitt "Start-Nukleinsäurekette" erläutert.

In einer bevorzugten Ausführungsform umfasst das zweite Primer-Oligonukleotid zumindest in seinem 3'-Segment Sequenzanteile, welche komplementär und sequenzspezifisch an ein Sequenzsegment einer Zielsequenz binden können und eine erfolgreiche Primer-Verlängerungsreaktion durch Polymerase initiieren / unterstützen. Die Länge eines solchen Sequenzsegments umfasst Bereiche von 6 und 40 Nukleotide, besser von 8 bis 30 Nukleotide, bevorzugt von 10 bis 25 Nukleotide.

In einer Ausführungsform umfasst das zweite Primer-Oligonukleotid in seinem 3'- und 5'-Segment kopierbare Sequenzabschnitte, welche bei Synthese des ersten Primer-Verlängerungsproduktes von Polymerase kopiert werden. Somit werden alle Sequenzabschnitte des zweiten Primers von Polymerase kopiert. Das führt zu Ausbildung einer Primer-Bindungsstelle im 3'-Segment des ersten Primer-Verlängerungsproduktes.

In einer weiteren Ausführungsform kann das zweite Primer-Oligonukleotid flankierend an sein 5'-Segment mit kopierbaren Sequenzabschnitten ein weiteres Sequenz-Segment umfassen, welches nicht kopierbare Anteile umfasst. Ein solcher Primer kann beispielsweise als "Skorpion-Primer" in der Detektion verwendet werden. In einer solchen Ausführungsform umfasst das zweite Primer-Oligonukleotid Modifikationen, welche Polymerase am Kopieren dieses flankierenden Sequenzsegmentes stören. Solche Modifikationen umfassen beispielsweise C3-Linker oder HEG-Linker oder andere Modifikationen, welche zum Polymerase-Stopp führen. Weitere Beispiele für solche Modifikationen sind im Abschnitt "Ausführungsformen des ersten Primer-Oligonukleotids" beschrieben. s.dort.

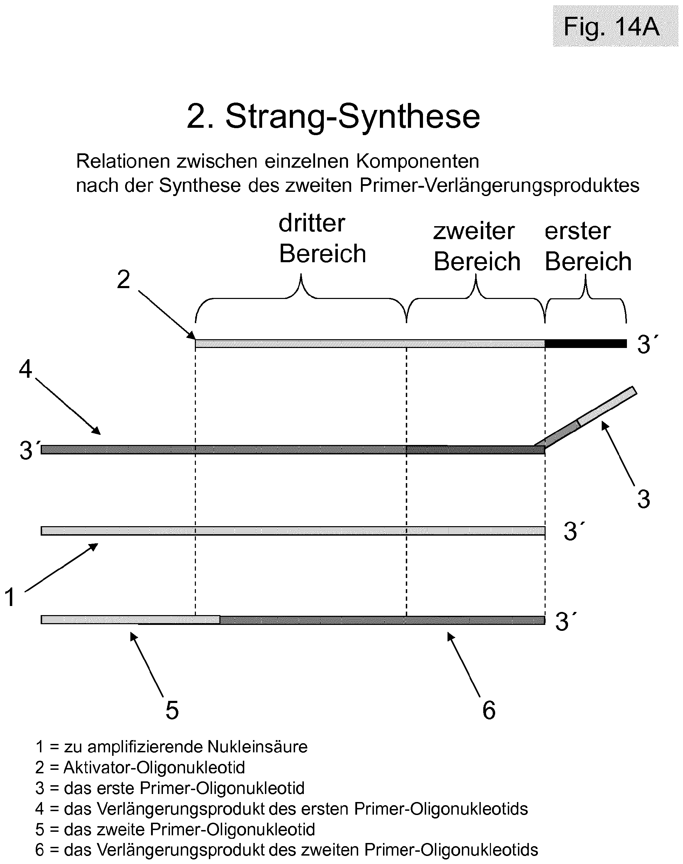

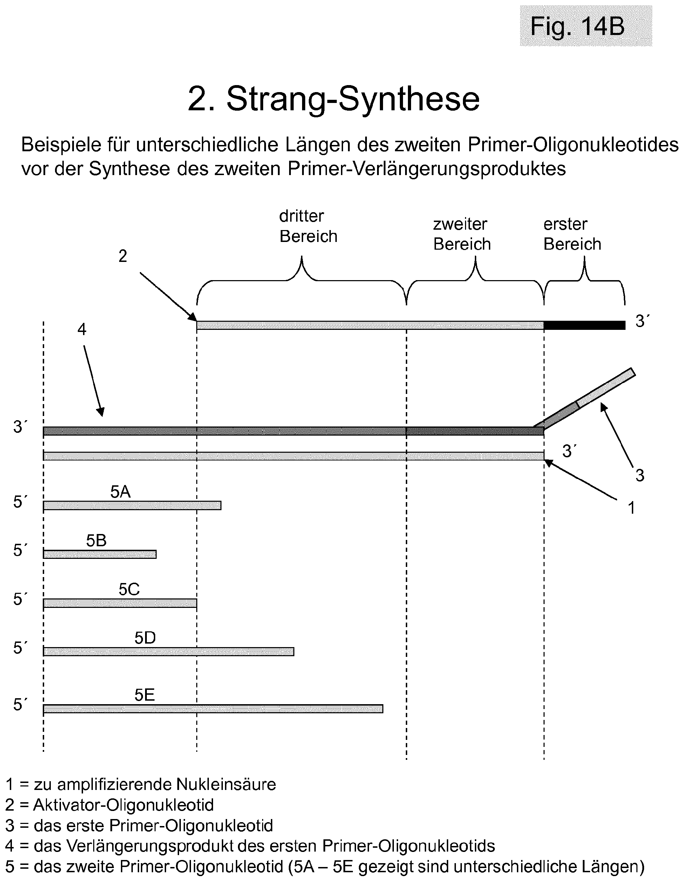

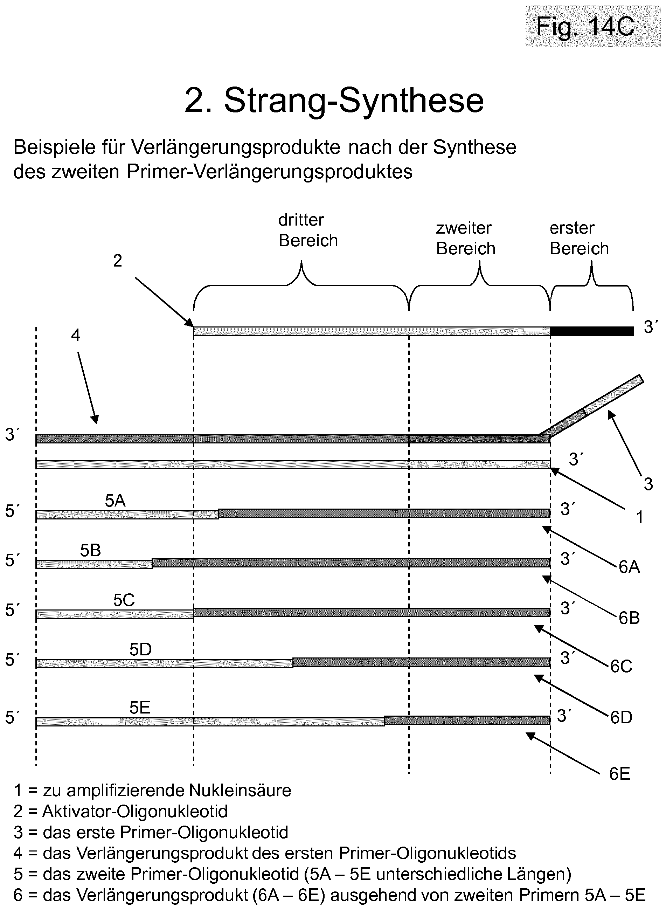

In einer Ausführungsform entspricht das zweite Primer-Oligonukleotid mit seinen kopierbaren Anteilen in ihrer Länge dem 3'-Segment des ersten Primer-Verlängerungsproduktes, welches nicht vom Aktivator-Oligonukleotid gebunden wird (Fig. 14B, Primer 5C). Im Komplex umfassend das zweite Primer-Oligonukleotid und das erste Primer-Verlängerungsprodukt grenzt das 3'-Ende eines solchen zweiten Primer-Oligonukleotides an das Aktivator-Oligonukleotid, welches an das erste Primer-Verlängerungsprodukt gebunden ist. Verlängerung eines solchen Primers erfolgt unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in Fig. 14C, abgebildet (Primer-Verlängerungsprodukt 6C).

In einer weiteren Ausführungsform ist das zweite Primer-Oligonukleotid mit seinen kopierbaren Sequenzanteilen kürzer als das 3'-Segment des ersten Primer-Verlängerungsproduktes, welches nicht vom Aktivator-Oligonukleotid gebunden wird (Fig. 14B, Primer 5B). Im Komplex umfassend das zweite Primer-Oligonukleotid und das erste Primer-Verlängerungsprodukt liegt somit zwischen dem 3'-Ende eines solchen Primers und dem an das erste Primer-Verlängerungsprodukt gebundenen Aktivator-Oligonukleotid ein einzelsträngiger Abschnitt des ersten Primer-Verlängerungsproduktes. Verlängerung eines solchen Primers erfolgt unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in in Fig. 14C, abgebildet (Primer-Verlängerungsprodukt 6B).

In einer weiteren Ausführungsform ist das zweite Primer-Oligonukleotid mit seinen kopierbaren Anteilen länger als das 3'-Segment des ersten Primer-Verlängerungsproduktes, welches nicht vom Aktivator-Oligonukleotid gebunden wird (Fig. 14B, Primer 5A, 5D, 5E). Im Komplex aus dem zweiten Primer-Oligonukleotid und dem ersten Primer-Verlängerungsprodukt konkurrieren das 3'-Segment des zweiten Primers und das 5'-Segment des Aktivator-Oligonukleotides um die Bindung an das das erste Primer-Verlängerungsprodukt. Die für eine Initiierung der Synthese erforderliche Bindung des 3'-Segments des zweiten Primers an das erste Primer-Verlängerungsprodukt erfolgt unter gleichzeitiger partiellen Verdrängung des 5'-Segmentes des Aktivator-Oligonukleotides. Nach Initiierung der Synthese durch Polymerase erfolgt die Verlängerung eines solchen Primers unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in Fig. 14C, abgebildet (Primer-Verlängerungsprodukt 6A, 6D, 6E). Die Sequenzlänge des 3'-Segment des zweiten Primer-Oligonukleotides, welches das 5'-Segment des Aktivator-Oligonukleotid verdrängt, kann folgende Bereiche umfassen: 1 bis 50 Nukleotide, besser 3 bis 30 Nukleotide, bevorzugt 5 bis 20 Nukleotide. Verwendung von zweiten Primer-Oligonukleotiden mit einer größeren Länge, welche die Länge des 3'-Segment des ersten Primer-Verlängerungsproduktes übersteigt, ist beispielsweise in einigen Ausführungsformen vorteilhaft. Solche Ausführungsformen umfassen beispielsweise ein erstes Primer-Verlängerungsprodukt mit einem 3'-Segment, welches nicht vom Aktivator-Oligonukleotid gebunden wird, mit einer Länge von 5 bis 40 Nukleotiden, besser von 10 bis 30 Nukleotiden. Besonders bei kürzeren 3'-Segmenten bietet ein längeres zweites Primer-Oligonukleotid eine verbesserte Sequenzspezifität bei Initiierung Synthese.

Die Bindungsstärke des zweiten Primer-Oligonukleotides an seine Primer-Bindungsstelle hängt von der Länge des Primers ab. Im Allgemeinen können längere zweite Primer-Oligonukleotide bei höheren Reaktionstemperaturen eingesetzt werden.

Vorzugsweise sind Sequenzen des ersten, des zweiten Primer-Oligonukleotides und des Aktivator-Oligonukleotides dermassen aneinander angepasst, dass Nebenreaktionen, z.B. Primer-Dimer-Ausbildung, minimiert sind. Zu diesem Zweck sind beispielsweise die Sequenz des ersten und des zweiten Primer-Oligonukleotides aneinander demassen angepasst, daß beide Primer-Oligonukleotide nicht in der Lage sind, eine Amplifikations-Reaktion in Abwesenheit einer passenden Matrize und/oder einer Zielsequenz und/oder einer Start-Nukleinsäurekette zu starten. Das kann beispielsweise dadurch erreicht werden, dass das zweite Primer-Oligonukleotid keine Primer-Bindungsstelle für das erste Primer-Oligonukleotid umfasst und das erste Primer-Oligonukleotid keine Primer-Bindungsstelle für das zweite Primer-Oligonukleotid umfasst. Weiterhin soll vermieden werden, dass die Primer-Sequenzen ausgedenhnte eigen-komplementäre Strukturen (Self-complement) umfassen.

Die Synthese des zweiten Primer-Verlängerungsproduktes ist eine Primer-Verlängerungsreaktion und bildet einen Teilschritt in der Amplifikation. Die Reaktionsbedinungen während dieses Schrittes sind entsprechend angepasst. Die Reaktions-Temperatur und die Reaktions-Zeit sind dermassen gewählt, dass die Reaktion erfolgreich stattfinden kann. Die jeweils bevorzugte Temperatur in diesem Schritt hängt von der verwendeten Polymerase ab und der Bindungsstärke des jeweiligen zweiten Primer-Oligonukleotides an seine Primer-Bindungsstelle und umfasst beispielsweise Bereiche von 15°C bis 75°C, besser von 20 bis 65°C, bevorzugt von 25°C bis 65°C. Die Konzentration des zweiten Primer-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

In einer Ausführungsform verlaufen alle Schritte der Amplifikation unter stringenten Bedinungen, welche Ausbildung von unspezifischen Produkten / Nebenprodukten verhindern bzw. verlangsamen. Zu solchen Bedinungen zählen beispielsweise höhere Temperaturen, beispielsweise über 50°C.

Falls mehr als eine spezifische Nukleinsäurekette in einem Ansatz amplifiziert werden müssen, werden in einer Ausführungsform bevorzugt jeweils sequenzspezifische Primer-Oligonukleotide für Amplifikation von entsprechenden jeweiligen Zielsequenzen verwendet.

In einer Ausführungsform verläuft die Synthese des ersten und des zweiten Primer-Verlängerungsproduktes bei gleicher Temperatur. In einer weiteren Ausführungsform verläuft die Synthese des ersten und des zweiten Primer-Verlängerungsproduktes bei unterschiedlichen Temperaturen. In einer weiteren Ausführungsform verläuft die Synthese des zweiten Primer-Verlängerungsproduktes und Strangverdrängung durch Aktivator-Oligonukleotid bei gleicher Temperatur. In einer weiteren Ausführungsform verläuft die Synthese des zweiten Primer-Verlängerungsproduktes und Strangverdrängung durch Aktivator-Oligonukleotid bei unterschiedlichen Temperaturen.

### Exponentielle vs. Lineare Amplifikation

Falls beide komplementäre Stränge (das erste Primer-Verlängerungs-Produkt und das zweite Primer-Verlängerungsprodukt, wobei beide Primer-Verlängerungsprodukte als Matrizen für Synthesen der komplementären Stränge auftreten können) im Wesentlichen parallel zu einander im gleichen Ansatz synthetisiert werden, kann es zu einer exponentiell ablaufenden Vermehrung beider Primer-Verlängerungsprodukte im Rahmen einer solchen Reaktion kommen.

Die während eines Synthese-Vorgangs neu synthetisierten Primer-Verlängerungsprodukte schließen die jeweils komplementäre Sequenzabschnitte zu verwendeten Primern, so dass Primer-Bindungsstellen neu generiert werden. Dadurch können neu synthetisierte Stränge in den darauf folgenden Synthese-Vorgängen ihrerseits als Matrizen dienen.

Falls im Wesentlichen nur ein Primer-Verlängerungs-Produkt als Folge zyklisch wiederholten Synthese-Vorgänge synthetisiert wird, so kommt es zu einer linear ablaufenden Amplifikation des besagten Primer-Verlängerungsproduktes.

Die Geschwindigkeit einer exponentiellen Amplifikation kann in der Regel höher als die Geschwindigkeit einer linearen Amplifikation sein, besonders zu Beginn einer Amplifikation, wenn Ausgangs-Mengen von Start-Nukleinsäureketten sehr gering sind und der Einfluss von Reaktions-Produkten auf Reaktions-Kinetik noch vernachlässigbar gering ist. Im Allgemeinen, sinkt die Geschwindigkeit der Reaktion mit steigender Menge von Synthese-Produkten, so dass es zu einer Verlangsamung der Amplifikation zu rechnen ist. Im Einzelnen, hat die Effizient und Geschwindigkeit einzelner Verfahrensschritte einen Einfluss auf die Gesamt-Gesamtgeschwindigkeit des erfindungsgemäßen Verfahrens. Durch Änderung/Anpassung der Effizienz einzelner Schritte kann das erfindungsgemäße Verfahren der Amplifikation in seiner Geschwindigkeit und seiner Effizienz beeinflusst werden.

Im Folgenden wird eine bevorzugte Ausführungsform des Verfahrens als Beispiel näher erläutert, nämlich eine exponentiell ablaufende Amplifikation einer zu amplifizierenden Nukleinsäurekette.

Eine exponentiell ablaufende Synthese-Reaktion (Amplifikation) hat als Ergebnis eine Akkumulierung von beiden neu synthetisierten Primer-Verlängerungsprodukten, welche als die zu amplifizierenden Nukleinsäureketten betrachtet werden. Sie ist bedingt durch eine zyklische Wiederholung von Synthese-Vorgängen der beiden Primer-Verlängerungsprodukte und Strang-Verdrängungs-Vorgängen (mit dem Ergebnis einer Öffnung von jeweiligen Primer-Bindungsstelle), welche zumindest teilweise durch sequenzspezifische Strangverdrängung des zweiten Primer-Verlängerungsproduktes via Aktivator-Oligonukleotid bedingt sind.

Dabei kann die Strang-Verdrängung eines zweiten Primer-Verlängerungsproduktes vermittelt durch komplementäre Bindung des Aktivator-Oligonukleotid an den ersten Primer-Verlängerungsprodukt zu einer dauerhaften oder vorübergehenden Trennung des ersten und des zweiten Primer-Verlängerungsproduktes führen. Diese Trennung kann vollständig oder partiell unter sequenzspezifischen Mitwirkung des Aktivator-Oligonukleotids erfolgen.

Bei erfolgreicher Bindung des Aktivator-Oligonukleotides an das erste Primer-Verlängerungsprodukt kommt es zu einer Verminderung der Anzahl von komplementären Basenpaarungen zwischen dem ersten und dem zweiten Primer-Verlängerungsprodukt. Bei gegeben Reaktionsbedingungen wird dabei vorzugsweise die Stabilität der Bindung zwischen dem ersten und dem zweiten Primer-Verlängerungsprodukt soweit herabgesetzt, dass es zu einer Ablösung der beiden Primer-Verlängerungsprodukte kommen kann (z.B. Reaktions-Temperatur in diesem Schritt liegt etwa in der Nähe der Schmelztemperatur des restlichen Komplexes aus dem ersten und dem zweiten Primer-Verlängerungsprodukt). Es resultiert dabei eine vollständige Dissoziation eines ersten Primer-Verlängerungsproduktes und eines zweiten Primer-Verlängerungsproduktes.

Aufgrund geringer Konzentrationen von synthetisierten Primer-Verlängerungsprodukten zu Beginn der Reaktion ist die Reassoziations-Kinetik dieser Stränge erheblich verlangsamt. Die Konzentration der Synthese-Produkte kann beispielsweise von sub-femtomolaren bis zu nanomolaren Konzentrationsbereichen reichen. In diesen Konzentrationsbereichen läuft die Reassoziierung von komplementären Strängen bekanntlich langsam ab. Dadurch kann eine vollständige Ablösung des zweiten Primer-Verlängerungsproduktes vom ersten Primer-Verlängrungsprodukt einen positiven Effekt auf die Reaktions-Kinetik haben. Durch molaren Überschuß des ersten und zweiten Primers binden diese an entsprechende frei gewordene Primer-Bindungsstellen und können somit bei einer neuen Synthese initiierend mitwirken.

In einer bevorzugten Ausführungsform wird Strangverdrängung des zweiten Primer-Verlängerungsprodukts durch Aktivator-Oligonukleotid bis zu seiner Trennung vom ersten Primer-Verlängerungsprodukts bei Reaktionsbedinungen durchgeführt, welche eine Temperatur-bedingte Strang-Ablösung im Bereich des 3'-Segmentes der ersten Primer-Verlängerungsproduktes vorteilhaft unterstützen.

Solche Reaktionsbedingungen sind allerdings dermaßen gewählt, dass keine vollständige Strang-Dissoziation zwischen dem ersten und dem zweiten Primer-Verlängerungsprodukt erfolgt, falls Bindung des Aktivator-Oligonukleotides an das erste Primer-Verlängerungsprodukt nicht erfolgt oder nur unvollständig erfolgt.

Durch die vollständige Ablösung des zwei Primer-Verlängerungsproduktes liegt nun dieser Strang in einzelsträngigen Form in der Reaktoins-Lösung vor, so dass Zugang eines neuen ersten Primers an seine entsprechende Primer-Bindungsstelle in diesem Strang im Wesetnlichen ungehindert ist. Im Allgemeinen wirkt sich das positiv auf die Reaktionskinetik der Initiation der Synthese-Reaktion des ersten Primer-Verlängerungsproduktes aus.

Im Einzelfall kann also eine vorteilhafte Kombination aus Reaktionsbedingungen während des Schrittes des Strangverdrängungs-Vorgangs durch das Aktivator-Oligonukleotid und dem Fortschritt/Ausmaß an Strangverdrängung des zweiten Primer-Verlängerungsproduktes bis hin zu Positionen in seiner Sequenz, bei welchen der restliche Doppelstrang aus dem 3'-Segment des ersten Primer-Verlängerungsproduktes und dem zweiten Primer-Verlängerungsproduktes zunehmend instabil wird und mit hoher Wahrscheinlichkeit unter solchen Reaktionsbedingungen zerfällt, so dass eine vollständige Ablösung vom ersten Primer-Verlängerungsprodukt erfolgt.

Aufgrund geringer Konzentrationen von Synthese-Produkten zu Beginn der Reaktion und damit verbundene beinahe vernachlässigbare Reassoziation von beiden synthetisierten Strängen, wird ein positiver Effekt auf die Geschwindigkeit der Synthese von solchen Strängen ausgeübt.

Mit steigender Konzentration von neu synthetisierten Strängen sinkt im Allgemeinen die Reaktionsgeschwindigkeit. Dieser Effekt wird zumindest zu einem Teil auf die beschleunigte Re-Assoziation beider Primer-Verlängerungsprodukte zurückgeführt, was zu einer verminderten Exposition von Primer-Bindungsstellen mit sich bringt.

Durch Änderung von Reaktions-Bedinungen, welche eine solche Reassoziation unterdrücken, kann die Geschwindigkeit der Reaktion begünstigt werden. Beispielsweise kann die Konzentration des zweiten Primers erhöht werden, was zu einer kompetetiven Besetzung der zweiten Primer-Bindungsstelle führen kann. Andererseits kann die Temperatur des Verfahrens mit zunehmendem Fortschritt der Reaktion erhöht werden.

In einer vorteilhaften Ausführungsform des Verfahrens werden beide Primer im Wesentlichen in gleich hohen Konzentrationen, oder in Konzentrationsbereichen, welche in etwas gleich hoch sind, eingesetzt.

In einer weiteren vorteilhaften Ausführungsfrom des Verfahrens wird mindestens eines der beiden Primer in einer höheren Konzentration eingesetzt, als sein Partnerprimer. Die Konzentrationsunterschiede können dabei in Bereichen liegen, welche zwischen 1:2 bis 1:50, vorteilhafter zwischen 1:2 bis 1:10 liegen.

Dabei kann eine asymmetrische Amplifikations-Reaktion resultieren, bei welcher Konzentration eines Primer-Verlängerungsproduktes entsprechend höher liegt als die des anderen Stranges.

Die im Folgenden aufgeführten Beispiele sollen lediglich zu Demonstrationszwecken des Verfahrens angeführt werden und nicht als Einschränkung verstanden werden.

Die in den Beispielen angeführten Strukturen, Sequenzen und Reaktiosbedinungen sollten lediglich Funktionsweise des Verfahrens darstellen und anschaulich machen, und dienen nicht als Einschränkung.

### Beispiele:

### Material und Methoden:

Reagenzien wurden von folgenden kommerziellen Anbietern bezogen:
- nicht modifizierte und modifizierte Oligonukleotide (Eurofins MWG, Eurogentec, Biomers, Trilink Technologies, IBA Solutions for Life Sciences)
- Polymerasen NEB (New England Biolabs)
- dNTP's: Jena Bioscience
- interkallierender EvaGreen Farbstoff: Jena Bioscience
- Puffer-Substanzen und andere Chemikalien: Sigma-Aldrich
- Plastikware: Sarstedt

Lösung 1 (Puffer-Lösung 1):
- Kalium Glutamat, 50 mmol/l, pH 8,0
- Magnesium Acetat, 10 mmol/I
- Triton X-100, 0,1% (v/v)
- EDTA, 0,1 mmol/I
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

Lösung 2 (Amplifikationsreaktion Lösung 2) (Standard-Reaktion):
- Kalium Glutamat, 50 mmol/l, pH 8,0
- Magnesium Acetat, 10 mmol/I
- dNTP (dATP, dCTP, dTTP, dGTP), je 200 µmol/l
- Polymerase (Bst 2.0 WarmStart, 120.000 U/ml NEB), 12 Units / 10 µl
- Triton X-100, 0,1% (v/v)
- EDTA, 0,1 mmol/I
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).
- Primer 1: 10 µmol/l
- Primer 2: 10 µmol/l
- Aktivator-Oligonukleotid: 10 µmol/l
- Matrizenstrang - variable Konzentrationen, wie in Beispielen indiziert.
-

Alle Konzentrationen sind Angaben der Endkonzentrationen in der Reaktion. Abweichungen von der Standard-Reaktion werden entsprechend angegeben.

Die Schmelztemperatur (Tm) von beteiligten Komponenten wurde bei Konzentration von 1 µmol/l von jeweiligen Komponenten in Lösung 1 bestimmt. Abweichende Parameter sind jeweils angegeben.

### Allgemeine Informationen zu Reaktionen

Primer-Verlängerungsreaktionen und Amplifikation wurden standardmäßig bei einer Reaktionstemperatur von 65°C durchgeführt. Abweichungen werden angegeben.

Der Reaktionsstart erfolgte durch Erhitzen der Reaktionslösungen auf Reaktionstemperatur, da Bst 2.0 Polymerase Warmstart bei niedrigeren Temperauren größtenteils in ihrer Funktion durch ein temperatur-sensitives Oligonukleotid gehemmt ist (laut Hersteller-Angaben). Die Polymerase wird zunehmend aktiver ab einer Temperatur von ca. 45°C, bei einer Temperatur von 65°C konnten keine Unterschiede zwischen Polymerase Bst 2.0 und Bst 2.0 Warmstart festgestellt werden. Um der extensiven Bildung von Nebenprodukten (z.B. Primer-Dimeren) während der Vorbereitungsphase einer Reaktion vorzubeugen, wurde Polymerase Bst 2.0 Warmstart verwendet. Abweichungen davon werden speziell angegeben.

Reaktionsstopp erfolgte durch Erhitzen der Reaktionslösung auf über 80°C, z.B. 10 min bei 95°C. Bei dieser Temperatur wird Polymerase Bst 2.0 irreversible denaturiert und das Ergebnis der Synthese-Reaktion kann nachträglich nicht geändert werden.

Reaktionen wurden in einem Thermostat mit einer Fluoreszenz-Messvorrichtung ausgeführt. Zu diesem Zweck wurde ein kommerzielles Real-Time PCR Gerät verwendet, StepOne Plus (Applied Biosystems, Thermofischer). Das Reaktionsvolumen betrug standardmäßig 10 µl. Abweichungen davon werden angegeben.

Sowohl Endpunkt-Bestimmung als auch kinetische Beobachtungen wurden vorgenommen. Bei Endpunktbestimmungen wurde das Signal beispielsweise von an Nukleinsäuren gebundenen Farbstoffen registriert, z.B. von TMR (Tetramethyl-Rhodamine, auch TAMRA genannt) oder von FAM (Fluorescein). Die Wellenlängen zur Anregung und Messung der Fluoreszenzsignale von FAM und TMR sind als Werkeinstellungen bei StepOne Plus Real-Time PCR Gerät gespeichert. Ebenfalls wurde ein interkallierender Farbstoff (EvaGreen) bei Endpunktmessungen vorgenommen, z.B. bei Messung einer Schmelzkurve). EvaGreen ist ein interkallierender Farbstoff und ist ein Analogon des häufig eingesetzten Farbstoff Sybrgreen, allerdings mit etwas geringerer Inhibierung von Polymerasen. Die Wellenlängen zur Anregung und Messung der Fluoreszenzsignale von SybreGreen und EvaGreen sind identisch und sind als Werkeinstellungen bei StepOne Plus Real-Time PCR Gerät gespeichert. Die Fluoreszenz kann mittels eingebauter Detektoren fortlaufend, d.h. "online" oder "Real-Time" detektiert werden. Da die Polymerase während ihrer Synthese einen Doppelstrang synthetisiert, konnte diese Technik zu kinetischen Messungen (Real-Time Monitoring) der Reaktion verwendet werden. Aufgrund von einem gewissen Cross-Talk zwischen Farb-Kanälen bei StepOne Plus Gerät wurde teilweise erhöhte basale Signal-Intensität bei Messungen beobachtet, bei welchen z.B. TMR-markierte Primer in Konzentration von über 1 µmol/l (z.B. 10 µmol/l) eingesetzt waren. Es wurde beobachtet, dass TMR-Signal in Sybre-Green-Kanal zu erhöhten Grundwerten führt. Diese erhöhten Grundwerte wurden bei Berechnungen berücksichtigt.

Die kinetischen Beobachtungen von Reaktionsverläufen wurden routinemäßig mittels Fluoreszenzsignalen von Fluoreszein (FAM-TAMRA Fret-Paar) bzw. interkallierenden Farbstoffen (EvaGreen) aufgenommen. Zeitabhängigkeit des Signal-Verlaufs wurde registriert (Real-Time Signal Erfassung bei StepOne plus PCR Gerät). Ein Anstieg des Signals während einer Reaktion vergleichen zu einer Kontroll-Reaktion wurde je nach Aufbau des Ansatzes interpretiert. Beispielsweise, eine Zunahme des Signals bei Verwendung von Evagreen-Farbstoff wurde als Hinweise auf Zunahme der Menge an doppelsträngigen Nukleinsäureketten während der Reaktion interpretiert, und somit als Ergebnis einer stattfindenden Synthese durch DNA-Polymerase gewertet.

Bei einigen Reaktionen wurde im Anschluss an die Reaktion eine Schmelzkurvenbestimmung durchgeführt. Solche Messungen erlauben Rückschlüsse auf das Vorliegen von Doppelsträngen, welche beispielsweise interkallierende Farbstoffe aufnehmen können und dadurch die Signalintensität von Farbstoffen deutlich verstärken. Mit der steigenden Temperatur sinkt der Anteil von Doppelsträngen und die Signal-Intensität sinkt ebenfalls. Das Signal ist von der Länge von Nukleinsäureketten und von der Sequenzzusammensetzung abhängig. Diese Technik ist einem Fachmann hinreichend bekannt.

Bei Verwendung der Schmelzkurven-Analyse im Zusammenhang mit Reaktionen, welche signifikante Anteile von modifizierten Nukleinsäureketten (z.B. Aktivator-Oligonukleotide oder Primer) enthielten, wurde festgestellt, dass sich das Signal von Farbstoff EvaGreen beispielsweise unterschiedlich zwischen B-Form der DNA und A-Form von modifizierten Nukleinsäureketten verhalten kann. Beispielsweise wurde bei B-Form der doppelsträngigen Nukleinsäureketten (gewöhnlich angenommen für klassische DNA-Abschnitte) eine höhere Signal-Intensität beobachtet, als bei doppelsträngigen Nukleinsäureketten mit der gleichen Sequenz von Nukleobasen, welche eine A-Form ähnliche Konformation annehmen können (z.B. durch mehrere 2'-O-Me Modifikationen von Nukleotiden). Diese Beobachtung wurde beim Einsatz von interkallierenden Farbstoffen berücksichtigt.

Bei Bedarf wurde die Reaktion mittels Kapillarelektrophorese analysiert und die Länge von gebildeten Fragmenten mit einem Standard verglichen. Als Vorbereitung auf die Kapillarelektrophorese wurde die Reaktionsmischung in einem Puffer (Tris-HCI, 20 mmol/l, pH 8,0, und EDTA, 20 mmol/l, pH 8,0) dermaßen verdünnt, dass die Konzentration von markierten Nukleinsäuren ca. 20 nmol/l betrug. Die Kapillar-Elektrophorese wurde bei Firma GATC-Biotech (Konstanz, Deutschland) als Auftragsleistung durchgeführt. Nach Angaben des Anbieters wurde die Kapillarelektrophorese auf einem ABI 3730 Cappilary Sequencer unter Standard-Bedingungen für eine Sanger-Sequenzierung unter Verwendung von POP7 Gelmatrix, bei ca. 50°C durchgeführt und konstanter Spannung (ca. 10 kV) durchgeführt. Die verwendeten Bedinungen führten zu Denaturierung von Doppelsträngen, so dass in der Kapillarelektrophorese die einzelsträngige Form von Nukleinsäureketten separiert wurde. Die Elektrophorese ist eine Standardtechnik in der genetischen Analyse. Die automatisierte Kapillar-Elektrophorese wird heute routinemäßig bei Sanger-Sequenzierung eingesetzt. Das Fluoreszenz-Signal wird während der Kapillar-Elektrophorese kontinuierlich aufgezeichnet (gewöhnlich unter Verwendung von virtuellen Filtern), so dass ein Elektrophorogramm entsteht, bei welchem die Signal-Intensität mit der Dauer der Elektrophorese korreliert. Bei kürzeren Fragmenten, z.B. nicht verbrauchte Primer, beobachtet man einen frühren Signal-Peak, bei verlängerten Fragmenten kommt es zu einer zeitlichen Verschiebung der Signale proportional der Länge des extendierten Bereichs. Dank mitgeführten Kontrollen mit bekannten Längen lässt sich die Länge von extendierten Fragmenten ausmessen. Diese Technik ist einem Fachmann bekannt und wird ebenfalls standardmäßig bei Fragment-Längen-Polymorphismus eingesetzt.

### Beispiel 1:

### Nukleinsäureabhängige Strangverdrängung unter isothermalen Bedingungen

Das Beispiel demonstriert die grundsätzliche Fähigkeit von einzelsträngigen komplementären Nukleinsäureketten zu einer nukleinsäureabhängigen, vorwiegend sequenzspezifischen Strangverdrängung in Abwesenheit von strangtrennenden Proteinen bzw. Enzymen (z.B. Helicasen oder Recombinase) und ohne Verbrauch von chemisch gespeicherten Energie in Form von ATP. Es wird demonstriert, dass ein unter Reaktionsbedinungen stabiler Doppelstrang (gebildet aus Strängen, hier genannt A1 und B1, so dass ein A1:B1-Doppelstrang resultiert) von einer einzelsträngigen Nukleinsäurekette (hier genannt C1-Oligonukleotid), welche im wesentlichen zu bestimmten Segmenten des Strang A1 komplementär ist, geöffnet werden kann. Als Ergebnis dieser Doppelstrang-Öffnung wird Strang B1 in einzelsträngige Form überführt, gleichzeitig entsteht ein neuer A1:C1-Doppeistrang. Die Sequenz der in diesem Beispiel verwendeten C1-Nukleinsäureketten wurde dermaßen konstruiert, dass eine nukleinsäurekettenvermittelte sequenzspezifische Strangverdrängungsreaktion an einem doppelsträngigen DNA-Fragment unter isothermalen Bedinungen stattfinden kann. In diesem Beispiel wurde keine Polymerase zur Reaktion zugegeben, es wurden somit keine neuen Nukleinsäurestränge synthetisiert und es fand keine Amplifikation von Nukleinsäureketten statt.

Zu Detektion der Strangverdrängung und Trennung eines vorgebildeten Doppelstranges wurden beide zu trennende Stränge mit Fluorophoren markiert, so dass ein FRET-Paar (bestehend aus Fluorescein und TAMRA) gebildet wurde. Die Fluorophore wurden an einem Ende des A1:B1-Doppeistranges, d.h. an komplementären Enden der beiden Stränge, positioniert. Die hier verwendeten Nukleinsäurestränge wurden chemisch synthetisiert, die Fluorophore wurden während der chemischen Synthese an die entsprechenden Strangenden chemisch gekoppelt. Die Distanz zwischen Fluophoren, wenn beide Stränge aneinander komplementär gebunden sind, wurde dermassen gewählt, dass ein effektives Quenching der Fluorescein-Fluoreszenz durch TAMRA-Farbstoff erfolgen kann. Bei Trennung der Stränge durch irgendeine Einwirkung, z.B. durch Temperatur oder durch Strangverdrängung, kommt es zu Vergößerung der Distanz zwischen einzelnen Fluorophoren, so dass Intensität des Fluoreszenz-Signals des Fluorescein-Markers zunimmt. Die maximale Intensität des Fluorescein-Markers wird erreicht, wenn beide Stränge (A1 und B1) vollständig getrennt sind. Durch Beobachtung der Intensität der Fluoreszenz und ihre Abhängigkeit von Einwirkung verschiedener Parameter (z.B. Temperatur, Zugabe von komplementären oder nicht-komplementären Sequenzen zwecks Strangverdrängung etc.) kann man Rückschlüsse auf die Menge der noch aneinander gebundenen markierten Stränge ziehen.

Im Beispiel 1 wird das Prinzip der nukleinsäureabhängigen sequenzspezifischen Strangverdrängungsreaktion veranschaulicht. Sowohl die Kinetik der Strangverdrängungsreaktion als auch Temperatur-Abhängigkeit der Öffnung des vorgebildeten Doppelstranges (A1:B1) wurde beobachtet, indem das Fluoreszenz-Signal während des Reaktionsablaufs aufgezeichnet wurde. Sowohl isothermale Reaktionsbedinungen als auch Bedingungen mit einem Temperatur-Gradient wurden ausgetestet.

### Vorbereitung des Ausgangsmaterials:

Bei den einzelnen Sequenzen wurden auch die laborinternen Bezeichnungen neben der Sequenz und der SEQ ID NO. angegeben.

### Doppelstrang (A1:B1)

Die Sequenzen für Doppelstrang A1:B1 sind künstliche Sequenzen, die zufällig ausgewählt wurden, wobei auf Abwesentheit von extensiven Hairpins und langen G-Segmenten, bzw. C-Segmenten geachtet wurde. Beide Stränge sind in der Lage, einen komplementären Doppelstrang auszubilden. Ein Doppelstrang hat eine Tm von ca. 75°C (ein weiterer Doppelstrang hat eine Tm von ca. 77°C s.u.), welche höher ist als die Reaktionstemperatur von 65°C. Damit ist solcher Doppelstrang unter Reaktionsbedinungen stabil. Strang A1 ist länger als jeweilige Strang B1, so dass ein 5'-Überhang entsteht. Dieser 5'-Überhang am A1-Strang wurde in Analogie zum Polynukleotid-Schwanz eines ersten Primer-Verlängerungsproduktes konzepiert. Zur Vereinfachung der Synthese enthielt dieser 5'-Überhang aber keine Modifikationen, welche Polymerase-Aktivität blockieren. Da im vorliegenden Beispiel keine Polymerase in der Reaktion vorhanden war, spielte die Ab- / Anwesentheit solcher Modifikationen eine untergeordnete Rolle. Dieser 5'-Überhang ist komplementär zum ersten Bereich der einzelsträngigen Nukleinsäurekette (C1), so dass eine spezifische Bindung der C1-Nukleinsäurekette an diesen Überhang unter Reaktionsbedinungen stattfinden kann. Dank dieser Bindung wird eine hinreichende räumliche Nähe zwischen einem Ende des A1:B1-Doppelstranges und entsprechenden komplementären Segmenten der einzelsträngigen C1-Nukleinsäureketten herbeigeführt, so dass eine Strangverdrängungsreaktion stattfinden kann. Als Ergebnis dieser Reaktion kann ein neuer Doppelstrang A1:C1 entstehen und B1-Strang kann in seine einzelstränige Form überführt werden.
**A1-Strang** SEQ ID NO: 1 **ES**-10-3504
5' CCGAAGCTCGCAGGAACTCAGAGTGTGGAGAGGACGATAGCTAGTCAGATGATGAAGTT 3'TMR

Die Sequenz besteht aus DNA. Eine 3'-endständige TAMRA-Modifikation (endständige TMR-Modifikation) wurde bereits während Synthese angebracht.
**B1-Strang** SEQ ID NO: 2 T-A6P-10-3506
   5'FAM 5' CATCATCTGACTAGCTATCGTCCTCTCCACACTCTGAGTTCCTGCGAG 3'
**B1-Strang** SEQ ID NO: 3 T-A6P-10-3507
   5' FAM 5' CATCATCTGACTAGCTATCGTCCTCTCCACACTCTGAGTTCCTGC 3'

Beide Stränge bestehen aus DNA. Eine 5'-endständige Fluorescein-Farbstoff-Modifikation (endständige FAM-Modifikation) wurde als Reporter ausgewählt und wurde bereits während Synthese an den jeweiligen Strang mit konventionellen Methoden der Oligonukleotid-Chemie angebracht.

Es wurden zwei Varianten des Doppelstranges generiert. Variante 1 enthält einen Doppelstrang mit einem kürzeren Überhang in 5'-Segment des A1-Stranges (6 Nukleotide), dieser Doppelstrang besteht aus SEQ ID NO: 1 und SEQ ID NO: 2), Variante 2 enthält einen Doppelstrang mit einem längeren Überhang in 5'-Segment des A1-Stranges (9 Nukleotide), dieser Doppelstrang besteht aus SEQ ID NO: 1 und SEQ ID NO: 3), die jeweils komplementäre Segmente sind unterstrichen. Überhänge am 3'-Segment des A1-Stranges wurden aus synthetischen Gründen designed und spielen in diesem Beispiel eine untergeordnete Rolle.
A1:B1-Doppe1strang (Variante 1)
   TMR 3' ttGAAGTAGTAGACTGATCGATAGCAGGAGAGGTGTGAGACTCAAGGACGCTCGAAGCC SEQ ID NO: 1 FAM 5' CATCATCTGACTAGCTATCGTCCTCTCCACACTCTGAGTTCCTGCGAG SEQ ID NO: 2
A1:B1-Doppelstrang (Variante 2)
   TMR 3' ttGAAGTAGTAGACTGATCGATAGCAGGAGAGGTGTGAGACTCAAGGACGCTCGAAGCC SEQ ID NO: 1 FAM 5' CATCATCTGACTAGCTATCGTCCTCTCCACACTCTGAGTTCCTGC SEQ ID NO: 3

### Einzelsträngige Nukleinsäureketten (C1-Oligonukleotide):

Eine einzelsträngige Nukleinsäurekette (in diesem Beispiel als C1-Oligonukleotid genannt) kann bei bestimmten Voraussetzungen zu einer Strangverdrängungs-Reaktion am Strang A1:B1 führen unter gleichzeitiger Ausbildung eines neuen Doppelstrangs mit A1 (A1:C1-Doppelstrang). Dabei wird Strang B1 in seine einzelsträngige Form zumindest teilweise überführt. Im Extremfall kann eine Strangverdrängung zu einer vollständigen Dissoziation von A1:B1 führen, so dass B1-Strang von A1-Strang komplett getrennt ist.

C1-Oligonukleotide und Aktivator-Oligonukleotide haben eine ähnliche Funktion: sie vermitteln nukleinsäureabhängige Strangverdrängung am entsprechenden Doppelstrang. Die Bezeichnung C1-Oligonukleotid wird im vorliegenden Beispiel verwendet. Wegen Abwesenheit der Polymerase in diesem Beispiel können zwar auch C1-Oligonukleotide Polymerase-Blockierende Nukleotid-Modifikationen umfassen, die Anwesenheit dieser Modifikationen stellt allerdings keine Voraussetzung für die Demonstration der Strangverdrängung dar (s.u.). Die Bezeichnung Aktivator-Oligonukleotid wird in Beispielen verwendet, in welchen die Reaktionsmischung eine Polymerase umfasst und welche eine exponentielle Amplifikation demonstrieren. Im Unterschied zu C1-Oligonukleotiden müssen Aktivator-Oligonukleotide Polymerase-blockierende Nukleotid-Modifikationen einschließen.

Aufgrund ähnlicher Funktion von C1-Oligonukleotiden und den Aktivator-Oligonukleotiden, wird die Sequenzzusammensetzung von C1-Oligonukleotiden ähnlich dem Aufbau von Aktivator-Oligonukleotide gestaltet. Die C1-Oligonukleotide müssen die zu A1-Strang komplementären Segmente umfassen, damit die Strangverdrängung ablaufen kann. C1-Oligonukleotide verfügen über einen einzelsträngigen ersten, zweiten und dritten Bereich (siehe Struktur von Aktivator-Oligonukleotid). Mit dem ersten Bereich können C1-Oligonukleotide an den einzelsträngigen 5'-Überhang des A1-Stranges binden, Bereiche zwei und drei binden die inneren Segmente des A1-Stranges, ähnlich wie bei Bindung eines Aktivator-Oligonukleotides und eines ersten Primer-Verlängerungsproduktes.

In diesem Beispiel soll auch die Fähigkeit von Aktivator-Oligonukleotiden-Strukturen zu einer effektiven Strangverdrängung demonstriert werden. Aus diesem Grund wurden einige C1-Nukleinsäureketten identisch zu Aktivator-Oligonukleotiden (z.B. SEQ ID NO: 4, 5, 6 und 7) gestaltet. Andere C1-Nukleinsäureketten (SEQ ID NO: 8, 9 und 10) wurden nur in diesem Beispiel zu Demonstrationszweicken der Strangverdrängung eingesetzt, sie bestehen aus DNA und enthalten keine internen Modifikationen, welche Polymerase-Aktivität blockieren können. Da im vorliegenden Beispiel keine Polymerase in der Reaktion vorhanden war, spielte die Ab- / Anwesentheit solcher Modifikationen in diesem Beispiel eine untergeordnete Rolle. Die Strukturen von jeweiligen einzelsträngigen Nukleinsäureketten (C1-Oligonukleotide) sind nachfolgend zusammengefasst:

### C1-Oligonukleotide (Gruppe 1, Oligonukleotide mit Struktur von Aktivator-Oligonukleotiden):

Folgende vier C1-Oligonukleotide können ggf. als Aktivator-Oligonukleotide auftreten.
SEQ ID NO: 4 A6 8504-001
   5'-[CGUCCUCUCCACACUCUGAGUUCCUG]CGAGCTTCGGTACAAG -3'
SEQ ID NO: 5 A6 8504-002
   5'- [AAAACAAACUAGCUAUCGUCCUCUCCACACUCUGAGUUCCUG]CGAGCTTC GGTACAAG -3'
SEQ ID NO: 6 A6 8507
   5'- CTAGCTATCGTCCTCTCCACA[CUCUGAGUUCCUG]CGAGCTTCGGTACAAG -3'
SEQ ID NO: 7 A26-1000-103 (negativ-kontrolle)
   5'- [AUUCAAAUGUGUUCUCAACGUCCUCU*ACUCAU*GUUCCUG]CGAGCTTCGGTAC AAAA - 3'

### Nummerierung von 5' zu 3'

Die Sequenz mit SEQ ID NO 4, 5, 6 und 7 wurden als Aktivator-Oligonukleotide konstruiert. Sie umfassen 2'-O-Me Nukleotid-Modifikationen (angegeben in [ ]-Klammern) und DNA Nukleotide (im 3'-Segment und ggf. im 5'-Segment), die 3'-OH-Gruppe des 3'-endständigen Nukleotides war durch Phosphat-Rest blockiert. Oligonukleotide wurden chemisch von einem kommerziellen Anbieter synthetisiert. Das unterstrichene Segment der Sequenz kann komplementär an den A1-Strang binden. Die Nukleinsäuren mit SEQ ID NO 4, 5 und 6 sind in ihren Bereichen ein, zwei und drei mit der Sequenz des Stranges A1 komplementär (komplementäre Sequenz des zweiten Bereichs gehen nahtlos in die komplementere Sequenzen des dritten Bereichs über). Solche durchgehende Anordnung von komplementären Nukleobasen soll eine Strangverdrängung unterstützen.

Die Nukleinsäure mit SEQ ID NO 7 ist komplementär im Bereich eins und zwei (von Position 33 bis 49), allerdings enthält sie eine zu A1-nicht komplementäre Sequenz (Positionen 1-18 und 27-32). Ein Teil dieser nicht-komplementären Sequenz ist platziert direkt am 5'-Segment des zweiten Bereichs, um das Fortschreiten der Strangverdrängung über diesen Bereich hinaus zu verhindern. Die Nukleinsäurekette diente als Negativ-Kontrolle.

### C1-Oligonukleotide (Gruppe 2, keine Aktivator-Oligonukleotide):

Folgende drei Oligonukleotide wurden ebenfalls in Strangverdrängungsreaktion eingesetzt.
SEQ ID NO: 8 A6 8510
   5'- TTTTTCTAGCTATCGTCCTCTCCACACTCTGAGTTCCTGCGAGCTTCGGTACAAG 3'
SEQ ID NO: 9 A6 8512
   5' TTTTTCTAGCTATCGTC*A*TCTCCACACTCTGAGTT*A*CTGCGAGCTTCGGTACAAG 3'
SEQ ID NO: 10 A6 8514 -1
   5' TTTTTCTAGCTATCGTCCTCTCCACA CTGAGTTCCTGCGAGCTTCGGTACAAG 3'

### Nummerierung von 5' zu 3'

Die Sequenz mit SEQ ID NO 8 - 10 wurde in ihrer Nukleobasenzusammensetzung ähnlich wie oben genannte Aktivator-Oligonukleotide konstruiert. Es wurden allerdings keine zu Polymerase-Blockade führenden Gruppe an entsprechender Stellen im inneren Segment der Oligonukleotide eingeführt: es fehlen Modifikationen des Zucker-Phosphat-Rückgrades (Polymerase-blockierender Modifikationen) im inneren Bereich der Sequenz, sowie eine 3'-blockierenden Gruppe. Diese C1-Oligonukleotide bestehen vollständig aus DNA-Nukleotiden. Oligonukleotide wurden chemisch von einem kommerziellen Anbieter synthetisiert. Das unterstrichene Segment der Sequenz kann komplementär an den A1-Strang binden. Dabei enthält die SEQ ID NO 8 einen durchgehend komplementeren Segment. Dieses Segment entspricht einem ersten, zweiten und dritten Bereich in einem Aktivator-Oligonukleotid. Die Nukleinsäuren mit SEQ ID NO 9 und 10 umfassend je zwei Sequenzabweichungen von durchgehend komplementären Sequenzen zu A1-Strang. SEQ ID NO 9 enthält an Positionen 18 und 36 je ein A-Nukleotid statt eines C-Nukleotids. Nukleotide an diesen Positionen stellen keine komplementäre Nukleobase zu entsprechenden Positionen in Strang A1 dar. Nukleinsäurekette mit SEQ ID NO 10 hat eine Abweichung in Komplementarität in Form einer Deletion: zwischen Positionen 26 und 27 fehlen 2 Nukleotide (CT). Diese Abweichung führt zu einer Unterbrechung einer komplementären Reihe bei Ausbildung von A1:C1 Doppelstrang.

### Erstes Primer-Oligonukleotid (Primer-1).

Das erste Primer-Oligonukleotid wurde in der Reaktion eingesetzt, um einen temperaturabhängigen Start der Strangverdrängung zu initiieren.
SEQ ID NO: 11 A6P-10-1001
5' - GTA[CCGAAGC]T*[CG]CAGGAAC - 3'

### Nummerierung von 5' zu 3'

Das erste Primer-Oligonukleotid umfasst 2'-O-Me Nukleotid-Modifikationen (angegeben in [ ]-Klammern) und DNA Nukleotide (im 3'-Segment und ggf. im 5'-Segment), die 3'-OH-Gruppe des 3'-endständigen Nukleotides ist frei. Das Oligonukleotid hat in seiner Position 11 einen mit TAMRA-Farbstoff modifizierten dT-Nukleotid (als T* in der Sequenz angezeigt). Das Oligonukleotid wurde chemisch von einem kommerziellen Anbieter synthetisiert. Der Primer kann mit seiner Sequenz komplementär an C1-Oligonukleotide binden. Dank dieser Bindung zwischen dem Primer-1 und C1-Oligonukleotid sind bestimmte Sequenzsegmente des jeweiligen C1-Oligonukleotides während der Assemblierung der Reaktionsmischung (durchgeführt unter Raumtemperatur) doppelsträngig und können nicht mit dem einzelsträngigen Überhang des A1-Stranges interagieren.

### Vorbereitung eines komplementären Doppelstranges (A1 und B1)

Die beiden jeweils komplementäre Stränge von Nukleinsäuren (A1 und B1) wurden in Konzentration von je 1 µmol/l in der Puffer-Lösung 1 (ohne EvaGreen-Farbstoff) zusammengeführt, kurzfristig erhitzt und bei Raumtemperatur zur Abkühlung stehen gelassen, so dass ein Doppelstrang (A1:B1) über die komplementäre Bereich ausgebildet werden kann. Es resultierten zwei Varianten von Doppelstrang:
Variante 1 enthält Sequenzen mit SEQ ID NO: 1 und SEQ ID NO: 2
Variante 2 enthält Sequenzen mit SEQ ID NO: 1 und SEQ ID NO: 3

**Vorbereitung von** einzelsträngigen Nukleinsäurekette (C1), **(ohne Primer-1 Komplex)** Die jeweilige einzelsträngige Nukleinsäurekette (C1), wurde in Puffer-Lösung 1 (ohne EvaGreen-Farbstoff) in Konzentration von 10 µmol/l vorgelegt.

### Vorbereitung von C1:Primer-1 Komplexen aus einzelsträngigen Nukleinsäurekette (C1) und Primer-1

Um den ersten und zweiten Bereich der einzelsträngigen Nukleinsäurekette (C1) reversibel zu blockieren wurde Primer-1 in equimolarer Konzentration zur jeweiligen C1-Oligonukleotid gegeben, so dass sich ein partiell doppelstranger C1:Primer-1 Komplex ausbilden konnte. Der Primer-1 bindet spezifisch an den ersten und zweiten Bereich des C1-Oligonukleotides und blockiert somit Sequenzsegment, welches mit dem Überhang des A1-Stranges während der Vorbereitungsphase der Reaktionsmischung (unter Raumtemperatur) interagieren kann. Dieser Komplex ist bei Raumtemperatur stabil und kann erst unter Reaktionsbedinungen in seine Einzelteile dissoziieren, so dass der erste und der zweite Bereiche des C1-Oligonukleotides einzelsträngig werden und somit mit entsprechenden Strukturen des Doppelstranges (A1:B1) interagieren können. Die Komplexe aus C1-Oligonukleotid und Primer 1 wurden ebenfalls in Puffer-Lösung 1 (ohne EvaGreen-Farbstoff) in Konzentration von 10 µmol/l vorgelegt.

### Vorbereitung der Reaktionsmischung, Reaktions-Start und Reaktionsbedingungen

Die Reaktionsmischung wurde durch Zusammenführung von gleichen Volumina (je 5 µl) einer Lösung mit einem Doppelstrang (A1:B1) und einer Lösung mit einem zu testenden C1-Oligonukleotid bzw. C1-Primer-1-Komplex bei Raumtemperatur vorbereitet. In der resultierenden Reaktionsmischung liegen die einzelnen Stränge in folgenden Konzentrationen vor:
Doppelstrang A1:B1 in Konzentration von 0,5 µmol/l
Einzelstrang (C1) bzw. Komplex C1-Primer-1 in Konzentration von 5 µmol/l
Einzelstrang B1 (als FAM-Signal-Kontrolle) in Konzentration von 0,5 µmol/l.

### Beobachtung des Verlaufs der Strangverdrängung unter isothermalen Bedingungen von 65°C

Die so vorbereitete Reaktionsmischung wurde in einem entsprechenden Reaktionsgefäß (Mikrotiter-Platte) in ein Thermonstat mit mit einer Fluoreszenz-Detektions-Funktion überführt. Als Thermostat wurde ein Real-Time-PCR Gerät ("StepOne Plus", StepOne Software v2.1 ABI, Themorfischer) verwendet. Die Reaktionsmischung wurde auf Temperatur von zunächst 50°C für ca. 50 sec vorgewärmt (Zyklus 1 bis 5) und anschließend auf 65°C erwärmt. Diese Temperatur wurde über ca. 10 min aufrechterhalten (Zyklus 6 bis 30 wurde aufgezeichnet), so dass eine Strangverdrängungsreaktion unter isothermalen Bedinungen verlaufen und beobachtet werden konnte. Das Fluoreszenzsignal von FAM wurde in Abhängigkeit von der Zeit (Real-Time Funktion des Geräts) aufgezeichnet. Dank Verwendung von Real-Time-PCR Gerät konnte ein vom Hersteller mitgeliefertes Software-Programm mit entsprechenden FAM-Filter-Einstellungen verwendet werden. Der minimale vom Hersteller vorgesehene Zeitinterval zwischen zwei Messungen betrug 10 sec (minimaler einstellbarer Zeitinterval), was insgesamt zu einem gerätebedingten Mess-Zyklus von ca. 15 bis 18 sec führte. Der Verlauf des FAM-Signals wurde mit dieser maximalen Aufzeichnungsgeschwindigkeit aufgenommen.

### Beobachtung der Trennung von A1 und B1 während eines thermalen Gradienen (50°C - 95°C) bei Anwesenheit / Abwesenheit von C1-Oligonukleotiden.

In einem weiteren Experiment wurde die Reaktionsmischung kontinuierlich von 50°C auf 95°C erwärmt (ein Temperatur-Gradient). Während dieser Aufwärmfphase wurde ebenfalls FAM-Signal detektiert, wobei pro 0,5° C Temperatur-Zuwachs eine FAM-Signalmessung erfolgte. Es resultierte ein Thermo-Profil der Reaktionsmischung, bei welchem Signal-Intensität des FAM-Reportes in Abhängigkeit von Temperatur aufgezeichnet wurde. Dieses Experiment wurde ebenfalls mit Hilfe von Real-Time PCR Gerätes (StepOne Plus) durchgeführt, wobei das Programm zur Schmelzkurvenbestimmung verwendet wurde (vorinstalliert vom Hersteller, Software Version StepOne v2.1).

Beide Experimente wurden für jeweilige Reaktionsmischung ausgewertet und Ergebnisse werden nachfolgend vorgestellt. Die Zusammensetzung einzeler Reaktionen wird an entsprechenden Stellen angegeben.

### Ergebnis:

### 1. Testung des Detektionssystems:

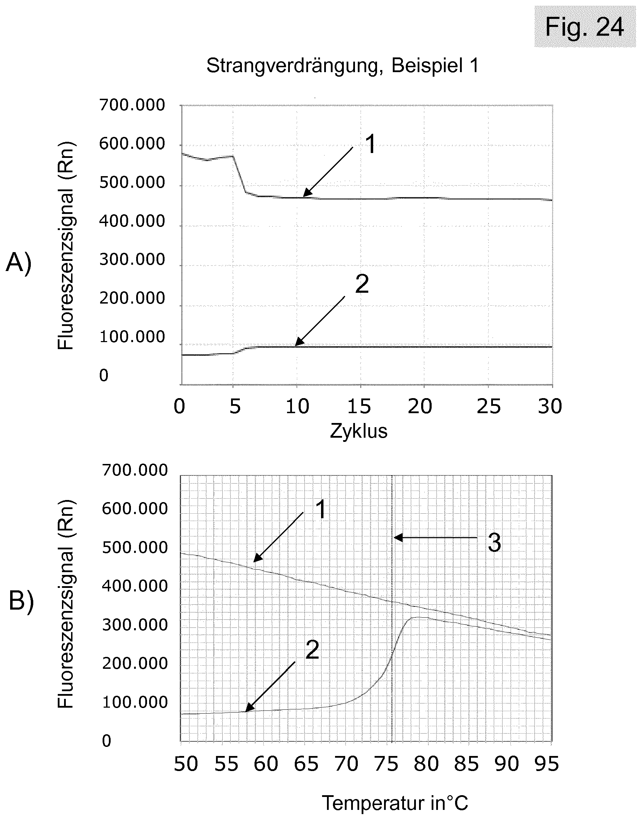

Die Funktionsfähigkeit des FRET-Paares aus FAM und TAMRA wurde in zwei Kontrollansätzen überprüft. Der erste Kontrollansatz enthielt nur einen B1-Strang markiert mit einem FAM-Farbstoff (Fig.24 Pfeil 1), der zweite Kontroll-Anzsatz enthielt sowohl einen mit TAMRA markierten A1-Strang als auch einen mit FAM markierten B1-Strang (A1:B1-Doppelstrang) (Fig. 24 Pfeil 2). Sowohl zeitlicher Verlauf des Signals unter isothermalen Bedingungen (Fig. 24 A) als auch das Verhalten des Signals im Temperatur-Gradient wurden aufgezeichnet (Fig. 24 B). Man erkennt jeweils eine stabile Signal-Linie bei isothermalen Bedinungen (65°C) für beide Kontroll-Ansätze (Fig. 24 A). Bei Aufzeichnung des Temperatur-Gradienten (Fig. 24 B) des Doppelstranges (Pfeil 2) sieht man einen Anstieg der Fluoreszenz des FAM-Reporters ab ca. 70°C. Das Signal erreicht bei ca. 80°C sein Maximum und verhält sich anschließend wie ein Einzelstrang (vergleiche Pfeil 1). Die errechnete Schmelztemperatur dieses Stranges (Variante 2 des Doppelstranges A1:B1 mit 9 Nukleotiden im 5'-Überhang A1-Stranges) liegt bei ca. 75°C (Pfeil 3). Bei dieser Temperaur ist definitionsgemäß die Hälfte des Doppelstranges geschmolzen, so dass Stränge A1 und B1 je zu Hälfte einzelsträngig vorliegen. Die Kontroll-Ansätze für Variante 1 des Doppelstranges A1:B1 mit 6 Nukleotiden im 5'-Überhang des A1-Stranges verhalten sich ähnlich, die Tm dieser Variante 1 liegt bei ca. 77°C.

Pfeile 1 und 2 zeigen auch in folgenden Figuren 24 bis Fig. 29 die jeweiligen Kontroll-Ansätze mit einem einzelsträngigen FAM-markierten B1-Strang (Pfeil 1) und einem markierten Doppelstrang A1:B1 (Pfeil 2).

### 2. Strangverdrängung durch ein einzelsträngiges komplementäres Oligonukleotid

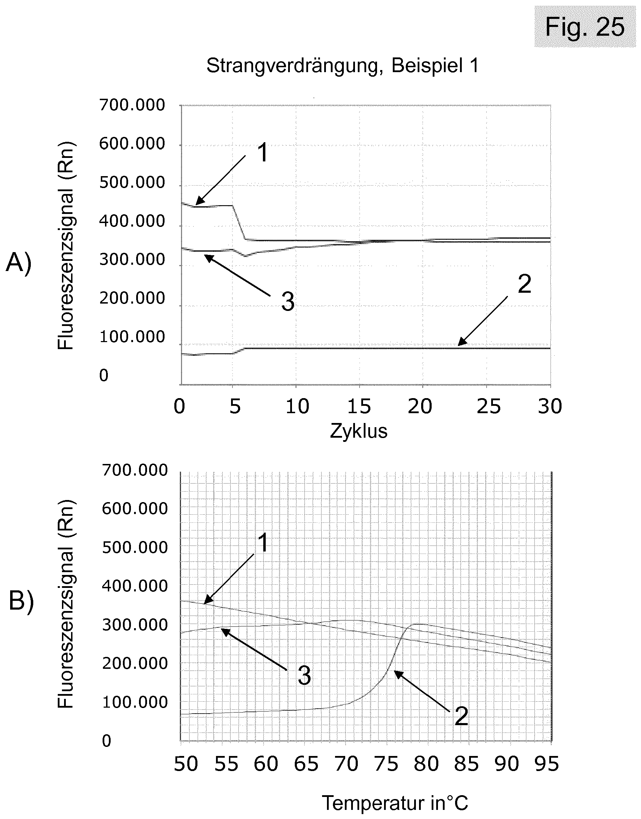

Bei durchgehend komlementären Zusammensetzung der Sequenzen der C1-Oligonukleotide und A1-Stränge (z.B. Reaktion zwischen A1:B1 Variante 2 und C1-Oligonukleotid SEQ ID NO 5 A6 8504) verlief die Strangverdrängung des B1-Strangesunter teilweise dermaßen schnell, dass ein signifikanter Anteil der B1-Stränge bereits während der Vorbereitung der Raktionsmischung bzw. noch vor Start der Aufzeichnung einzelsträngig vorlag. Das sieht man anhand der gestiegenen Signalintensität in Fig. 25 (Pfeil 3). Bereits die erste Messung (Zyklus 1) weist einen deutlich höheres Signalniveau als die Kontroll-Reaktion mit einem A1:B1-Doppelstrang ohne C1-Oligonukleotid. Somit liegt B1-Strang größtenteils bereits vor der ersten Messung in einzelsträngiger Form. Ein ähnliches Verhalten zeigen auch Reaktionen mit A1:B1-Doppelstrang Variante 1.

Ein Austausch des C1-Oligonukleotids durch ein anderes durchgehend komplementäres C1-Oligonukleotid (z.B. SEQ ID NO 4, 6 oder 8) zeigen grundsätzlich ähnliches Verhalten. Die hohe Intensität des Fluoreszenz-Signals von FAM-Reporter bereits zu Beginn der Messung deutet auf Vorliegen von B1-Stranges in einzelsträngiger Form noch vor der ersten Messung. Dieses Verhalten von entsprechenden C1-Oligonukleotide zeigt, dass die ggf. vorhandenen Unterschiede in Zusammensetzung des Zucker-Phosphat-Rückgrades (DNA vs. 2'-O-Methyl Modifikationen) im vorliegenden Beispiel nur unwesentliche Unterschiede im Ergebnis der Strangverdrängung durch einzelne C1-Oligonukleotide bewirkten. Sowohl Oligonukleotide mit längeren 2'-O-Me Segmenten als auch komplett aus DNA bestehende Oligonukleotide führten zum gleichen Ergebnis. Die durchgehende Reihenfolge von komplementären Basen insbesondere in Bereichen zwei und drei von C1-Oligonukleotiden sorgte für eine Strangverdrängung mit hohen Ausbeuten.

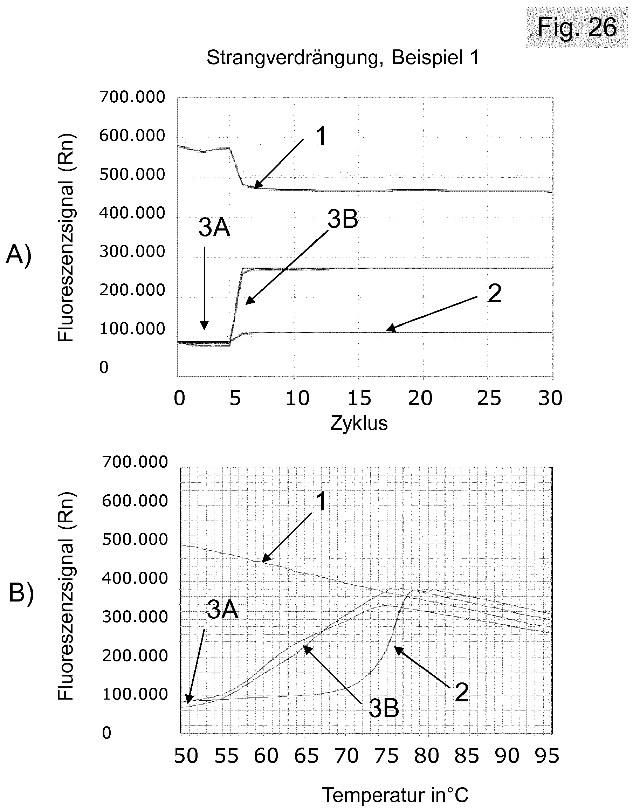

Verwendung von C1-Strang mit SEQ ID NO: 4 (8504 kurz) mit einer durchgehenen komplementären Zusammensetzung einzelner Bereiche allerdings mit einem kürzeren komplementären dritten Bereich führte zum Ergebnis dargestellt in Fig. 26. Ansätze mit C1 und A1:B1-Doppelstrang Varianten 1, sowie C1 und A1:B1-Doppelstrang Variante 2 verhalten sich ähnlich (Pfeil 3): erst nach Erhöhung der Temperatur auf 65°C kommt es zu einer Strangverdrängung.

Eine Verkürzung der C1-Sequenz am seinem 5'-Ende (entspricht dem dritten Bereich eines Aktivator-Oligonukleotides) (SEQ ID NO: 4) führte dazu, dass ein längerer Sequenzabschnitt im 3'-Segement des A1-Stranges nicht von C1-Oligonukleotid komplementär gebunden wird, so dass dieses 3'-ständige Segment des A1-Stranges in Kontakt mit 5'-Segment des B1-Strang bleiben kann (Positionen 1 bis 18 des 5'-Segment des B1 Stranges). Dieses doppelsträngige Fragment ist bei 50°C hinreichend stabil, so dass A1:B1-Doppelstrang an seinem markierten Ende auch bei 50°C hinreichend Kontakt behält, und das Signal von FAM bei dieser Temperatur hinreichend gequenched (Pfeil 3, Zyklus 1 bis 5, Fig. 26) ist. Mit Erhöhung der Temperatur auf 65°C (Pfeil 3B Fig. 26) führte die Anwesenheit von C1-Oligonukleotid dazu, dass ca. die Hälfte der Population von A1:B1-Doppelstrangen in Einzelstränge zerfiel. Es bildet sich ein Gleichgewicht zwischen einer A1:B1-Doppelstrang-Form und Einzelstrangform von B1-Strang (3-Strang-Komplex). Die Fluoreszenzintensität von FAM-Reporter im Ansatz entspricht ca. 50% der Intensität eines freien Stranges. Dieses Verhalten wird darauf zurückgeführt, dass bei angenommenen voll ausgeprägten Strangverdrängung durch C1-Oligonukleotid (d.h. vollständige Bindung zwischen komplementären Bereichen von A1-Strang und C1-Oligonukleotid) der restlich verbleibende Kontakt zwischen dem 3'-Segment von A1-Strang und 5'-Segment des B1-Stranges bei dieser Temperatur nicht mehr hinreichend stabil, so dass es zu einer spontanen, temperatur-induzierten Trennung von A1 und B1 Strängen in diesem Segment kommt. Unter Reaktionsbedinungen kann ein einzelsträngiger B1-Strang wiederum an das 3'-Segment des A1-Strang erneut binden und zur Strangverdrängung von C1-Oligonukleotid führen. Das System befindet sich somit im Gleichgewicht zwischen A1:B1-Doppelstrang, C1:A1-Doppelstrang und ihren Zwischenformen (3-Strang-Komplex).

### 3. Strangverdrängung durch ein einzelsträngiges Oligonukleotid mit Abweichungen in der Sequenzzusammensetzung

Die Testung des Einflusses von Sequenzabweichungen wurde mit C1-Oligonukleotiden ausgeführt, welche in ihrer Zusammensetzung der Nukleobasen zwei oder mehr nicht-komplementären Basen umfassten, welche im zweiten oder dritten Bereich des C1-Oligonukleotides lagen. Diese abweichende Basenzusammensetzung von C1 führte dazu, dass keine durchgehend komplementäre Reihenfolge von Basenpaarungen in einem zu erwartenden Doppelstrang aus A1 und C1 entstehen würde.

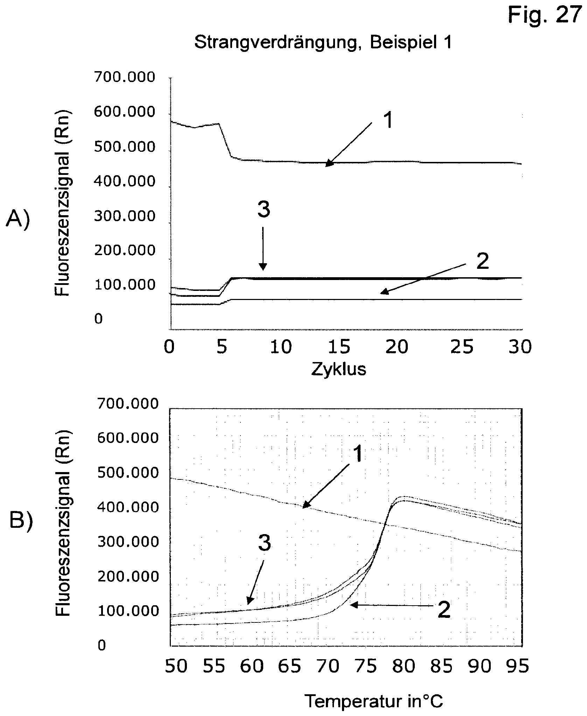

In Fig. 27 ist Ergebnis der Interaktion zwischen C1-Oligonukleotid SEQ ID NO 5 und A1:B1-Doppelstrang Variante 1 gezeigt. Die Abweichungen von der durchgehend komplementären Sequenz sind in Positionen 18 und 36 (SEQ ID NO 9) und Deletion zwischen Positionen 26 und 27 (SEQ ID NO 10) zu sehen. Beide C1-Oligonukleotide führen zu einer geringfügig ausgeprägten Änderung in der Fluoreszenz des FAM-Reporters (Fig. 27, Pfeil 3). Eine solch geringe Signalintensität des FAM-Reportes ist assoziiert mit einer vorwiegend gebundenen Form des B1-Stranges an A1-Strang. Mit anderen Worten, ein C1-Oligonukleotid mit Abweichungen in der Nukleobasenzusammensetzung im zweiten und dritten Bereich führte zu Verringerung von Ausbeuten bei der Strangverdrängung: der A1:B1-Doppelstrang wurde kaum geöffnet und nur geringer Anteil von B1-Strängen wurde in einzelsträngige Form überführt. Das Gleichgewicht der Reaktion lag überwiegend auf der Seite des A1:B1-Doppelstranges.

Eine weitere Erhöhung der Anzahl von Abweichungen in Nukleobasenzusammensetzung führte zu einer vollständigen Aufhebung der Strangverdrängung. Das C1-Oligonukleotid mit SEQ ID NO: 7 umfasst durchgehend komplementäre Nukleobasensequenz im ersten und zweiten Bereich, weist allerdings signifikante Abweichungen im dritten Bereich seiner Sequenz auf. Diese Sequenzabweichungen liegen teilweise im 3'-Segement des dritten Bereichs des C1-Oligonukleotids (Positionen 26 - 32) und sind somit unmittelbar am zweiten Bereich lokalisiert. Die Zugabe eines solchen C1-Oligonukleotid zum vorgelegten A1:B1-Doppelstrang führte zu keiner messbaren Änderung in Fluoreszenz des FAM-Reporters unter 65°C; das Signal ist auf dem Niveau des intakten A1:B1-Doppelstranges geblieben. Das bedeutet, dass obwohl C1-Oligonukleotid in der Lage war, mit seinem ersten und zweiten Bereich an das A1-Strang komplementär zu binden (diese Bereiche umfassen komplementäre Sequenzen zwischen C1 und A1 Strängen), führte Abweichung der Sequenzzusammensetzung im Dritten Bereich zu vollständigen Aufhebung einer erfolgreichen Strangverdrängung unter gegebenen Reaktionsbedinungen, der A1:B1-Doppelstrang blieb weitgehend intakt. Dies wird im Zusammenhang mit der Stabilität des restlichen A1:B1-Doppelstrang gesehen. Im Fall einer komplementären Bindung des ersten und zweiten Bereichs des C1-Oligonukleotides an A1-Strang bleiben A1-Strang und B1-Strang über ein signifikant langes Segment aneinander komplementär gebunden. Unter gegebenen Bedinungen ist diese Stabilität dieses restlichen, nicht verdrängten doppelsträngigen Segmentes (A1:B1) hinreichend hoch, um beide Stränge zusammenzuhalten und eine messbare Trennung des B1 von A1 zu verhindern. Das Gleichgewicht der Reaktion liegt in diesem Fall vollständig auf der Seite der A1:B1-Doppelstranges, es findet keine messbare Trennung von diesen Strängen statt.

### 4. Kinetik der Strangverdrängung

Zur Messung der Kinetik der Strangverdrängung wurden C1:Primer-1 Komplexe und vorbereitete A1:B1-Doppelstränge verwendet. Der Vorteil bei der Verwendung von C1-Primer-1 Komplexen bestand darin, dass Primer 1 an komplementäre Bereiche des C1-Oligonukleotides bereits im Vorfeld binden konnte, was zu einem bei Raumtemperatur stabilen Doppelstrang führte. Dank dieser Reaktionsanordnung konnte eine Strangverdrängung erst gestartet werden, wenn Primer-1 von C1-Strängen unter Reaktionsbedinungen getrennt war, z.B. im Rahmen der Erhöhung der Reaktionstemperatur auf 65°C (entspricht in etwa der Tm von Primer-1 (Tm plus / minus 5°C). Durch Freisetzung der komplementären Bereiche im C1-Oligonukleotid konnte dieses nun unter Reaktionsbedinungen mit dem A1:B1-Doppelstrang interagieren und ggf. zu Strangverdrängung führen.

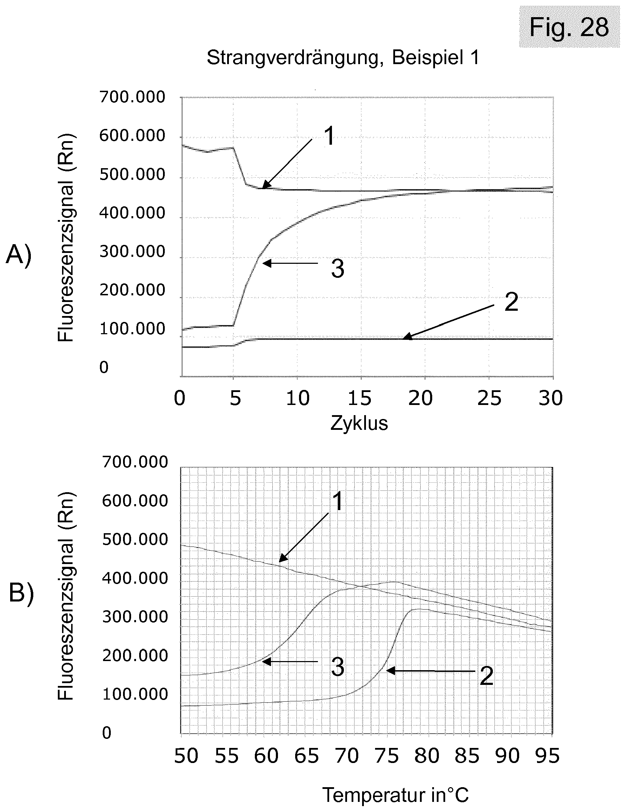

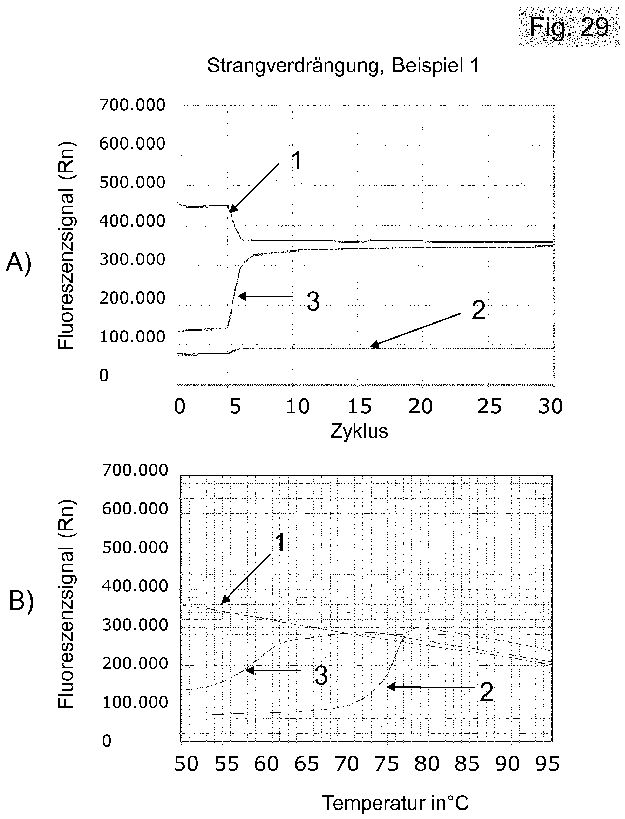

Zu Demonstration der Kinetik der sequenzabhängigen Strangverdrängung wurde Variante 1 des A1:B1-Stranges mit C1-Primer-1 Komplex (bestehend aus SEQ ID NO 5 und 11) in Kontakt gebracht und zunächst auf 50°C (Zyklus 1 - 5) und anschließend auf 65°C (Zyklus 6 und weiter) erhitzt, wie oben dargestellt. Der Verlauf des Signals vom FAM-Reporter ist in Fig. 28 zu sehen. Man beobachtet eine nahe konstante Linie zwischen Zyklus 1 und 5 (50°C) und danach eine sprunghafte Zunahme der Fluoreszenz von FAM-Reporter ab dem 6. Mess-Zyklus (65°C). In darauf folgenden 10 Zyklen (6 bis 16) erreicht die Signalintensität des FAM-Reporters ca. 90% der maximal möglichen Fluoreszenz. Die Zunahme der Signalintensität spricht für Trennung von Strängen A1 und B1. Die daraus errechnete Halbwertzeit der Trennung des A1:B1-Doppelstranges lag bei ca. 40 - 50 sec. Im Vergleich dazu, erfolgte der Anstieg der Fluoreszenz von FAM-Reporter bei Verwendung der Variante 2 des A1:B1-Stranges mit gleichem C1:Primer-1 Komplex (bestehend aus SEQ ID NO 5 und 11) wesentlich schneller (Fig. 29, Pfeil 3). Die errechnete Halbwertzeit der Trennung des A1:B1-Doppelstranges lag in diesem Ansatz unter 10 sec bei gegebenen Reaktionsbedingungen.

Der Unterschied zwischen Variante 1 und 2 des A1:B1-Doppelstrangs besteht in erster Linie in der Länge des 5'-Überhangs des A1-Stranges, mit welchem A1-Strang in den initialen Kontakt mit C1-Oligonukleotid kommt. Der B1-Strang der Variante 1 ist länger als in Variante 2, der dabei resultierende Unterschied ergibt 6-Nukleotide langen 5'-Überhang in der Variante 1 und 9-Nukleotide langen 5'-Überhang in der Variante 2. Durch komplementäre Zusammensetzung entsprechender Segmente im C1-Oligonukleotid ist davor auszugehen, dass die Bindung an 9-Nukleotid-Überhang etwas stabiler ist als die Bindung an einen 6-Nukleotid-Überhang.

Vergleich der Kinetik der Fluoreszenzzunahme zwischen Variante 1 und Variante 2 des A1:B1-Doppelstranges führte zu Schlußfolgerung, dass die Länge des 5'-Überhangs am A1-Strang und somit auch Stabilität eines während der Reaktion geformten Zwischenkomplexes, bestehend aus A1-Überhang und C1-Oligonukleotid, eine Rolle spielen kann: je stabiler dieser Komplex aus A1-Überhang und C1-Oligonukleotid ist, umso effektiver bzw. schneller die Strangverdrängung bei sonst gleichen Parametern erfolgen kann. Dieser Zwischenkomplex aus A1-Überhang und dem ersten Bereich des C1-Oligonukleotides bringt das C1-Oligonukleotid in hinreichende räumliche Nähe zum entsprechenden Duplex-Ende des A1:B1-Oligonukleotids, so dass eine Strangverdrängung erfolgen kann. Im vorliegenden Ansatz resultiert eine Komplex-Bildung zwischen C1-Oligonukleotid und 5'-Überhang des A1-Stranges in annehrend identischer räumlicher Nähe des komplexierten C1-Oligonukleotides zum Duplex-Ende des A1:B1-Doppelstranges sowohl bei Variante 1 als auch bei Variante 2. Die Unterschiede in der Kinetik der Strangverdrängung zeigen jedoch, dass höhere Stabilität der Bindung des Zwischenkomplexes bei gleicher räumlicher Nähe zu Effizienzsteigerung des Vorgangs der Strangverdrängung führen kann. Das Gleichgewicht kann schneller verschoben werden.

In folgenden Beispielen werden einzelne Ausführungsformen einer exponentiellen Amplifikation veranschaulicht. Diese Beispiele dienen zur Veranschaulichtung der Funktion einzelner Elemente der exponentiellen Amplifikation und sollen nicht als Einschränkung verstanden werden. Das Verfahren wird unter Verwendung von isothermalen Bedingungen demonstriert.

Während einer exponentiellen Amplifikation läuft eine polymerasen-abhängige Nukleinsäuresynthese zeitlich gleichzeitig mit der nukleinsäureabhängigen Strangverdrängung ab. Die Reaktionsansätze umfassen Polymerasen und dNTP's als Substrate. Statt eines C1-Oligonukleotides werden Aktivator-Oligonukleotide verwendet, welche in ihren Strängen Nukleotid-Modifikationen umfassen, die zu lokalen Blockade der Polymerase-Tätigkeit führen, so dass Aktivator-Oligonukleotide nicht als Matrize während der Amplifikation auftreten. Es werden je zwei Primer in einer Amplifikation eingesetzt: das erste Primer-Oligonukleotid und das zweite Primer-Oligonukleotid. Das erste Primer-Oligonukleotid umfasst einen Polynukleotid-Schwanz, welcher Aktivator-Oligonukleotide spezifisch binden kann und damit räumliche Nähe zum Duplex-Ende einer zu amplifizierenden Nukleinsäure herbeiführen, so dass eine Strangverdrändung des zweiten Primer-Verlängerungsproduktes stattfinden kann. Das zweite Primer-Oligonukleotid kann an das 3'-Segment des ersten Primer-Verlängerungsproduktes binden und initiiert somit die Synthese des zweiten Primer-Verlängerungsproduktes.

### Beispiel 2:

Dieses Beispiel demonstriert eine exponentiell ablaufende Amplifikation eines artifiziellen DNA-Fragments unter isothermalen Bedinungen. Dabei dienen die neu synthetisierten Stränge als Matrizen für die spätere Synthese der komplementären Partner-Stränge. Die Synthese findet in Abhängigkeit von Präsenz einer spezifischen Matrize statt. Die Vermehrung von zu amplifizierenden Nukleinsäuren erfolgte um ca. Faktor 10⁹ bezogen auf die Ausgangsmenge der eingesetzten Matrize.

### Ausgangsmaterial:

### Matrize:

Als Teil einer zu amplifizierenden Nukleinsäure wurde eine einzelsträngie DNA-Matrize genommen. Die Sequenz der Matrize-001 (SEQ ID NO 12) wurde zufällig ausgewählt und wurde im Hinblick auf Vermeidung von Strecken (mehr als 6 Basen), welche zu einer Doppelstrangbildung innerhalb des gleichen Strangs überprüft, um die Formierung von sog. Hairpins zu vermeiden. Diese DNA Sequenz wurde künstlich von einem kommerziellen Anbieter synthetisiert. Die DNA Sequenz trägt keine Nukleotid-Modifikationen. Das 3'-Ende des Matrizenstrangs ist offen und ist nicht blockiert. Diese Matrize soll als Beispiel für eine einzelsträngige zu amplifizierende Nukleinsäure dienen.

Matrize 001: (SEQ ID NO 12)
T35-4401-601
5'- ATACTACAATGTCACTTACGTCCTCTCCACACTCTGAGTTCCTGCGAG - 3' (Reihenfolge der Nummerierung: 1 - 48)

Die gemessene Schmelztemperatur eines DNA-Doppelstranges, welcher Matrize-001 als ein Strang einschließt, beträgt 77°C (Tm gemessen in Lösung 1).

### Primer-1 (erstes Primer-Oligonukleotid)

Primer 001-01:
SEQ ID NO: 13 A6P-10-1001
5' - GTA[CCGAAGC]T*[CG]CAGGAAC - 3' Nummerierung von 5' zu 3'

Das erste Primer-Oligonukleotid umfasst 2'-O-Me Nukleotid-Modifikationen (angegeben in [ ]-Klammern) und DNA Nukleotide (im 3'-Segment und ggf. im 5'-Segment), die 3'-OH-Gruppe des 3'-endständigen Nukleotides ist frei. Das Oligonukleotid hat in seiner Position 11 einen mit TAMRA-Farbstoff modifizierten dT-Nukleotid (als T* in der Sequenz angezeigt).

### Primer-2 (zweites Primer-Oligonukleotid)

Primer 001-02: (SEQ ID NO 14)
PA6-35-4401-608 5'- AAAAAAAC ATACTACAATGTCACTTAC - 3' (Reihenfolge der Nummerierung: 1 - 27)

Dieser Primer ist für die Initiierung der Synthese des 2. Stranges notwendig. Die Nukleotide in Positionen 9 - 27 sind mit der Matrize-001 identisch.

### Aktivator-Oligonukleotid-001 -01

Aktivator-Olgonukleotid-001-01: (SEQ ID NO 15)
A6 8505
5'- [CUAGCUAUCGUCCUCUCCACACUCUGA GUUCC1TGCGAGCTTCGGTACAAG X -3' (Reihenfolge der Nummerierung: 1 - 50)

Das Aktivator-Oligonukleotid umfaßt 2'-O-Me Nukleotid-Modifikationen (angegeben in [ ]-Klammern) und DNA Nukleotide (im 3'-Segment). Die 3'-OH-Gruppe des 3'-endständigen Nukleotides war durch Phosphat-Rest blockiert (hier mit X gekennzeichnet). Oligonukleotid wurde chemisch von einem kommerziellen Anbieter synthetisiert. Das unterstrichene Segment der Sequenz kann komplementär an das erste Primer-Verlängerungsprodukt binden. Diese durchgehende Anordnung von komplementären Nukleobasen soll eine Strangverdrängung unterstützen. Die Nukleotide und Nukleotid-Modifikationen sind untereinander mit Phosphodiesterbindung verknüpft.

Das Aktivator-Oligonukleotid schließt drei Bereiche ein:
Erster Bereich = Position 35 - 50
Zweiter Bereich = Position 28 - 34
Dritter Bereich = Position 1 - 27

Diese Komponenten wurden wie folg in der Reaktionsmischung eingesetzt:

| | |
|---|---|
| • Matrize-001 | 2 fmol/l |
| • Aktivator-Oligonukleotid-001-01: | 10 µmol/l |
| • Primer 001-01: | 10 µmol/l |
| • Primer 001-02: | 10 µmol/l |

Weitere Komponenten der Reaktion wurden als Amplifikationsreaktion Lösung 2 eingesetzt:
- Kalium Glutamat, 50 mmol/l, pH 8,0
- Magnesium Acetat, 10 mmol/I
- dNTP (dATP, dCTP, dTTP, dGTP), je 200 µmol/l
- Polymerase (Bst 2.0 WarmStart, 120.000 U/ml NEB), 12 Units / 10 µl
- Triton X-100, 0,1% (v/v)
- EDTA, 0,1 mmol/I
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

### Start der Reaktion und Reaktionsbedingungen

Die Vorbereitung der Reaktionsmischung erfolgte bei Raumtemperatur. Die Matrize wurde im Reaktionsansatz als letzte Komponente zugegeben. Ein Kontroll-Ansatz enthielt keine Matrize.

Die so vorbereiteten Reaktionsmischung wurden in je einem entsprechenden Reaktionsgefäß (Mikrotiter-Platte) in ein Thermonstat mit mit einer Fluoreszenz-Detektions-Funktion überführt. Als Thermostat wurde ein Real-Time-PCR Gerät ("StepOne Plus", StepOne Software v2.1 ABI, Themorfischer) verwendet. Der Start der Reaktion erfolgte durch Erhitzung der Reaktionsmischung auf 65°C.

Die Reaktionstemperatur wurde während der gesamten Amplifikation (40 min) bei 65°C konstant gehalten (isothermale Amplifikation). Anschließend wurde Reaktion auf 95°C für 10 min erhitzt. Während der Reaktion wurde die Intensität des Fluoreszenz-Signal von EvaGreen Farbstoff aufgezeichnet. Die Mess-Intervale zwischen benachbarten Messungen betrugen je eine Minute.

### Auswertung:

### Die zu erwartenden Primer-Verlängerungsprodukte:

Das zu erwartende erste Primer-Verlängerungsprodukt-001-01 nach der ersten Primer-Verlängerung an der Matrize-001: (SEQ ID NO 16)
5'-GTACCGAAGCTCGCAGGAACTCAGAGTGTGGAGAGGACGTAAGT GACATTGTAGTAT -3'
(Reihenfolge der Nummerierung: 1 - 57)

Unterstrichene Bereiche sind durch 2'-O-Me Modifikationen des ersten Primer-Oligonukleotid dargestellt. Nicht unterstrichene Bereiche sind 2'-deoxy-Nukleotide (DNA). Dieses erste Primer-Verlängerungsprodukt kann das zweite Primer-Oligonukleotid im 3'-Bereich komplementär binden und damit selbst als Matrize für die Synthese des zweiten Primer-Produktes dienen.

Die Synthese des zweiten Primer-Verlängerungsproduktes erfolgt bis hin zum modifizierten Bereich des ersten Primer-Oligonukleotides. Polymerase kann dabei entweder unmittelbar vor dem modifizierten Bereich gestoppt werden oder erster nach Kopierung von einigen Nukleotid-Modifikationen. Das zu erwartende zweite Primer-Verlängerungsprodukt 001-02 (SEQ ID NO 17) unter Verwendung des ersten Primer-Verlängerungsproduktes als Matrize:
5'- AAAAAAACATACTACAATGTCACTTACGTCCTCTCCACACTC TGAGTTCCTGCGAG - 3'
(Reihenfolge der Nummerierung: 1 - 56)

Die vier unterstrichenen 3'-ständigen Nukleotide werden hier angezeigt als angenommene maximale Verlängerung des zweiten Primer-Verlängerungsproduktes an der Sequenz des ersten Primer-Verlängerungsproduktes. Diese Nukleotide bilden komplementäre Positionen zu einigen 2'-O-Me modifizierten Nukleotiden des ersten Primer-Oligonukleotids. Polymerase kann auch kürzere Stränge unter Reaktionsbedinungen synthetisieren, so dass es eine Mischung aus Primer-Verlängerungsprodukten resultieren kann, da Stopp der Polymerase-Synthese bereits vor dem modifizierten Bereich des ersten Primer-Oligonukleotides oder erst nach Kopierung von einigen Modifikationen erfolgen kann. Solche Primer-Verlängerungsprodukte sind entsprechend kürzer (sie schließen beispielsweise Positionen 1 bis 52 oder 1 bis 53 oder 1 bis 54 oder 1 bis 55 ein). Die genaue Position der Terminierung der Synthese des zweiten Primer-Verlängerugnspdoruktes und somit seine die exakte Länge des zweiten Primer-Verlängerungsproduktes an seinem 3'-Ende ist an dieser Stelle von untergeorneter Bedeutung, solange alle amplifikationsfähigen zweiten Primer-Verlängerungsprodukte in der Lage sind, das erste Primer-Oligonukleotid zu binden und selbst als Matrize für die Synthese des ersten Primer-Verlängerungsproduktes auftreten. Die polymeraseabhängige Kopierung des zweiten Primer-Verlängerungsproduktes führt zu Generierung eines weiteren ersten Primer-Verlängerungsproduktes, welches auch komplementäre Sequenz zum 5'-Segment des zweiten Primer-Oligonukleotids einschließt.

Als Ergebnis einer exponentiellen Amplifikation entstehen zwei Primer-Verlängerungsprodukte, welche einen komplementären Doppelstrang bilden können. Die dabei zu erwartende Struktur des Doppelstranges, welcher aus beiden maximal verlängerten Primer-Verlängerungsprodukten besteht, ist nachfolgend abgebildet:
5'-GTACCGAAGCTCGCAGGAACTCAGAGTGTGGAGAGGACGTAAGTGACA TTGTAGTATGTTTTTTT -3' (SEQ ID NO 18)
3'- GAGCGTCCTTGAGTCTCACACCTCTCCTGCATTCACTGT AACATCATACAAAAAAA -5' (SEQ ID NO 19)

Im Laufe der exponentiellen Amplifikation kommt es zu Akkumulierung von diesen Primer-Verlängerungsprodukten, welche das Hauptprodukt der exponentiellen Amplifikation darstellen. Die so amplifizierten Stränge stellen die zu amplifizierende Nukleinsäure dar. Sie schließen die Sequenz der in die Reaktion eingesetzten Matrize ein. Unterstrichene Bereiche sind durch 2'-O-Me Modifikationen dargestellt. Nicht unterstrichene Bereiche sind 2'-deoxy-Nukleotide (DNA).

Der während der Reaktion entstehende Doppelstrang kann EvaGreen Farbstoff binden, so dass die Signal-Intensität des Farbstoffs unter fortschreitender Reaktion ansteigen kann.

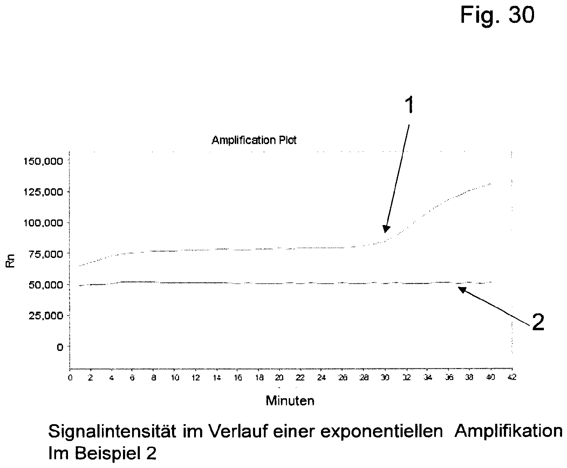

Das Ergebnis des Verlaufs der Signalintensität ist in Fig. 30 zu sehen. Pfeil 1 zeigt den Verlauf des Signals in Reaktion mit Matrize. Pfeil 2 zeigt den Verlauf der Reaktion ohne Matrize. Nach einer anfänglichen Plato-Phase wurde ein Signalanstieg des Fluoreszenzsignals ab ca. 30 min in der Reaktion beobachtet, welche Matrize-001 enthielt. Die Kontroll-Reaktion ohne Matrize-001 zeigte keine Änderung in der Signal-Intensität über den gesamten aufgezeichneten Zeitraum von 40 min. Das durchgehend hohe Niveau der Grundfluoreszenz hängt mit der Tatsache zusammen, dass der Primer-001-01 einen Fluoreszenzmarker enthält (dT-TMR in Position 11).

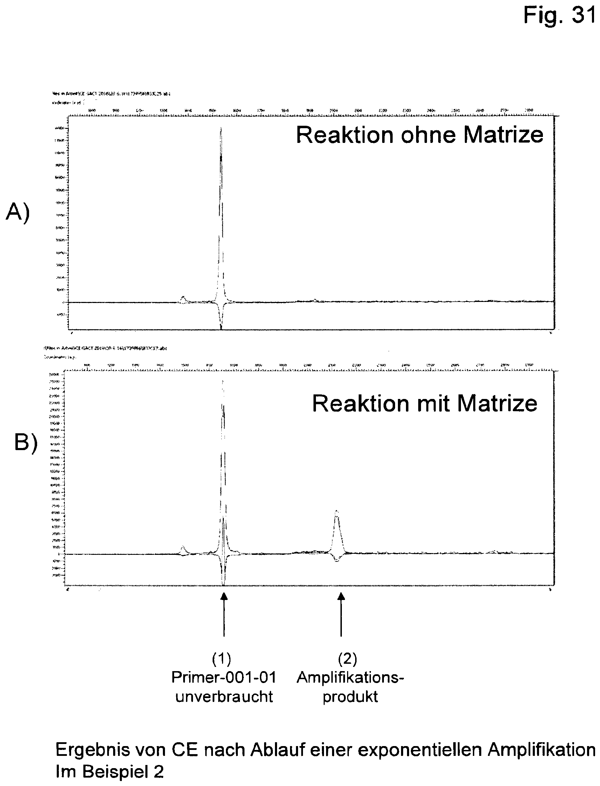

Zur Überprüfung der Identität des Signals wurde je ein Aliquot von beiden Reaktionen mittels CE analysiert. Da das erste Primer-Oligonukleotid einen Fluoreszenzmarker einschließt (dT-TMR in Position 11) konnte das Signal während CE aufgezeichnet werden. Das Ergebnis der CE ist in Fig. 31 zu sehen.

In Fig. 31 (A) ist CE-Verlauf der Kontroll-Reaktion zu sehen. Man sieht einen Peak, welcher dem nicht verbrauchten Primer-001-01 zugeordnet wurde. In Fig. 31 (B) ist CE-Verlauf der Reaktion mit Matrize zu sehen. Man sieht einen Peak (1), welcher dem unverbrauchten Primer-001-01 entspricht, sowie einen weiteren Peak (2), welcher um ca. 460 CE-Einheiten nach rechts verschoben ist (was einem längeren Molekül entspricht). In parallelen Kontroll-Experimenten wurde festgestellt, dass unter gegebenen CE-Bedingungen 10,2 bis ca. 10,3 CE-Längen-Einheiten einer Sequenzdifferenz von einem Nukleotid entsprechen. Somit entspricht die in CE gemessene Distanz zwischen dem Peak (1) und dem Peak (2) einer Differenz in der Länge von ca. 44 - 45 Nukleotiden zwischen beiden aufgetrennten markierten Sequenzen. Diese Sequenzdifferenz stimmt mit der Länge des zu erwartenden Verlängerungsproduktes des ersten Primer-Verlängerungsproduktes (SEQ ID NO 20) in einer exponentiellen Amplifikation überein, sie beträgt 45 Nukleotide.
5'- GTACCGAAGCTCGCAGGAAC
TCAGAGTGTGGAGAGGACGTAAGTGACATTGTAGTATGTTTTTTT -3' (SEQ ID NO 20) (das zu erwartete erste Verlängerungsprodukt des ersten Primer-Oligonukleotids ist unterstrichen)

Die Intensität des Signals in Peak (2) zeigt, dass weniger als 50% des anfänglich eingesetzten Primer-001-01 in der Reaktion verlängert wurde. Die Konzentration des entstandenen Produktes wurde auf etwa 1 µmol/l bis 2 µmol/l geschätzt, ausgehend von der Signalintensität des verbrauchten Primers. Somit betrug die Ausbeute ca. 10-20 % gemessen nach dem Primer-001-01 Verbrauch.

In der exponentiellen Amplifikation erfolgte somit eine Matrizen-induzierte Vermehrung der zu amplifizierenden Sequenz, wobei auf die Ausgangskonzentration der Matrize-001 von 2 fmol/l entsprach und die beobachtete Konzentration des Produktes der Amplifikationsreaktion auf ca. 1 bis 2 µmol/l geschätzt wurde. Der daraus errechnete Vermehrungsfaktor beträgt somit ca. 5x10^8 bis 10^9. Die exponentielle Amplifikation verlief unter isothermalen Bedinungen.

Das Ergebnis der exponentiellen Reaktion ist abhängig von Anwesenheit der Matrize und Aktivator-Oligonukleotid, sowie Primer-1, Primer-2, Polymerase, dNTP's und passenden Puffer-Bedinungen. Weiterhin ist die Reaktion abhängig von der SequenzZusammensetzung einzelner beteiligten Nukleinsäurekomponenten: Aktivator-Oligonukleotid und Primer-1 und Primer-2. Im Folgenden wird auf das Struktur-Wirkung-Prinzip dieser Komponenten eingegangen.

### Erläuterungen zum Design von Sequenzen

### Matrize:

Die Sequenz der zu amplifizierenden Nukleinsäure dient als Ausgangspunkt für Design der Sequenzen von Primer-1, Primer-2 und Aktivator-Oligonukleotid. Diese zu amplifizierende Nukleinsäure umfasst eine Zielsequenz, welche spezifisch amplifiziert werden muss.

Die Sequenzlänge des zu erwartenden Verlängerungsprodukts des ersten Primer-Oligonukleotids beträgt 45 Nukleotide. Die Gesamtlänge des ersten Primer-Verlängerungsproduktes beträgt 65 Nukleotide. Die zu erwartende Länge des Verlängerungsproduktes liegt zwischen 25 und 29 Nukleotide. Die Gesamtlänge des zweiten Primer-Verlängerungsproduktes liegt zwischen 52 und 56 Nukleotiden (je nach Position des Stopps der Polymerase während der Synthese des zweiten Primer-Verlängerungsproduktes). Die Länge des komplementären Doppelstranges der zu amplifizierenden Nukleinsäure, bestehend aus dem ersten Primer-Verlängerungsprodukt und dem zweiten Primer-Verlängerungsprodukt, liegt somit zwischen 52 und 56 Nukleotide. Das erste Primer-Verlängerungsprodukt hat in seinem 5'-Segment einen einzelsträngigen Bereich, welcher dem Polynukleotid-Schwanz des ersten Primer-Oligonukleotides entspricht.

Die Sequenzlänge der zu amplifizierenden Nukleinsäure ist im vorliegenden Beispiel hinreichend groß, so dass die Schmelztemperatur des während der Reaktion gebildeten spezifischen Doppelstranges aus dem ersten und dem zweiten Primer-Verlängerungsprodukt ca. 78°C beträgt und somit über der Reaktionstemperatur von 65°C liegt. Es wird angenommen, dass während der Reaktion gebildeter Doppelstrang im Wesentlichen stabil ist und nicht spontan in seine Einzelstränge zerfällt.

### Primer 1

Das erste Primer-Oligonukleotid schließt einen ersten Primer-Bereich und einen zweiten Bereich mit einem Polynukleotid-Schwanz, welcher nicht von Polymerase kopiert wird und in der Lage ist, Aktivator-Oligonukleotid zu binden.

### Erster Primer-Bereich:

Positionen 14-20 bilden den ersten Primer-Bereich des ersten Primer-Oligonukleotid.

Diese 3'-ständigen Nukleotide von Primer-001-01 (Positionen 14 - 20) sollen an die Matrize spezifisch binden und eine Primer-Verlängerungs-Reaktion ermöglichen. Daher wird die Sequenz dieser Positionen der Matrize 001 angepasst (Positionen 38 - 44). Diese sieben 3'-endständigen Nukleotide sind DNA-Nukleotide und sind mittels Phosphodiester-Verbindungen verknüpft. Dieses Segment initiiert die Primer-Verlängerung durch eine Polymerase bei der Synthese des ersten Stranges und dient als Matrize bei der Synthese des zweiten Stranges durch die Polymerase, so dass ein zu diesem Segment komplementärer Strang gebildet werden kann.

### Zweiter Primer-Bereich:

Der zweite Bereich ist an das 5'-Seite an den ersten Primer-Bereich angeschlossen (Positionen 1 - 13). Die Strukturen im zweiten Bereich sollen unter anderem Polymerase bei der Synthese des zweiten Stranges am Kopieren des Polynukleotid-Schwanzes hindern. Weiterhin sollen diese Strukturen in der Lage sein, Aktivator-Oligonukleotid zu binden und es bei Strangverdrängung unterstützen. Aus diesem Grund wurden vorwiegend 2'-O-Methyl Nukleotid-Modifikationen verwendet (Positionen 4 bis 10 und 12 bis 13) welche untereinander via 5'-3' Phosphodiester-Bindung verknüpft sind. Diese 2'-O-Methyl Nukleotid-Modifikationen stellen Beispiel für Modifikationen dar, welche in der Lage sind, Polymerase am Kopieren des Polynukleotid-Schwanzes zu hindern.

Die Sequenz von 5'-ständigen Nukleotide von Primer-001-01 (Positionen 1 - 9) ist zufällig gewählt, wobei darauf geachtet wurde, dass diese Sequenz keine extensive sebstkomplementäre Segmente, weiterhin keine komplementäre Bindungsstellen in der zu amplifizierenden Matrize hat, sowie keine multiple G-Nukleotide hat, welche in benachbarten Positionen stehen und somit G-Quadruplexe bilden könnten. Die Positionen 10 bis 13 sind komplementär zu Matrize-001 und sollen unter anderem Bindung des ersten Bereichs an die Matrize unterstützen.

Die Sequenz (Positionen 1 - 9) dient als Polynukleotid-Schwanz und soll während der Reaktion in der Lage sein, Aktivator-Oligonukleotid zu binden und dadurch räumliche Nähe zum Duplex-Ende herbeiführen, so dass Strangverdrängung stattfinden kann. Positionen 1 bis 3 sind durch DNA-Nukleotide dargestellt, Positionen 4 bis 9 sind 2'-O-Me Nukleotid-Modifikationen. Alle Nukleotide und Nukleotid-Modifikationen sind unter einander via Phosphodiester-Bindung verknüpft.

Der Übergang zwischen dem ersten Primer-Bereich und dem zweiten Bereich mit Polynukleotid-Schwanz ist in diesem Beispiel nahtlos: der zweite Bereich ist direkt an den ersten Bereich via Phosphordiester-Bindung angekoppelt. Das schafft günstige Voraussetzung für die Initiierung einer Strangverdrängung an einem Duplex-Ende durch ein Aktivator-Oligonukleotid. Beide Bereiche sind in gleicher Richtung angeordnet: von 5' zu 3'. Der zweite Bereich mit Polynukleotid-Schwanz liegt im 5'-Segment, der erste Primer-Bereich liegt im 3'-Segment.

Bei der Synthese des zweiten komplementären Stranges ist Polymerase ab Position 13 des ersten Primer-Oligonukleotids mit 2'-O-Methyl-Nukleotid-Modifikationen konfrontiert, welche weitere Synthese blockieren bzw. erschweren. Zwar führt eine einzelne 2'-O-Me-Nukleotid-Modifikation (wenn im Matrizenstrang eingefügt) nicht zwingend zur vollständigen Blockade der Polymerase-Synthese, so kommt es zur Erschwerung bzw. Verlangsamung der weiteren Synthese. Dank sequenzieller Kopplung von mehreren Nukleotid-Modifikationen wird Polymerase in ihrer Synthesfähigkeit erfolgreich gehemmt und kann den restlichen Polynukleotid-Schwanz nicht kopieren. Ob die vollständige Hemmung der Polymerase im Bereich des zweiten Bereichs des Primer-Oligonukleotid bereits nach einer Kopierung einer einzigen 2'-O-Methyl-Nukleotid-Modifikation auftritt oder erst nach Kopierung von mehreren 2'-O-Methyl-Nukleotid-Modifikationen ist zweitrangig, solange genügend nicht kopierte Nukleotide bzw. Nukleotid-Modifikationen im zweiten Bereich bleiben (Polynukleotid-Schwanz) und eine Bindung mit Aktivator-Oligonukleotid unter Reaktionsbedinugnen eingehen können, um eine erfolgreiche Strangverdrängung zu initiieren.

Aus Gründen der Anschaulichkeit und nicht als Einschränkung wird hiermit festgehalten, dass die Polymerase bis einschließlich Position 10 des ersten Primer-Oligonukleotides kopieren kann. Das bedeutet, dass das an den ersten Primer-Bereich angrenzende Segment des zweiten Bereichs von Polymerase teilweise kopiert wird. Bei der schematischen Darstellung werden deshalb vier Nukleotid-Positionen des zweiten Bereichs als von Polymerase kopiert betrachtet. Die restlichen Nukleotid-Positionen des zweiten Bereichs bleiben unkopiert und ermöglichen eine spezifische Bindung von Aktivator-Oligonukleotid.

### Primer 2:

Dieser Primer-2 ist für die Initiierung der Synthese des zweiten Stranges notwendig. Die Nukleotide in Positionen 9 - 27 sind mit der Matrize-001 identisch. Diese Positionen sollten zu Beginn der Amplifikation an das 3'-Segment des ersten Primer-Verlängerungsproduktes komplementär binden und damit die Synthese des zweiten Stranges initiieren. Der Primer 2 besteht aus DNA-Nukleotiden, welche untereinander via konventionelle 5'-3'-Phospordiesterbindungen verknüpft sind. Das 5'-Segment des zweiten Primers ist in diesem Beispiel bildet zunächst einen Überhang (Position 1 bis 8), welcher aber von einer Polymerase kopiert werden kann. Im Laufe der Reaktion kommt es zu Verlängerung der zu amplifizierenden Nukleinsäurekette um den 5'-Überhang des zweiten Primers.

Primer 2 nutzt das erste Primer-Verlängerungsprodukt als Matrize, so dass kopierbare Anteile des ersten Primer-Verlängerungsproduktes von Polymerase kopiert werden. Dadurch kommt es zu Verlängerung des zweiten Primers und Bildung eines Doppelstranges im kopierbaren Segment des ersten Primer-Verlängerungsproduktes. Der Polynukleotid-Schwanz des zweiten Bereichs des ersten Primers bleibt allerdings unkopiert. Das Primer-Verlängerungsprodukt schließt in seinem 3'-Segment eine Sequenz ein, welche in der Lage ist, das erste Primer-Oligonukleotid komplementär zu binden.

### Aktivator-Oligonukleotid:

### Erster Bereich:

Der erste Bereich (Position 35 - 50) schließt eine komplementäre Sequenz (Position 35 - 47) zum zweiten Bereich des ersten Primer-Oligonukleotides (Positionen 1 - 13) ein. Die drei 3'-endständigen Nukleotide bilden einen 3'-Überhang und haben im vorliegenden Beispiel keine funktionelle Bedeutung. Der erste Bereich besteht in diesem Beispiel aus 2'-Deoxynukleotiden, welche via eine konventionelle 5'-3' Phosphodiester-Bindung gekoppelt sind. Die endständige 3'-OH-Gruppe ist durch Phosphat-Rest blockiert und kann somit nicht von Polymerase verlängert werden.

### Zweiter Bereich:

Der zweite Bereich des Aktivator-Oligonukleotids schließt eine komplementäre Sequenz (Position 28 - 34) zum ersten Bereich des ersten Primer-Oligonukleotides (Positionen 14 - 20) ein. Der zweite Bereich des Aktivator-Oligonukleotids schließt in seinem 5'-Segment 2'-O-Methyl-Nukleotid-Modifikationen ein (Position 28 - 32) ein. Positionen 34 und 35 sind durch 2'-Deoxynukleotiden gebildet. Die einzelnen Nukleotide und Nukleotid-Modifikationen sind untereinander via eine konventionelle 5'-3'- Phosphodiester-Bindung gekoppelt. Der erste und der zweite Bereich des Aktivator-Oligonukleotides sind nahtlos aneinander via eine konventionelle 5'-3'- Phosphodiester-Bindung gekoppelt.

Die Sequenz-Länge und die ihre Beschaffenheit des ersten und zweiten Bereichs des Aktivator-Oligonukleotids ist an komplementäre Bereiche des ersten Primer-Oligonukleotides abgestimmt, so dass unter Reaktionsbedingungen das erste Primer-Oligonukleotid reversibel an das Aktivator-Oligonukleotid binden kann. Somit besteht ein Gleichgewicht zwischen freier, einzelsträngiger und gebundener, doppelsträngiger Form zwischen diesen beiden Komponenten.

### Dritter Bereich:

Der dritte Bereich des Aktivator-Oligonukleotids schließt eine komplementäre Sequenz (Position 9-27) zu einem Segment des zu erwartenden ersten Primer-Verlängerungsprodukt (Positionen 21-39), welches durch Polymerase während der Amplifikation synthetisiert werden soll. Der dritte Bereich besteht auf seiner gesamten Länge aus 2'-O-Methyl-Nukleotid-Modifikationen (Position 1-27). Die einzelnen Nukleotid-Modifikationen sind untereinander via eine konventionelle 5'-3'-Phosphodiester-Bindung gekoppelt. Der zweite und der dritte Bereich des Aktivator-Oligonukleotides sind nahtlos aneinander via eine konventionelle 5'-3'-Phosphodiester-Bindung gekoppelt. Die acht 5'-endständigen Nukleotid-Modifikationen bilden einen 5'-Überhang, welcher nicht zum ersten Primer-Verlängerungsprodukt komplementär ist.

Die Sequenz des Aktivator-Oligonukleotids ist dermaßen zusammengesetzt, dass es mit seinen Positionen 9-47 mit einem Teil des ersten Primer-Verlängerungsprodukts (Positionen 1 bis 39) einen komplementären Doppelstrang bilden kann. Positionen 1 bis 9, sowie Positionen 48-50 nehmen an der Ausbildung eines Doppelstranges mit dem ersten Primer-Verlängerungsprodukt nicht teil. Gleichzeitig ist die Sequenz des Aktivator-Oligonukleotids dermaßen zusammengesetzt, dass es mit seinen Nukleotid-Positionen 28-47 mit dem ersten Primer-Oligonukleotid einen komplementären Doppelstrang bilden kann. Dabei kann 3'-Ende des ersten Primers an das Aktivator-Oligonukleotid komplementär bindem. Um die Verlängerung des 3'-Ende des ersten Primers-Oligonukleotides durch eine Polymerase zu verhindern, schließt das Aktivator-Oligonukleotid Nukleotid-Modifikationen ein, welche die synthetische Wirkung der Polymerase verhindern. In diesem Beispiel sind es 2'-O-Methyl-Nukleotid-Modifikationen. Das Aktivator-Oligonukleotid in diesem Beispiel besteht an Positionen 1-32 aus 2'-O-Methyl-Nukleotid-Modifikationen. Dadurch bindet das 3'-Ende des ersten Primers bei korrekter Hybridisierung im Sequenz-Segment, welcher Nukleotid-Modifikationen umfasst. Eine solche Anordnung der Interaktion zwischen dem Aktator-Oligonukleotid und dem ersten Primer-Oligonukleotid soll eine ungewollte Verlängerung des ersten Primer-Oligonukleotids verhindern, wenn es am Aktivator-Oligonukleotid komplementär gebunden hat. Dank Modifizierungen im dritten Bereich des Aktivator-Oligonukleotides wird auch vorbeugend die Möglichkeit einer unspezifischen Verlängerung durch Polymerase generell reduziert. Um die Strandverdrängung zu fördern, wurden in entsprechenden Segmenten des Aktivator-Oligonukleotides Sequenzen eingesetzt, welche ihren Bindungspartnern im ersten Primer-Verlängerungsprodukt komplementär sind. Da sowohl Sequenz-Mismatches als auch Kettenunterbrechung durch Modifikationen ohne Nukleobasen (z.B. C3-Linker oder C18-Linker) die Effektivität einer Strandverdrängung und/oder ihre Geschwindigkeit reduzieren können, wurden solche Struktur-Varianten in diesem Beispiel vermieden.

Aktivator-Oligonukleotid soll unter Reaktionsbedingungen in Kontakt mit dem Doppelstrang der zu amplifizierenden Nukleinsäure kommen, über eine Bindung an den Polynukleotid-Schwanz des ersten Primer-Verlängerungsprodukts. In diesem Beispiel ist die Bindung vorzugsweise unter Reaktionbedingungen reversibel. Der Doppelstrang besteht dabei entweder am Beginn der Reaktion aus dem ersten Primer-Verlängerungsprodukt und der Matrize-001 und im späteren Verlauf der Reaktion aus dem ersten und dem zweiten Primer-Verlängerungsprodukt.

Die Beschaffenheit und die Länge des Aktivator-Oligonukleotids sollte hinreichend sein, um eine Strangverdrängung durchzuführen, welche zu einer Trennung der Primer-Verlängerungsprodukte in hinreichenden Ausbeuten führt. Die hinreichende Trennung der beiden Primer-Verlängerungsprodukte soll gewährleisten, dass Sequenzsegmente der beiden Primer-Verlängerungsprodukte mit entsprechenden Sequenzabschnitten mit Primer-Bindungs-Stellen für die Bindung von jeweils komplementären Primer-Oligonukleotide zugängig werden. Im vorliegenden Beispiel beträgt die Länge des Aktivator-Oligonukleotides 50 Nukleotide (Deoxynukleotide plus Nukleotid-Modifikationen), davon können 39 eine komplementäre Bindung mit dem ersten Primer-Verlängerungsprodukt einhergehen. Aktivator-Oligonukleotid war dadurch in der Lage, zunächst die Matrize-001 und im späteren Verlauf der Reaktion auch das zweite Primer-Verlängerungsprodukt unter Reaktionsbedingungen mittels Strangverdrängung zumindest teilweise aus der Bindung mit dem ersten Primer-Verlängerungsprodukt zu verdrängen.

Dabei wurden Matrize-001 bzw. das zweite Primer-Verlängerungsprodukt vollständig oder teilweise einzelsträngig, so dass ein neues erstes Primer-Oligonukleotid an das entsprechend komplementäres Sequenzsegment binden konnte und mittels Polymerase verlängert werden konnte. Im vorliegenden Beispiel bindet der dritte Bereich nur ein 5'-ständiges Segment des ersten Primer-Verlängerungsproduktes. Im vorliegenden Beispiel beträgt die Länge dieses komplementären Segments 19 Nukleotide.

Die 3'-ständigen Sequenzanteile des ersten Primer-Verlängerungsproduktes wurden von Aktivator-Oligonukleotid nicht gebunden. Dieses 3'-ständige Segment des ersten Primer-Verlängerungsprodukts soll in seiner einzelsträngiger Form in der Lage sein, das zweite Primer-Oligonukleotid zu binden, um eine Primer-Verlängerungsreaktion zu ermöglichen. Damit im Verlauf der Reaktion ein weiteres zweites Primer-Oligonukleotid an das erste Primer-Verlängerungsfragment binden kann, muss das 3'-ständiges Segment des ersten Primer-Verlängerungsproduktes in einzelsträngige Form überführt werden und aus der Bindung mit der Matrize oder mit einem zweiten Primer-Verlängerungsprodukt getrennt werden. Im vorliegenden Beispiel wird dies durch Trennung der beiden synthetisierten Stränge unter Reaktionsbedingungen während einer isothemalen Reaktion erreicht. Während der Strangverdrängung durch das Aktivator-Oligonukleotids kommt es zu Ausbildung eines Zwischenprodukts der Strangverdrängungsreaktion, welches aus einem ersten Primer-Verlängerungsprodukt, dem Aktivator-Oligonukleotid und einem zweiten Primer-Verlängerungsprodukt besteht. Das zweite Primer-Verlängerungsprodukt ist in diesem Zwischenprodukt an das 3'-ständige Segment des ersten Primer-Verlängerungsproduktes komplementär gebunden. Die Stabilität dieses resultierenden Doppelstranges ist allerdings im Vergleich zu einem vollständig gebundenen zweiten Primer-Verlängerungsprodukt deutlich herabgesetzt. Unter Reaktionsbedingungen kommt es zu einer spontanen Trennung dieses Doppelstrangs (Dissoziation des ersten und des zweiten Primer-Verlängerungsproduktes), so dass das zweite Primer-Verlängerungsprodukt vollständig vom ersten Primer-Verlängerungsprodukt abgelöst wird. Das 3'-Segment des ersten Primer-Verlängerungsproduktes ist unmittelbar nach der Trennung vom zweiten Primer-Verlängerungsprodukt einzelsträngig.

Das einzelsträngige 3'-endständige Segment des ersten Primer-Verlängerungsproduktes kann sowohl in Kontakt mit der Matrize bzw. mit dem zweiten Primer-Verlängerungsprodukt, als auch eine neue Bindung mit dem zweiten Primer-Oligonukleotid eingehen. Aufgrund eines molaren Überschusses des eingesetzten zweiten Primer-Oligonukleotid ist die Wahrscheinlichkeit des Kontaktes mit dem zweiten Primer-Oligonukleotid wesentlich höher. Dies ist insbesondere zu Beginn der Reaktion der Fall: die Matrize wird im vorliegenden Beispiel in Konzentration von 1 fmol/l eingesetzt. Während der ersten Verdoppelungszyklen der exponentiellen Amplifikation bleibt die Konzentration von synthetisierten zweiten Primer-Verlängerungsprodukten ebenfalls längere Zeit deutlich unter dem Wert von 1 µmol/l. Das zweite Primer-Oligonukleotid dagegen in Konzentration 10 µmol/l. Es resultiert somit ein 10¹⁰ Überschuss an zweiten Primer-Oligonukleotiden über die Matrize und über die synthetisierten zweiten Primer-Verlängerungsprodukte insbesondere während der anfänglichen Phasen der exponentiellen Amplifikation. In späteren Phasen der Amplifikation konkurriert das nun in höhreren Konzentrationen vorliegende zweite Primer-Verlängerungsprodukt um die Bindung an das 3'-Segment des ersten Primer-Verlängerungsproduktes, so dass es zu einer Verlangsamung der Reaktion kommen kann.

### Beispiel 3:

### Überprüfung der Spezifität der Amplifikations-Reaktion

In diesem Beispiel wurde Einfluss einer Sequenzänderung in der Matrize auf die Amplifikation untersucht. Drei Nukleotide aus dem mittleren Bereich der Matrize wurden entfernt. Die randständigen Bereiche, welche für die Bindung von Primern entscheidend sind wurden identisch gestaltet wie im Beispiel 2. Bei Verlängerung des ersten Primer-Oligonukleotides wird ein komplementärer Strang gebildet, welcher eine komplementäre Sequenz zur Matrize aufweist und somit diese Abweichungen in der Sequenz umfasst. Auf diese Weise sollte überprüft werden, welche Auswirkung ein solcher Mismatch zwischen einem dabei entstehenden ersten Primer-Verlängerungsprodukt und einem Aktivator-Oligonukleotid auf die Amplifikation hat. Die Gestaltung von Sequenzen erfolgte in ähnlicher Art und Weise wie im Beispiel 2.

Folgende Matrizen wurden verwendet:
- Matrize (SEQ ID NO 21) mit Sequenzzusammensetzung, welche zu einem ersten Primer-Verlängerungsprodukt mit einem Perfekt-Match Übereinstimmung mit Aktivator-Oligonukleotid führt:
   T-A6P-15-4401 5' GTAGTTCTCACTTACGTCCTCTCCACACTCTGAGTTCCTGCGAG 3'

Bindungssequenz für das erste Primer-Oligonukleotid ist unterstrichen.
- Matrize (SEQ ID NO 22) mit Sequenzzusammensetzung, welche zu einem ersten Primer-Verlängerungsprodukt mit einem MisMatch mit Aktivator-Oligonukleotid führt: T-A6P-15-4401-5 5' GTAGTTCTCACTTACGTCCTCTCCCTCTGAGTTCCTGCGAG 3'

Bindungssequenz für das erste Primer-Oligonukleotid ist unterstrichen.

Bei beiden Matrizen sind jeweilige Primer-Bindungsstellen identisch, so dass zu erwarten ist, dass bei Primer-Oligonukleotiden jeweils eine erfolgreiche erste Primer-Verlängerungsreaktion initiieren kann.

Beide Matrizen unterscheiden sich nur an einer Stelle:
T-A6P-15-4401 5' GTAGTTCTCACTTACGTCCTCTCCACACTCTGAGTTCCTGCGAG 3' (SEQ ID NO 21)
T-A6P-15-4401-5 5' GTAGTTCTCACTTACGTCCTCTCC_CTCTGAGTTCCTGCGAG 3' (SEQ ID NO 22)

### Die Differenz-Stelle ist unterstrichen.

Somit sind auch Unterschiede in jeweiligen Primer-Verlängerungsprodukten des ersten und des zweiten Primer-Oligonukleotide zu erwarten. Die Differenzstelle liegt im 5'-Segment des Verlängerungsprodukt des ersten Primer-Oligonukleotides.

Folgende Primer wurden verwendet:
- Das erste Primer-Oligonukleotid (SEQ ID NO 23):
A6P-10-1002 TMR7 5'7765567876 CAGGAAC 3'
A = 2'-deoxy-Adenosin
C = 2'-deoxy-Cytosin
G = 2'-deoxy-Guanosin
T = 2'-deoxy-Thymidin (Thymidin)

Modifikationen:
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
8 = dT-TMR
   - Das zweite Primer-Oligonukleotid (SEQ ID NO 24): PA6-15-4401-10 5' CACGAGTAGTTCTCACTTAC 3'

Folgendes Aktivator-Oligonukleotid-002-01 (SEQ ID NO 25) wurde verwendet:
A6 8504
5'- CTAGCTAT CGTC**CTCT CCA**CAC**T C**TGA GTTCCTGCGAG CTTC GGTAC AAG X 3' 68576858 6786**6868 665**656**8 6**875 788668 7CGAG CTTC GGTAC AAG
A = 2'-deoxy-Adenosin
C = 2'-deoxy-Cytosin
G = 2'-deoxy-Guanosin
T = 2'-deoxy-Thymidin (Thymidin

5 = 2'-O-Me A (2'-O-Methyl-Adenosine)
6 = 2'-O-Me C (2'-O-Methyl-Cytosine)
7 = 2'-O-Me G (2'-O-Methyl-Guanosine)
8 = 2'-O-Me U (2'-O-Methyl-Uridine)
X = 3'-Phosphat-Gruppe zur Blockade einer möglichen Verlängerung durch Polymerase.

Die Nukleotide und Nukleotid-Modifikationen sind untereinander mit Phosphodiesterbindung verknüpft. Das 3'-Ende ist mit einer Phosphat-Gruppe blockiert, um eine mögliche Verlängerung durch die Polymerase zu verhindern.

Die Matrize mit Perfekt-Match Sequenzzusammensetzung wurde in Konzentration von 1 nmol/l, 1 pmol/l und 1 fmol/l eingesetzt.

Die Matrize mit Mismatch Sequenzzusammensetzung wurde in Konzentration von 1 nmol/l verwendet.

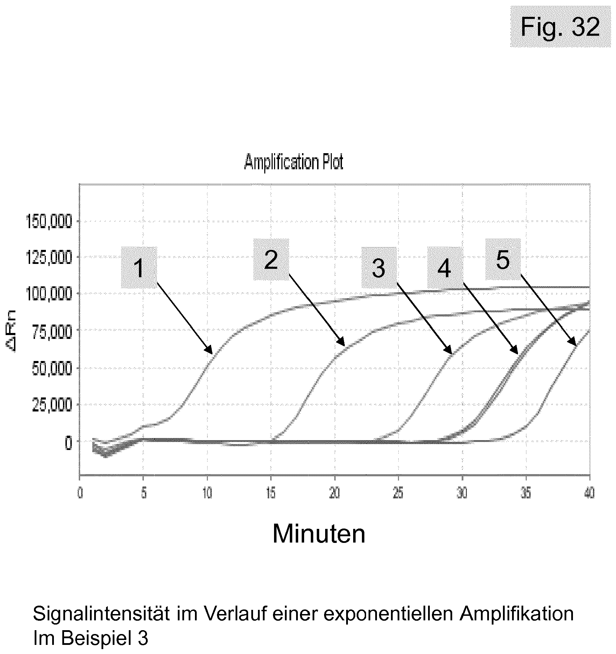

In der Kontroll-Reaktion wurde keine Matrize eingesetzt. Sonstige Reaktionsbedinungen wurden wie im Beispiel 2 gehalten. Fig. 32 zeigt das Ergebnis der Reaktion.

Bei Verwendung einer Perfect-Match Matrize (SEQ ID NO 21) kommt es zur Synthese eines komplementären Stranges eines Primer-Verlängerungsproduktes. Dieses Verlängerungsprodukt ist sowohl zu Perfect-Match-Matrize komplementär als auch zum verwendeten Aktivator-Oligonukleotid. Diese Konstellation wurde im Beispiel 2 ausführlich dargestellt. Sie dient als Grundlage für eine erfolgreiche Amplifikation.

Im Gegensatz dazu, unter Verwendung einer Mismatch-Sequenz (SEQ ID NO 22) kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der Komplementarität mit dem dritten Bereich des Aktivator-Oligonukleotid abweicht. Diese Abweichung findet im 5'-ständigen Segment des Verlängerungsproduktes statt, welches mit Aktivator-Oligonukleotid reagieren sollte, damit Strangverdrängungsvorgang voranschreiten kann. Wie im vorliegenden Beispiel gezeigt wird, stört das Fehlen von drei Nukleotiden in entsprechenden Positionen eine Strangverdrängung durch das Aktivator-Oligonukleotid.

Die Fig. 32 zeigt diesen Einfluss der Sequenzunterschiede zwischen Aktivator-Oligonukleotid und dem Verlängerungsprodukt des ersten Primer-Oligonukleotids. Abweichungen an 3 Positionen in der Sequenz des Aktivator-Oligonukleotides von der Sequenz des an der Mismatch-Matrize synthetisierten Primer-Verlängerungsproduktes zeigt die Abhängigkeit der Amplifikation von der Übereinstimmung in der Reihenfolge der komplementären Basen im Aktivator-Oligonukleotid und Matrize bzw. dem ersten Primer-Verlängerungsprodukt. Eine Wegnahme von nur 3 Basen aus dem Bereich des Verlängerungsproduktes führte bereits zu einer Unterdrückung der Amplifikation auf ein Niveau, was im vorliegenden Beispiel sogar unter der Negativkontrolle liegt.

Die Kontrollreaktionen mit einer Perfect Match Matrize (Pfeile 1 - 3) zeigten eine Konzentrationsabhängigkeit der Amplifikation. Mit sinkender Konzentration brauchte Reaktion längere Zeit, um eine ausreichende Menge der zu amplifizierenden Nukleinsäure zu synthetisieren, damit das Signal über Niveau der Base-line ansteigt.

Die Negativ-Kontroll Reaktion ohne Matrize zeigte ebenfalls ein Anstieg im Signal, allerdings in einem signifikant späteren Zeitintervall zu Reaktionen mit einer Perfect-Match Matrize.

Die Pfeile in Fig. 32 zeigen:
Perfect Match Matrize (SEQ ID NO 21) 1 nmol/l Konzentration (Pfeil 1)
Perfect Match Matrize (SEQ ID NO 21) 1 pmol/l Konzentration (Pfeil 2)
Perfect Match Matrize (SEQ ID NO 21) 1 fmol/l Konzentration (Pfeil 3)
Negativ-Kontrolle: Keine Matrize in der Reaktion (Pfeil 4) (angezeigt sind 2 KontrollReaktionen, beide ohne Matrize)
Mismatch Matrize (SEQ ID NO 22) 1 nmol/l Konzentration (Pfeil 5)

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt können zu Verlangsamung oder sogar Unterbrechung der Amplifikation führen.

In diesem Beispiel wurde gezeigt, dass obwohl Sequenzenden von Perfect-Match-Matrize und Mismatch-Matrize komplett übereinstimmten und somit das Potenzial zu Bindung von beiden Primer-Oligonukleotide gleich war, sind beide Reaktionen vollkommen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall fehlten drei Nukleotide im 5'-Segment des Verlängerungsproduktes des ersten Primers) führte zur starken Unterdrückung der Amplifikation.

### Beispiel 4:

### Verwendung einer anderen Matrizen-Sequenz mit einem anderen passenden Aktivator-Oligonukleotid

Zunächst wurde eine künstliche Sequenz von 49 Nukleotiden synthetisch (Eurofins) generiert, welche eine Sequenz des MIP-Gens von *Legionella pneumophila* zwischen dem Primer-1 und Primer-2 einschließt (SEQ ID NO 26), Matrize-003-01. Als Kontrolle wurde Matrize-003-02 (SEQ ID NO 27) mit Sequenzzusammensetzung synthetisch generiert (Eurofins), welche zu einem ersten Primer-Verlängerungsprodukt mit einem Mismatch mit Aktivator-Oligonukleotid führt (5 Nukleotide). Zur Amplifikation einer solchen neuen Matrize-003 wurden zwei Primer (Primer-1, erstes Primer-Oligonukleotid (identisch mit SEQ ID NO 13 im Beispiel 2) und Primer-2, zweites Primer-Oligonukleotid (SEQ ID NO 28) sowie ein zur Matrize-003-01 passendes Aktivator-Oligonukleotid (SEQ ID NO 29) generiert. Die Gestaltung von Sequenzen erfolgte in ähnlicher Art und Weise wie im Beispiel 2 beschrieben.

Folgende Matrizen wurden verwendet:
- Matrize-003-01 (SEQ ID NO 26) mit Sequenzzusammensetzung, welche zu einem ersten Primer-Verlängerungsprodukt mit einem Perfekt-Match Übereinstimmung mit Aktivator-Oligonukleotid führt:
   LP-T1-1000-103
   5' CTACAATGTCACTTACACGTTCTTAACAAGTTTCAG**CC**GTTCCTGCGAG 3' Sequenzsegment des MIP-Gens von *L.pneumonia* ist unterstrichen.

Die Matrize besteht aus DNA Nukleotiden.
- Matrize (SEQ ID NO 27) mit Sequenzzusammensetzung, welche zu einem ersten Primer-Verlängerungsprodukt mit einem MisMatch mit Aktivator-Oligonukleotid führt:
   LP-T1-1001-118
   5' CTACAATGTCACTTACACGTTCTTAACAAGTTTGTTCCTGCGAG 3'
   Sequenzsegment des MIP-Gens von *L.pneumonia* ist unterstrichen (dieses Segment ist um 5 Nukleotide kürzer als in der SEQ ID NO 26).

Die Matrize besteht aus DNA Nukleotiden.

Bei beiden Matrizen sind jeweilige Primer-Bindungsstellen identisch, so dass zu erwarten ist, dass eine erfolgreiche erste Primer-Verlängerungsreaktion durch Primer-Oligonukleotide jeweils initiieren kann.
LP-T1-1000-103 5' CTACAATGTCACTTACACGTTCTTAACAAGTTTCAG**CC**GTTCCTGCGAG 3' (SEQ ID NO 26)
LP-T1-1001-118 5' CTACAATGTCACTTACACGTTCTTAACAAGTTT GTTCCTGCGAG 3' (SEQ ID NO 27)
   Bindungsstelle für Primer-1 unterstrichen.

Beide Matrizen unterscheiden sich nur an einer Position:
LP-T1-1000-103 5'
   CTACAATGTCACTTACACGTTCTTAACAAGTTTCAG**CC**GTTCCTGCGAG 3' (SEQ ID NO 26)
LP-T1-1001-118 5' CTACAATGTCACTTACACGTTCTTAACAAGTTT_GTTCCTGCGAG 3' (SEQ ID NO 27)
   Die Differenz-Stelle zwischen Matrizen ist unterstrichen.

Diese Position liegt im Bereich, welcher bei der Synthese des ersten Primer-Verlängerungsproduktes durch Polymerase synthetisiert wird. Die Differenzstelle liegt im 5'-Segment des Verlängerungsprodukt des ersten Primer-Oligonukleotides. Somit sind auch Unterschiede in jeweiligen Primer-Verlängerungsprodukten des ersten und des zweiten Primer-Oligonukleotide zu erwarten.

Folgende Primer wurden verwendet:
Das erste Primer-Oligonukleotid (SEQ ID NO 13) ist identisch mit Primer 1 in Beispiel 2:

Primer 1
Primer001-01 (20 Nukleotide):
Die Sequenz des ersten Primer-Oligonukleotides:
A6P-10-1001 5' GTACCGAAGC**T**CGCAGGAAC 3' (SEQ ID NO 13)
(Reihenfolge der Nummerierung: 1 - 20)

Unterstrichene Bereiche sind durch 2'-O-Me Modifikationen dargestellt. Nicht unterstrichene Bereiche sind 2'-deoxy-Nukleotide (DNA).
- Das zweite Primer-Oligonukleotid (SEQ ID NO 28):
   LP-P2-1000-211 5' CTACAATGTCACTTAC ACGTTCTTAACAAGTTT 3' (SEQ ID NO 17):

Das erste und das zweite Primer-Oligonukleotide können beide Matrizen (SEQ ID NO 26 und 27) an entsprechenden Primer-Bindungsstellen binden.

Folgendes Aktivator-Oligonukleotid-003 (SEQ ID NO 29) wurde verwendet:
LP-A1-1000-104

Sequenz am 3'-ständigen Segment besteht aus DNA-Nukleotiden
A = 2'-deoxy-Adenosin
C = 2'-deoxy-Cytosin
G = 2'-deoxy-Guanosin
T = 2'-deoxy-Thymidin (Thymidin)

Die Sequenz zwischen eckigen Klammern [], Positionen 9-58, besteht aus 2'-O-Me-Nukleotid-Modifikationen, welche via Phosphordiester-Bindung verknüpft sind: 2'-O-Me A (2'-O-Methyl-Adenosine), 2'-O-Me C (2'-O-Methyl-Cytosine), 2'-O-Me G (2'-O-Methyl-Guanosine), 2'-O-Me U (2'-O-Methyl-Uridine).

Verbindungen zwischen 5'-ständigen Nukleotiden (Positionen 1-8) sind via Phosphorothioat-Verbindungen (PTO) verknüpft; entsprechende Positionen sind mit (*) markiert.

X = 3'-Phosphat-Gruppe zur Blockade einer möglichen Verlängerung durch Polymerase.

Das 3'-Ende ist mit einer Phosphat-Gruppe blockiert, um eine mögliche Verlängerung durch die Polymerase zu verhindern.

Die Konzentration von Matrizen zu Beginn der Reaktion betrug 10 pmol/l (für perfect match und mismatch). In einer weiteren Kontroll-Reaktion wurde keine Matrize eingesetzt. Die Reaktion wurde 60 min bei 65°C durchgeführt. Sonstige Reaktionsbedinungen wurden wie im Beispiel 1 gehalten.

### Ergebnisse

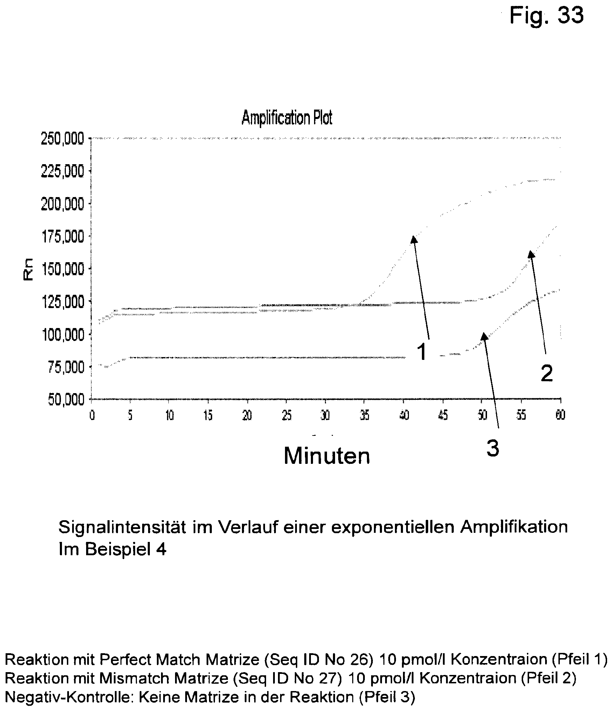

Der Verlauf der Reaktion ist in Fig. 33 abgebildet. Man erkennt, dass die Reaktion, welche eine perfekt-Match Matrize enthielt, führt ab 35 min zum Anstieg des Fluoreszenzsignals (Pfeil 1). Reaktion mit Mismatch und Negativ-Kontrolle führen zwar ebenfalls zu einem Signal, welches allerdings signifikant später kommt (ca. 45 min bzw. 50 min).

### Auswertung:

Der Signalanstieg in Fig. 33 korreliert mit der Anwesenheit einer Perfect-Match Matrize-003-01. Eine sehr ähnliche Sequenz, mit gleichen Primer-Bindungsstellen und einem signifikant ähnlichen Abschnitt zwischen den Primern, allerdings mit einer Deletion von 5 Nukleotiden, unterscheidet sich im Verlauf der Amplifikation und liegt im Bereich der Negativ-Kontrolle.

Dieser Unterschied wird auf folgende Sachverhältnisse zurückgeführt:
Bei Verwendung einer Perfect-Match Matrize (SEQ ID NO 26) kommt es zur Synthese eines komplementären Stranges eines Primer-Verlängerungsproduktes. Dieses Verlängerungsprodukt ist sowohl zu Perfect-Match-Matrize komplementär als auch zu entsprechenden Positionen des verwendeten Aktivator-Oligonukleotids (SEQ ID NO 29). Diese Konstellation wurde im Beispiel 1 ausführlich dargestellt. Sie dient als Grundlage für eine erfolgreiche Amplifikation.

Nachfolgend sind Sequenzen gegenübergestellt und jeweilige Segmente markiert, welche unter der Reaktion einen komplementären Doppelstrang mit entsprechenden Reaktionspartnern bilden:

### Matrizen

LP-T1-1000-103 5'
   CTACAATGTCACTTACACGTTCTTAACAAGTTTCAG**CC**GTTCCTGCGAG 3' (SEQ ID NO 26)
LP-T1-1001-118 5' CTACAATGTCACTTACACGTTCTTAACAAGTTT GTTCCTGCGAG 3' (SEQ ID NO 27)
   Bindungsstelle für Primer-1 ist unterstrichen.

**Primer-1 und Primer 2:**
Primer 1: A6P-10-1001 5' GTACCGAAGC**T**CGCAGGAAC 3' (SEQ ID NO: 13)
   Bindungsstelle an Matrize ist unterstrichen.
Primer 2: LP-P2-1000-211 5' CTACAATGTCACTTAC ACGTTCTTAACAAGTTT 3' (SEQ ID NO 28):
   (unterstrichen ist 5'-Segment des zweiten Primer-Oligonukleotides, welches an das 3'-Segment des ersten Primer-Verlängerungsproduktes komplementär binden kann, wobei dieses 3'-Segment des ersten Primer-Verlängerungsproduktes nicht komplementär zum dritten Bereich des Aktivator-Oligonukleotid ist.)

### Aktivator-Oligonukleotid

LP-A1-1000-104 (SEQ ID NO 29)

(Positionen des dritten Bereichs des Aktivator-Oligonukleotides, welche mit dem Verlängerungsprodukt des ersten Primer-Oligonukleotides an der Matrize-003-01 (SEQ ID NO 14) perfekt übereinstimmen sind unterstrichen.)

LP-A1-1000-104 (SEQ ID NO 29)

(Positionen des Aktivator-Oligonukleotides, welche mit dem Primer-Verlängerungsprodukt des ersten Primer-Oligonukleotides an der Matrize-003-01 (SEQ ID NO 14) perfekt übereinstimmen, sind unterstrichen.) Dieses Fragment ist länger als das Fragment, welches mit Verlängerungsprodukt des ersten Primer-Oligonukleotides interagiert, da das gesamte Primer-Verlängerungsprodukt aus dem ersten Primer-Oligonukleotid und dem Verlängerungsprodukt besteht.

Man erkennt, dass es einen nahtlosen Übergang in der Komplementarität zwischen dem ersten Primer-Verlängerungsprodukt (welches an der perfect-match Matrize-003-01 synthetisiert wurde) und den Segmenten des zweiten Bereiches und des dritten Bereich des Aktivator-Oligonukleotides gibt. Diese Konstellation unterstützt die Ausbildung des neuen Doppelstrangs zwischen dem Aktivator-Oligonukleotid und dem ersten Primer-Verlängerungsprodukt, so dass eine hinreichende Strangverdrängung des Matrizenstranges, bzw. einen zweiten Primer-Verlängerungsproduktes stattfinden kann. Insgesamt führt das zu einem positiven Amplifikationsergebnis.

Im Gegensatz dazu, unter Verwendung einer Mismatch-Sequenz (SEQ ID NO 15) kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der Komplementarität mit dem dritten Bereich des Aktivator-Oligonukleotid abweicht. Diese Abweichung findet im 5'-ständigen Segment des Verlängerungsproduktes statt, welches mit Aktivator-Oligonukleotid reagieren sollte, damit Strangverdrängungsvorgang voranschreiten kann. Wie im vorliegenden Beispiel gezeigt wird, stört das Fehlen von fünf Nukleotiden in entsprechenden Positionen eine Strangverdrängung durch das Aktivator-Oligonukleotid, was zu Störung der Amplifikation führt.

LP-A1-1000-104 (SEQ ID NO 29)

(Positionen des dritten Bereichs des Aktivator-Oligonukleotides, welche mit dem Verlängerungsprodukt des ersten Primer-Oligonukleotides an der Matrize-003-02 (SEQ ID NO 26) perfekt übereinstimmen sind unterstrichen.

LP-A1-1000-104 (SEQ ID NO 29)

(Positionen des Aktivator-Oligonukleotides, welche mit dem Primer-Verlängerungsprodukt des ersten Primer-Oligonukleotides an der Matrize-003-01 (SEQ ID NO 27) perfekt übereinstimmen sind unterstrichen.

Man erkennt eine zu erwartende Unterbrechung in der Bindung eines komplementären Doppelstranges zwischen dem Aktivator-Oligonukleotid-003 und dem ersten Primer-Verlängerungsprodukt, welches an der Mismatch Matrize gebildet wurde. Diese Unterbrechung entspricht dem Anteil des Primer-Verlängerungsproduktes der Matrize-003-02 (SEQ ID NO 27), welcher dem weggelassenen Segment der Matrize-003-02 entspricht. Diese Position liegt zwischen dem zweiten und dem dritten Bereich des Aktivator-Oligonukleotides.

Eine solche Differenz in der Komplementarität der potenziell zu erwartenden Doppelstränge zwischen Aktivator-Oligonukleotid-003 (SEQ ID NO 29) und dem ersten Primer-Verlängerungsprodukt einer perfect-match Matrize-003-01 bzw. dem ersten Primer-Verlängerungsprodukt einer mismatch Matrize-003-02 kann zu Unterschieden in der Strangverdrängungsreaktion führen, welche in einer Hinderung der Amplifikation resultieren.

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt können zur Unterbrechung der Amplifikation führen.

In diesem Beispiel wurde gezeigt, dass obwohl Sequenzenden von Perfect-Match-Matrize und Mismatch-Matrize komplett übereinstimmten und somit das Potenzial zu Bindung von beiden Primer-Oligonukleotide gleich war, sind beide Reaktionen vollkommen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall fehlten fünf Nukleotide im 5'-Segment des Verlängerungsproduktes des ersten Primers) führte zur Unterdrückung der Amplifikation.

### Beispiel 5:

In diesem Beispiel wird gezeigt, dass weitere Struktur-Varianten des ersten Primer-Oligonukleotides in der Lage sind, eine exponentielle Amplifikation einer zu amplifizierenen Nukleinsäurekette zu unterstützen. Diese Strukturvarianten unterscheiden sich in der zum Stopp der Polymerase verwendeten Gruppe bei der Synthese des zweiten Primer-Verlängerungsproduktes. Es wurden je eine Modifikation als eine zum Stopp der Polymerase führende Grupp verwendet: C3-Linker, C18-Linker (auch als HEG-Linker, Hexaethylenglycol-Linker, bekannt) oder Iso-dC-5-Me.

Weiterhin wird in diesem Beispiel gezeigt, dass ein Primer-2 mit seinem zum ersten Primer-Verlängerungsprodukt komplementären 3'-Segment das Aktivator-Oligonukleotid aus der Bindung mit dem ersten Primer-Verlängerungsprodukt teilweise verdrängen kann und eine erfolgreiche Amplifikation unterstützen kann.

Zu Testzwecken wurde die gleiche zu amplifizierende Nukleinsäure genommen, wie in Beispiel 2 beschrieben.

Matrize 001-01: (SEQ ID NO 12)
T35-4401-601
5'- ATACTACAATGTCACTTACGTCCTCTCCACACTCTGAGTTCCTGCGAG - 3'
(Reihenfolge der Nummerierung: 1 - 48)
Aktivator-Oligonukleotid: Es wurde das gleiche Aktivator-Oligonukleotid-001-01 verwendet, wie im Beispiel 2 beschrieben:
Aktivator-Olgonukleotid-001-01: (SEQ ID NO: 15)
A6 8505
5'- [CUAGCUAUCGUCCUCUCCACACUCUGA GUUCC] TGCGAGCTTCGGTACAAG X - 3'
(Reihenfolge der Nummerierung: 1 - 50)

Das Aktivator-Oligonukleotid umfaßt 2'-O-Me Nukleotid-Modifikationen (angegeben in [ ]-Klammern) und DNA Nukleotide (im 3'-Segment). Die 3'-OH-Gruppe des 3'-endständigen Nukleotides war durch Phosphat-Rest blockiert (hier mit X gekennzeichnet).

Folgende Varianten des ersten Primer-Oligonukleotids wurden verwendet:
P-1001-100 TMR C3 (SEQ ID NO 30)
   5'- 6T57765568 CTCGCAGGAAC - 3'
   8 = C3-Linker
P-1001-103 TMR C18 (SEQ ID NO 31)
   5'- 6T57765568 7T76CAGGAAC -3'
   8 = C18-Linker (HEG-Linker, Hexaethylenglycol)
P-10-1002-IsoC4 (SEQ ID NO 32)
   5'- 7765568TCGCAGGAAC - 3'
   8 = Iso-dC-5-Me (Modifikation)
weitere Positionen wurden wie folgt belegt:
A = 2'-deoxy-Adenosin
C = 2'-deoxy-Cytosin
G = 2'-deoxy-Guanosin
T = 2'-deoxy-Thymidin (Thymidin)

Modifikationen:
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
5'- wurde von ersten Primer-Oligonukleotiden wurden mit TAMRA-Farbstoff modifiziert. (TMR -Modifikation).

Der jeweilige zweite Bereich eines jeden ersten Primer-Oligonukleotid ist unterstrichen.

Es wurden jeweils konventionelle 5'-3'-Verknüpfung zwischen benachbarten Nukleotiden bzw. Nukleotid-Modifikationen gewählt. Auch Modifikationen wurden in 5'-3'- Anordnung angebracht. Alle Sequenzen wurden durch kommerzielle Anbieter mit einer Standard-Synthese Chemie (Phosphoroamidit-Chemie) synthetisiert.

Das folgende zweite Primer-Oligonukleotid wurde verwendet:
Primer 001-02:
(SEQ ID NO: NN 33)
PA6-35-4401-604 5'- GCTCATACTACAATGTCACTTACGTCCTCT - 3'
(Reihenfolge der Nummerierung in 5'-3': 1 - 30)

Der zweite Primer besteht aus DNA.

Zusammensetzung der Reaktionsmischung und Start der Reaktion wurde durchgeführt wie im Beispiel 2 beschrieben.

Diese Komponenten wurden wie folg in der Reaktionsmischung eingesetzt:

| | |
|---|---|
| • Matrize 001-01: | 10 pmol/l |
| • Aktivator-Oligonukleotid-001-01: | 10 µmol/l |
| • Der jeweilige Primer-1: | 10 µmol/l |
| • Primer 001-02: | 10 µmol/l |

Weitere Komponenten der Reaktion wurden als Amplifikationsreaktion Lösung 2 eingesetzt:
- Kalium Glutamat, 50 mmol/l, pH 8,0
- Magnesium Acetat, 10 mmol/l
- dNTP (dATP, dCTP, dTTP, dGTP), je 200 µmol/l
- Polymerase (Bst 2.0 WarmStart, 120.000 U/ml NEB), 12 Units / 10 µl
- Triton X-100, 0,1% (v/v)
- EDTA, 0,1 mmol/l
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

Die Reaktion wurde in der Amplifikationsreaktion Lösung 2 unter jeweils isothermalen Reaktionsbedingungen bei 65°C über die Zeitdauer (wie jeweils angegeben) durchgeführt. Das Fluoreszenzsignal von EvaGreen wurde während Reaktion aufgezeichnet, wie im Beispiel 2 beschrieben. Das Ergebnis der Aufzeichnungen der Signalintensität während der Reaktion ist in Fig. 34 dargestellt.

### Auswertung:

Unter Einsatz von angeführten ersten Primer-Oligonukleotiden ist die Entstehung des folgenden ersten Primer-Verlängerungsprodukts zu erwarten:
(SEQ ID NO: NN 34)
5 - XXXXXXX TCAGAGTGTGGAGAGGACGTAAGTGACATTGTAGTATGAGC - 3'

Mit "XXXXXXX" ist die jeweilige Sequenz des entsprechenden ersten Primer-Oligonukleotides markiert. Direkt anschließend ist die Sequenz des zu erwartenden ersten Verlängerungsproduktes angegeben (41 NT). Das Verlängerungsprodukt ist unterstrichen.

Es wurden unterschiedliche Primer-1 Strukturen auf ihre Fähigkeit getestet, eine exponentielle Amplifikation unter isothermalen Bedingungen zu unterstützen. Dabei wurden im zweiten Bereich des ersten Primer-Oligonukleotides unterschiedliche zum Stopp der Polymerase führende Gruppen integriert: C3-Linker, C18-Linker (auch als HEG-Linker, Hexaethylenglycol-Linker, bekannt) oder Iso-dC-5-Me. Bei der Synthese des zweiten Primer-Verlängerungsproduktes kommt es zu einer positionsspezifischen Unterbrechung der Synthese des zweiten Primer-Verlängerungsproduktes, so dass Polynukleotid-Schwanz des zweiten Bereichs unkopiert bleibt.

Das jeweilige Signalprofil der Reaktion ist in Fig.34 abgebildet.

Die Reaktion mit Primer-1 (C3-Linker) mit SEQ ID NO: 30 ist in Fig. 34 (A) abgebildet.

Die Reaktion mit Primer-1 (C18-Linker) mit SEQ ID NO: 31 ist in Fig. 34 (B) abgebildet.

Die Reaktion mit Primer-1 (Iso-dC-5-Me) mit SEQ ID NO: 32 ist in Fig. 34 (C) abgebildet.

Alle angeführten Struktur-Varianten des ersten Primer-Oligonukleotides führten zu einer erfolgreichen exponentiellen Amplifikation. Beim Design der ersten Primer-Oligonukleotide wurden folgende Aspekte berücksichtigt:
- Erster Primer-Bereich des ersten Primer-Oligonukleotides muss spezifisch an die zu amplifizierende Nukleinsäure binden können und eine messbare Primer-Verlängerungsreaktion durch die verwendete Polymerase initiieren können.
- Erster Primer-Bereich des ersten Primer-Oligonukleotides muss mit dem zweiten Bereich des Aktivator-Oligonukleotides komplementär binden, damit eine Strangverdrängungsreaktion effektiv stattfinden kann.
- Zweiter Bereich des ersten Primer-Oligonukleotides umfasst einen Polynukleotid-Schwanz, welcher den ersten Bereich des Aktivator-Oligonukleotids spezifisch binden soll
- Zwischen dem ersten Bereich und dem Polynukleotid-Schwanz des zweiten Bereichs des ersten Primer-Oligonukleotides ist jeweils eine zur Blockade der Polymerase-abhängigen Synthese bzw. zur Termination bzw. zum Stopp der Polymerase-abhängigen Synthese führende erste Blockieungs-Einheit integriert, welche die Polymerase am Kopieren des Polynukleotid-Schwanzes hindert.
- Bindung zwischen dem ersten Primer-Oligonukleotid und dem Aktivator-Oligonukleotid soll unter verwendeten isothermalen Bedinungen reversiblel sein. Insgesamt soll unter gewählten Reaktionsbedingungen ein Gleichgewicht zwischen einer einzelsträngigen Form des ersten Primer-Oligonukleotids und einer an das Aktivator-Oligonukleotid komplementär gebundenen Form des ersten Primer-Oligonukleotid bestehen.

Bei der Wahl der Sequenzlänge und Sequenzkomposition des zweiten Primer-Oligonukleotides wurden folgende Aspekte berücksichtigt:
- Die Primer-Länge umfasst 30 Nukleotide. Positionen 5-30 der Sequenz des zweiten Primer-Oligonukleotides sind komplementär zum zu erwartenden sequenzspezifischen ersten Primer-Verlängerungsprodukt, welches bereits nach der ersten Primer-Verlängerungsreaktion entsteht. Der Primer-2 kann somit eine Primer-Verlängerungsreaktion für das zweite Primer-Verlängerungsprodukt initiieren.
- Das 3'-Segment (Positionen 23-30) des zweiten Primer-Oligonukleotides konkurriert im vorliegenden Beispiel um die Bindung an das erste sequenzspezifische Primer-Verlängerungsprodukt mit dem Aktivator-Oligonukleotid. Dieses 3'-Segment kann zu einer Nukleinsäure-abhängigen Strangverdrängung des entsprechenden Segmentes des Aktivator-Oligonukleotides führen. Bei einer solchen Strangverdrängung kommt das 3'-endständige Nukleotid des zweiten Primer-Oligonukleotides in Kontakt mit dem ersten sequenzspezifischen Primer-Verlängerungsprodukt, so dass die Polymerase eine effektive sequenzspezifische zweite Primer-Verlängerungsreaktion initiieren kann, welche zur Synthese des zweiten sequenzspezifischen Stranges führt. Diese Synthese findet unter gleichzeitiger Strangverdrängung des Aktivator-Oligonukleotides durch die Polymerase statt. Die Länge dieses 3'-Segmentes wurde in diesem Beispiel dermassen gewählt, dass keine Überlappung von komplementären Sequenzabschnitten mit dem ersten Primer-Oligonukleotid stattfinden kann.
- Im vorliegenden Beispiel kann das 5'-Segment (22 Nukleotide, Position 1 bis 22) des zweiten Primer-Oligonukleotides an das 3'-Segment des ersten sequenzspezifischen Primer-Verlängerungsproduktes komplementär binden. Dieses 5'-Segment konkurriert nicht um die Bindung an das erste Primer-Verlängerungsprodukt mit dem Aktivator-Oligonukleotid und führt somit nicht zu einer Strangverdrängung des Aktivator-Oligonukleotides aus der Bindung mit dem ersten Primer-Verlängerungsprodukt. Die Tm dieses 5'-Segmentes des zweiten Primer-Oligonukleotides ist so gewählt, dass eine temperatur-induzierte spontane Strangtrennung zwischen einem ersten sequenzspezifischen Primer-Verlängerungsprodukt und einem zweiten sequenzspezifischen Primer-Verlängerungsprodukt im Falle einer vollständigen Bindung des Aktivator-Oligonukleotides an das erste sequenzspezifische Primer-Verlängerungsprodukt begünstigt wird. Die Trennung der beiden Stränge resultiert in einzelsträngigen Form des 3'-Segments des ersten Primer-Verlängerungsproduktes.

### Beispiel 6:

### Einfluss von einzelnen Nukleotid-Veränderungen in der Matrize auf Geschwindigkeit der Amplifikations-Reaktion

In diesem Beispiel wurde Einfluss einer Sequenzänderung in der Matrize auf die Amplifikation untersucht, wobei einzelne Nukleotid-Monomere durch andere Nukleotid-Monomere ersetzt wurden. Die randständigen Bereiche, welche für die Bindung von Primern entscheidend sind, wurden identisch gestaltet. Auf diese Weise sollte überprüft werden, welche Auswirkung solche Mismatch-Positionen zwischen einem dabei entstehenden ersten Primer-Verlängerungsprodukt und einem vorgegebenen Aktivator-Oligonukleotid auf die Amplifikation hat. Die Gestaltung von Sequenzen erfolgte in ähnlicher Art und Weise wie im Beispiel 2 und 3.

Folgende Matrizen wurden verwendet:
- Matrize (SEQ ID NO 21) mit Sequenzzusammensetzung, welche zu einem ersten Primer-Verlängerungsprodukt mit einem Perfekt-Match Übereinstimmung mit Aktivator-Oligonukleotid führt:
   T-A6P-15-4401 5' GTAGTTCTCACTTACGTCCTCTCCACACTCTGA GTTCCTGCGAG 3' (SEQ ID NO:21)
   Bindungssequenz für das erste Primer-Oligonukleotid ist unterstrichen.

Darüber hinaus wurden weitere Matrizen (SEQ ID NO 36-38) mit Sequenzzusammensetzung, welche zu einem ersten Primer-Verlängerungsprodukt mit einem Mismatch mit Aktivator-Oligonukleotid führt:
T-A6P-15-4401-1
   5' GTAGTTCTCACTTACGTCCTCTCCACACT***A***TGA GTTCCTGCGAG 3'(SEQ ID NO:35)
T-A6P-15-4401-2
   5' GTAGTTCTCACTTACGTCCTCTCCA***A***ACTCTGA GTTCCTGCGAG 3' (SEQ ID NO:36)
J-A6P-15-4401-3
   5' GTAGTTCTCACTTACGTCCT***A***TCCACACTCTGA GTTCCTGCGAG 3' (SEQ ID NO:37)
T-A6P-15-4401-4
   5' GTAGTTCTCACTTACGT***A***CTCTCCACACTCTGA GTTCCTGCGAG 3' (SEQ ID NO:38)

Bindungssequenz für das erste Primer-Oligonukleotid ist unterstrichen.

Abweichende Basen (zu SEQ ID NO 21) sind ***FETT KURSIV*** markiert.

Bei allen Matrizen (mit Perfekt-Match Übereinstimmung und integrierten Mismatches) sind jeweilige Primer-Bindungsstellen identisch, so dass eine erfolgreiche erste Primer-Verlängerungsreaktion durch Primer-Oligonukleotide jeweils initiiert werden konnte.

Durch die Amplifikation mit der Polymerase entstehen Unterschiede in den amplifizierten Sequenzen gegenüber den unveränderten Matrizen (SEQ ID NO:21). Die entsprechenden Differenzstellen liegen im 5'-Segment des Verlängerungsprodukt des ersten Primer-Oligonukleotides.

Folgende Primer wurden verwendet:
- Das erste Primer-Oligonukleotid (SEQ ID NO 23):
   (A6P-10-1002 TMR7) 5'7765567876 CAGGAAC 3'
   mit der Bedeutung von:
   A = 2'-deoxy-Adenosin
   C = 2'-deoxy-Cytosin
   G = 2'-deoxy-Guanosin
   T = 2'-deoxy-Thymidin (Thymidin) und den
Modifikationen:
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
8 = dT-TMR
   - Das zweite Primer-Oligonukleotid (SEQ ID NO 24):
      (PA6-15-4401-10) 5' CACGAGTAGTTCTCACTTAC 3'

Folgendes Aktivator-Oligonukleotid-002-01 (SEQ ID NO 25) wurde verwendet:
A6 8504
5'- CTAGCTAT CGTC**CTCT CCA**CAC**T C**TGA GTTCCTGCGAG CTTC GGTAC AAG X 3' 68576858 6786**6868 665**656**8 6**875 788668 7CGAG CTTC GGTAC AAG
mit folgender Bedeutung:
A = 2'-deoxy-Adenosin
C = 2'-deoxy-Cytosin
G = 2'-deoxy-Guanosin
T = 2'-deoxy-Thymidin (Thymidin)

5 = 2'-O-Me A (2'-O-Methyl-Adenosine)
6 = 2'-O-Me C (2'-O-Methyl-Cytosine)
7 = 2'-O-Me G (2'-O-Methyl-Guanosine)
8 = 2'-O-Me U (2'-O-Methyl-Uridine)
X = 3'-Phosphat-Gruppe zur Blockade einer möglichen Verlängerung durch Polymerase.

Die Nukleotide und Nukleotid-Modifikationen sind untereinander mit Phosphodiesterbindung verknüpft. Das 3'-Ende ist mit einer Phosphat-Gruppe blockiert, um eine mögliche Verlängerung durch die Polymerase zu verhindern.

Die Matrize mit Perfekt-Match Sequenzzusammensetzung wurde in Konzentration von 1 nmol/l, 1 pmol/l und 1 fmol/l eingesetzt. Die Matrize mit Mismatch Sequenzzusammensetzung wurde in Konzentration von 1 nmol/l verwendet.

In der Kontroll-Reaktion wurde keine Matrize eingesetzt. Sonstige Reaktionsbedingungen wurden wie im Beispiel 2 gehalten. Fig. 35 zeigt das Ergebnis der Reaktion.

Bei Verwendung einer Perfect-Match Matrize (SEQ ID NO 21) kommt es zur Synthese eines komplementären Stranges eines Primer-Verlängerungsproduktes. Dieses Verlängerungsprodukt ist sowohl zu Perfect-Match-Matrize komplementär als auch zum verwendeten Aktivator-Oligonukleotid. Diese Konstellation wurde im Beispiel 2 ausführlich dargestellt. Sie dient als Grundlage für eine erfolgreiche Amplifikation.

Im Gegensatz dazu, unter Verwendung einer Mismatch-Sequenzen (SEQ ID NO 35-38) kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der vollständigen Komplementarität mit dem dritten Bereich des Aktivator-Oligonukleotid abweicht. Diese Abweichung in der Komplementatirät liegen im 5'-ständigen Segment des Verlängerungsproduktes des ersten Primer-Verlängerungsproduktes, welches mit Aktivator-Oligonukleotid reagieren soll, damit Strangverdrängungsvorgang voranschreiten kann. Wie im vorliegenden Beispiel gezeigt wird, können Mismatches zwischen korrespondierenden Stellen die Strangverdrängung durch das Aktivator-Oligonukleotid stören. Die Amplifikation verläuft dadurch ingesamt langsamer.

Die Fig. 35 zeigt diesen Einfluss der Sequenzunterschiede zwischen Aktivator-Oligonukleotid und dem Verlängerungsprodukt des ersten Primer-Oligonukleotids. Abweichungen an jeweils nur einer Position in der Sequenz des Aktivator-Oligonukleotides von der Sequenz des an der Mismatch-Matrize synthetisierten Primer-Verlängerungsproduktes zeigt die Abhängigkeit der Amplifikation von der Übereinstimmung in der Reihenfolge der komplementären Basen im Aktivator-Oligonukleotid und Matrize bzw. dem ersten Primer-Verlängerungsprodukt. Die jeweiligen Mutationen verursachen eine Verlangsamung der Amplifikationsreaktion.

Die Kontrollreaktionen mit einer Perfect Match Matrize (Pfeile 1 - 3, Fig. 35 A) zeigten eine Konzentrationsabhängigkeit der Amplifikation. Mit sinkender Konzentration brauchte Reaktion längere Zeit, um eine ausreichende Menge der zu amplifizierenden Nukleinsäure zu synthetisieren, damit das Signal über Niveau der Base-line ansteigt.

Die Negativ-Kontroll-Reaktion ohne Matrize zeigte ebenfalls ein Anstieg im Signal, allerdings in einem signifikant späteren Zeitintervall zu Reaktionen mit einer Perfect-Match Matrize.

Die Pfeile in Fig. 35 A zeigen:
Perfect Match Matrize (SEQ ID NO 21) 1 nmol/l Konzentration (Pfeil 1)
Perfect Match Matrize (SEQ ID NO 21) 1 pmol/l Konzentration (Pfeil 2)
Perfect Match Matrize (SEQ ID NO 21) 1 fmol/l Konzentration (Pfeil 3)
Negativ-Kontrolle: Keine Matrize in der Reaktion (Pfeil 4)

Die Pfeile in Fig. 35 B zeigen:
Perfect Match Matrize (SEQ ID NO 21) 1 nmol/l Konzentration (Pfeil 1)
Negativ-Kontrolle: Keine Matrize in der Reaktion (Pfeil 4)
Mismatch Matrize (SEQ ID NO 35) 1 nmol/l Konzentration (Pfeil 5) T-A6P-15-4401-1
Mismatch Matrize (SEQ ID NO 36) 1 nmol/l Konzentration (Pfeil 6) T-A6P-15-4401-2
Mismatch Matrize (SEQ ID NO 37) 1 nmol/l Konzentration (Pfeil 7) T-A6P-15-4401-3
Mismatch Matrize (SEQ ID NO 38) 1 nmol/l Konzentration (Pfeil 8) T-A6P-15-4401-4

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt um nur eine komplementare Base können zu einer teilweise signifikanten Verzögerung der exponentiellen Amplifikation führen.

In diesem Beispiel wurde gezeigt, dass obwohl endständige Sequenzsegmente von Perfect-Match-Matrize und Mismatch-Matrize komplett übereinstimmten und somit das Potenzial zu Bindung von beiden Primer-Oligonukleotide im Verlauf der exponentiellen Amplifikation gleich war, sind beide Reaktionen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Vorkommen von einzelnen Mismatches zwischen Aktivator-Oligonukleotid und einem Primer-Verlängerungsprodukt können zu einer Verlangsamung einer exponentiellen Amplifikations-Reaktion führen.

## Patentansprüche

1. Verfahren zur Amplifikation einer Nukleinsäure,das folgende Schritte umfasst:
a) Hybridisierung eines ersten Primer-Oligonukleotids an das 3'-Segment eines Stranges einer zur amplifizierenden Nukleinsäurekette, wobei die zu amplifizierende Nukleinsäurekette eine Zielsequenz umfasst,
wobei das erste Primer-Oligonukleotid umfasst:
• Einen ersten Primer-Bereich im 3'-Segment des ersten Primer-Oligonukleotides, der an einen Strang einer zu amplifizierenden Nukleinsäurekette sequenzspezifisch binden kann
• Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedinungen im Wesentlichen unkopiert bleibt.
b) Extension des ersten Primer-Oligonukleotids mit Hilfe einer Polymerase zur Bildung eines ersten Primer-Verlängerungsproduktes, das eine zur Zielsequenz der zu amplifizierenden Nukleinsäurekette (a) komplementäre Sequenz umfasst,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
• Einen ersten einzelsträngigen Bereich, welcher an den Polynukleotid-Schwanz des zweiten Bereichs des ersten Primer-Oligonukleotides binden kann
• Einen zweiten einzelsträngigen Bereich, welcher zu dem ersten Bereich des ersten Primer-Oligonukleotides im Wesentlichen komplementär ist und an diesen binden kann
• Einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, wobei das 3'-Segment des ersten Primer-Verlängerungsproduktes einzelsträngig wird,
f) Hybridisierung eines zweiten Oligonukleotid-Primers an das erste Primer-Verlängerungsprodukt, wobei das 3'-Segment des zweiten Oligonukleotid-Primers eine Sequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist.

2. Verfahren nach Anspruch 1, **dadurch gekennzeichnet, dass** das Verfahren im Wesentlichen isothermal durchgeführt wird.

3. Verfahren nach einem der Ansprüche 1 oder 2, **dadurch gekennzeichnet, dass** der dritte einzelsträngige Bereich des Aktivator-Oligonukleotids zum Segment des von der Polymerase synthetisierten Verlängerungsproduktes des ersten Primer-Verlängerungsprodukts, welches sich unmittelbar an den ersten Primer-Bereich anschließt, im Wesentlichen komplementär ist.

4. Verfahren nach Anspruch 1, **dadurch gekennzeichnet, dass** Schritt (e) des Verfahrens dahingehend modifiziert wird, dass es umfasst die Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, bis dieser komplementäre Strang der zu amplifizierenden Nukleinsäure vom ersten Primer-Verlängerungsprodukt abgelöst wird, wobei das 3'-Segment des ersten Primer-Verlängerungsproduktes einzelsträngig wird.

5. Verfahren nach Anspruch 1, **dadurch gekennzeichnet, dass** Schritt (f) des Verfahrens dahingehend modifiziert wird, dass es umfasst die Hybridisierung eines zweiten Oligonukleotid-Primers an das erste Primer-Verlängerungsprodukt, wobei gleichzeitig zumindest eine partielle Verdrängung des Aktivator-Oligonukleotides aus der Bindung mit dem ersten Verlängerungsprodukt durch Strangverdrängung erfolgt.

6. Verfahren nach Anspruch 1, **dadurch gekennzeichnet, dass** Schritt (g) des Verfahrens so modifiziert wird, dass er eine Verdrängung des Aktivator-Oligonukleotides aus der Bindung mit dem ersten Primer-Verlängerungsprodukt unter Mitwirkung der Polymerase umfasst.

7. Verfahren nach Anspruch 1, **dadurch gekennzeichnet, dass** Schritt (h) des Verfahrens dahingehend modifiziert wird, dass es die Bindung des Aktivator-Oligonukleotides an den unkopierten Polynukleotid-Schwanz des ersten verlängerten Primer-Oligonukleotids und eine Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oligonukleotides umfasst.

8. Verfahren nach Anspruch 1, **dadurch gekennzeichnet, dass** die Wiederholung der Schritte unter solchen Bedingungen durchgeführt wird, die die Wiederholung der Schritte (a) bis (g) zulassen.

9. Verfahren nach einem der vorhergehenden Ansprüche, **dadurch gekennzeichnet, dass** es die gleichzeitige Amplifikation des ersten und zweiten Primer-Verlängerungsproduktes in einer exponentiellen Reaktion unter Verwendung der ersten und zweiten Primer-Oligonukleotide und des Aktivator-Oligonukleotides umfasst, wobei die gebildeten Primer-Verlängerungsprodukte als Matrizen für die gegenseitige Synthese fungieren.

10. Verwendung eines Kits zur Durchführung eines Verfahrens nach einem der Ansprüche 1 bis 9 zur Amplifikation einer Nukleinsäurekette, **dadurch gekennzeichnet, dass** der Kit wenigstens ein erstes Primer-Oligonukleotid enthält,
wobei das erste Primer-Oligonukleotid umfasst:
• Einen ersten Primer-Bereich im 3'-Segment des ersten Primer-Oligonukleotides, der an einen Strang einer zu amplifizierenden Nukleinsäurekette sequenzspezifisch binden kann
• Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedinungen im Wesentlichen unkopiert bleibt
und wenigstens ein Aktivator-Oligonukleotid, wobei das Aktivator-Oligonukleotid umfasst:
• Einen ersten einzelsträngigen Bereich, welcher an den Polynukleotid-Schwanz des zweiten Bereichs des ersten Primer-Oligonukleotides binden kann
• Einen zweiten einzelsträngigen Bereich, welcher zu dem ersten Bereich des ersten Primer-Oligonukleotides im Wesentlichen komplementär ist und an diesen binden kann
• Einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
wobei das Aktivator-Oligonukleotid nicht als Matrize für eine Primerverlängerung des ersten Primer-Oligonukleotid dient und wenigstens eine Polymerase zur Amplifikation enthält.

11. Verwendung eines Kits nach Anspruch 10, **dadurch gekennzeichnet, dass** es weiterhin einen zweiten Oligonukleotid-Primer enthält, wobei das 3'-Segment des zweiten Oligonukleotid-Primers eine Sequenz umfasst, die an das erste Primer-Verlängerungsprodukt hybridisieren kann.

## Claims

1. A method for amplification of a nucleic acid comprising the following steps:
a) hybridizing a first primer oligonucleotide to the 3' segment of a strand of a nucleic acid chain to be amplified, wherein the nucleic acid chain to be amplified comprises a target sequence,
wherein the first primer oligonucleotide comprises:
• a first primer region in the 3' segment of the first primer oligonucleotide that can sequence-specifically bind to a strand of a nucleic acid chain to be amplified,
• a second region that is directly or via a linker linked to the 5' end of the first primer region of the first primer oligonucleotide and that comprises a polynucleotide tail which is suitable for binding an activator oligonucleotide and supporting strand displacement (step c) by the activator oligonucleotide, wherein the polynucleotide tail remains substantially uncopied by polymerase under the selected reaction conditions,
b) extending the first primer oligonucleotide by means of a polymerase to form a first primer extension product comprising a sequence that is complementary to the target sequence of the nucleic acid chain (a) to be amplified,
c) binding the activator oligonucleotide to the polynucleotide tail of the second region of the first extended primer oligonucleotide, wherein the activator oligonucleotide comprises:
• a first single-stranded region that can bind to the polynucleotide tail of the second region of the first primer oligonucleotide,
• a second single-stranded region that is substantially complementary and can bind to the first region of the first primer oligonucleotide,
• a third single-stranded region that is substantially complementary to at least a segment of the extension product, which has been synthesized by polymerase, of the first primer extension product,
wherein the activator oligonucleotide does not serve as template for a primer extension of the first primer oligonucleotide,
d) binding the activator oligonucleotide to the first primer region of the first extended primer oligonucleotide by displacing the strand of the nucleic acid chain to be amplified that is complementary to said first primer region,
e) binding the activator oligonucleotide to the complementary segment of the extension product of the first extended primer oligonucleotide by displacing the strand of the nucleic acid chain to be amplified that is complementary to said extension product, wherein the 3' segment of the first primer extension product becomes single-stranded,
f) hybridizing a second oligonucleotide primer to the first primer extension product, wherein the 3' segment of the second oligonucleotide primer comprises a sequence that can hybridize to the first primer extension product; and
g) extending the second oligonucleotide primer with polymerase to form a second primer extension product, wherein the extension takes place up to and including the first primer region of the first primer oligonucleotide and said first primer region is copied by the polymerase, wherein the polynucleotide tail of the second region remains uncopied,
h) repeating steps a)-g) until the desired degree of amplification has been achieved.

2. The method according to claim 1, **characterized in that** the method is carried out substantially isothermal.

3. The method according to any of claims 1 or 2, **characterized in that** the third single-stranded region of the activator oligonucleotide is substantially complementary to the segment of the extension product, which has been synthesized by the polymerase, of the first primer extension product, which immediately follows the first primer region.

4. The method according to claim 1, **characterized in that** step (e) of the method is modified **in that** it comprises the binding of the activator oligonucleotide to the complementary segment of the extension product of the first extended primer oligonucleotide by displacing the strand of the nucleic acid chain to be amplified that is complementary to said extension product until said complementary strand of the nucleic acid to be amplified is detached from the first primer extension product, wherein the 3' segment of the first primer extension product becomes single-stranded.

5. The method according to claim 1, **characterized in that** step (f) of the method is modified **in that** it comprises the hybridization of a second oligonucleotide primer to the first primer extension product, wherein at the same time there is at least a partial displacement of the activator oligonucleotide from the binding with the first extension product by strand displacement.

6. The method according to claim 1, **characterized in that** step (g) of the method is modified such that it comprises a displacement of the activator oligonucleotide from the binding with the first primer extension product with the participation of the polymerase.

7. The method according to claim 1, **characterized in that** step (h) of the method is modified **in that** it comprises the binding of the activator oligonucleotide to the uncopied polynucleotide tail of the first extended primer oligonucleotide and a displacement of the second primer extension product from the binding to the first primer extension product with the simultaneous formation of a complementary double strand with a segment of the first specific extension product of the first primer oligonucleotide.

8. The method according to claim 1, **characterized in that** the repetition of the steps is performed under such conditions that allow the repetition of the steps (a) to (g).

9. The method according to any of the preceding claims, **characterized in that** it comprises the simultaneous amplification of the first and second primer extension products in an exponential reaction by using the first and second primer oligonucleotides and the activator oligonucleotide, wherein the formed primer extension products function as a template for the mutual synthesis.

10. Use of a kit for carrying out a method according to any of claims 1 to 9 for amplification of a nucleic acid chain, **characterized in that** the kit contains at least one first primer oligonucleotide,
the first primer oligonucleotide comprises:
• a first primer region in the 3' segment of the first primer oligonucleotide that can sequence-specifically bind to a strand of a nucleic acid chain to be amplified,
• a second region that is directly or via a linker linked to the 5' end of the first primer region of the first primer oligonucleotide and that comprises a polynucleotide tail which is suitable for binding an activator oligonucleotide and supporting strand displacement (step c) by the activator oligonucleotide, wherein the polynucleotide tail remains substantially uncopied by polymerase under the selected reaction conditions,
and at least one activator oligonucleotide wherein the activator oligonucleotide comprises:
• a first single-stranded region that can bind to the polynucleotide tail of the second region of the first primer oligonucleotide,
• a second single-stranded region that is substantially complementary and can bind to the first region of the first primer oligonucleotide,
• a third single-stranded region that is substantially complementary to at least a segment of the extension product, which has been synthesized by polymerase, of the first primer extension product,
wherein the activator oligonucleotide does not serve as template for a primer extension of the first primer oligonucleotide, and at least one polymerase for amplification.

11. Use of a kit according to claim 10, **characterized in that** it further contains a second oligonucleotide primer, wherein the 3' segment of the second oligonucleotide primer comprises a sequence that can hybridize to the first primer extension product.

## Revendications

1. Procédé pour l'amplification d'un acide nucléique, qui comprend les étapes suivantes :
a) hybridation d'un premier oligonucléotide d'amorce sur le segment 3' d'un brin d'une chaîne d'acide nucléique à amplifier, dans lequel la chaîne d'acide nucléique à amplifier comprend une séquence cible,
dans lequel l'oligonucléotide d'amorce comprend :
• une première zone d'amorce dans le segment 3' du premier oligonucléotide d'amorce, qui peut se lier de manière spécifique à une séquence à un brin d'une chaîne d'acide nucléique à amplifier
• une seconde zone, couplée directement ou via un lieur à l'extrémité 5' de la première zone d'amorce du premier oligonucléotide d'amorce, lequel comprend une queue polynucléotidique, laquelle est appropriée pour la liaison d'un oligonucléotide activateur et le soutien du déplacement de chaîne (étape c) par des oligonucléotides activateurs, dans lequel la queue polynucléotidique de la polymérase reste sensiblement non copiée dans les conditions de réaction choisies.
b) extension du premier oligonucléotide d'amorce à l'aide d'une polymérase pour la formation d'un premier produit d'allongement d'amorce, qui comprend une séquence complémentaire par rapport à la séquence cible de la chaîne d'acide nucléique à amplifier (a),
c) liaison de l'oligonucléotide activateur à la queue polynucléotidique de la seconde zone du premier oligonucléotide d'amorce allongé, dans lequel l'oligonucléotide activateur comprend :
• une première zone monobrin, laquelle peut se lier à la queue polynucléotidique de la seconde zone du premier oligonucléotide d'amorce
• une deuxième zone monobrin, laquelle est sensiblement complémentaire par rapport à la première zone du premier oligonucléotide d'amorce et peut se lier à celle-ci
• une troisième zone monobrin, laquelle est sensiblement complémentaire par rapport à au moins un segment du produit d'allongement du premier produit d'allongement d'amorce synthétisé par la polymérase,
dans lequel l'oligonucléotide activateur ne sert pas de matrice pour un allongement d'amorce du premier oligonucléotide d'amorce,
d) liaison de l'oligonucléotide activateur à la première zone d'amorce du premier oligonucléotide d'amorce allongé, par déplacement du brin complémentaire par rapport à cette première zone d'amorce de la chaîne d'acide nucléique à amplifier
e) liaison de l'oligonucléotide activateur au segment complémentaire du produit d'allongement du premier oligonucléotide d'amorce allongé, par déplacement du brin complémentaire par rapport à ce produit d'allongement de la chaîne d'acide nucléique à amplifier, dans lequel le segment 3' du premier produit d'allongement d'amorce devient monobrin,
f) hybridation d'une seconde amorce oligonucléotidique au premier produit d'allongement d'amorce, dans lequel le segment 3' de la seconde amorce oligonucléotidique comprend une séquence qui peut s'hybrider au premier produit d'allongement d'amorce, et
g) allongement de la seconde amorce oligonucléotidique avec une polymérase pour former un second produit d'allongement d'amorce, dans lequel l'allongement se produit jusqu'à la première zone d'amorce incluse du premier oligonucléotide d'amorce et cette première zone d'amorce est copiée par la polymérase, dans lequel la queue polynucléotidique de la seconde zone reste non copiée,
h) répétition des étapes a) à g) jusqu'à ce que le degré souhaité d'amplification soit atteint.

2. Procédé selon la revendication 1, **caractérisé en ce que** le procédé est effectué de manière sensiblement isotherme.

3. Procédé selon l'une quelconque des revendications 1 ou 2, **caractérisé en ce que** la troisième zone monobrin de l'oligonucléotide activateur est sensiblement complémentaire par rapport au segment du produit d'allongement du premier produit d'allongement d'amorce synthétisé par la polymérase, lequel se raccorde directement à la première zone d'amorce.

4. Procédé selon la revendication 1, **caractérisé en ce que** l'étape (e) du procédé est modifiée afin de comprendre la liaison de l'oligonucléotide activateur au segment complémentaire du produit d'allongement du premier oligonucléotide d'amorce allongé, par déplacement du brin de la chaîne d'acide nucléique à amplifier complémentaire par rapport à ce produit d'allongement jusqu'à ce que ce brin complémentaire de l'acide nucléique à amplifier soit détaché du premier produit d'allongement d'amorce, dans lequel le segment 3' du premier produit d'allongement d'amorce devient monobrin.

5. Procédé selon la revendication 1, **caractérisé en ce que** l'étape (f) du procédé est modifiée afin de comprendre l'hybridation d'une seconde amorce oligonucléotidique au premier produit d'allongement d'amorce, dans lequel, simultanément, au moins un déplacement partiel de l'oligonucléotide activateur à partir de la liaison avec le premier produit d'allongement se produit par déplacement de brin.

6. Procédé selon la revendication 1, **caractérisé en ce que** l'étape (g) du procédé est modifiée de manière à comprendre un déplacement de l'oligonucléotide activateur à partir de la liaison avec le premier produit d'allongement d'amorce avec la participation de la polymérase.

7. Procédé selon la revendication 1, **caractérisé en ce que** l'étape (h) du procédé est modifiée de façon à comprendre la liaison de l'oligonucléotide activateur à la queue polynucléotidique non copiée du premier oligonucléotide d'amorce allongé et un déplacement du second produit d'allongement d'amorce à partir de la liaison au premier produit d'allongement d'amorce tout en réalisant simultanément un double brin complémentaire avec un segment du premier produit d'allongement spécifique du premier oligonucléotide d'amorce.

8. Procédé selon la revendication 1, **caractérisé en ce que** la répétition des étapes est effectuée dans des conditions telles qu'elles permettent la répétition des étapes (a) à (g).

9. Procédé selon l'une quelconque des revendications précédentes, **caractérisé en ce qu'**il comprend l'amplification simultanée des premier et second produits d'allongement d'amorce dans une réaction exponentielle en utilisant les premier et second oligonucléotide d'amorce et l'oligonucléotide activateur, dans lequel les produits d'allongement d'amorce formés fonctionnent comme des matrices pour la synthèse mutuelle.

10. Utilisation d'un kit pour effectuer un procédé selon l'une quelconque des revendications 1 à 9 pour l'amplification d'une chaîne d'acide nucléique, **caractérisée en ce que** le kit contient au moins un premier oligonucléotide d'amorce,
dans lequel le premier oligonucléotide d'amorce comprend :
• une première zone d'amorce dans le segment 3' du premier oligonucléotide d'amorce, qui peut se lier de manière spécifique à une séquence à un brin d'une chaîne d'acide nucléique à amplifier
• une seconde zone, couplée directement ou par un lieur à l'extrémité 5' de la première zone d'amorce du premier oligonucléotide d'amorce, lequel comprend une queue polynucléotidique, laquelle est appropriée pour la liaison d'un oligonucléotide activateur et le soutien du déplacement de chaîne (étape c) par des oligonucléotides activateurs, dans lequel la queue polynucléotidique de la polymérase reste sensiblement non copiée dans les conditions de réaction choisies,
et au moins un oligonucléotide activateur, dans lequel l'oligonucléotide activateur comprend :
• une première zone monobrin, laquelle peut se lier à la queue polynucléotidique de la seconde zone du premier oligonucléotide d'amorce
• une deuxième zone monobrin, laquelle est sensiblement complémentaire par rapport à la première zone du premier oligonucléotide d'amorce et peut se lier à celle-ci
• une troisième zone monobrin, laquelle est sensiblement complémentaire par rapport à au moins un segment du produit d'allongement du premier produit d'allongement d'amorce synthétisé par la polymérase,
dans lequel l'oligonucléotide activateur ne sert pas de matrice pour un allongement d'amorce du premier oligonucléotide d'amorce et contient au moins une polymérase pour l'amplification.

11. Utilisation d'un kit selon la revendication 10, **caractérisé en ce qu'**il contient en outre une seconde amorce oligonucléotidique, dans laquelle le segment 3' de la seconde amorce oligonucléotidique comprend une séquence qui peut s'hybrider au premier produit d'allongement d'amorce.
